(19) Europäisches Patentamt
European Patent Office
Office européen des brevets

(11) EP 4 741 400 A1

(12) EUROPEAN PATENT APPLICATION

(43) Date of publication:
**13.05.2026 Bulletin 2026/20**

(21) Application number: **24212482.4**

(22) Date of filing: **12.11.2024**

(51) International Patent Classification (IPC):
***C07D 498/08*** *(2006.01)* ***A61P 43/00*** *(2006.01)*
***A61K 31/395*** *(2006.01)*

(52) Cooperative Patent Classification (CPC):
**A61P 43/00; C07D 498/08; C07D 519/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Bayer Aktiengesellschaft**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

# (54) MULTIMERIC MANGANESE CONTRAST AGENTS

(57) The present disclosure relates to new manganese ($Mn^{2+}$) chelate compounds, to methods of preparing said compounds, to the use of said compounds as contrast agents in diagnostic imaging such as magnetic resonance imaging (MRI) and to their use in a mammalian body.

EP 4 741 400 A1

## Description

## FIELD

The present disclosure relates to new manganese ($Mn^{2+}$) chelate compounds, to methods of preparing said compounds, to the use of said compounds as contrast agents in diagnostic imaging such as magnetic resonance imaging (MRI) and to their use in a mammalian body.

## BACKGROUND

The Magnetic Resonance Imaging (MRI) technique is non-invasive and can provide information on the anatomy, function and metabolism of tissues in vivo. Unenhanced MRI scans of tissue anatomy and function make use of the hydrogen atoms in water to generate the image. Apart from differences in the local water content, the basic contrast in the MR image mainly results from regional differences in the intrinsic relaxation times T1 and T2, each of which can be chosen to dominate image contrast. However, the intrinsic contrast provided by the water T1 and T2 and changes in their values brought about by tissue pathology are often too limited to enable a sensitive and specific diagnosis. To overcome these limits the proton relaxation times can be influenced by the presence of paramagnetic ions. Commercial Gadolinium-based Contrast Agents (GBCAs) contain at least one paramagnetic ion of the rare earth metal Gadolinium ($Gd^{3+}$), which possesses the highest number of unpaired electrons of any stable ion (seven), creating a high magnetic moment that is effective at enhancing proton relaxation.

Paramagnetic contrast media shorten the T1 (longitudinal) and T2 (transversal) relaxation times of surrounding water protons to indirectly produce a signal-enhancing effect. The efficacy of an agent to shorten relaxation times is called relaxivity (r1 and r2), which is dependent on the ligand surrounding the paramagnetic ion and influenced by extrinsic factors including temperature, magnetic field strength and the matrix (water, solid tissue, or blood) (Lauffer RB et al., Paramagnetic metal complexes as water proton relaxation agents for NMR imaging: theory and design. Chem Rev. 1987;87(5):901-27; Caravan P et al., Gadolinium(III) chelates as MRI contrast agents: structure, dynamics, and applications. Chem Rev. 1999;99(9):2293-352).

Since unchelated $Gd^{3+}$ ions are toxic, extracellular GBCAs require ligands chelating the $Gd^{3+}$ ion. Gd-DTPA (gadopentetic acid, marketed as Magnevist, Bayer), Gd-DTPA-BMA (gadodiamide, marketed as Omniscan, Ge Healthcare) and Gd-BOPTA (gadobenate, marketed as MultiHance, Bracco) are classified as linear GBCAs based on the chain-like ligand structure. Gd-DOTA (gadoterate, e.g., marketed as Dotarem, Guerbet), Gd-HP-DO3A (gadoteridol, marketed as ProHance, Bracco) and Gd-DO3A-butrol (gadobutrol, e.g., marketed as Gadovist, Bayer) contain cage-like ligands and are classified as macrocyclic agents. Macrocyclic agents are more stable against dissociation and $Gd^{3+}$ release compared to linear agents (Frenzel et al. Invest Radiol. 2008 Dec;43(12):817-28.)

In 2014, a first study showed an increased signal intensity (SI) in the dentate nucleus (DN) and globus pallidus (GP) brain areas on unenhanced T1-weighted (T1w) MR images in patients with normal renal function who received multiple linear gadolinium-based contrast agent (GBCA) administrations (Kanda et al. Radiology 2014 Mar;270(3):834-41). This led to a thorough evaluation of GBCA safety including regulatory activities and intensive research on Gd retention in organs after use of GBCA. In March 2017, the European Union (EU) suspended the marketing authorization for all multi-purpose linear GBCAs while supporting the continued use of macrocyclic GBCAs.

Following this decision, the MRI market moved away from linear Gadolinium-based agents and increased efforts and strategies to reduce or replace Gd. Nonetheless, a new high relaxivity GBCA received market authorization in the US (gadopiclenol, marketed as Elucirem, Guerbet, and VUEWAY, Bracco) in 2022, which allows for a reduced dose while maintaining diagnostic efficacy. Other efforts include artificial intelligence approaches to further reduce gadolinium dose by creating virtual enhanced images comparable to full-dose images.

Thus, there is a long-standing desire to abandon GBCAs and to replace them with alternative contrast agents which remains unfulfilled to date. In particular, there is an increased long-standing medical need for new non-Gd contrast agents for magnetic resonance imaging. Endogenous paramagnetic elements have been considered as non-Gd alternatives for MRI. Manganese, one such endogenous paramagnetic element, is an essential trace element for several enzymes in the human body and possesses five unpaired electrons in its bivalent state.

To date, no multi-purpose contrast agent based on endogenous manganese is commercially available. Only one liver-specific Mn complex has been approved as a contrast agent for MRI: Mn-DPDP (mangafodipir, marketed as TeslaScan). Mn-DPDP (GE Healthcare) was approved for liver imaging at clinical doses of 0.005 mmol/kg but was withdrawn by the manufacturer from both the US and EU markets in 2003 and 2012, respectively. Mn-DPDP is metabolized in plasma and rapidly releases the Mn ions due to dephosphorylation and transmetalation with zinc. The released manganese ions are cleared via biliary excretion resulting in the image contrast of the liver and detection of focal liver lesions (Toft KG et al. Metabolism and pharmacokinetics of MnDPDP in man. Acta Radiol. 1997 Jul; 38 (4 Pt 2): 677-89.). So far, no suitable alternative for intravenous injection has been found.

Thus, there is an increased medical need to provide new contrast agents for radiological imaging. In particular, there is an increased medical need to provide new contrast agents based on endogenous elements replacing gadolinium. Given the low complex stability experienced with manganese (see Mn-DPDP above) and the clinically observed side effects, manganese-based contrast agents have been challenged as suitable and stable alternatives to multi-purpose GBCAs; instead, research and development efforts have focused on the use of other paramagnetic metal ions such as iron. Superparamagnetic iron oxide nanoparticles (SPIONS) have been investigated as MRI agents with very limited success: Two examples are Ferumoxytol (marketed as Feraheme and approved only for the treatment of iron deficiency and not for imaging purposes) and Ferucarbotran (marketed as Resovist, Bayer AG, discontinued in 2009). The pharmacokinetic clearance and biodistribution of SPIONS differ from those of GBCAs rendering them therefore not fully comparable (Jin et al. Superparamagnetic iron oxide nanoparticles for MR imaging and therapy: design considerations and clinical applications. Curr Opin Pharmacol. 2014 Oct;18:18-27). Iron chelates are alternatives that are still in a very early stage (Boehm-Sturm et al. Low-Molecular-Weight Iron Chelates May Be an Alternative to Gadolinium-based Contrast Agents for T1-weighted Contrast-enhanced MR Imaging; Radiology 2018 Feb;286(2):537. Snyder et al. A Class of FeIII Macrocyclic Complexes with Alcohol Donor Groups as Effective T1 MRI Contrast Agents. Angew Chem Int Ed Engl. 2020 Feb 3;59(6):2414-2419). Thus, there is a long-standing, increased medical need to provide Gd-free, multipurpose contrast agents.

WO2017/027834 describes examples of metal chelating ligands that have high affinity for manganese; the resulting complexes can be used as MRI contrast agents.

WO2021/043926 describes Mn-based chelates, stereoisomers thereof and their properties including their potential use as contrast agents in MRI.

There is clearly an unmet medical need to provide new contrast agents for radiological imaging, particularly for magnetic resonance imaging which are not based on the use of Gadolinium. The long-standing desire to abandon GBCAs and to replace them with alternative contrast agents remains unfulfilled to date. In particular, there is an increased long-standing medical need for new non-Gd contrast agents for magnetic resonance imaging. In addition, there is an increased long-standing medical need for new non-Gd contrast agents which applicability is not restricted to specific uses (such as liver imaging), i.e., multipurpose applications. Specifically, there is an unmet medical need to provide new contrast agents for radiological imaging which are not based on the use of Gadolinium and fulfil as many of the below-listed criteria as possible:

- exhibit high water solubility,
- are physically and chemically stable,
- are sufficiently stable against metal release from the chelate,
- exhibit high relaxivity,
- have low protein binding,
- are fast and completely excreted,
- exhibit no long-term retention of Mn both in tissues and in organs,
- are stable against metabolic degradation,
- are well tolerated,
- can be produced in large quantities.

The state of the art described above does not disclose the compounds of general formula (I) of the present disclosure as defined herein, or a stereoisomer, a tautomer, an N-oxide, a hydrate, a solvate, or a salt thereof, or a mixture of the same, as described and defined herein. Nor does the art disclose the compounds of general formula (I) of the present disclosure in the form of a metal complex with $Mn^{2+}$, and stereoisomers, tautomers, N-oxides, hydrates, solvates, or salts thereof, or mixtures of same. Together or separately, the compounds of general formula (I) of the present disclosure and the compounds of general formula (I) of the present disclosure in the form of a complex with $Mn^{2+}$, and stereoisomers, tautomers, N-oxides, hydrates, solvates, or salts thereof, or mixtures of same are hereinafter referred to as "compounds of the present disclosure". The compounds of general formula (I) of the present disclosure in the form of a complex with $Mn^{2+}$, and stereoisomers, tautomers, N-oxides, hydrates, solvates, or salts thereof, or mixtures of same may also be referred to

as "$Mn^{2+}$-containing compounds of the present disclosure"

It has been found, and this constitutes the basis of the present disclosure, that the compounds of the present disclosure have surprising and advantageous properties.

In particular, the compounds of general formula (I) of the present disclosure allow for the preparation of complexes with $Mn^{2+}$, *i.e.* compounds of general formula (I) of the present disclosure in the form of a metal complex $Mn^{2+}$, respectively, as well as stereoisomers, tautomers, N-oxides, hydrates, solvates, or salts thereof, or mixtures of same. In particular, the $Mn^{2+}$-containing compounds of the present disclosure display the favourable stability of macrocyclic GBCAs without many of the drawbacks associated with the use of Gadolinium. The stability of the $Mn^{2+}$-containing compounds of the present disclosure can be determined by a number of physical and chemical tests, including, but not limited to, heat, oxidation, pH and light, among others.

Further, the $Mn^{2+}$-containing compounds of the present disclosure show high tolerability, sufficient relaxivity, excellent water solubility and a fast and complete excretion, making them well suited for diagnostic imaging, in particular for magnetic resonance imaging. The increased tolerability - as shown, for example, by cell toxicity assays - of the $Mn^{2+}$-containing compounds of the present disclosure is particularly advantageous, since toxicity has been a major concern for radiologists and patients. The ongoing evaluation of GBCA safety has raised public awareness on potential toxicity and tolerability issues of contrast agents and has increased the demand for safer, more tolerable products.

**DESCRIPTION**

In accordance with a first aspect, the present disclosure covers compounds of general formula (I),

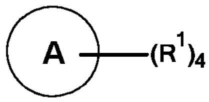

**(I)**

wherein

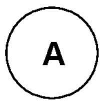

represents a group selected from

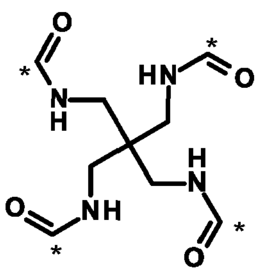

,

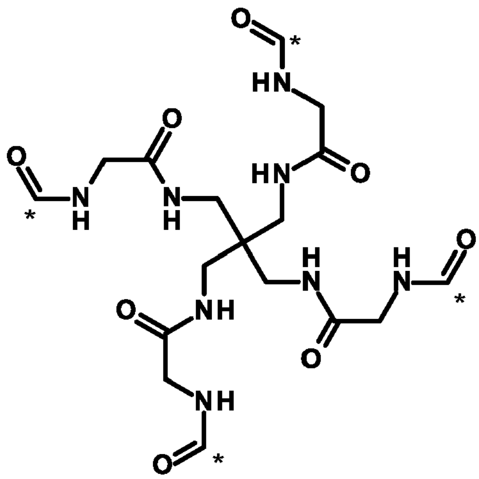

,

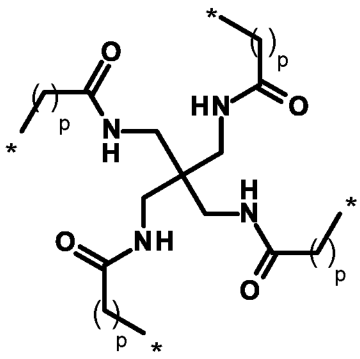

and

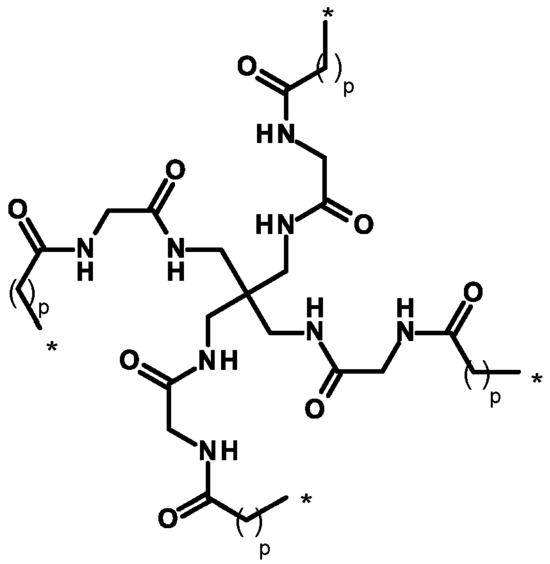

in which * represents the point of attachment to $R^1$ and p is an integer selected from 1 and 2,

$R^1$ represents a group selected from

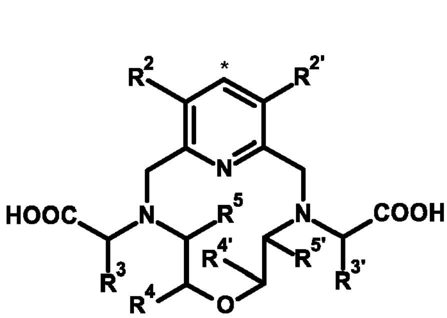

and

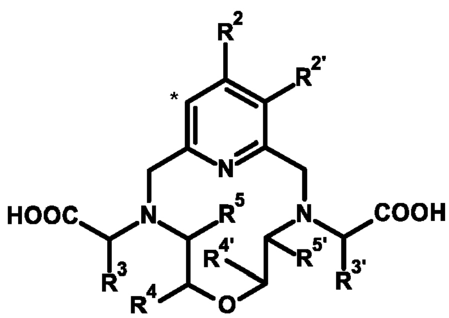

,

in which * represents the point of attachment to A,
$R^2$ and $R^{2'}$ are each independently selected from a hydrogen atom and a methyl group;
$R^3$ and $R^{3'}$ are each independently selected from a hydrogen atom, a $C_1$-$C_3$ alkyl group and a $-(CH_2)_m-(C=O)(NH)-(CH_2)-(CH(OH))_n-CH_2OH$ group, wherein m is an integer of from 1 to 3 and n is an integer of from 0 to 5;
$R^4$, $R^{4'}$, $R^5$ and $R^{5'}$ are each independently selected from a hydrogen atom and a $C_1$-$C_3$ alkyl group;
and stereoisomers, tautomers, N-oxides, hydrates, solvates, or salts thereof, or mixtures of same.

In accordance with a second aspect, the present disclosure covers compounds of general formula (I), *supra,* in the form of a complex with $Mn^{2+}$, and stereoisomers, tautomers, N-oxides, hydrates, solvates, or salts thereof, or mixtures of same.

As described in the present disclosure, compounds of general formula (I) in the form of a complex with $Mn^{2+}$, i.e., $Mn^{2+}$-containing compounds of the present disclosure refer to compounds in which $R^1$ forms a complex with $Mn^{2+}$ as depicted herein below:

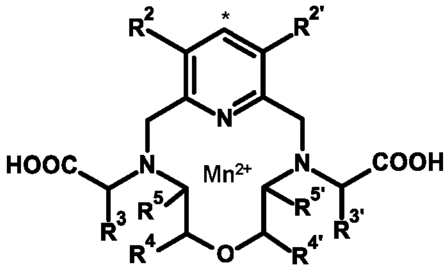

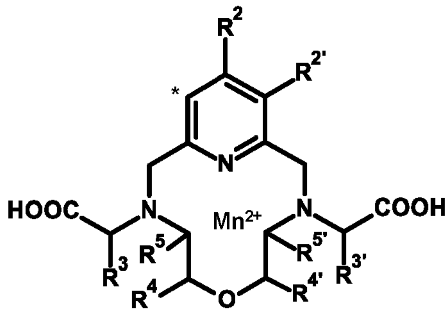

,

wherein * represents the point of attachment to A and A, $R^2$, $R^{2'}$, $R^3$, $R^{3'}$, $R^4$, $R^{4'}$, $R^5$ and $R^{5'}$ are as defined throughout the present disclosure.

Thus, in accordance to said second aspect, the present disclosure covers compounds of general formula (I) in the form of a complex with $Mn^{2+}$,

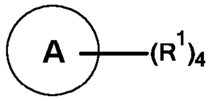

**(I)**

wherein

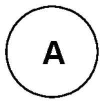

represents a group selected from

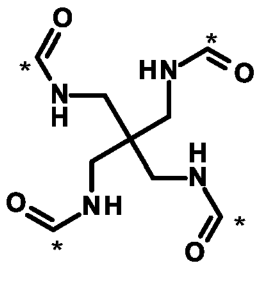

,

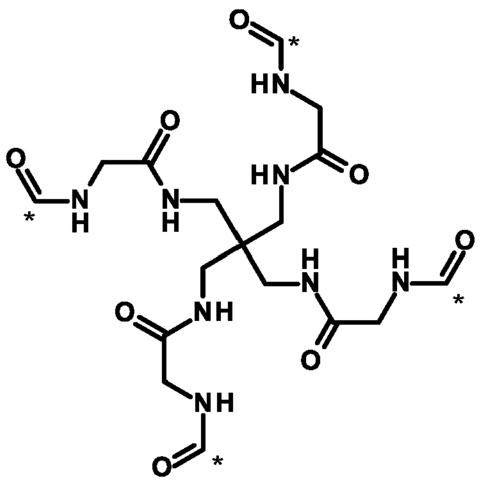

,

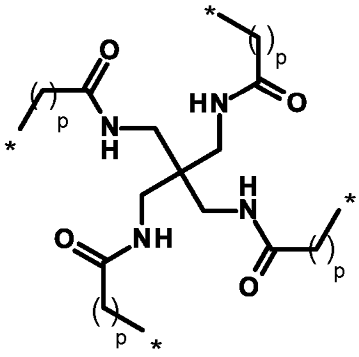

and

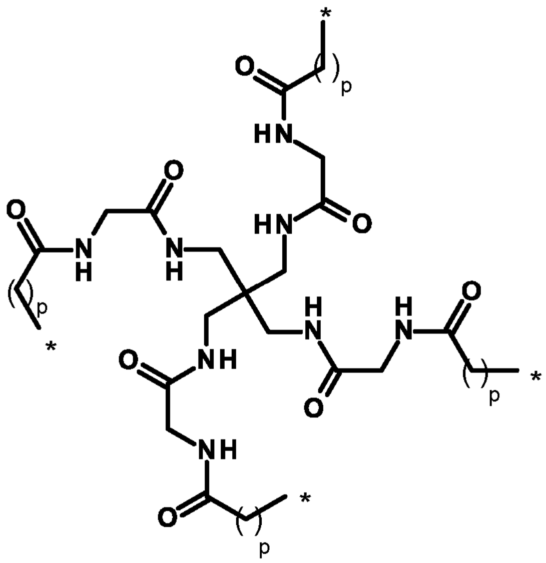

in which * represents the point of attachment to $R^1$ and p is an integer selected from 1 and 2,

$R^1$ represents a group selected from

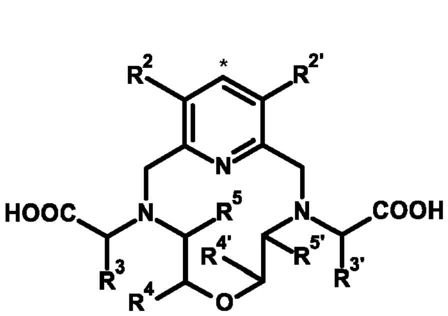

and

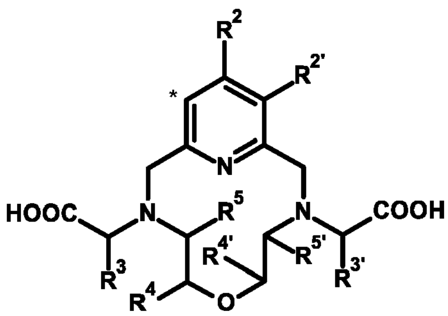

,

in which * represents the point of attachment to A,
$R^2$ and $R^{2'}$ are each independently selected from a hydrogen atom and a methyl group;
$R^3$ and $R^{3'}$ are each independently selected from a hydrogen atom, a $C_1$-$C_3$ alkyl group and a $-(CH_2)_m-(C=O)(NH)-(CH_2)-(CH(OH))_n-CH_2OH$ group, wherein m is an integer of from 1 to 3 and n is an integer of from 0 to 5;
$R^4$, $R^{4'}$, $R^5$ and $R^{5'}$ are each independently selected from a hydrogen atom and a $C_1$-$C_3$ alkyl group;
and stereoisomers, tautomers, N-oxides, hydrates, solvates, or salts thereof, or mixtures of same.

**DEFINITIONS**

In the context of the present disclosure, the term "oxapyclen" refers to an optionally substituted 6-oxa-3,9,15-triazabicyclo[9.3.1]pentadeca-1(15),11,13-triene moiety, i.e., to a chelating moiety which corresponds to the group $R^1$ as described throughout the present disclosure

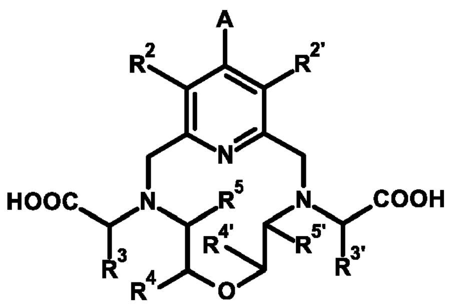
,
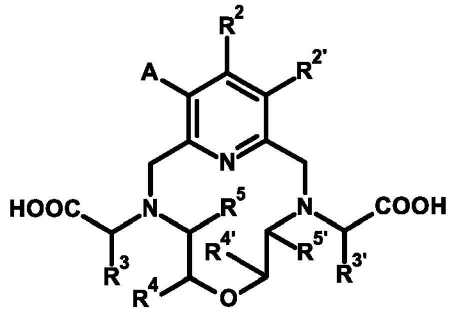
,

wherein A, $R^2$, $R^{2'}$, $R^3$, $R^{3'}$, $R^4$, $R^{4'}$, $R^5$ and $R^{5'}$ are as defined throughout the present disclosure.

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

When groups in the compounds according to the present disclosure are substituted, it is possible for said groups to be mono-substituted or poly-substituted with substituent(s), unless otherwise specified. Within the scope of the present disclosure, the meanings of all groups which occur repeatedly are independent from one another. It is possible that groups in the compounds according to the present disclosure are substituted with one, two or three identical or different substituents, particularly with one substituent.

Should a composite substituent be composed of more than one parts, e.g. ($C_1$-$C_3$-alkoxy)-($C_2$-$C_6$-alkyl)-, it is possible for the position of a given part to be at any suitable position of said composite substituent, *i.e.* the $C_1$-$C_3$-alkoxy part can be attached to any carbon atom of the $C_2$-$C_6$-alkyl part of said ($C_1$-$C_3$-alkoxy)-($C_2$-$C_6$-alkyl)- group. A hyphen at the beginning or at the end of such a composite substituent indicates the point of attachment of said composite substituent to the rest of the molecule.

For example, in the context of the present disclosure, the compounds of formula (I) may comprise a group $R^1$ being a $-(CH_2)_m-(C{=}O)(NH)-(CH_2)-(CH(OH))_n-CH_2OH$ group, wherein m is an integer of from 1 to 3 and n is an integer of from 0 to 5. The hyphen at the beginning of the definition of $R^3$ and/or $R^{3'}$ indicates that this is the point of attachment of said group $R^3$ and/or $R^{3'}$ to the rest of the molecule, i.e., via the methylene "$(CH_2)_m$" group as shown below, in which * represents the point of attachment to $R^1$:

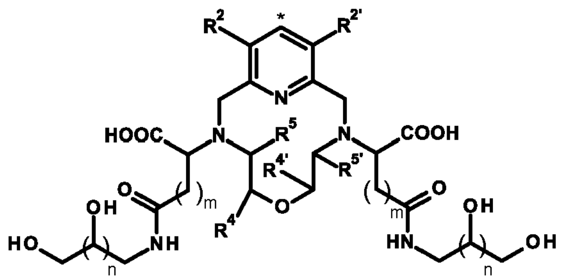

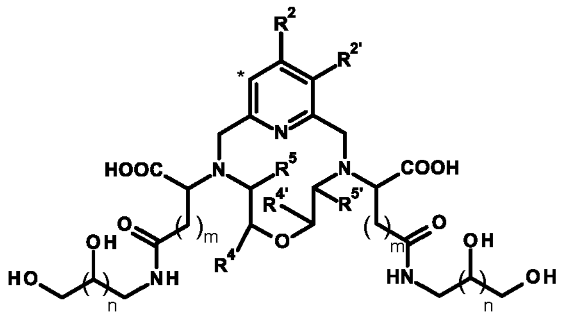

The term "comprising" when used in the specification includes "consisting of" and "consisting essentially of".

If within the present text any item is referred to as "as mentioned herein", it means that it may be mentioned anywhere in the present text.

The terms as mentioned in the present text have the following meanings:

The term "halogen atom" means a fluorine, chlorine, bromine or iodine atom, particularly a fluorine, chlorine or bromine atom.

The term "$C_1$-$C_3$-alkyl" means a linear or branched, saturated, monovalent hydrocarbon group having 1, 2 or 3 carbon atoms, e.g. a methyl, ethyl, propyl, isopropyl, or an isomer or stereoisomer thereof.

The compounds of the present disclosure may contain one or more asymmetric centers, depending upon the location and nature of the various substituents desired. Asymmetric carbon atoms may be present in the (R) or (S) configuration, which can result in racemic mixtures, mixtures in which one enantiomer is present in a greater amount than the other enantiomer, or single enantiomers in the case of a single asymmetric center. In the case of multiple stereogenic centers, diastereomeric mixtures, single diastereomers or single enantiomers can be synthesized. In certain instances, asymmetry may also be present due to restricted rotation about a given bond, axial chirality or coordination of the metal center.

Preferred compounds are those which produce the more desirable activity. Separated, pure or partially purified isomers and stereoisomers or racemic or diastereomeric mixtures of the compounds of this present disclosure are also included within the scope of the present disclosure. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallization. The optically active bases or acids are then liberated from the separated diastereomeric salts. A different process for separation of optical isomers involves the use of chiral chromatography (e.g., chiral HPLC columns), with or without conventional derivatization, optimally chosen to maximize the separation of the enantiomers. Suitable chiral HPLC columns are manufactured by Daicel, e.g., Chiracel OD and Chiracel OJ among many others, all routinely selectable. Enzymatic separations, with or without derivatization, are also useful. The optically active compounds of this present disclosure can likewise be obtained by chiral syntheses utilizing optically active starting materials and/or reagents and catalysts.

In order to describe different types of isomers reference is made to IUPAC Rules Section E (Pure Appl Chem 45, 11-30, 1976).

The present disclosure includes all possible stereoisomers of the compounds of the present disclosure as single stereoisomers, or as any mixture of said stereoisomers, e.g. R- or S-isomers, or diastereoisomers, in any ratio. Isolation of a single stereoisomer, e.g. a single enantiomer or a single diastereomer, of a compound of the present disclosure may be achieved by any suitable state of the art method as described herein, such as chromatography, especially chiral chromatography, for example.

In particular, the compounds of general formula (I) and the compounds of general formula (I) in the form of a complex with $Mn^{2+}$, i.e. $Mn^{2+}$ complexes of the compounds of general formula (I), as described throughout this document and including their salts as described below, may exist in different stereochemical configurations (i.e., as different stereoisomers). The present disclosure therefore includes all possible stereoisomers of the compounds of general formula (I) in the form of a complex with $Mn^{2+}$, i.e. all stereoisomers of $Mn^{2+}$ complexes of the compounds of general formula (I) either as single stereoisomers or as mixtures of two or more of said stereoisomers, in any ratio.

In particular, the compounds of general formula (I) and the compounds of general formula (I) in the form of a complex with $Mn^{2+}$, i.e. $Mn^{2+}$ complexes of the compounds of general formula (I), as described throughout this document and including their salts, comprise four oxapyclen chelating units which are held together by the central tetraamine core A. When one or more of $R^3/R^{3'}$, $R^4/R^{4'}$, $R^5/R^{5'}$ is different from a hydrogen atom, the corresponding carbon atom to which $R^3/R^{3'}$, $R^4/R^{4'}$, $R^5/R^{5'}$ is attached exhibits chirality, as denoted by * below:

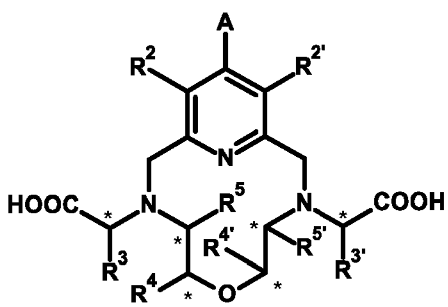

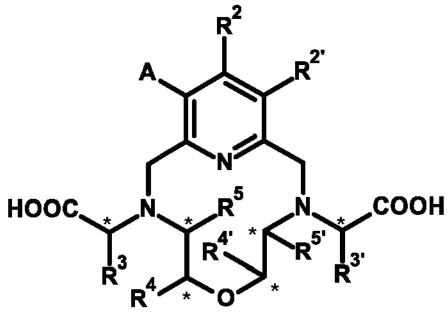

,

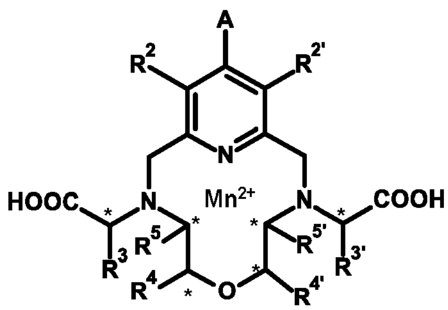
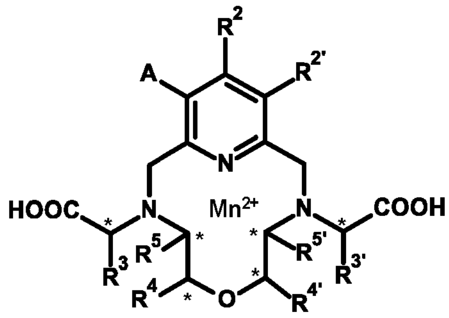
,

These carbon atoms can be in the (R) or (S) configuration. Thus, the present disclosure includes all compounds of general formula (I) and the compounds of general formula (I) in the form of a complex with $Mn^{2+}$, i.e. $Mn^{2+}$ complexes of the compounds of general formula (I), as described throughout this document and including their salts as described below, in which these carbon atoms are in the (R) or (S) configuration independently of each other. This includes cases where the carbon atoms to which $R^3/R^{3'}$, $R^4/R^{4'}$, $R^5/R^{5'}$ are attached have the same or different configurations (i.e., (R) or (S)) in each one of the oxapyclen chelators. Thus, depending on the configuration of the carbon atoms to which $R^3/R^{3'}$, $R^4/R^{4'}$, $R^5/R^{5'}$ are attached, different diastereomers and/or enantiomers may exist. The present disclosure includes all possible configurations of the carbon atoms to which $R^3/R^{3'}$, $R^4/R^{4'}$, $R^5/R^{5'}$ are attached and therefore all possible stereoisomers of the compounds of formula (I), and of the compounds of general formula (I) in the form of a complex with $Mn^{2+}$, i.e. $Mn^{2+}$ complexes of the compounds of general formula (I), as described throughout this document.

Upon complexation of the oxapyclen chelator(s) with $Mn^{2+}$, the two nitrogen atoms to which the $Mn^{2+}$ attaches may become chiral. The present disclosure comprises all possible isomers and combinations of isomers of the compounds of general formula (I) in the form of complexes with $Mn^{2+}$ where the nitrogen atoms attached to the $Mn^{2+}$ center exhibit chirality.

Given the possible chiral nature of the chelating moieties, it is important that the stereochemical centers remain stable under the different chemical and/or physical conditions that they might be subjected to in the course of development, production, transport and/or administration of any drug product they might be contained in. In case a conformational change in any - i.e., one or more, if present - of the chiral centers should nonetheless occur, it is crucial that no diastereomer is formed which has unacceptable properties. For example, it is important that no compounds - be it by-products or other diastereomers - with unfavorable or even harmful properties are formed during autoclaving - an important step in the preparation of sterile solutions of contrast agents - at high temperatures and/or high pressures.

Further, the compounds of the present disclosure may exist as N-oxides, which are defined in that at least one nitrogen of the compounds of the present disclosure is oxidized. The present disclosure includes all such possible N-oxides.

The present disclosure also relates to useful forms of the compounds as disclosed herein, such as hydrates, solvates, salts, in particular pharmaceutically acceptable salts, and co-precipitates.

The compounds of the present disclosure can exist as a hydrate, or as a solvate, wherein the compounds of the present disclosure contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, e.g. a hydrate, hemi-, (semi-), mono-, sesqui-, di-, tri-, tetra-, penta- *etc.* solvates or hydrates, respectively, are possible. The present disclosure includes all such hydrates or solvates.

Further, the compounds of the present disclosure may exist in the form of a salt depending on the nature of one or more of the substituents present in the compounds of formula (I) and in the compounds of general formula (I) in the form of a complex with $Mn^{2+}$, i.e. $Mn^{2+}$ complexes of the compounds of general formula (I), as described throughout this document, i.e., $R^2/R^{2'}$, $R^3/R^{3'}$, $R^4/R^{4'}$, $R^5/R^{5'}$. Said salt may be either an inorganic or organic addition salt, particularly any pharmaceutically acceptable inorganic or organic addition salt, customarily used in pharmaceutical formulations.

The term "pharmaceutically acceptable salt" refers to a relatively non-toxic, inorganic or organic acid addition salt of a compound of the present disclosure. For example, see S. M. Berge, et al. "Pharmaceutical Salts," J. Pharm. Sci. 1977, 66, 1-19. The production of especially neutral salts is described in US Patent No. 5,560,903.

Pharmaceutically acceptable salts of the compounds according to the present disclosure include salts with inorganic and/or organic bases or amino acids, in particular physiologically tolerable cations of inorganic and/or organic bases or amino acids, such as, inter alia, those of primary, secondary or tertiary amines. Examples may be, without being limited thereto, salts of sodium, lithium, potassium, calcium, magnesium, arginine, lysine, ammonia, creatinine, diethanolamine, ethanol amine, morpholine, glucamine, N,N-dimethylglucamine, N-methylglucamine, ornithine, histidine, imidazole, tromethamine, meglumine and the like. Particularly preferred pharmaceutically acceptable salts of the compounds according to the present disclosure are their corresponding sodium salts.

Those skilled in the art will further recognize that salts of the claimed compounds may be prepared by reaction of the compounds with the appropriate inorganic or organic base via any of a number of known methods.

The present disclosure includes all possible salts of the compounds of the present disclosure as single salts, or as any mixture of said salts, in any ratio.

In the present text, in particular in the Experimental Section, for the synthesis of intermediates and of examples of the present disclosure, when a compound is mentioned as a salt form with the corresponding base or acid, the exact stoichiometric composition of said salt form, as obtained by the respective preparation and/or purification process, is, in most cases, unknown.

This applies analogously to cases in which synthesis intermediates or example compounds or salts thereof have been obtained, by the preparation and/or purification processes described, as solvates, such as hydrates with (if defined) unknown stoichiometric composition.

**FURTHER EMBODIMENTS OF THE PRESENT DISCLOSURE**

In accordance with a further embodiment of the first aspect, the the present disclosure covers compounds of general formula (I), supra, wherein

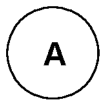

represents a group selected from

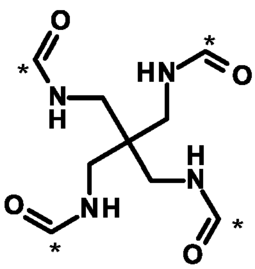

,

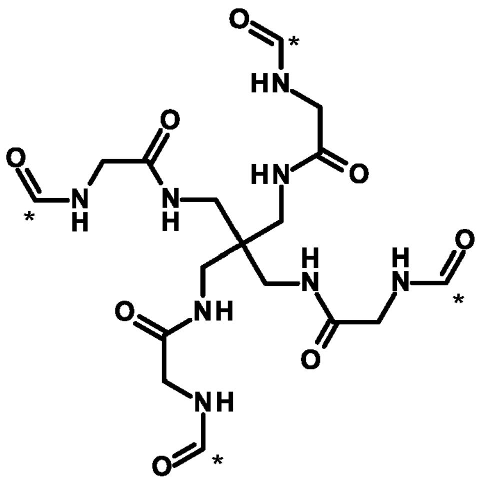

,

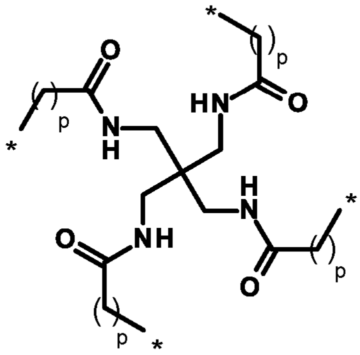

and

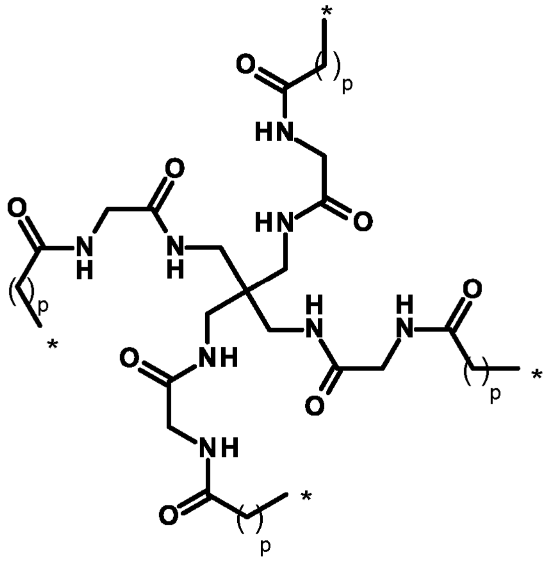

in which * represents the point of attachment to $R^1$ and p is an integer selected from 1 and 2,

$R^1$ represents a group selected from

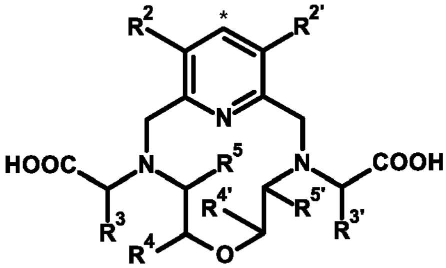

and

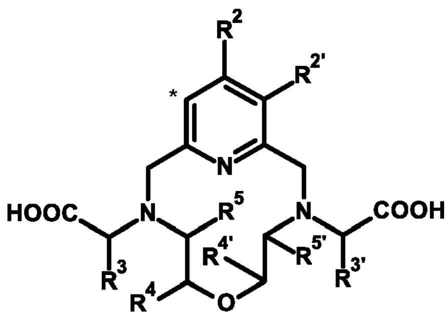

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

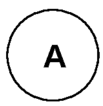

represents a group selected from

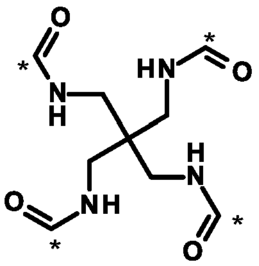
,
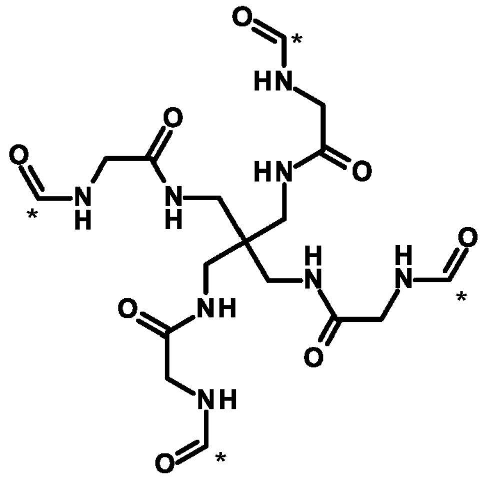
,

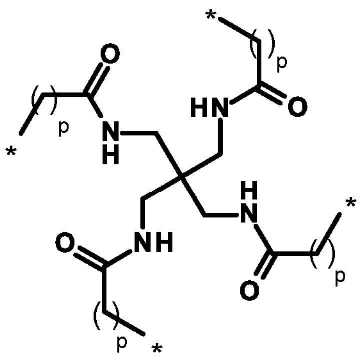
and
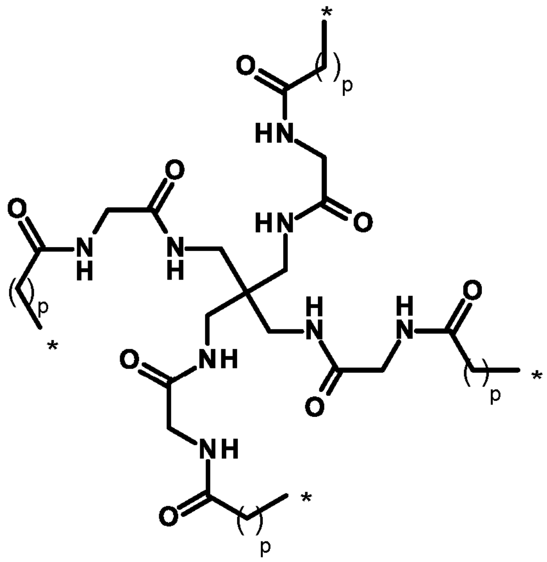

in which * represents the point of attachment to $R^1$ and p is an integer selected from 1 and 2,

$R^1$ represents a group selected from

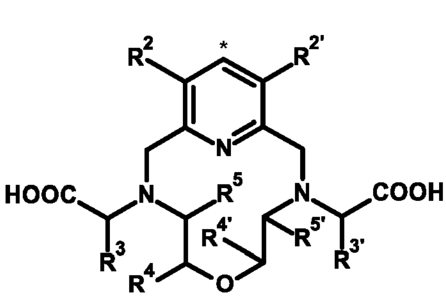
and
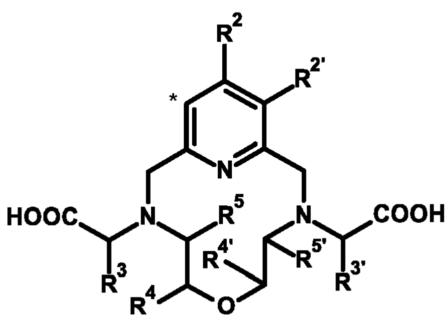
,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

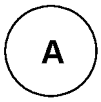

represents a group selected from

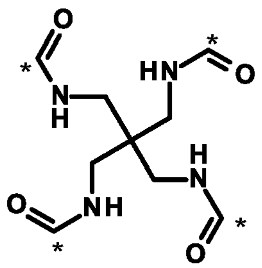

,

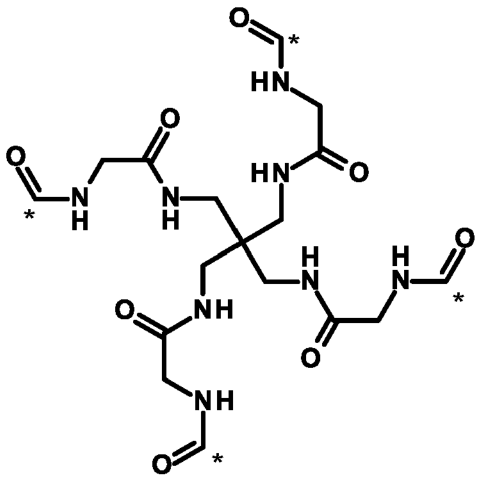

,

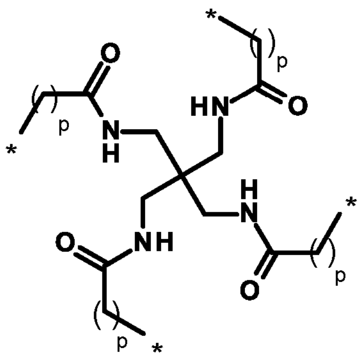

and

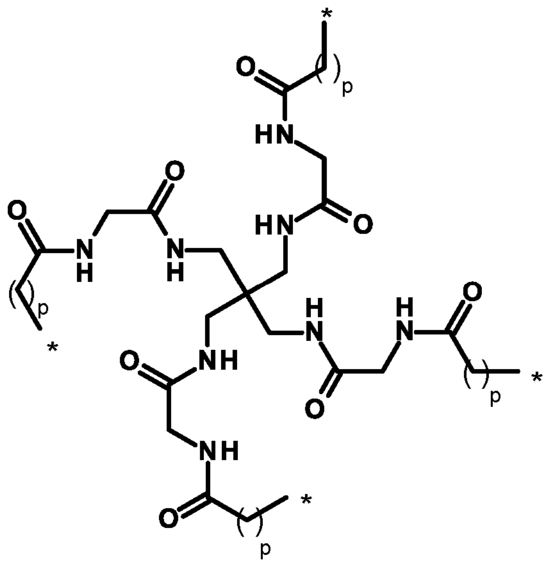

in which * represents the point of attachment to $R^1$ and p is an integer selected from 1 and 2,

$R^1$ represents a group selected from

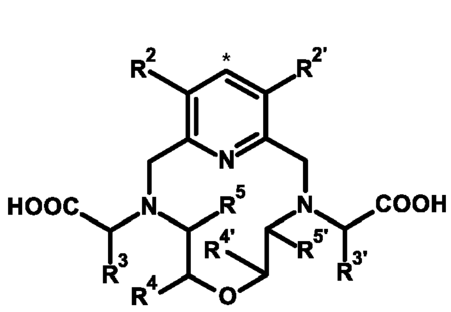

and

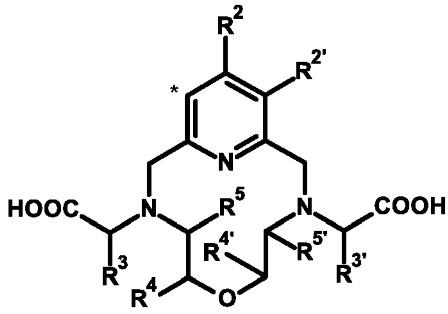

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

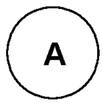

represents a group selected from

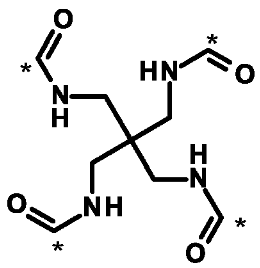

,

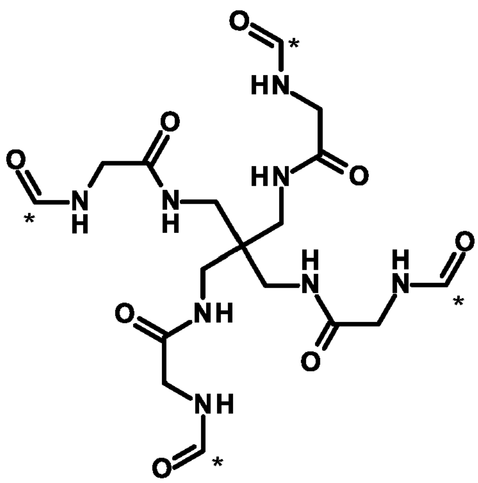

,

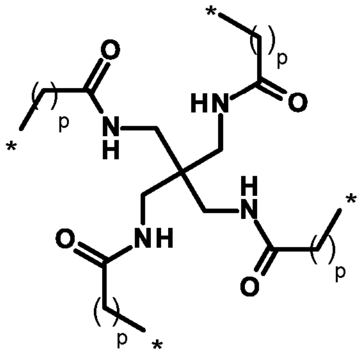

and

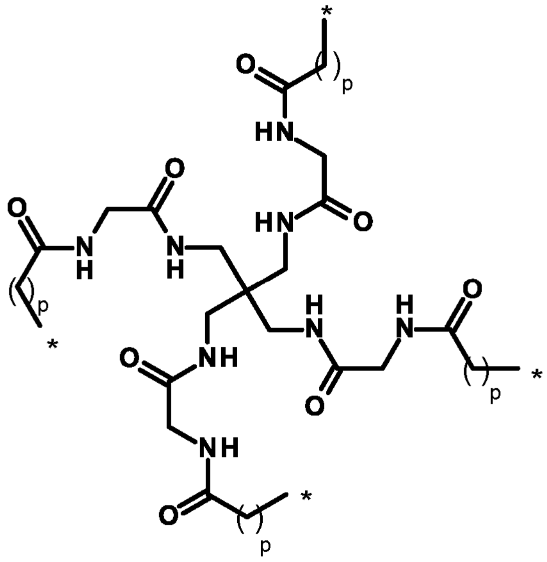

in which * represents the point of attachment to $R^1$ and p is an integer selected from 1 and 2,

$R^1$ represents a group selected from

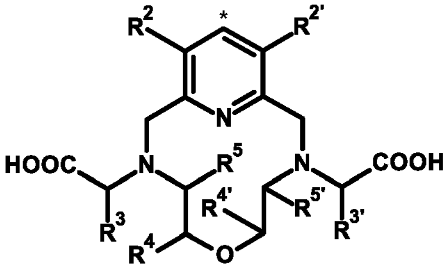

and

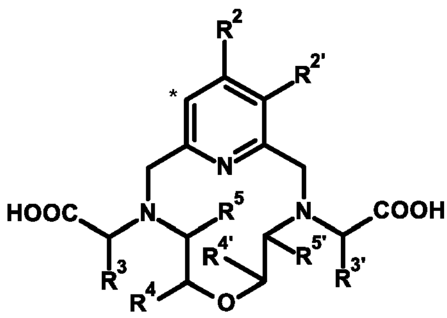

,

in which * represents the point of attachment to A,
$R^2$ and $R^{2'}$ are each independently selected from a hydrogen atom and a methyl group;
$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least one of $R^3$, $R^{3'}$, $R^4$, $R^{4'}$, $R^5$ and $R^{5'}$ is a $C_1$-$C_3$ alkyl group;
and stereoisomers, tautomers, N-oxides, hydrates, solvates, or salts thereof, or mixtures of same.

In accordance with a further embodiment of the first aspect, the the present disclosure covers compounds of general formula (I), supra, wherein

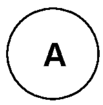

represents a group selected from

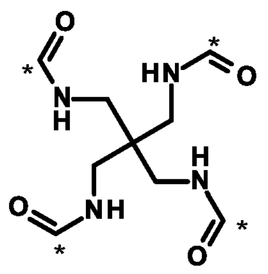
,
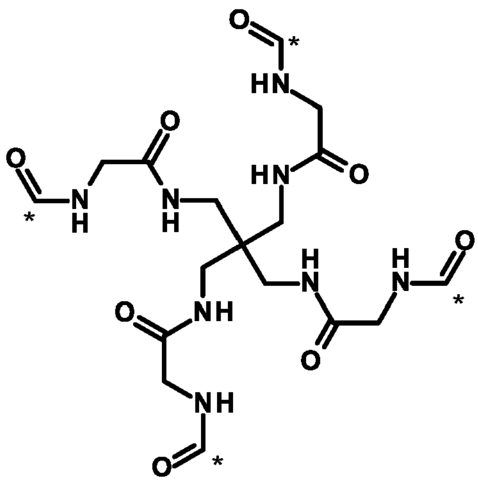
,

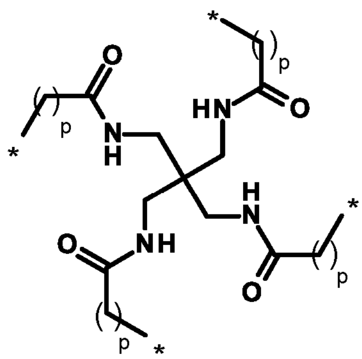
and
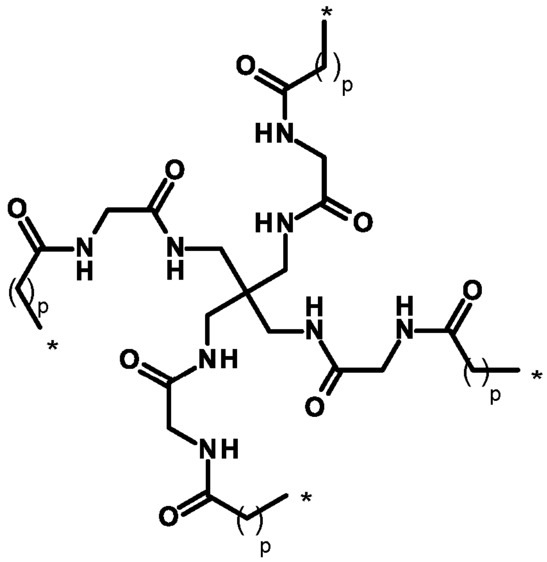

in which * represents the point of attachment to $R^1$ and p is an integer selected from 1 and 2,

$R^1$ represents a group selected from

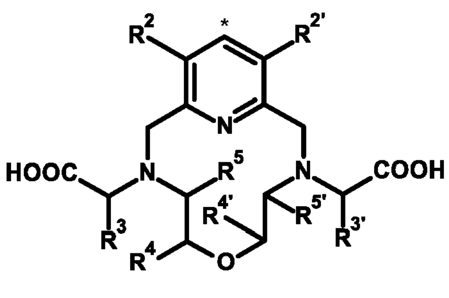
and
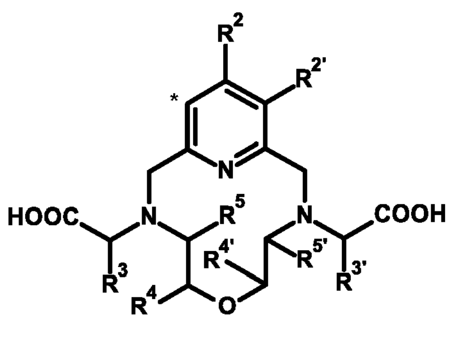
,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

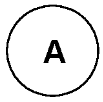

represents a group selected from

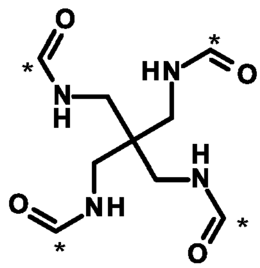

,

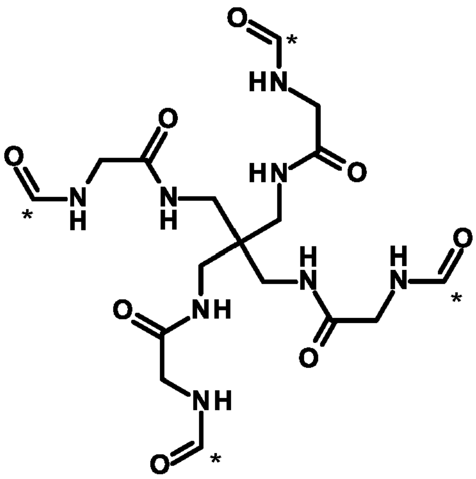

,

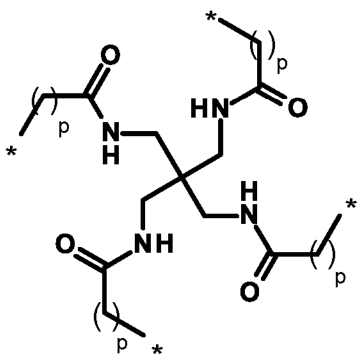

and

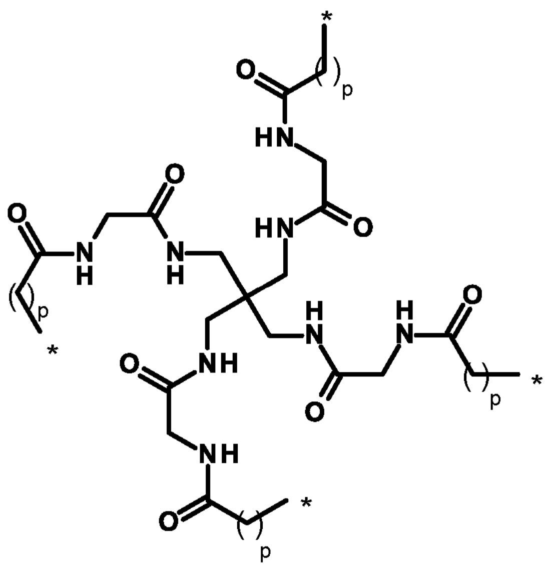

in which * represents the point of attachment to $R^1$ and p is an integer selected from 1 and 2,

$R^1$ represents a group selected from

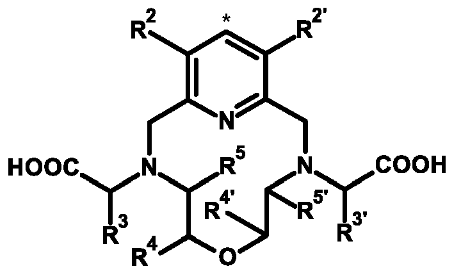

and

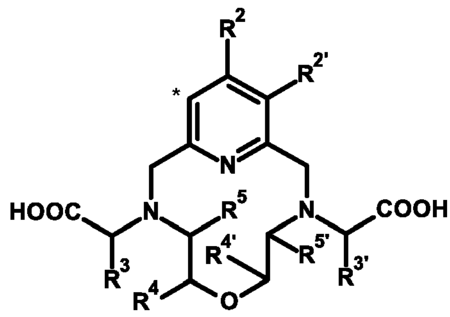

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

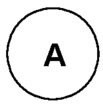

represents a group selected from

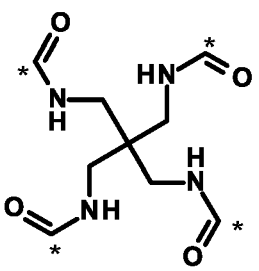

,

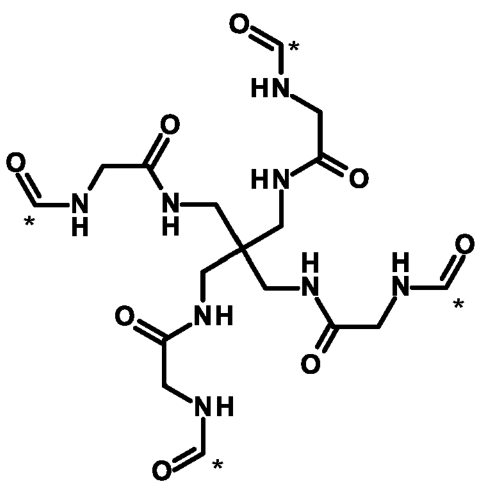

,

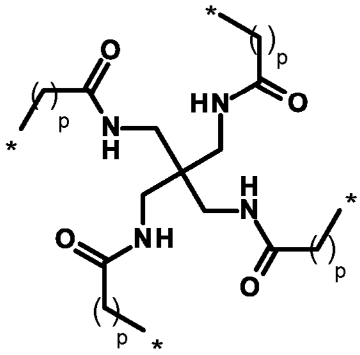

and

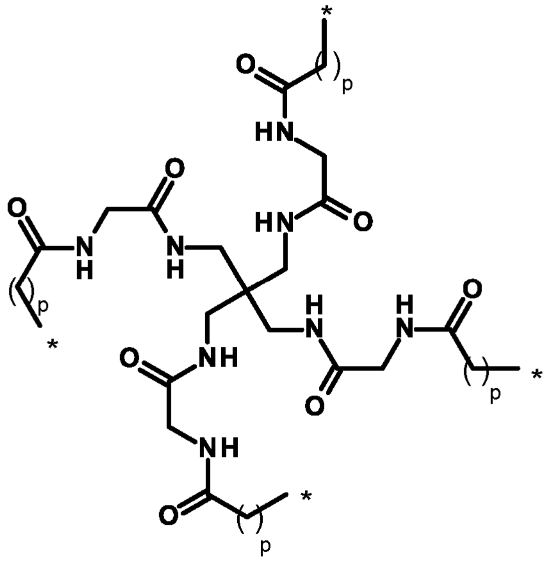

in which * represents the point of attachment to $R^1$ and p is an integer selected from 1 and 2,

$R^1$ represents a group selected from

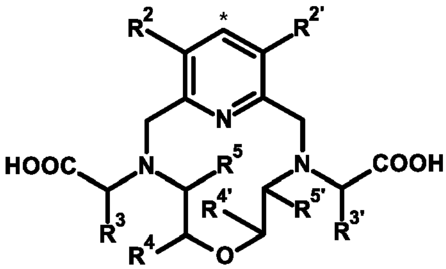

and

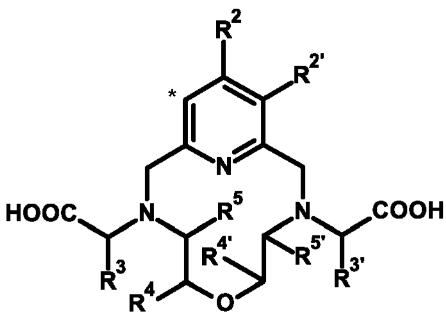

,

in which * represents the point of attachment to A,
$R^2$ and $R^{2'}$ are each independently selected from a hydrogen atom and a methyl group;
$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least two of $R^3$, $R^{3'}$, $R^4$, $R^{4'}$, $R^5$ and $R^{5'}$ are a methyl group;
and stereoisomers, tautomers, N-oxides, hydrates, solvates, or salts thereof, or mixtures of same.

In accordance with a further embodiment of the first aspect, the the present disclosure covers compounds of general formula (I), supra, wherein

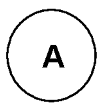

represents a group selected from

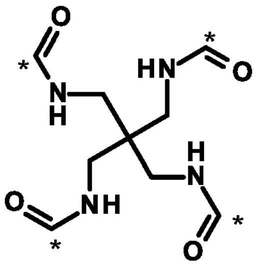 and 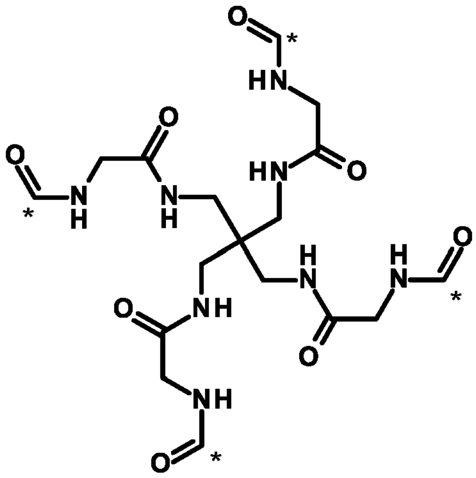 ,

in which * represents the point of attachment to $R^1$,

$R^1$ represents a group selected from

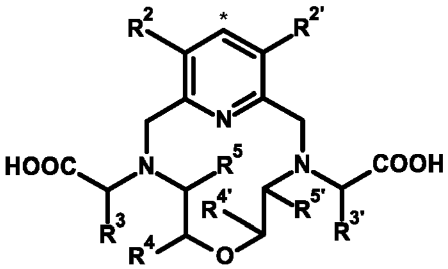 and 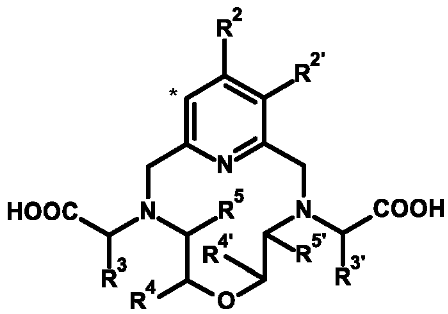 ,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

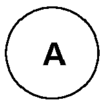

represents a group selected from

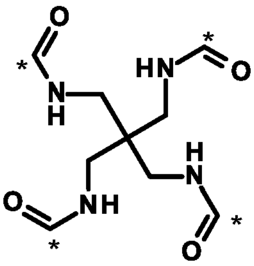

and

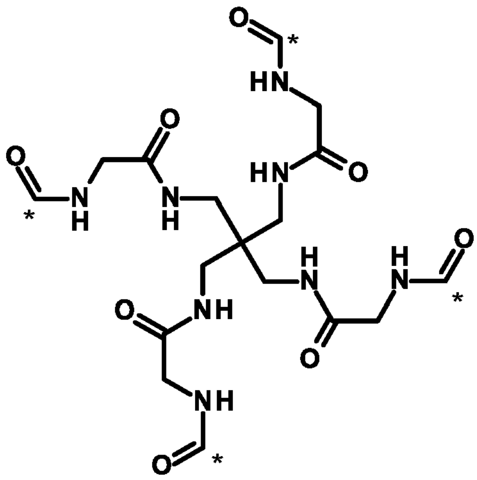

,

in which * represents the point of attachment to $R^1$,

$R^1$ represents a group selected from

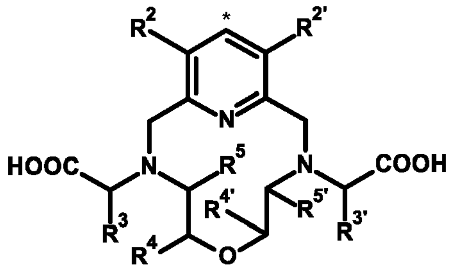

and

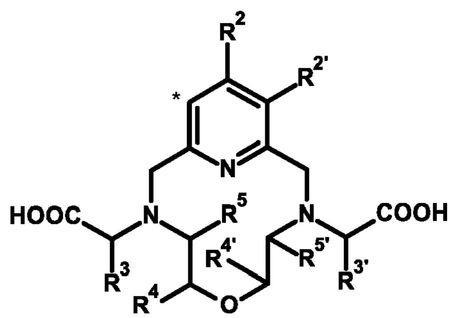

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

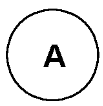

represents a group selected from

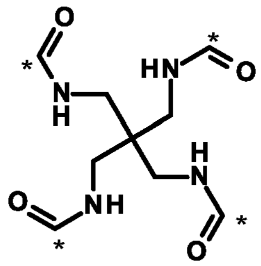

and

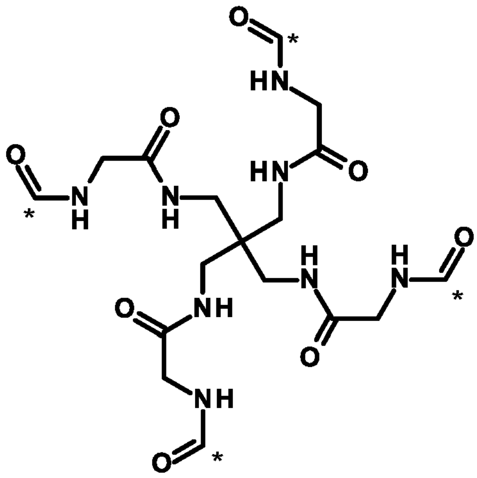

,

in which * represents the point of attachment to $R^1$,

$R^1$ represents a group selected from

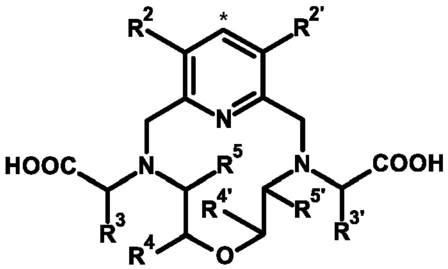

and

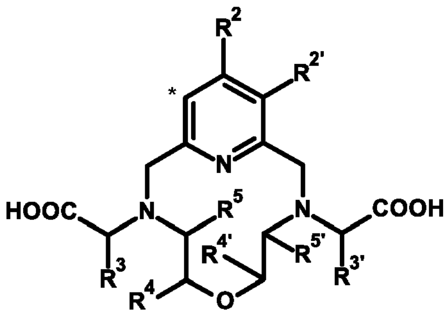

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

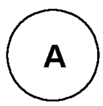

represents a group selected from

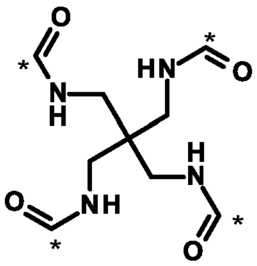 and 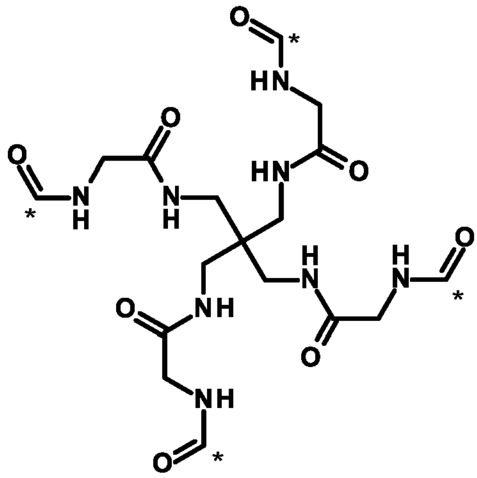 ,

in which * represents the point of attachment to $R^1$,

$R^1$ represents a group selected from

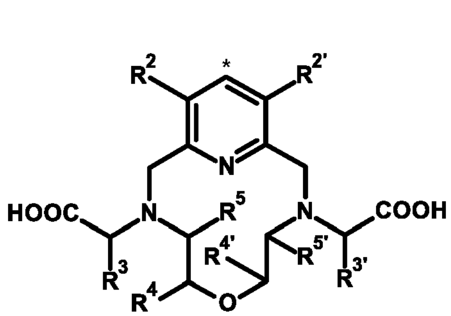 and 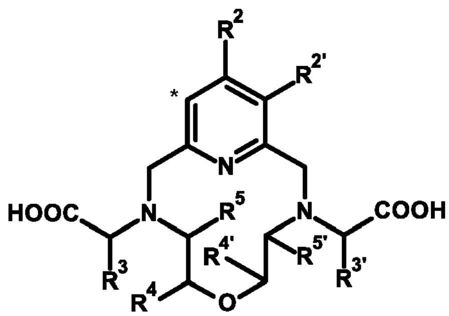 ,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

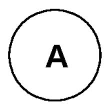

represents a group selected from

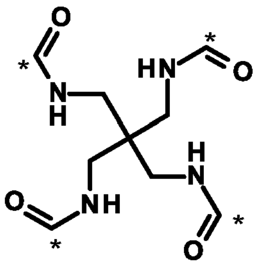

and

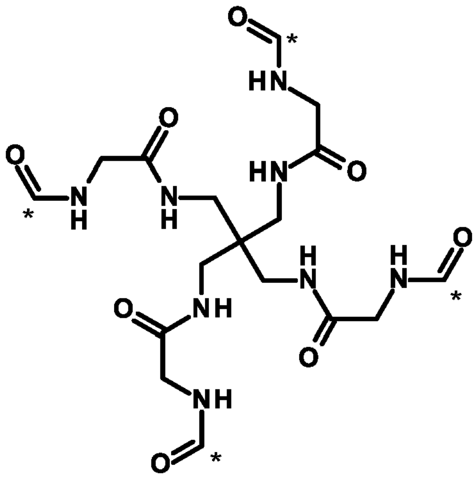

,

in which * represents the point of attachment to $R^1$,

$R^1$ represents a group selected from

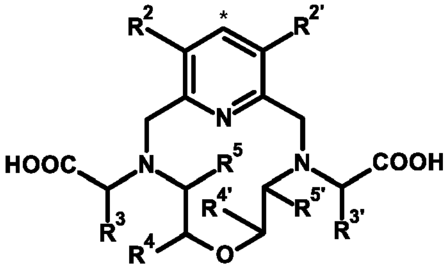

and

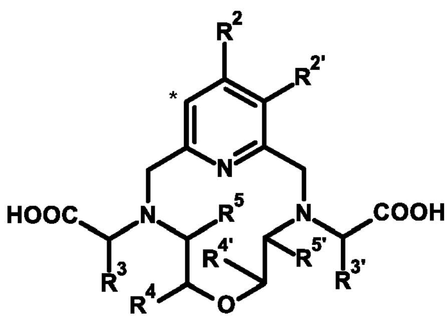

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

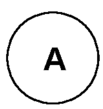

represents a group selected from

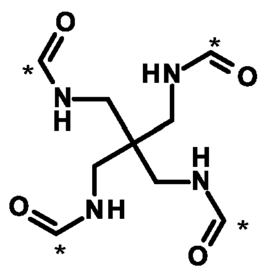

and

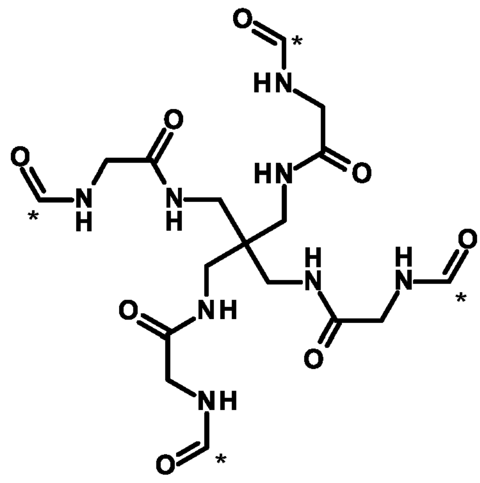

,

in which * represents the point of attachment to $R^1$,

$R^1$ represents a group selected from

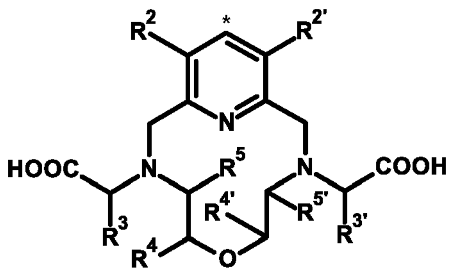

and

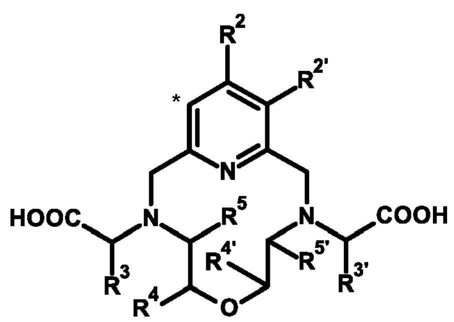

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

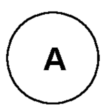

represents a group selected from

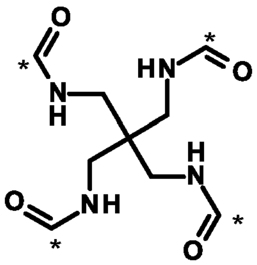 and 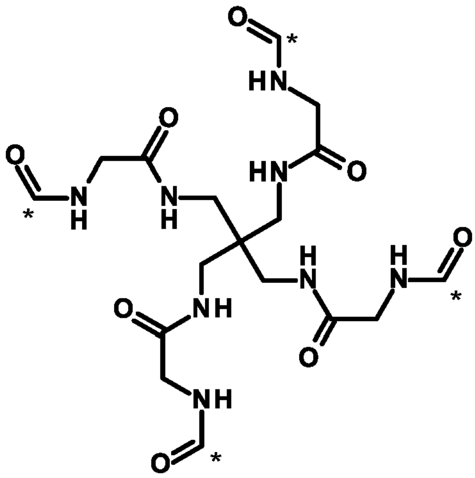 ,

in which * represents the point of attachment to $R^1$,

$R^1$ represents a group selected from

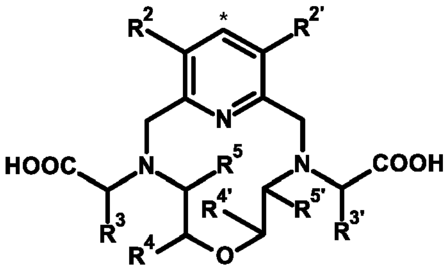 and 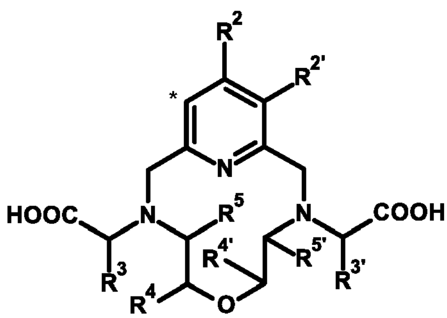 ,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

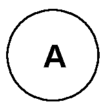

represents a group selected from

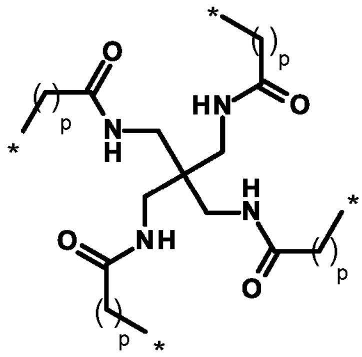

and

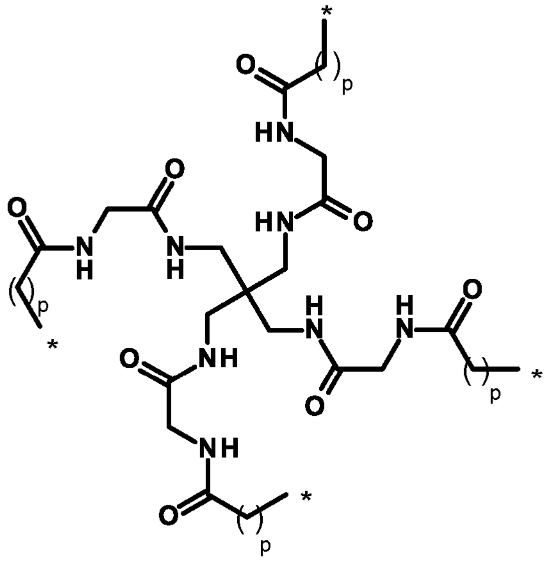

in which * represents the point of attachment to $R^1$ and p is an integer selected from 1 and 2,

$R^1$ represents a group selected from

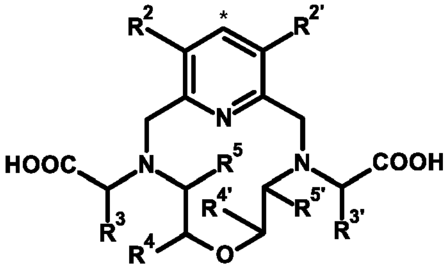

and

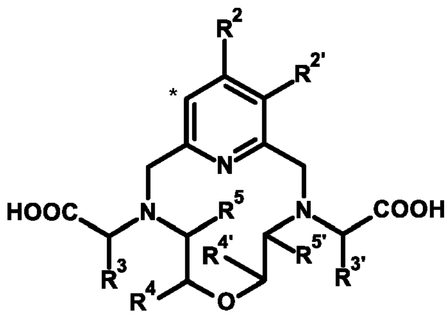

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

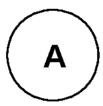

represents a group selected from

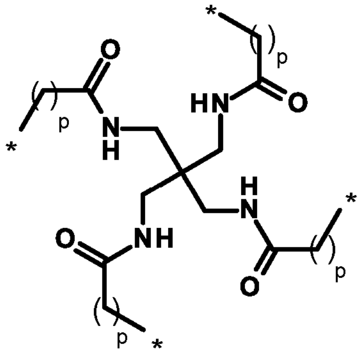

and

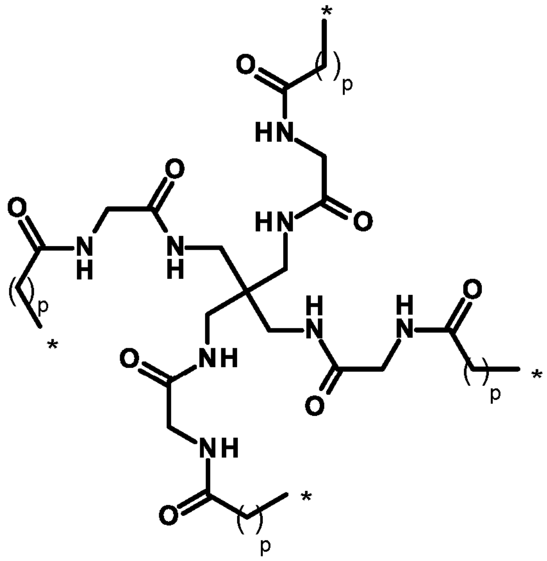

in which * represents the point of attachment to $R^1$ and p is an integer selected from 1 and 2,

$R^1$ represents a group selected from

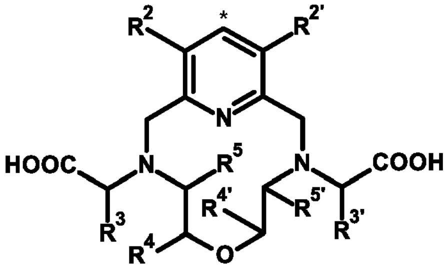

and

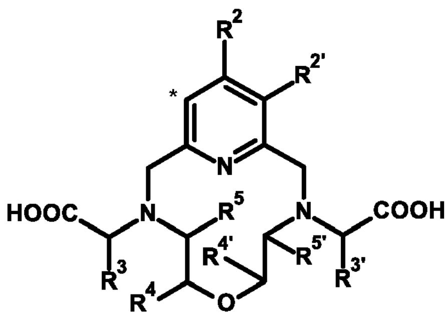

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

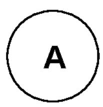

represents a group selected from

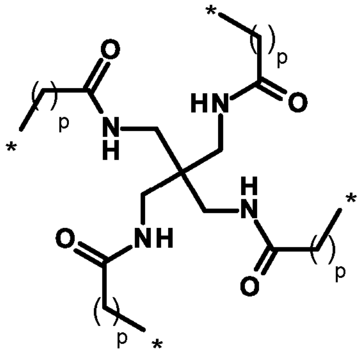

and

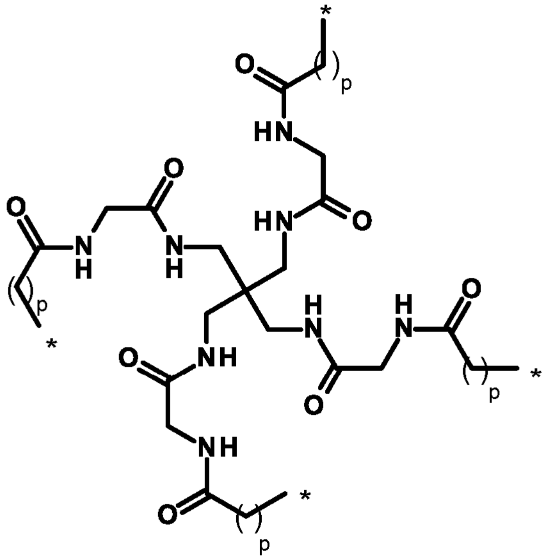

in which * represents the point of attachment to $R^1$ and p is an integer selected from 1 and 2,

$R^1$ represents a group selected from

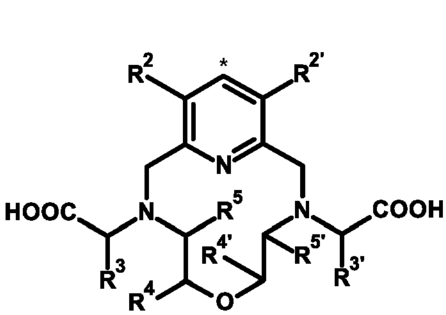

and

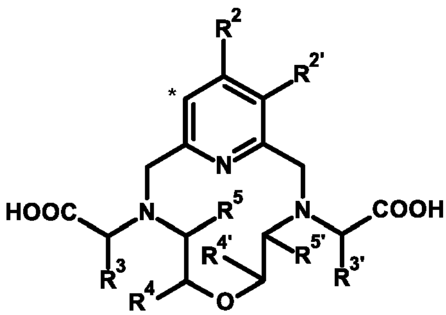

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

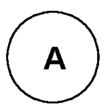

represents a group selected from

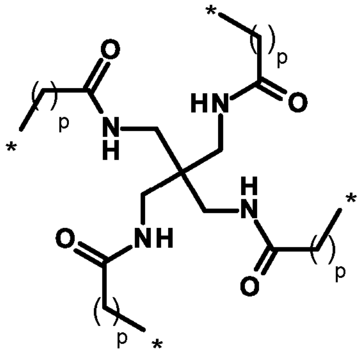

and

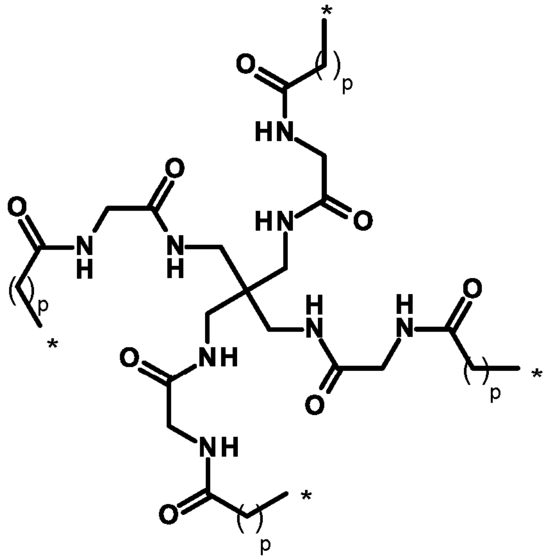

in which * represents the point of attachment to $R^1$ and p is an integer selected from 1 and 2,

$R^1$ represents a group selected from

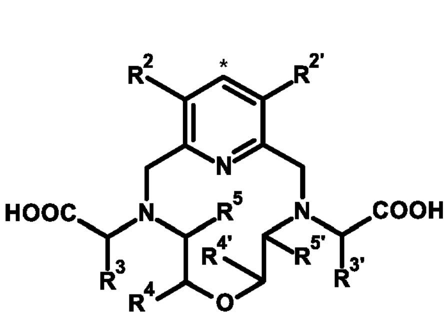

and

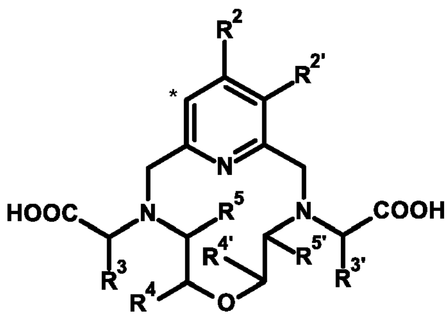

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

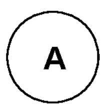

represents a group selected from

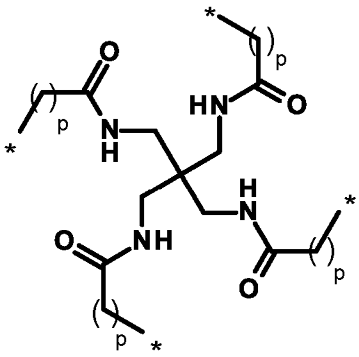

and

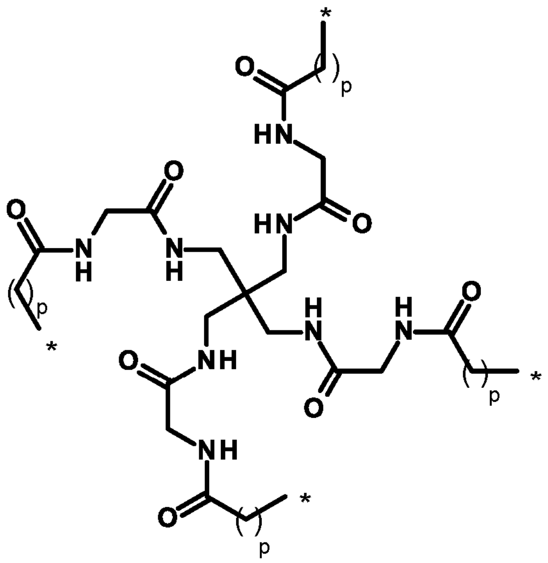

in which * represents the point of attachment to $R^1$ and p is an integer selected from 1 and 2,

$R^1$ represents a group selected from

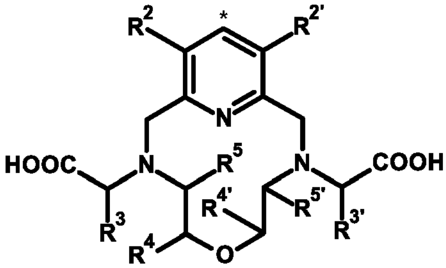

and

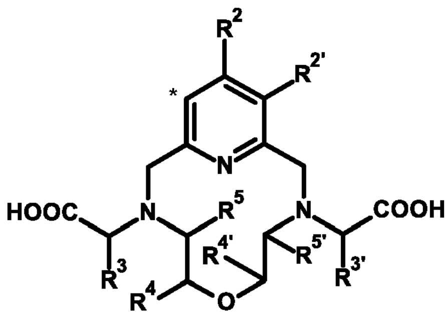

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

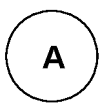

represents a group selected from

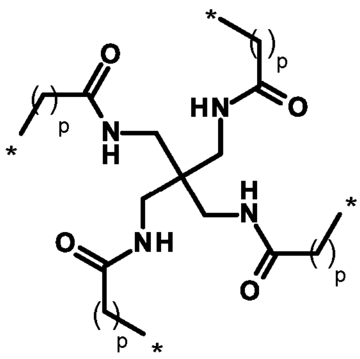

and

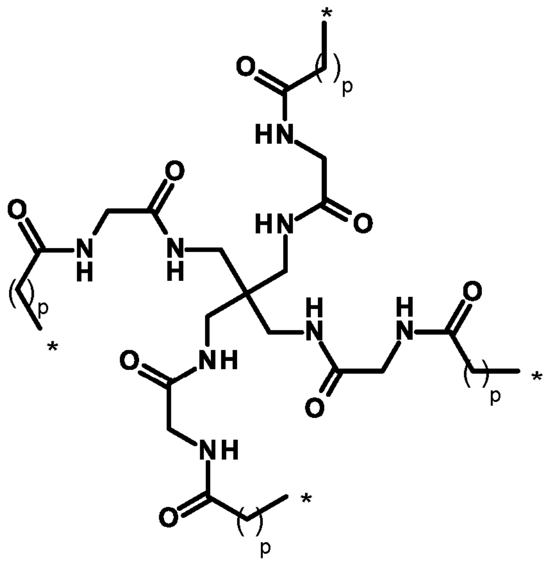

in which * represents the point of attachment to $R^1$ and p is an integer selected from 1 and 2,

$R^1$ represents a group selected from

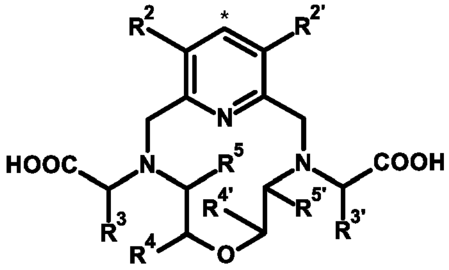

and

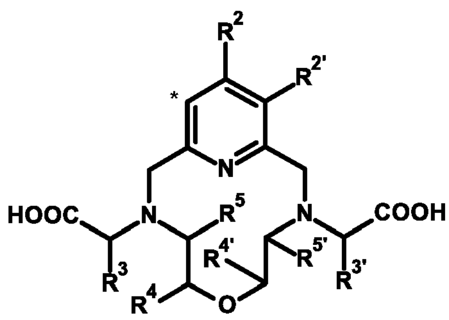

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

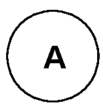

represents a group selected from

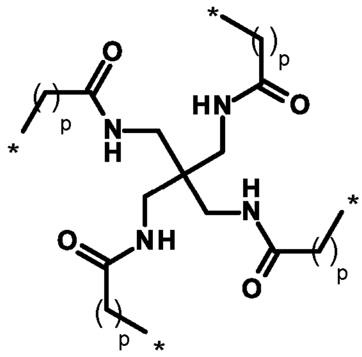

and

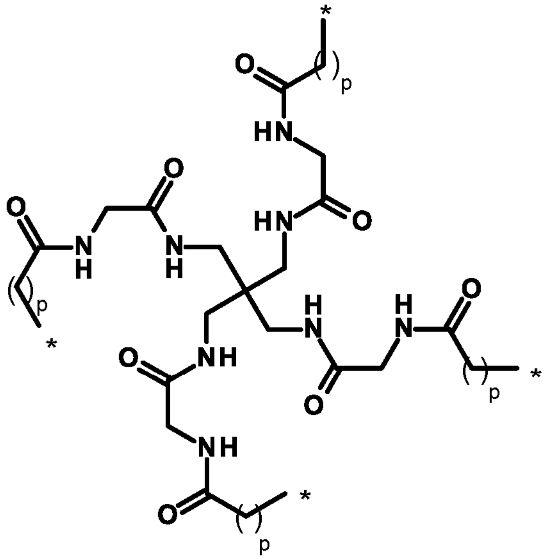

in which * represents the point of attachment to $R^1$ and p is an integer selected from 1 and 2,

$R^1$ represents a group selected from

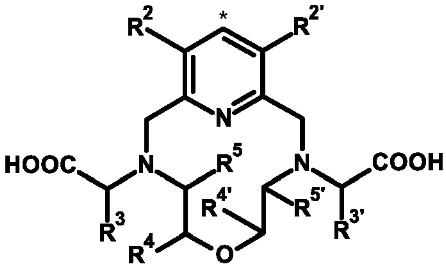

and

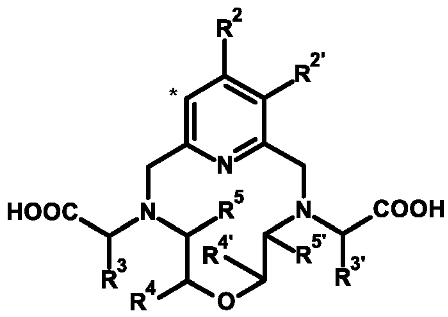

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

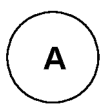

represents a group selected from

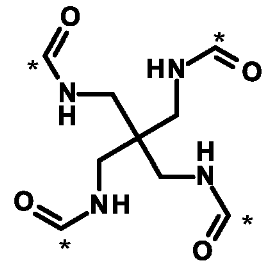
,
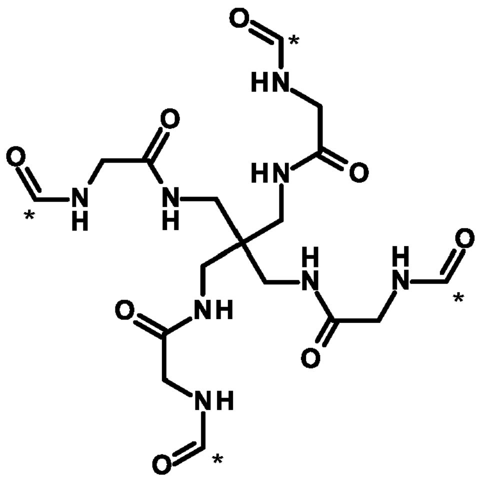
,

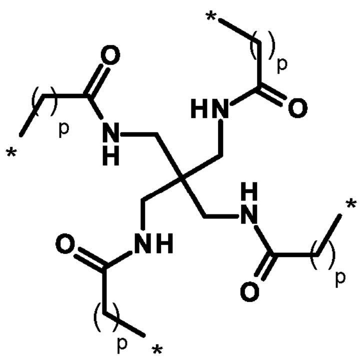
and
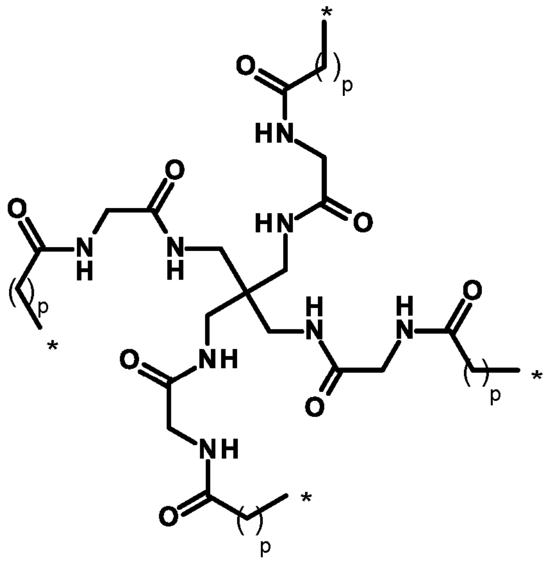

in which * represents the point of attachment to $R^1$ and p is an integer selected from 1 and 2,

$R^1$ represents a group selected from

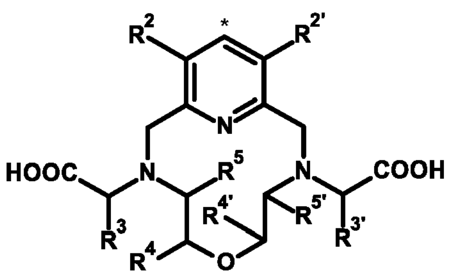
and
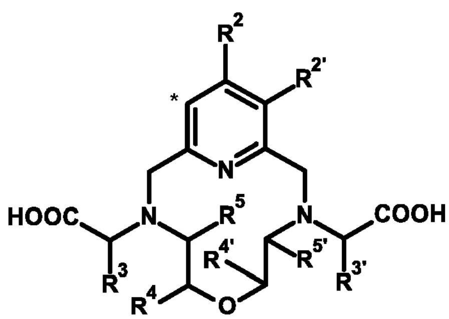
,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

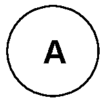

represents a group selected from

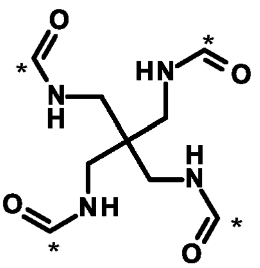

,

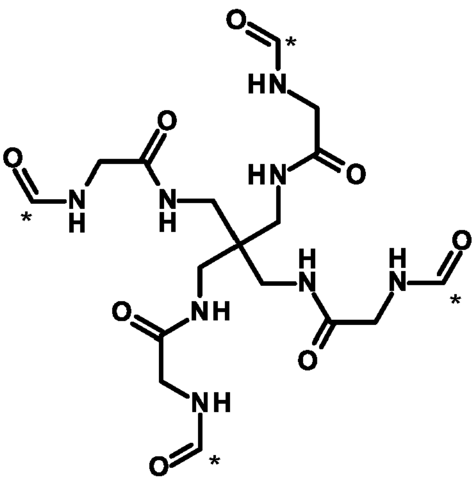

,

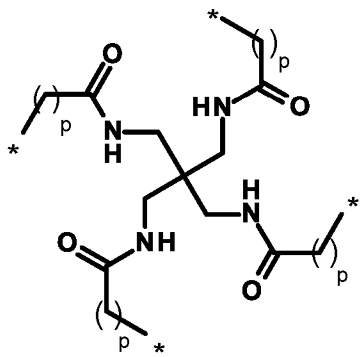

and

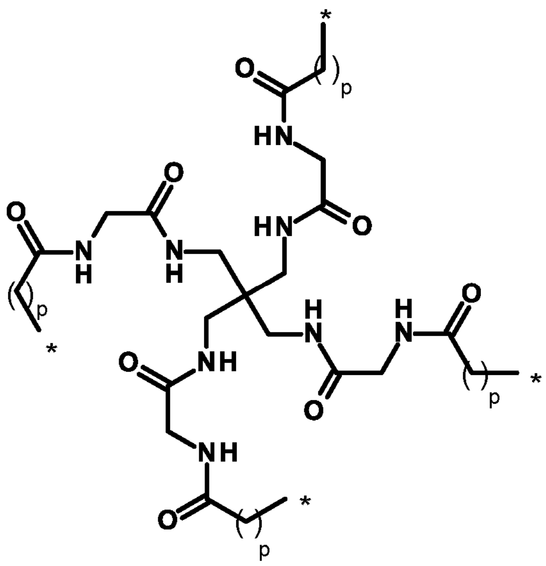

in which * represents the point of attachment to $R^1$ and p is an integer selected from 1 and 2,

$R^1$ represents a group selected from

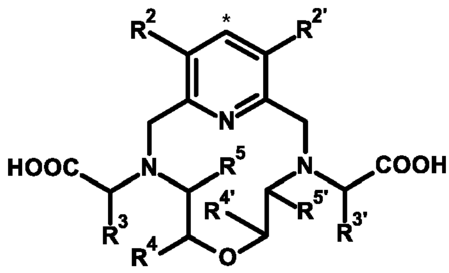

and

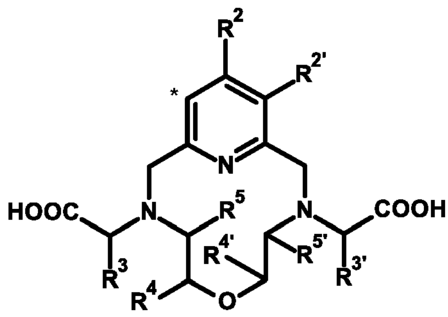

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

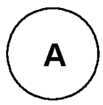

represents a group selected from

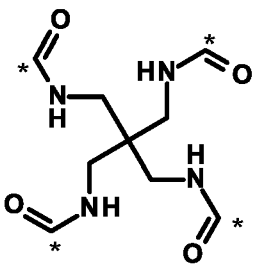

,

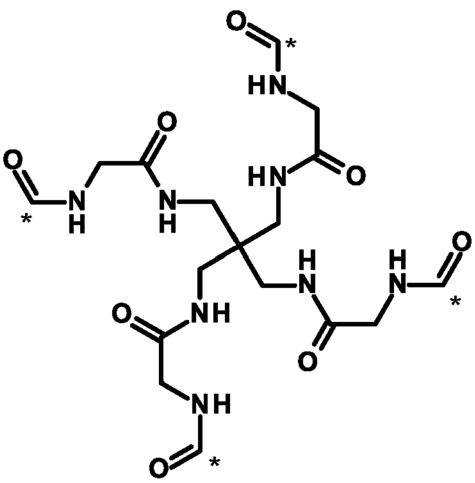

,

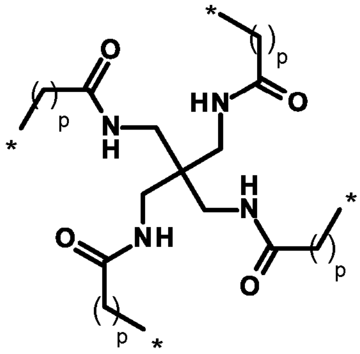

and

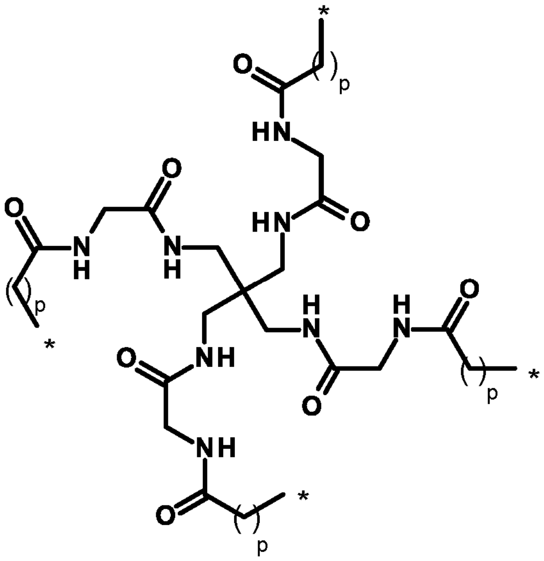

in which * represents the point of attachment to $R^1$ and p is an integer selected from 1 and 2,

$R^1$ represents a group selected from

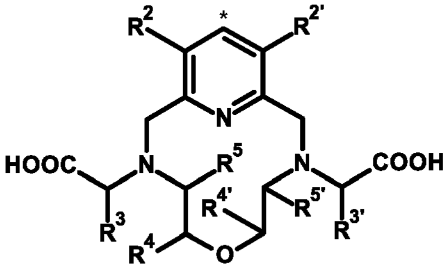

and

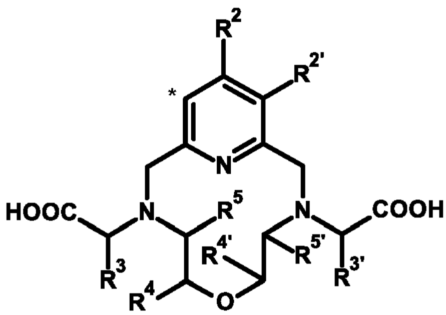

,

in which * represents the point of attachment to A,
$R^2$ and $R^{2'}$ are each independently selected from a hydrogen atom and a methyl group;
$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least two of $R^3$, $R^{3'}$, $R^4$, $R^{4'}$, $R^5$ and $R^{5'}$ are not a hydrogen atom;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

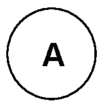

represents a group selected from

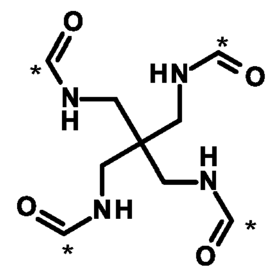
,
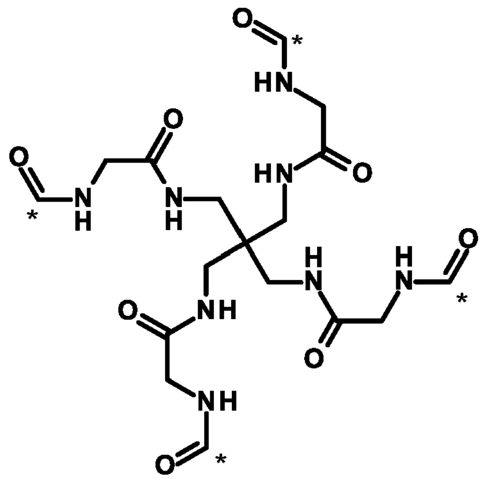
,

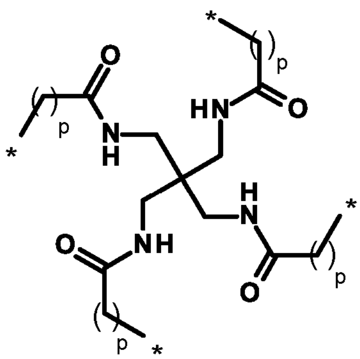
and
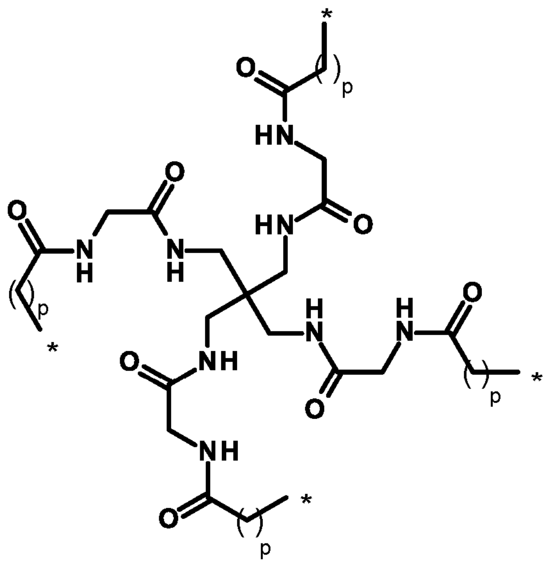

in which * represents the point of attachment to $R^1$ and p is an integer selected from 1 and 2,

$R^1$ represents a group selected from

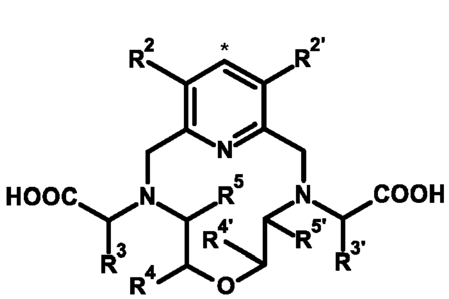
and
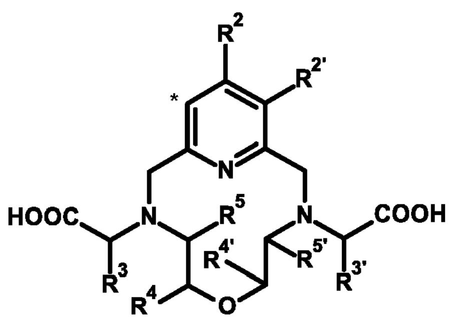
,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

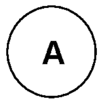

represents a group selected from

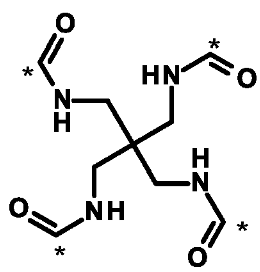

,

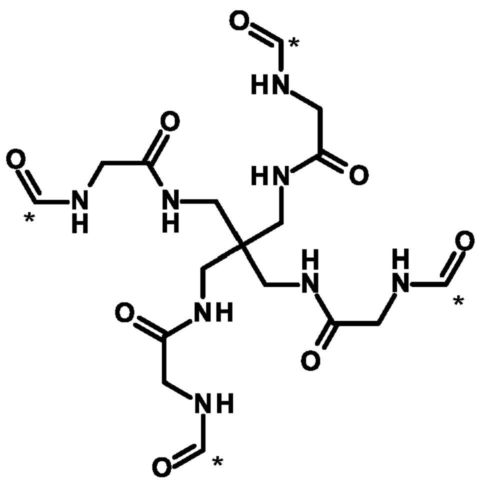

,

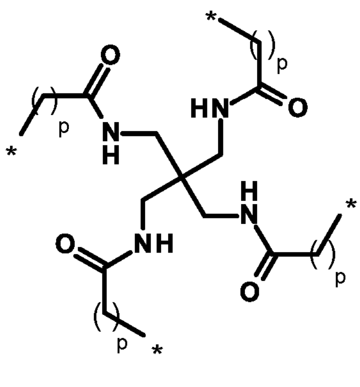

and

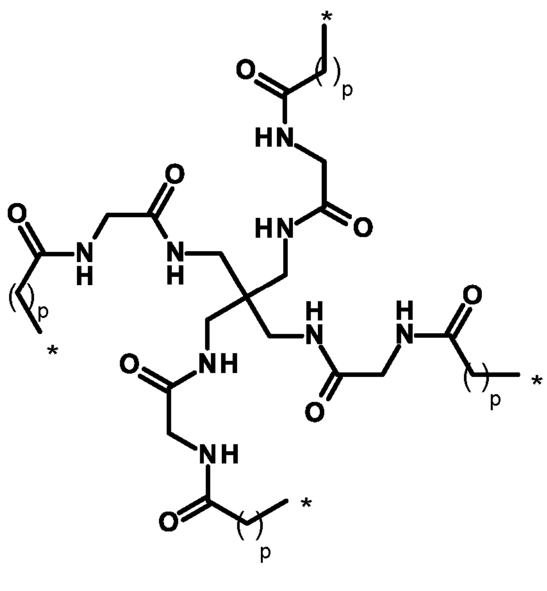

in which * represents the point of attachment to $R^1$ and p is an integer selected from 1 and 2,

$R^1$ represents a group selected from

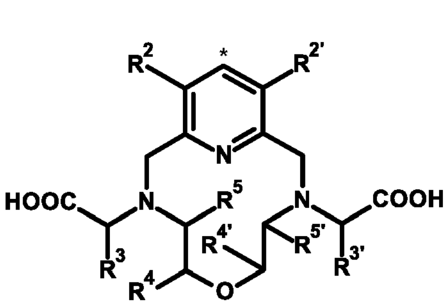

and

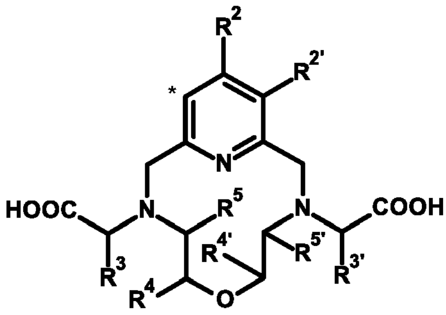

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

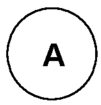

represents a group selected from

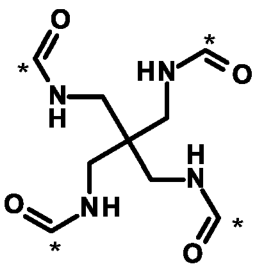

,

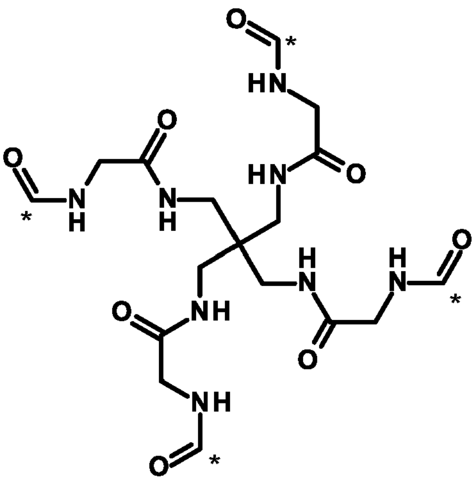

,

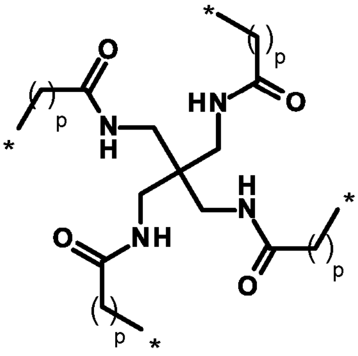

and

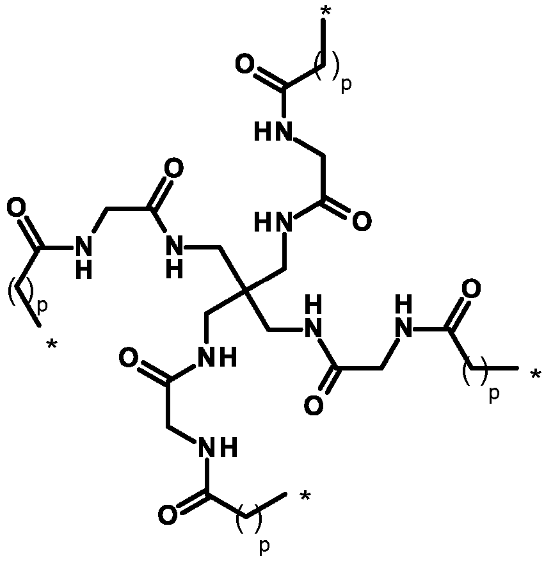

in which * represents the point of attachment to $R^1$ and p is an integer selected from 1 and 2,

$R^1$ represents a group selected from

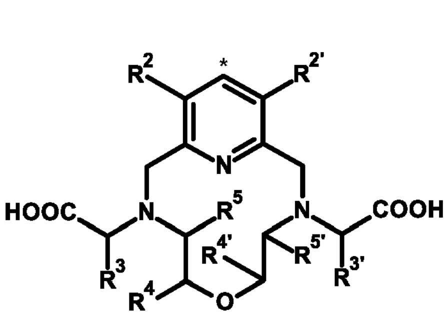

and

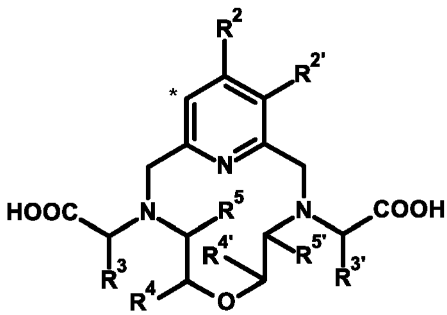

,

in which * represents the point of attachment to A,
$R^2$ and $R^{2'}$ are each independently selected from a hydrogen atom and a methyl group;
$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least one of $R^3$, $R^{3'}$, $R^4$, $R^{4'}$, $R^5$ and $R^{5'}$ is a methyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

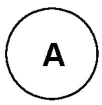

represents a group selected from

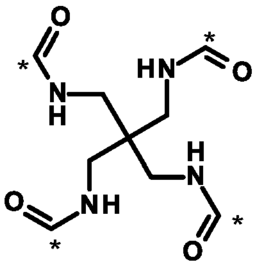
,
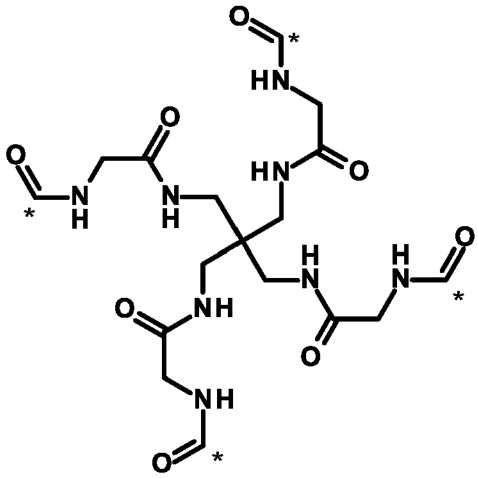
,

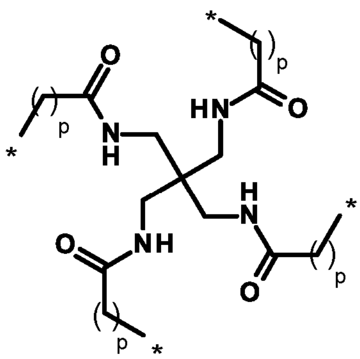
and
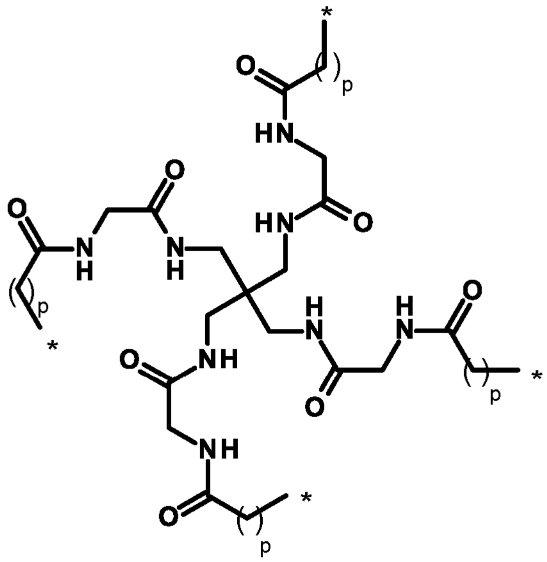

in which * represents the point of attachment to $R^1$ and p is an integer selected from 1 and 2,

$R^1$ represents a group selected from

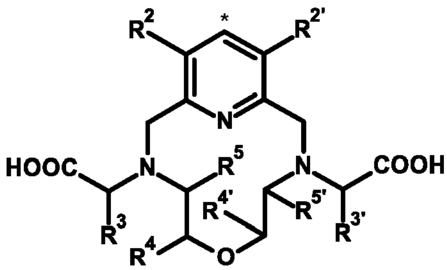
and
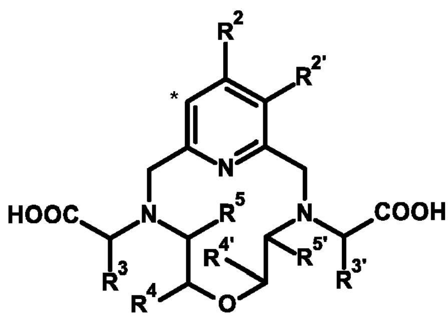
,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

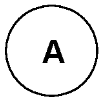

represents a group selected from

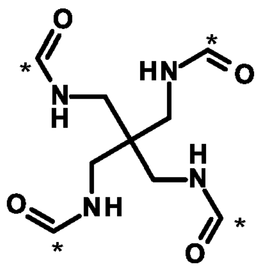

and

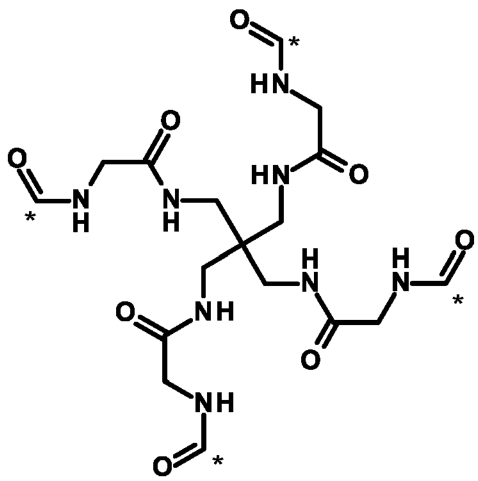

,

in which * represents the point of attachment to $R^1$,

$R^1$ represents a group selected from

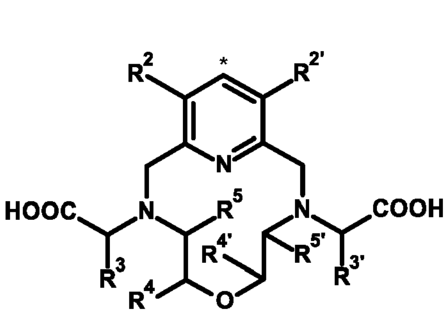

and

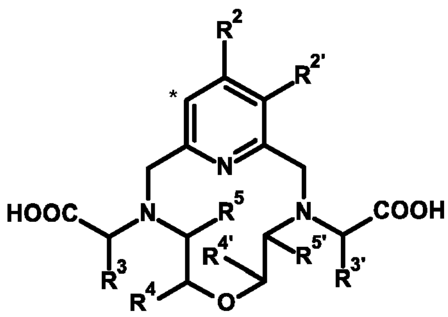

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), supra, in the form of a complex with $Mn^{2+}$, wherein

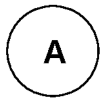

represents a group selected from

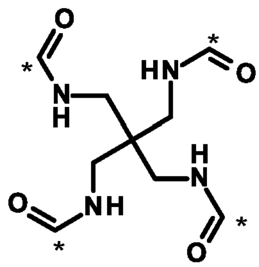

and

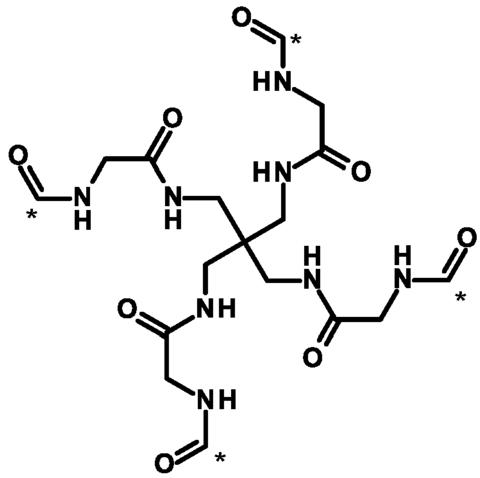

,

in which * represents the point of attachment to $R^1$,

$R^1$ represents a group selected from

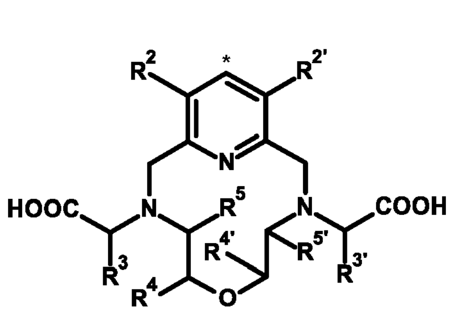

and

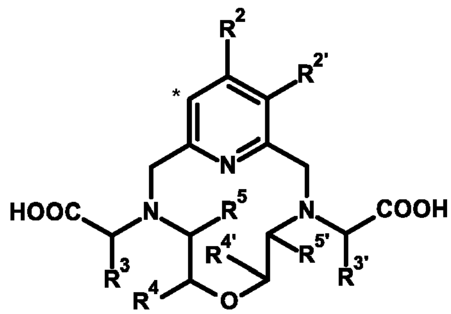

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

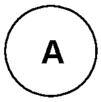

represents a group selected from

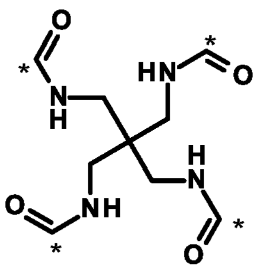

and

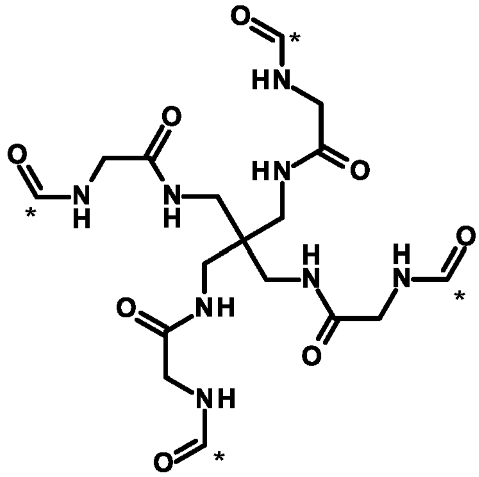

,

in which * represents the point of attachment to $R^1$,

$R^1$ represents a group selected from

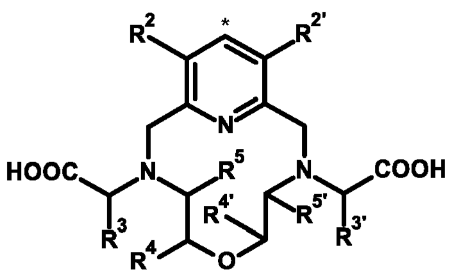

and

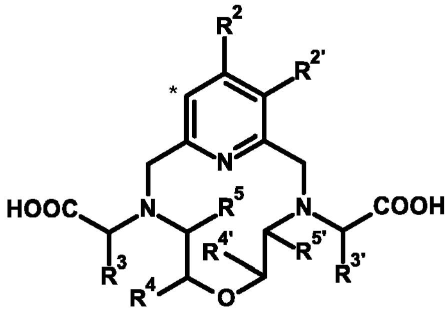

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are not a hydrogen atom;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

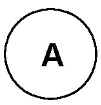

represents a group selected from

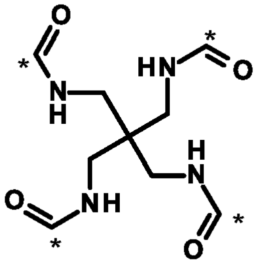 and 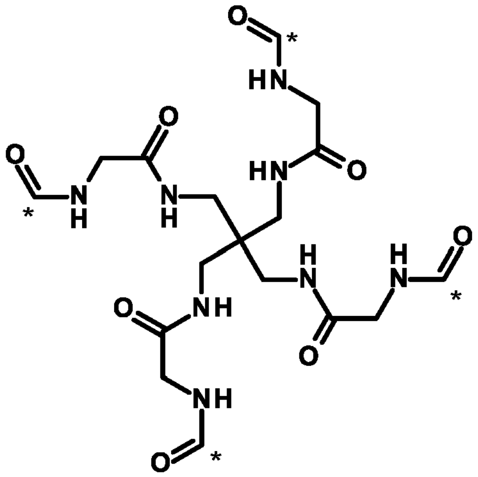 ,

in which * represents the point of attachment to $R^1$,

$R^1$ represents a group selected from

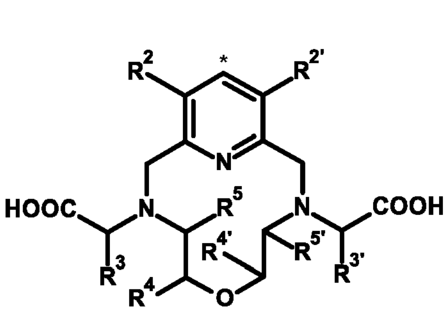 and 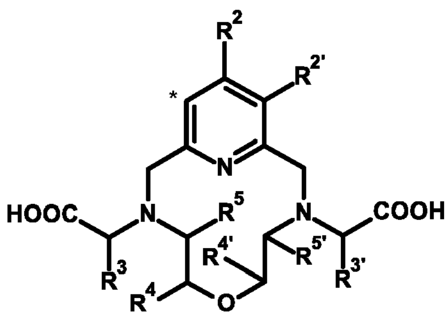 ,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

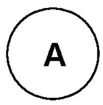

represents a group selected from

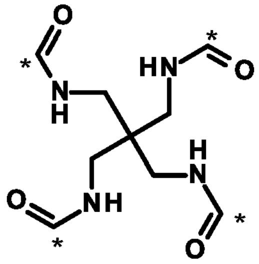 and 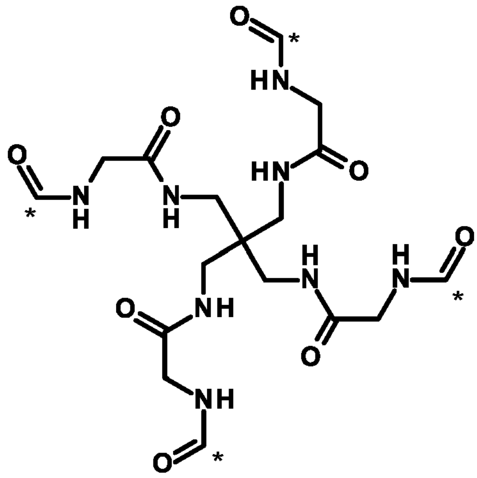,

in which * represents the point of attachment to $R^1$,

$R^1$ represents a group selected from

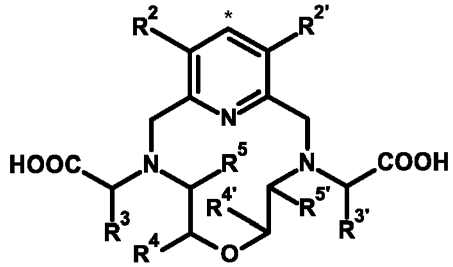 and 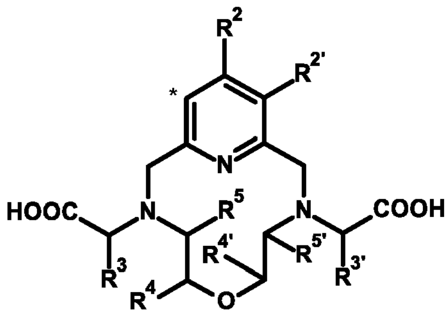,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

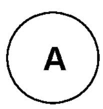

represents a group selected from

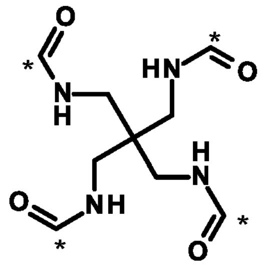

and

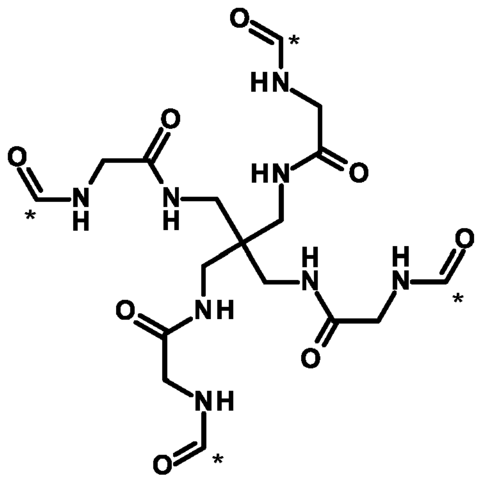

,

in which * represents the point of attachment to $R^1$,

$R^1$ represents a group selected from

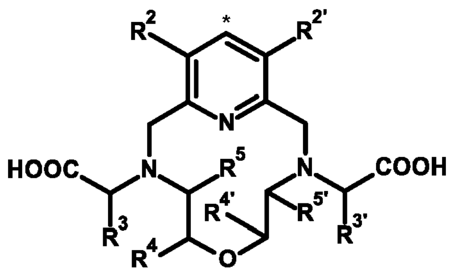

and

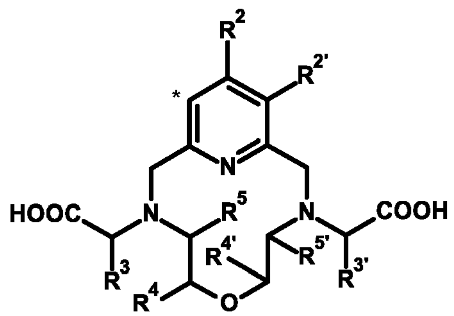

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

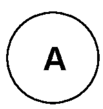

represents a group selected from

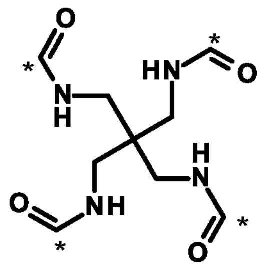

and

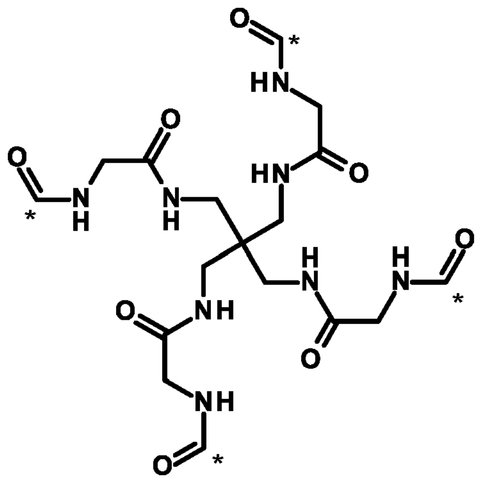

,

in which * represents the point of attachment to $R^1$,

$R^1$ represents a group selected from

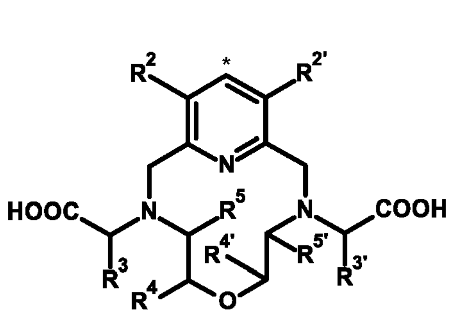

and

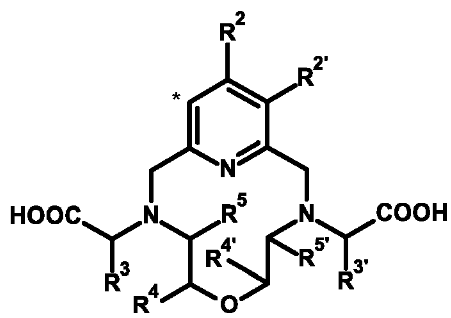

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

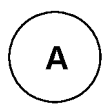

represents a group selected from

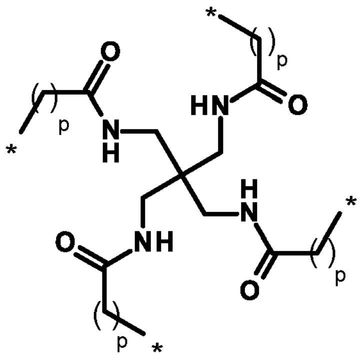

and

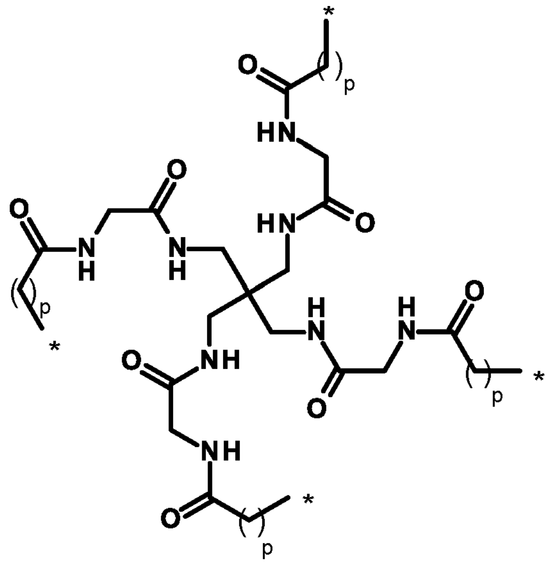

in which * represents the point of attachment to $R^1$ and p is an integer selected from 1 and 2,

$R^1$ represents a group selected from

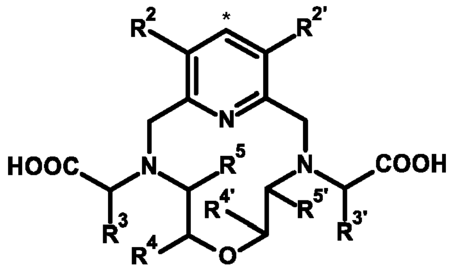

and

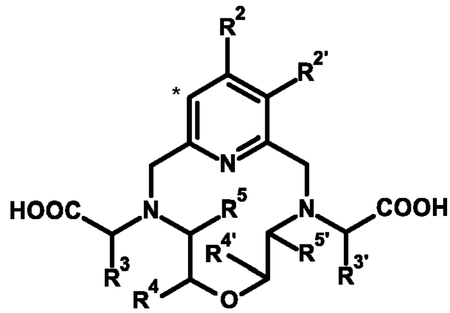

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

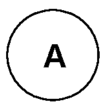

represents a group selected from

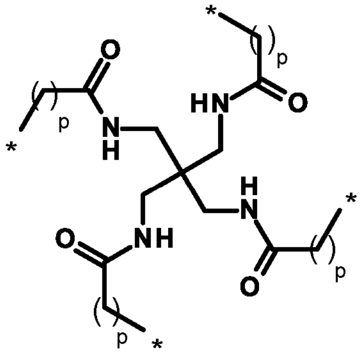

and

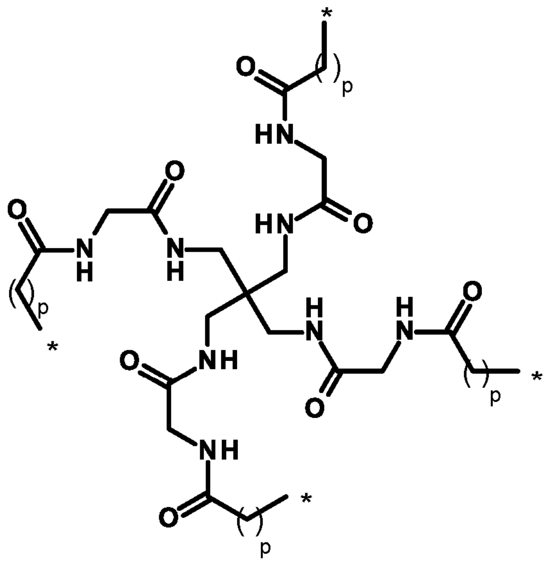

in which * represents the point of attachment to $R^1$ and p is an integer selected from 1 and 2,

$R^1$ represents a group selected from

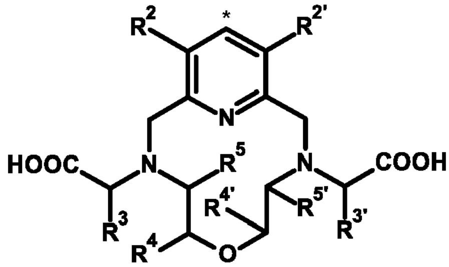

and

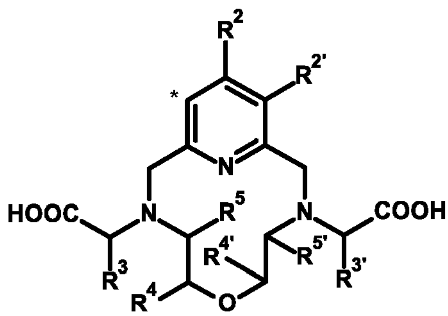

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

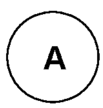

represents a group selected from

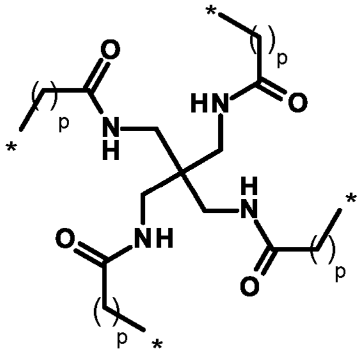

and

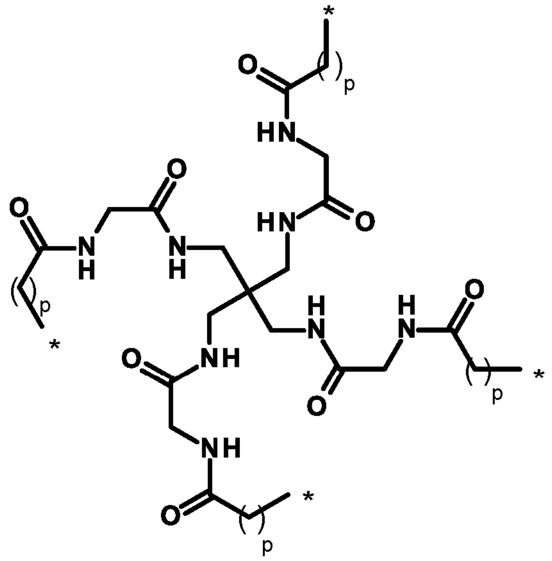

in which * represents the point of attachment to $R^1$ and p is an integer selected from 1 and 2,

$R^1$ represents a group selected from

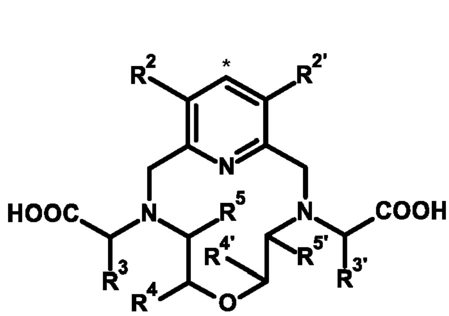

and

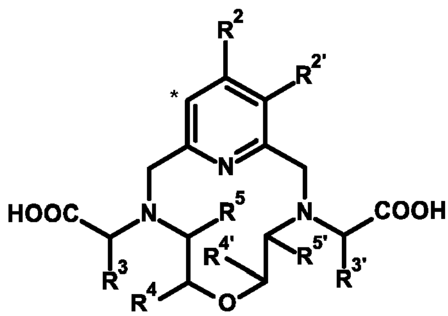

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are not a hydrogen atom;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

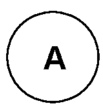

represents a group selected from

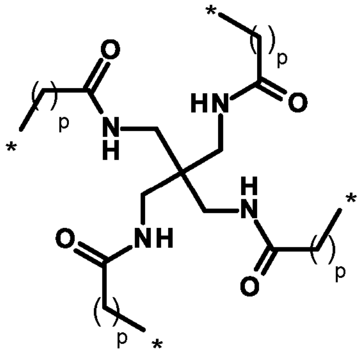

and

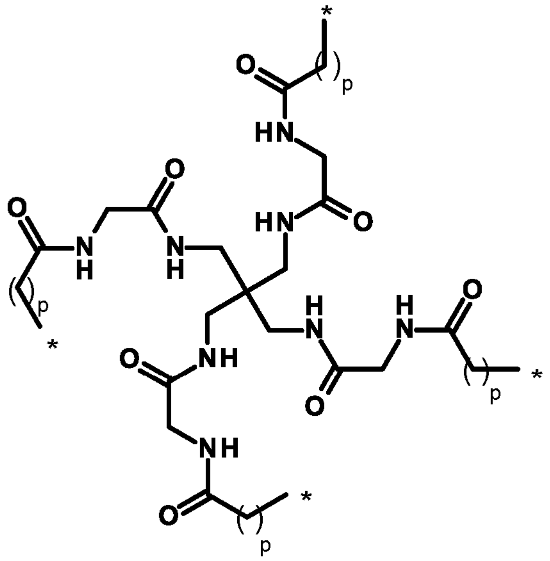

in which * represents the point of attachment to $R^1$ and p is an integer selected from 1 and 2,

$R^1$ represents a group selected from

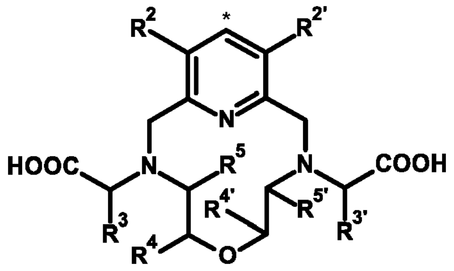

and

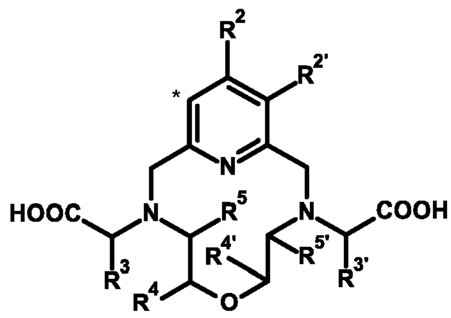

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

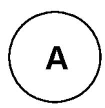

represents a group selected from

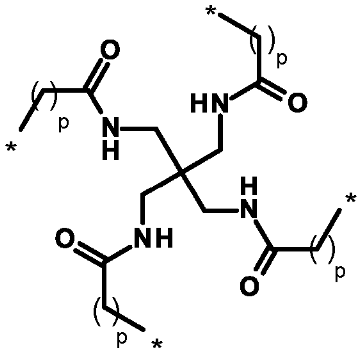

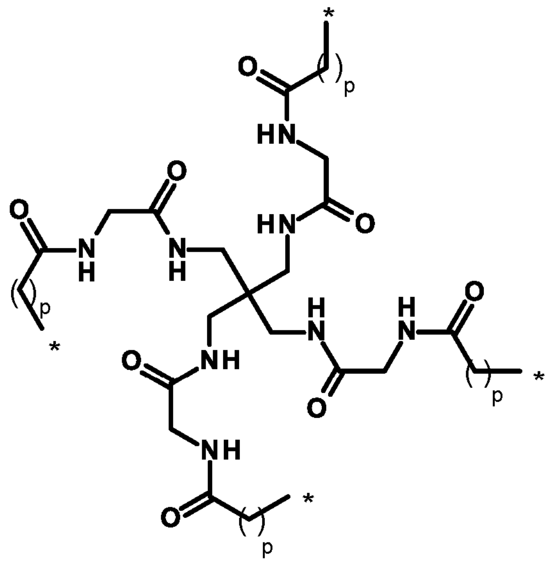

and

in which * represents the point of attachment to $R^1$ and p is an integer selected from 1 and 2,

$R^1$ represents a group selected from

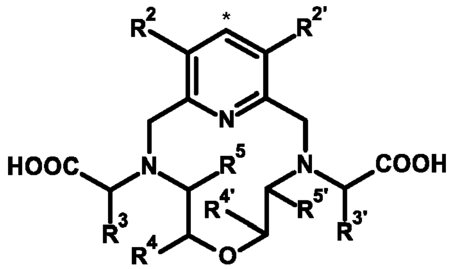

and

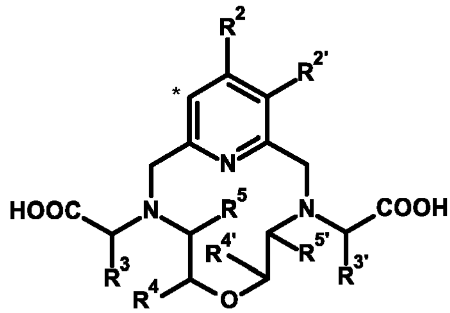

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

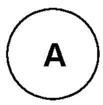

represents a group selected from

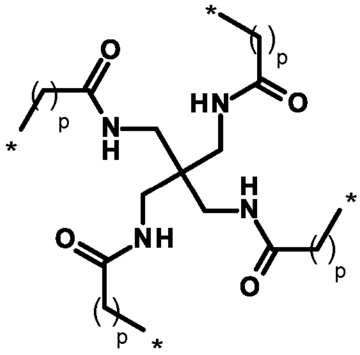

and

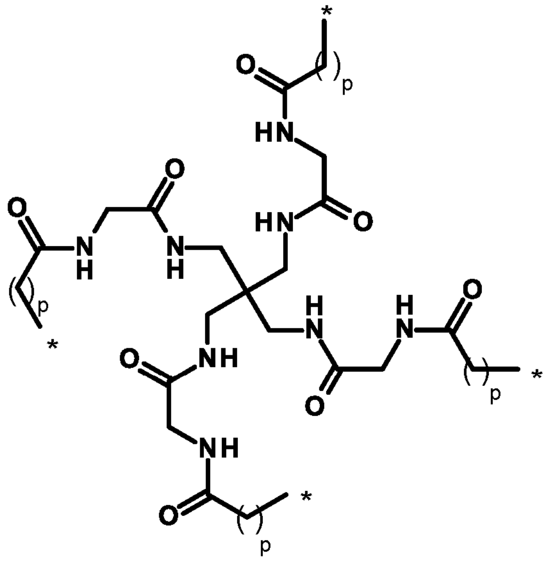

in which * represents the point of attachment to $R^1$ and p is an integer selected from 1 and 2,

$R^1$ represents a group selected from

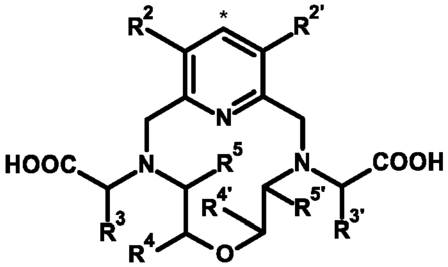

and

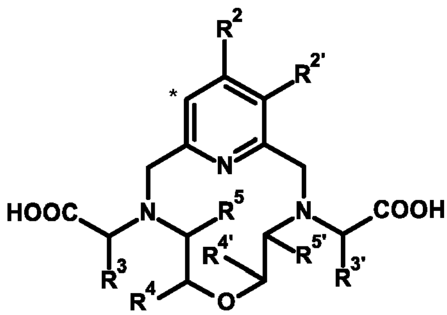

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the second aspect, the present disclosure covers compounds of general formula (I), supra, in the form of a complex with $Mn^{2+}$, wherein

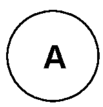

represents a group selected from

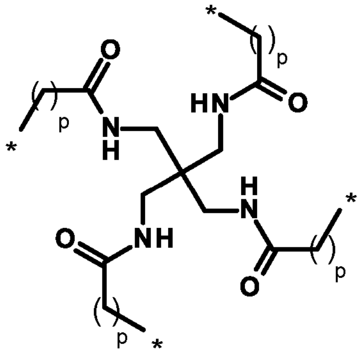

and

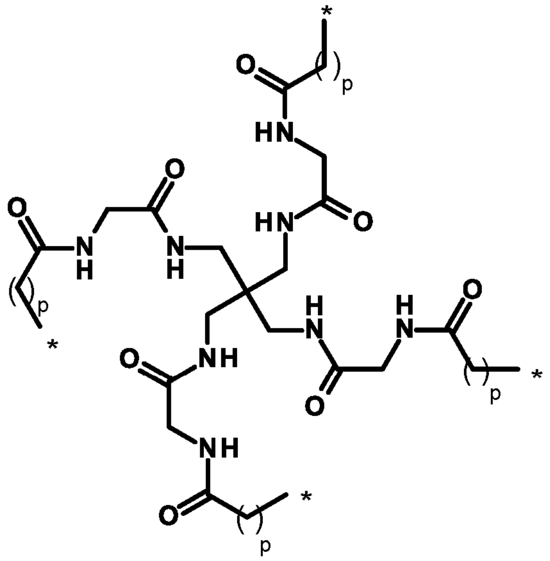

in which * represents the point of attachment to $R^1$ and p is an integer selected from 1 and 2,

$R^1$ represents a group selected from

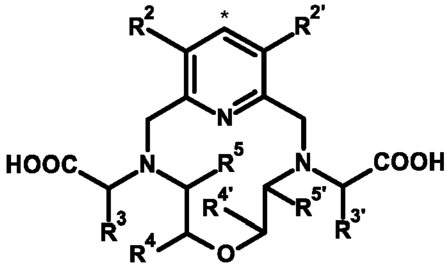

and

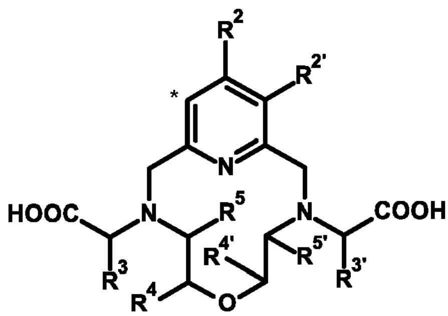

,

in which * represents the point of attachment to A,

$R^2$ and $R^{2'}$ are each independently selected from a hydrogen atom and a methyl group;

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

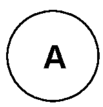

represents a group selected from

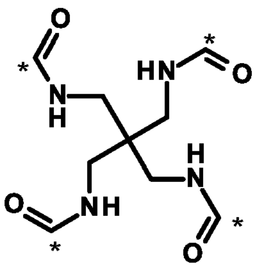
,
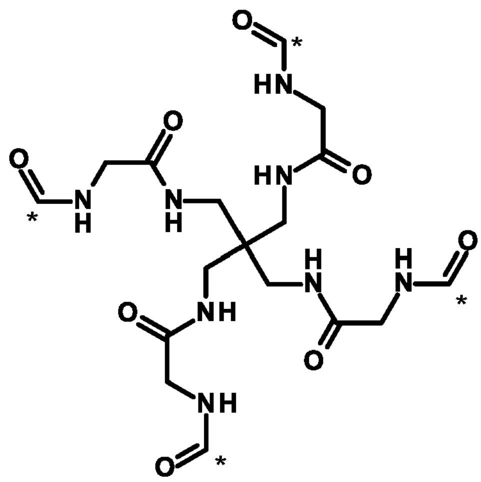
,

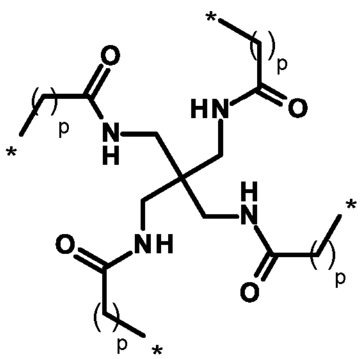
and
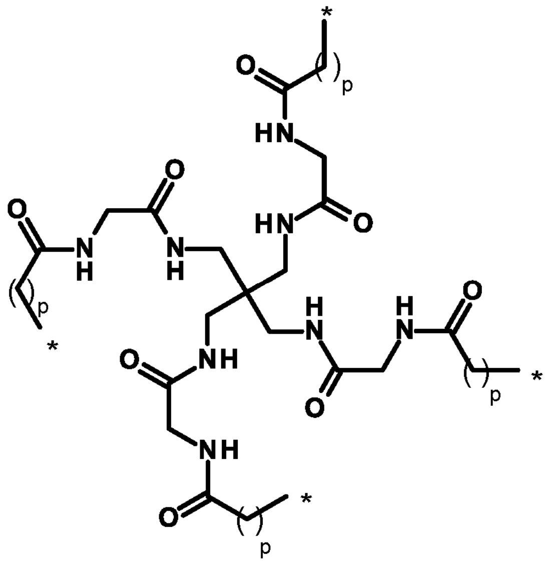

in which * represents the point of attachment to $R^1$ and p is an integer selected from 1 and 2,
and stereoisomers, tautomers, N-oxides, hydrates, solvates, or salts thereof, or mixtures of same.

In accordance with a further embodiment of the first aspect, the the present disclosure covers compounds of general formula (I), supra, wherein

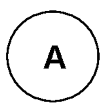

represents a group selected from

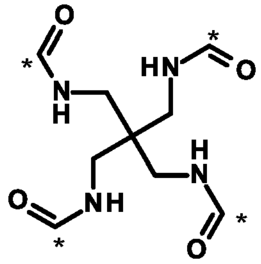

and

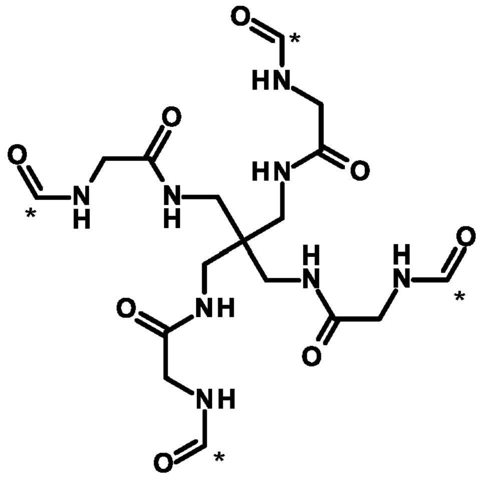

,

in which * represents the point of attachment to $R^1$,
and stereoisomers, tautomers, N-oxides, hydrates, solvates, or salts thereof, or mixtures of same.

In accordance with a further embodiment of the first aspect, the the present disclosure covers compounds of general formula (I), supra, wherein

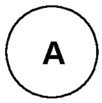

represents a group selected from

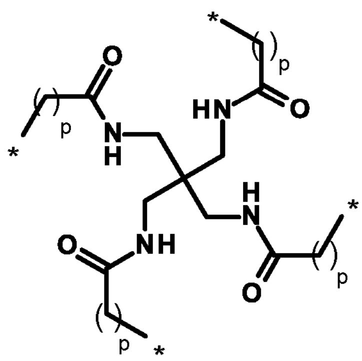

and

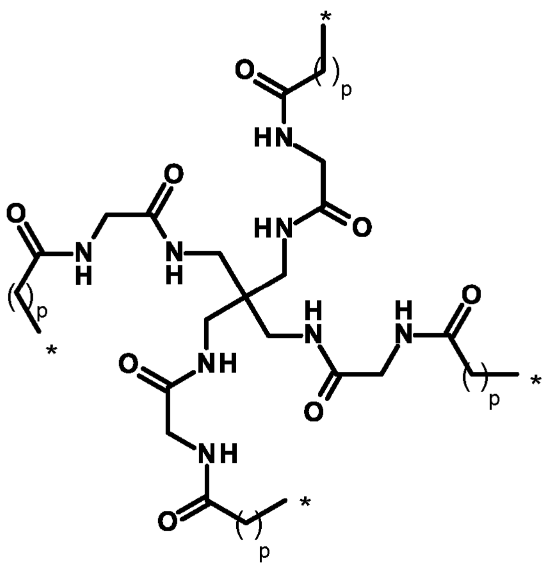

in which * represents the point of attachment to $R^1$ and p is an integer selected from 1 and 2, and stereoisomers, tautomers, N-oxides, hydrates, solvates, or salts thereof, or mixtures of same.

In accordance with a further embodiment of the first aspect, the the present disclosure covers compounds of general formula (I), supra, wherein

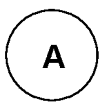

represents a group which is

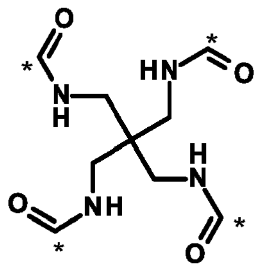

,

in which * represents the point of attachment to $R^1$,
and stereoisomers, tautomers, N-oxides, hydrates, solvates, or salts thereof, or mixtures of same.

In accordance with a further embodiment of the first aspect, the the present disclosure covers compounds of general formula (I), supra, wherein

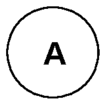

represents a group which is

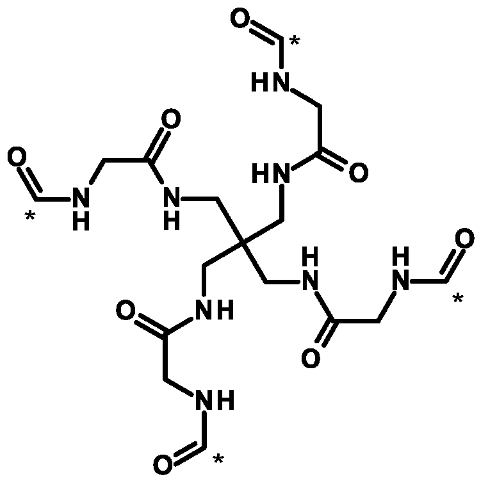

,

in which * represents the point of attachment to $R^1$,

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^1$ represents a group selected from

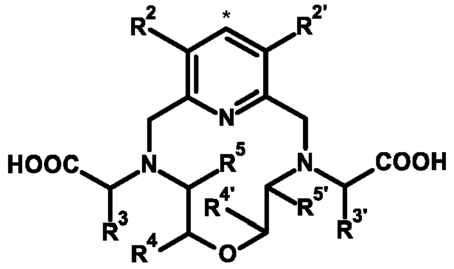

and

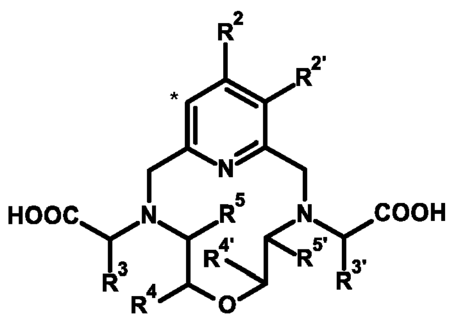

,

in which * represents the point of attachment to A,

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^1$ represents a group which is

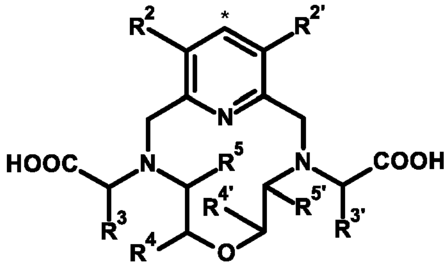

,

in which * represents the point of attachment to A,

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^1$ represents a group which is

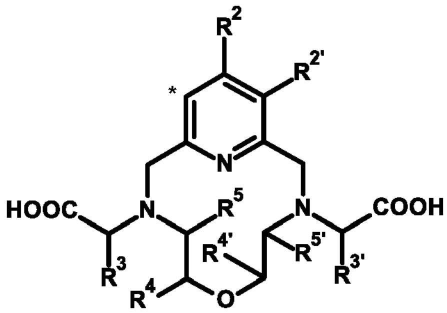

,

in which * represents the point of attachment to A,

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^2$ and $R^{2'}$ are each independently selected from a hydrogen atom and a methyl group;
and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^2$ and $R^{2'}$ are each a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^2$ and $R^{2'}$ are each a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each a hydrogen atom;
and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^4$, $R^{4'}$, $R^5$ and $R^{5'}$ are each independently selected from a hydrogen atom and a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^4$, $R^{4'}$, $R^5$ and $R^{5'}$ are each independently selected from a hydrogen atom and a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^4$, $R^{4'}$, $R^5$ and $R^{5'}$ are each a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^4$, $R^{4'}$, $R^5$ and $R^{5'}$ are each a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are not a hydrogen atom;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a $C_1$-$C_3$ alkyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the first aspect, the the present disclosure covers compounds of general formula (I), supra, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

It is to be understood that the present disclosure relates also to any combination of the embodiments described above.

In accordance with a further embodiment of the first aspect, the the present disclosure covers compounds of general formula (I) selected from the group consisting of

2,2',2",2'''-{[2,2-bis({[3,9-bis(carboxymethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1]pentadeca-1(15),11,13-triene-13,3,9-triyl]}tetraacetic acid,

2,2',2",2'''-({2,2-bis[(2-{[3,9-bis(carboxymethyl)-6-oxa-3,9,15-triazabicyclo[9.3.1]pentadeca-1(15),11,13-triene-13-carbonyl]amino}acetamido)methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-13,3,9-triyl])tetraacetic acid,

(2*R*,2'*S*,2"*R*,2'''*S*)-2,2',2",2'''-{(2,2-bis[({3-[(1*R*)-1-carboxyethyl]-9-[(1*S*)1-carboxyethyl]-6-oxa-3,9,15-triazabicyclo [9.3.1]pentadeca-1(15),11,13-triene-13-carbonyl}amino)methyl]propane-1,3-diyl)bis[carbamoyl-6-oxa-3,9,15-triazabicyclo [9.3.1]pentadeca-1(15),11,13-triene-13,3,9-triyl]}tetrapropanoic acid,

2,2',2",2'''-{[2,2-bis({[3,9-bis(1-carboxyethyl)-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo[9.3.1]pentadeca-1 (15), 11, 13-triene-13,3,9-triyl]}tetrapropanoic acid,

(2*R*,2'*S*,2"*R*,2'''*S*)-2,2',2",2'''-{(2,2-bis{[2-({3-[(1*R*)-1-carboxyethyl]-9-[(1*S*)-1-carboxyethyl]-6-oxa-3,9,15-triazabicyclo[9.3.1]pentadeca-1(15),11,13-triene-13-carbonyl}amino)acetamido] methyl}propane-1,3-diyl)bis[azanediyl(2-oxoethane-2,1-diyl)carbamoyl{6-oxa-3,9,15-triazabicyclo[9.3.1]pentadeca-1(15),11,13-triene-13,3,9-triyl}]}tetrapropanoic acid,

2,2',2",2'''-{[2,2-bis({[3,9-bis(carboxymethyl)-6-oxa-3,9,15-triazabicyclo[9.3.1]pentadeca-1(15),11,13-triene-12-carbonyl]amino}methyl)propane-1,3-diyl]bis[carbamoyl-6-oxa-3,9,15-triazabicyclo[9.3.1]pentadeca-1(15),11,13-triene-12,3,9-triyl]}tetraacetic acid,

2,2',2",2'''-({2,2-bis[(2-{[3,9-bis(carboxymethyl)-6-oxa-3,9,15-triazabicyclo[9.3.1]pentadeca-1(15),11,13-triene-12-carbonyl]amino}acetamido) methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-12,3,9-triyl])tetraacetic acid,

2,2',2",2'''-{[2,2-bis({2-[3,9-bis(carboxymethyl)-6-oxa-3,9,15-triazabicyclo[9.3.1] pentadeca-1 (15),11,13-trien-13-yl] acetamido}methyl)propane-1,3-diyl]bis[azanediyl(2-oxoethane-2,1-diyl)-6-oxa-3,9,15-triazabicyclo[9.3.1]pentadeca-1 (15),11,13-triene-13,3,9-triyl]}tetraacetic acid,

(2*R*,2'*S*,2"*R*,2'''*S*)-2,2',2",2'''-({2,2-bis[(2-{3-[(1*R*)-1-carboxyethyl]-9-[(1*S*)-1-carboxyethyl]-6-oxa-3,9,15-triazabicyclo[9.3.1]pentadeca-1(15),11,13-trien-13-yl}acetamido)methyl]propane-1,3-diyl}bis[azanediyl(2-oxoethane-2,1-diyl){6-oxa-3,9,15-triazabicyclo[9.3.1]pentadeca-1(15),11,13-triene-13,3,9-triyl}])tetrapropanoic acid,

(2*R**,2'*S**,2"*RS*,2'''*SR*)-2,2',2",2'''-{(2,2-bis{[({3-[(1*RS*)-1-carboxyethyl]-9-[(1*SR*)-1-carboxy ethyl]-6-oxa-3,9,15-triazabicyclo[9.3.1] pentadeca-1(15),11,13-trien-12-yl} carbonyl)amino] methyl}propane-1,3-diyl)bis[carbamoyl-6-oxa-3,9,15-triazabicyclo[9.3.1] pentadeca-1(15),11, 13-triene-12,3,9-triyl]}tetrapropanoic acid,

(2*R**,2'*R**,2"*RS*,2'''*RS*)-2,2',2",2'''-({2,2-bis[({3,9-bis[(1*RS*)-1-carboxyethyl]-6-oxa-3,9,15-triazabicyclo[9.3.1]pentadeca-1(15),11,13-triene-12-carbonyl}amino)methyl]propane-1,3-diyl}bis[carbamoyl-6-oxa-3,9,15-triazabicyclo [9.3.1]pentadeca-1(15),11,13-triene-12,3,9-triyl]) tetrapropanoic acid,

2,2’,2",2‴-{[2,2-bis({3-[3,9-bis(1-carboxyethyl)-6-oxa-3,9,15-triazabicyclo[9.3.1]pentadeca-1(15),11,13-trien-13-yl] propanamido}methyl)propane-1,3-diyl]bis[azanediyl(3-oxopropane-3,1-diyl)-6-oxa-3,9,15-triazabicyclo[9.3.1]pentadeca-1(15),11,13-triene-13,3,9-triyl]}tetra propanoic acid,

2,2’,2",2‴-([2,2-bis({[3,9-bis(carboxymethyl)-4-methyl-6-oxa-3,9,15-triazabicyclo[9.3.1]penta deca-1(15), 11,13-triene-13-carbonyl]amino}methyl)propane-1,3-diyl]bis{carbamoyl[4-methyl-6-oxa-3,9,15-triazabicyclo[9.3.1]pentadeca-1(15),11,13-triene-13,3,9-triyl]})tetraacetic acid,

2,2’,2",2‴-([2,2-bis({[3,9-bis(carboxymethyl)-4-ethyl-6-oxa-3,9,15-triazabicyclo[9.3.1]penta deca-1(15),11,13-triene-13-carbonyl]amino}methyl) propane-1,3-diyl] bis{carbamoyl[4-ethyl-6-oxa-3,9,15-triazabicyclo[9.3.1]pentadeca-1(15),11,13-triene-13,3,9-triyl]})tetraacetic acid,

2,2’,2",2‴-{[2,2-bis({[3,9-bis(carboxymethyl)-5-methyl-6-oxa-3,9,15-triazabicyclo[9.3.1]penta deca-1(15), 11,13-triene-13-carbonyl]amino}methyl)propane-1,3-diyl]bis[carbamoyl(5-methyl-6-oxa-3,9,15-triazabicyclo[9.3.1]pentadeca-1(15),11,13-triene-13,3, 9-triyl)]}tetraacetic acid,

(2*R*,2’*S*,2"*R*,2‴*S*)-2,2’,2",2‴-{(2,2-bis[({3-[(1*R*)1-carboxypropyl]-9-[(1*S*)-1-carboxypropyl]-6-oxa-3,9,15-triazabicyclo[9.3.1]pentadeca-1(15),11,13-triene-13-carbonyl}amino)methyl] propane-1,3-diyl)bis[carbamoyl-6-oxa-3,9,15-triazabicyclo[9.3.1]pentadeca-1(15),11,13-triene-13,3,9-triyl]}tetrabutanoic acid,

(2*R**,2’*S**,2"*RS*,2‴*SR*)-2,2’,2",2‴-{(2,2-bis{[({3-[(1*RS*)-1-carboxypropyl]-9-[(1*SR*)-1-carboxy propyl]-6-oxa-3,9,15-triazabicyclo[9.3.1] pentadeca-1(15),11, 13-trien-13-yl}carbonyl)amino] methyl}propane-1,3-diyl)bis [carbamoyl-6-oxa-3,9,15-triazabicyclo[9.3.1]pentadeca-1(15),11, 13-triene-13,3,9-triyl]}tetrabutanoic acid,

2,2’-({2,2-bis({[3-(1-carboxyethyl)-9-(carboxymethyl)-6-oxa-3,9, 15-triazabicyclo [9.3.1] pentadeca-1(15),11,13-triene-13-carbonyl]amino}methyl)propane-1,3-diyl}bis {carbamoyl[9-(carboxymethyl)-6-oxa-3,9,15-triazabicyclo [9.3.1]pentadeca-1(15),11,13-triene-13,3-diyl]}) dipropanoic acid,

2,2’,2",2‴-{[2,2-bis({[3,9-bis(carboxymethyl)-8-methyl-6-oxa-3,9,15-triazabicyclo[9.3.1] pentadeca-1(15), 11,13-triene-12-carbonyl]amino}methyl)propane-1,3-diyl]bis[carbamoyl(8-methyl-6-oxa-3,9,15-triazabicyclo[9.3.1]pentadeca-1(15),11,13-triene-12,3,9-triyl)]} tetraacetic acid,

(2*S*,2’*S*,2"*S*,2‴*S*)-2,2’,2",2‴-{(2,2-bis[({3,9-bis[(1*S*)-1-carboxyethyl]-6-oxa-3,9,15-triazabicyclo [9.3.1]pentadeca-1(15),11,13-triene-13-carbonyl}amino)methyl]propane-1,3-diyl)bis [carbamoyl-6-oxa-3,9,15-triazabicyclo[9.3.1] pentadeca-1(15),11,13-triene-13,3,9-triyl]} tetrapropano and

(2*R*, 2’*R*, 2"*R*,2‴*S*)-2,2’,2",2‴-{(2,2-bis[({3,9-bis[(1*R*)-1-carboxyethyl]-6-oxa-3,9,15-triaza bicyclo[9.3.1]pentadeca-1(15), 11,13-triene-13-carbonyl}amino)methyl]propane-1,3-diyl)bis [carbamoyl-6-oxa-3,9,15-triazabicyclo [9.3.1] pentadeca-1(15),11,13-triene-13,3,9-triyl]} tetrapropanoic acid

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

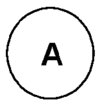

represents a group selected from

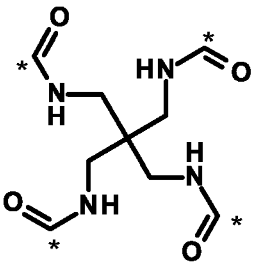

,

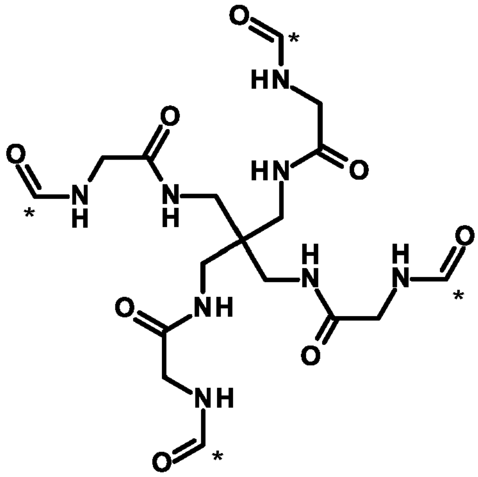

,

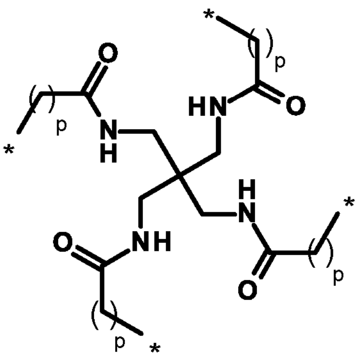

and

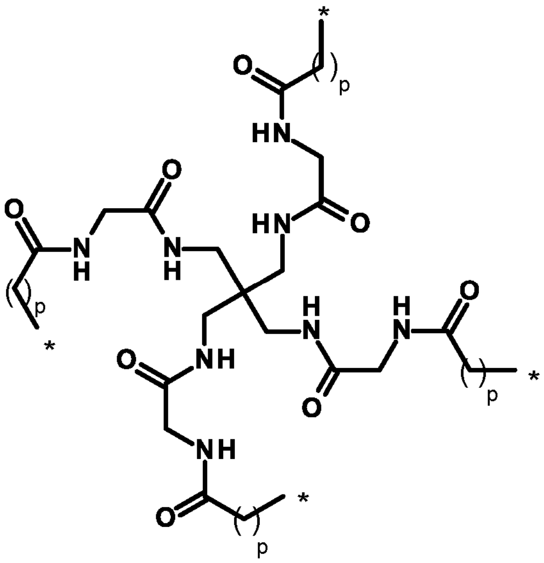

in which * represents the point of attachment to $R^1$ and p is an integer selected from 1 and 2, and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

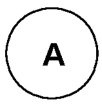

represents a group selected from

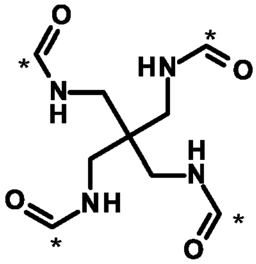 and 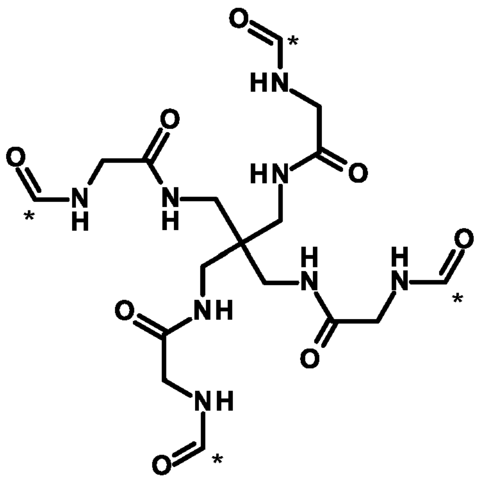 ,

in which * represents the point of attachment to $R^1$,
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

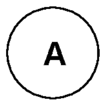

represents a group selected from

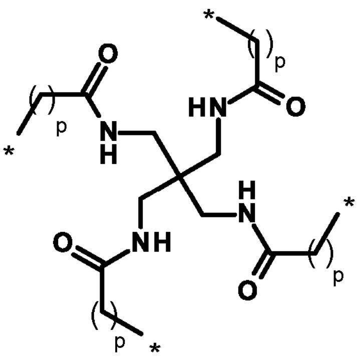 and 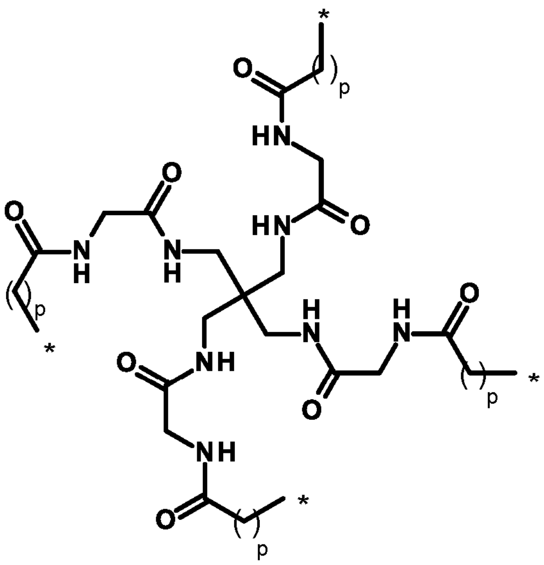

in which * represents the point of attachment to $R^1$ and p is an integer selected from 1 and 2, and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

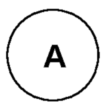

represents a group which is

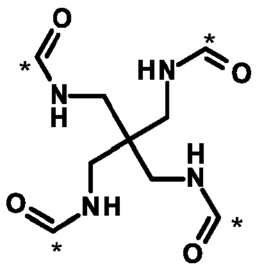

,

in which * represents the point of attachment to $R^1$,
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

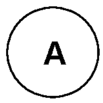

represents a group which is

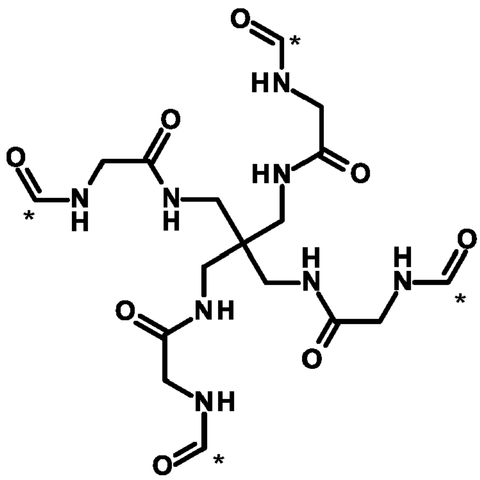

,

in which * represents the point of attachment to $R^1$,
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^1$ represents a group selected from

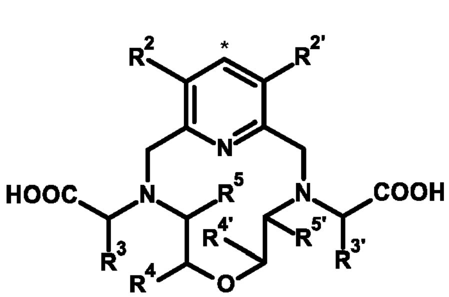

and

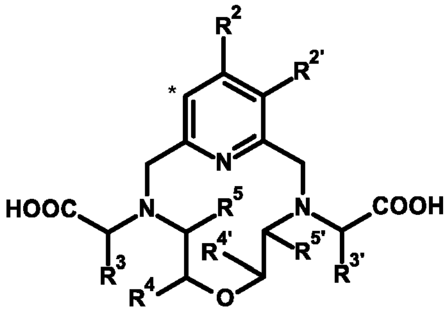

,

in which * represents the point of attachment to A,

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^1$ represents a group which is

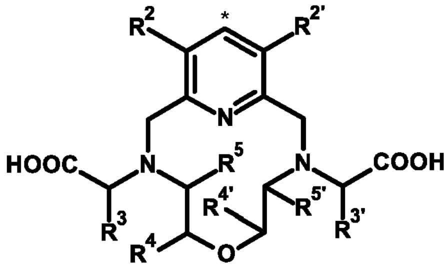

,

in which * represents the point of attachment to A,

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^1$ represents a group which is

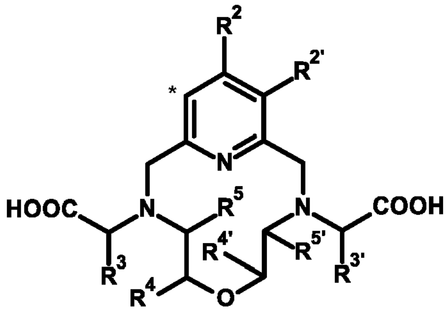

,

in which * represents the point of attachment to A,
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^2$ and $R^{2'}$ are each independently selected from a hydrogen atom and a methyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^2$ and $R^{2'}$ are each a hydrogen atom;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^2$ and $R^{2'}$ are each a methyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a methyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each a hydrogen atom;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each a $C_1$-$C_3$ alkyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^4$, $R^{4'}$, $R^5$ and $R^{5'}$ are each independently selected from a hydrogen atom and a $C_1$-$C_3$ alkyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^4$, $R^{4'}$, $R^5$ and $R^{5'}$ are each independently selected from a hydrogen atom and a methyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^4$, $R^{4'}$, $R^5$ and $R^{5'}$ are each a hydrogen atom;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^4$, $R^{4'}$, $R^5$ and $R^{5'}$ are each a $C_1$-$C_3$ alkyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least one of $R^3$, $R^{3'}$, $R^4$, $R^{4'}$, $R^5$ and $R^{5'}$ is not a hydrogen atom;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least two of $R^3$, $R^{3'}$, $R^4$, $R^{4'}$, $R^5$ and $R^{5'}$ are not a hydrogen atom;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least one of $R^3$, $R^{3'}$, $R^4$, $R^{4'}$, $R^5$ and $R^{5'}$ is a $C_1$-$C_3$ alkyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;
$R^4$, $R^{4'}$, $R^5$ and $R^{5'}$ are each independently selected from a hydrogen atom and a $C_1$-$C_3$ alkyl group;
wherein at least two of $R^3$, $R^{3'}$, $R^4$, $R^{4'}$, $R^5$ and $R^{5'}$ are a $C_1$-$C_3$ alkyl group;
and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least one of $R^3$, $R^{3'}$, $R^4$, $R^{4'}$, $R^5$ and $R^{5'}$ is a methyl group;

and stereoisomers, tautomers, N-oxides, hydrates, solvates, or salts thereof, or mixtures of same.

In accordance with a further embodiment of the second aspect, the present disclosure covers compounds of general formula (I), *supra,* in the form of a complex with $Mn^{2+}$, wherein

$R^3$ and $R^{3'}$ are each independently selected from a hydrogen atom and a $C_1$-$C_3$ alkyl group;

$R^4$, $R^{4'}$, $R^5$ and $R^{5'}$ are each independently selected from a hydrogen atom and a $C_1$-$C_3$ alkyl group;

wherein at least two of $R^3$, $R^{3'}$, $R^4$, $R^{4'}$, $R^5$ and $R^{5'}$ are a methyl group;

and stereoisomers, tautomers, N-oxides, hydrates, solvates, or salts thereof, or mixtures of same.

It is to be understood that the present disclosure relates also to any combination of the embodiments described above.
In accordance with a further embodiment of the second aspect, the present disclosure covers compounds of general formula (I), *supra,* in the form of a complex with $Mn^{2+}$, selected from the group consisting of:

tetramanganese(2+) 2,2',2'',2'''-{[2,2-bis({[3,9-bis(carboxylatomethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1]pentadeca-1(15),11,13-triene-13,3,9-triyl]}tetraacetate,

tetramanganese(2+) 2,2',2'',2'''-({2,2-bis[(2-{[3,9-bis(carboxylatomethyl)-6-oxa-3,9,15-triazabicyclo[9.3.1]pentadeca-1(15),11,13-triene-13-carbonyl]amino}acetamido)methyl] propane-1,3-diyl}bis[azanediyl(2-oxoethane-2,1-diyl) carbamoyl-6-oxa-3,9,15-triazabicyclo[9.3.1]pentadeca-1(15),11,13-triene-13,3,9-triyl])tetraacetate,

tetramanganese(2+) (2*R*,2'*S*,2"*R*,2'''*S*)-2,2',2",2'''-{(2,2-bis[({3-[(1*R*)-1-carboxylatoethyl]-9-[(1*S*)1-carboxylatoethyl]-6-oxa-3,9,15-triazabicyclo[9.3.1]pentadeca-1(15),11,13-triene-13-carbonyl}amino)methyl] propane-1,3-diyl)bis[carbamoyl-6-oxa-3,9,15-triazabicyclo [9.3.1]pentadeca-1(15),11,13-triene-13,3,9-triyl]}tetrapropanoate,

tetramanganese(2+) 2,2',2",2'''-{[2,2-bis({[3,9-bis(1-carboxylatoethyl)-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo[9.3.1] pentadeca-1(15),11,13-triene-13,3,9-triyl]}tetrapropanoate,

tetramanganese(2+) (2*R*,2'*S*,2"*R*,2'''*S*)-2,2',2",2'''-{(2,2-bis{[2-({3-[(1*R*)-1-carboxylato ethyl]-9-[(1*S*)-1-carboxylatoethyl]-6-oxa-3,9,15-triazabicyclo[9.3.1]pentadeca-1(15), 11,13-triene-13-carbonyl}amino)acetamido]methyl}propane-1,3-diyl)bis[azanediyl(2-oxoethane-2,1-diyl)carbamoyl{6-oxa-3,9,15-triazabicyclo[9.3.1] pentadeca-1(15),11,13-triene-13,3,9-triyl}]}tetrapropanoate,

tetramanganese(2+) 2,2',2",2'''-{[2,2-bis({[3,9-bis(carboxylatomethyl)-6-oxa-3,9,15-triazabicyclo[9.3.1]pentadeca-1(15),11,13-triene-12-carbonyl]amino}methyl)propane-1,3-diyl]bis[carbamoyl-6-oxa-3,9,15-triazabicyclo[9.3.1] pentadeca-1(15),11,13-triene-12,3,9-triyl]}tetraacetate,

tetramanganese(2+) 2,2',2",2'''-({2,2-bis[(2-{[3,9-bis(carboxylatomethyl)-6-oxa-3,9,15-triazabicyclo[9.3.1]pentadeca-1(15),11,13-triene-12-carbonyl]amino}acetamido) methyl]propane-1,3-diyl}bis[azanediyl(2-oxoethane-2,1-diyl) carbamoyl-6-oxa-3,9,15-triazabicyclo[9.3.1]pentadeca-1(15),11,13-triene-12,3,9-triyl])tetraacetate,

tetramanganese(2+) 2,2',2",2'''-{[2,2-bis({2-[3,9-bis(carboxylatomethyl)-6-oxa-3,9,15-triazabicyclo[9.3.1]pentadeca-1(15),11,13-trien-13-yl]acetamido}methyl)propane-1,3-diyl]bis[azanediyl(2-oxoethane-2,1-diyl)-6-oxa-3,9,15-triazabicyclo[9.3.1]pentadeca-1(15),11,13-triene-13,3,9-triyl]}tetraacetate,

tetramanganese(2+) (2*R*,2'*S*,2"*R*,2'''*S*)-2,2',2",2'''-({2,2-bis[(2-{3-[(1*R*)-1-carboxylatoethyl]-9-[(1*S*)-1-carboxylatoethyl]-6-oxa-3,9,15-triazabicyclo[9.3.1]pentadeca-1(15),11,13-trien-13-yl}acetamido)methyl]propane-1,3-diyl}bis [azanediyl(2-oxoethane-2,1-diyl){6-oxa-3,9,15-triazabicyclo[9.3.1]pentadeca-1(15),11,13-triene-13,3,9-triyl}])tetrapropanoate,

tetramanganese(2+) (2*R**,2'*S**,2"*RS*,2'''*SR*)-2,2',2",2'''-{(2,2-bis{[({3-[(1*RS*)-1-carboxylato ethyl]-9-[(1SR)-1-carboxylatoethyl]-6-oxa-3,9,15-triazabicyclo[9.3.1] pentadeca-1(15),11,13-trien-12-yl}carbonyl)amino]methyl}propane-1,3-diyl)bis[carbamoyl-6-oxa-3,9,15-triazabicyclo [9.3.1]pentadeca-1(15),11,13-triene-12,3,9-triyl]}tetrapropanoate,

tetramanganese(2+) (2*R**,2'*R**,2"*RS*,2'''*RS*)-2,2',2",2'''-({2,2-bis[({3,9-bis[(1*RS*)-1-carboxylato ethyl]-6-oxa-3,9,15-triazabicyclo[9.3.1]pentadeca-1(15),11,13-triene-12-carbonyl}amino) methyl]propane-1,3-diyl}bis[carbamoyl-6-oxa-3,9,15-triazabicyclo[9.3.1]pentadeca-1(15),11, 13-triene-12,3,9-triyl])tetrapropanoate,

tetramanganese(2+) 2,2',2",2'''-{[2,2-bis({3-[3,9-bis(1-carboxylatoethyl)-6-oxa-3,9,15-triazabicyclo[9.3.1]pentadeca-1(15),11,13-trien-13-yl]propanamido}methyl)propane-1,3-diyl] bis[azanediyl(3-oxopropane-3,1-diyl)-6-oxa-3,9,15-triazabicyclo[9.3.1] pentadeca-1(15),11, 13-triene-13,3,9-triyl]}tetrapropanoate,

tetramanganese(2+) 2,2',2",2'''-([2,2-bis({[3,9-bis(carboxylatomethyl)-4-methyl-6-oxa-3,9,15-triazabicyclo[9.3.1] pentadeca-1(15),11,13-triene-13-carbonyl]amino}methyl)propane-1,3-diyl] bis{carbamoyl[4-methyl-6-oxa-3,9,15-triazabicyclo[9.3.1]pentadeca-1(15),11,13-triene-13,3,9-triyl]})tetraacetate,

tetramanganese(2+) 2,2',2",2'''-([2,2-bis({[3,9-bis(carboxylatomethyl)-4-ethyl-6-oxa-3,9,15-triazabicyclo[9.3.1]pentadeca-1(15),11,13-triene-13-carbonyl]amino}methyl)propane-1,3-diyl] bis{carbamoyl[4-ethyl-6-oxa-3,9,15-triazabicyclo[9.3.1]pentadeca-1(15),11,13-triene-13,3,9-triyl]})tetraacetate,

tetramanganese(2+) 2,2',2",2'''-{[2,2-bis({[3,9-bis(carboxylatomethyl)-5-methyl-6-oxa-3,9,15-triazabicyclo[9.3.1] pentadeca-1(15),11,13-triene-13-carbonyl]amino}methyl)propane-1,3-diyl] bis[carbamoyl(5-methyl-6-oxa-3,9,15-triazabicyclo[9.3.1]pentadeca-1(15),11,13-triene-13,3, 9-triyl)]}tetraacetate,

tetramanganese(2+) (2*R*,2'*S*,2"*R*,2'''*S*)-2,2',2",2'''-{(2,2-bis[({3-[(1*R*)1-carboxylatopropyl]-9-[(1*S*)-1-carboxylatopropyl]-6-oxa-3,9,15-triazabicyclo[9.3.1] pentadeca-1(15),11,13-triene-13-carbonyl}amino)methyl] propane-1,3-diyl) bis [carbamoyl-6-oxa-3,9,15-triazabicyclo[9.3.1] pentadeca-1(15),11,13-triene-13,3,9-triyl]}tetrabutanoate,

tetramanganese(2+) (2*R**,2'*S**,2"*RS*,2'''*SR*)-2,2',2",2'''-{(2,2-bis{[({3-[(1*RS*)-1-carboxylato propyl]-9-[(1*SR*)-1-carboxylatopropyl]-6-oxa-3,9,15-triazabicyclo[9.3.1] pentadeca-1(15),11, 13-trien-13-yl}carbonyl)amino]methyl}propane-1,3-diyl)bis [carbamoyl-6-oxa-3,9,15-triazabicyclo[9.3.1]pentadeca-1(15),11,13-triene-13,3,9-triyl]}tetrabutanoate,

tetramanganese(2+) 2,2'-({2,2-bis({[3-(1-carboxylatoethyl)-9-(carboxylatomethyl)-6-oxa-3,9, 15-triazabicyclo[9.3.1] pentadeca-1(15),11,13-triene-13-carbonyl]amino}methyl)propane-1,3-diyl}bis{carbamoyl[9-(carboxylatomethyl)-6-oxa-3,9,15-triazabicyclo[9.3.1]pentadeca-1(15), 11,13-triene-13,3-diyl]})dipropanoate,

tetramanganese(2+) 2,2',2",2'''-{[2,2-bis({[3,9-bis(carboxylatomethyl)-8-methyl-6-oxa-3,9,15-triazabicyclo[9.3.1] pentadeca-1(15),11,13-triene-12-carbonyl]amino}methyl)propane-1,3-diyl]bis[carbamoyl(8-methyl-6-oxa-3,9,15-triazabicyclo[9.3.1] pentadeca-1(15),11,13-triene-12,3,9-triyl)]}tetraacetate,

tetramanganese(2+) (2S,2'*S*,2"*S*,2'''*S*)-2,2',2",2'''-{(2,2-bis[({3,9-bis[(1*S*)-1-carboxylatoethyl]-6-oxa-3,9,15-triazabicyclo[9.3.1]pentadeca-1(15),11,13-triene-13-carbonyl}amino)methyl] propane-1,3-diyl)bis[carbamoyl-6-oxa-3,9,15-triazabicyclo[9.3.1]pentadeca-1(15),11,13-triene-13,3,9-triyl]}tetrapropanoate and

tetramanganese(2+) (2*R*,2'*R*,2"*R*,2'''*S*)-2,2',2",2'''-{(2,2-bis[({3,9-bis[(1*R*)-1-carboxylatoethyl]-6-oxa-3,9,15-triazabicyclo[9.3.1]pentadeca-1(15),11,13-triene-13-carbonyl} amino)methyl] propane-1,3-diyl)bis[carbamoyl-6-oxa-3,9,15-triazabicyclo[9.3.1]pentadeca-1(15),11,13-triene-13,3,9-triyl]}tetrapropanoate.

and stereoisomers, tautomers, N-oxides, hydrates, solvates, or salts thereof, or mixtures of same.

In accordance with another aspect, the present disclosure covers methods of preparing compounds of the present disclosure, said methods comprising the steps as described in the Experimental Section herein.

In accordance with a further aspect, the present disclosure covers intermediate compounds which are useful for the preparation of the compounds of general formula (I), and particularly for the preparation of the compounds of general formula (I) in the form of a complex with $Mn^{2+}$, *supra.*

More particularly still, the present disclosure covers the intermediate compounds which are disclosed in the Experimental Section of this text, *infra.*

In particular, the compounds of formula (I) in the form of a complex with $Mn^{2+}$ of the present disclosure can be used as contrast agents in contrast-enhanced MRI (CE-MRI), preferably for multi-purpose MRI.

A further aspect of the disclosure is the use of a compound of general formula (I), *supra,* for diagnostic imaging.

A person skilled in the art would recognize that the compounds of general formula (I) of this present disclosure can be used in combination with other MR-active metal ions instead of $Mn^{2+}$, such compounds also being encompassed by the present disclosure.

A further aspect of the disclosure is the use of a compound of general formula (I), *supra,* in the form of a complex with $Mn^{2+}$ for diagnostic imaging.

A further aspect of the disclosure is the use of a $Mn^{2+}$ complex of a compound of general formula (I), *supra,* for diagnostic imaging.

Preferably, the use of a compound of general formula (I), *supra* and/or of a compound of general formula (I), *supra,* in the form of a complex with $Mn^{2+}$ and/or of a $Mn^{2+}$ complex of a compound of general formula (I), *supra,* in the diagnosis is performed using magnetic resonance imaging (MRI).

A further aspect of the disclosure are compounds of general formula (I) for use in diagnostic imaging.

A further aspect of the disclosure are compounds of general formula (I) in the form of a complex with $Mn^{2+}$ for use in diagnostic imaging.

A further aspect of the disclosure are $Mn^{2+}$ complexes of compounds of general formula (I), *supra,* for use in diagnostic imaging.

A person skilled in the art would recognize that the compounds of general formula (I) of this present disclosure can be used in combination with other MR-active metal ions instead of $Mn^{2+}$, such compounds also being encompassed by the present disclosure.

The present disclosure also comprises compounds of general formula (I) for the manufacture of diagnostic agents.

The present disclosure also comprises compounds of general formula (I) in the form of a complex with $Mn^{2+}$ for

the manufacture of diagnostic agents.

A further aspect of the disclosure is the use of the compounds of general formula (I) or mixtures thereof for the manufacture of diagnostic agents.

A further aspect of the disclosure is the use of the compounds of general formula (I) in the form of a complex with $Mn^{2+}$ or mixtures thereof for the manufacture of diagnostic agents.

A further aspect of the disclosure is the use of the compounds of general formula (I) or mixtures thereof for the manufacture of diagnostic agents for magnetic resonance imaging (MRI).

A further aspect of the disclosure is the use of the compounds of general formula (I) in the form of a complex with $Mn^{2+}$ or mixtures thereof for the manufacture of diagnostic agents for magnetic resonance imaging (MRI).

A further aspect of the disclosure is a method of imaging body tissue in a patient, comprising the steps of administering to the patient an effective amount of one or more compounds of general formula (I) in the form of a complex with $Mn^{2+}$ in a pharmaceutically acceptable carrier, and subjecting the patient to NMR tomography.

A further aspect of the disclosure is a method of imaging body tissue in a patient, comprising the steps of administering to the patient an effective amount of one or more $Mn^{2+}$ complexes of the compounds of general formula (I) in a pharmaceutically acceptable carrier, and subjecting the patient to NMR tomography.

For the manufacture of diagnostic agents, for example the administration to human or animal subjects, the compounds of general formula (I) in the form of complexes with $Mn^{2+}$, *i.e.* $Mn^{2+}$ complexes of the compounds of general formula (I) or mixtures will conveniently be formulated together with pharmaceutical carriers or excipients. The contrast media of the disclosure may conveniently contain pharmaceutical formulation aids, for example stabilizers, antioxidants, pH-adjusting agents, metal scavengers, electrolytes (e.g. sodium chloride), flavors and the like. The diagnostic agents of the disclosure may be formulated for parenteral or enteral administration or for direct administration into body cavities. For example, in the case of $Mn^{2+}$ complexes of the compounds of general formula (I), parenteral formulations contain a sterile solution or suspension in a dose of 0.0001-5 mmol manganese/kg body weight, preferably 0.001-0.5 mmol manganese/kg body weight, more preferably 0.005-0.1 mmol manganese/kg body weight of the compound of formula (I) according to this disclosure. Thus, the media of the disclosure may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

In a further aspect, the present disclosure is directed to a method of diagnosing and health monitoring of patients. This method comprises a) administering to a human in need of such diagnosis a compound of the present disclosure for detecting the compound in the human as described above and herein, and b) measuring the signal arising from the administration of the compound to the human, preferably by magnetic resonance imaging (MRI).

In a further aspect, the present disclosure is directed to a method of diagnosing and health monitoring of patients. This method comprises a) administering to a human in need of such diagnosis a compound of the present disclosure, *i.e.* a compound of general formula (I) in the form of a complex with $Mn^{2+}$ for detecting the compound in the human as described above and herein, and b) measuring the signal arising from the administration of the compound to the human, preferably by magnetic resonance imaging (MRI).

In a further aspect, the present disclosure is directed to a method of diagnosing and health monitoring of patients. This method comprises a) administering to a human in need of such diagnosis a compound of the present disclosure, *i.e.* a $Mn^{2+}$ complex of a compound of general formula (I) for detecting the compound in the human as described above and herein, and b) measuring the signal arising from the administration of the compound to the human, preferably by magnetic resonance imaging (MRI).

## GENERAL SYNTHESIS

Appropriate protecting groups and their introduction and cleavage are well-known to the person skilled in the art (see for example T.W. Greene and P.G.M. Wuts in Protective Groups in Organic Synthesis, 5th edition, Wiley 2014).

The compounds of general formula (I) described in the present disclosure, including the compounds of general formula (I) in the form of a complex with $Mn^{2+}$ and/or the $Mn^{2+}$ complexes of a compound of general formula (I) can be produced using the general procedures depicted in Schemes 1 to 3 below. Unless otherwise specified, the groups $R^1$, $R^2$, $R^{2'}$, $R^3$, $R^{3'}$, $R^4$, $R^{4'}$, $R^5$ and $R^{5'}$ displayed therein have the meaning given throughout the present disclosure. In case protective group chemistry is required for the introduction of $R^3CHCOOH$, a protecting group PG can be employed.

A route for the preparation of intermediates of general formula 7 is described in Scheme 1.

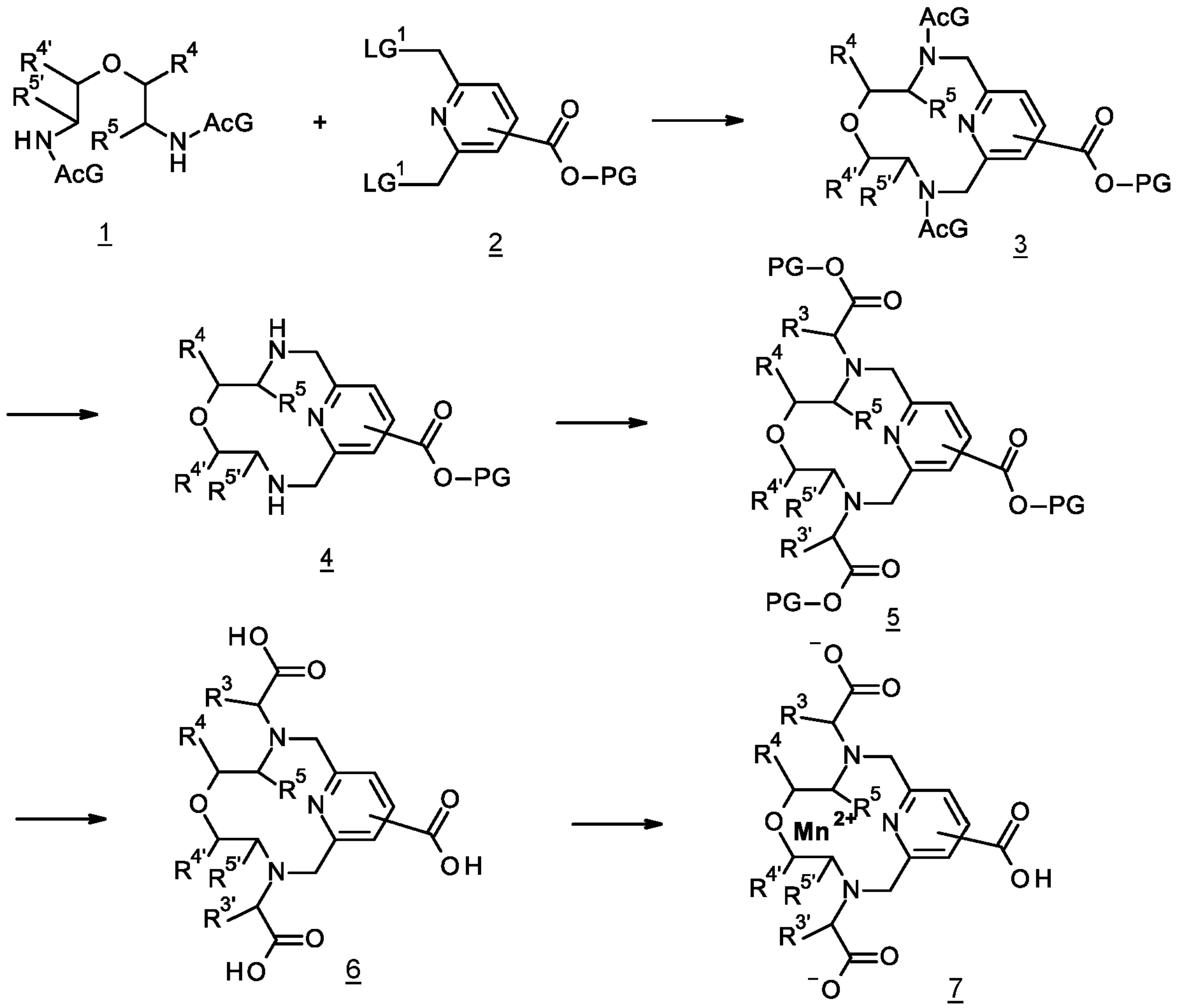

1

2

3

4

5

6

7

**Scheme 1**: Route for the preparation of intermediates of general formula 7, wherein $R^3$, $R^4$, $R^5$, $R^{3'}$, $R^{4'}$ and $R^{5'}$ have the meaning as given for the compounds of general formula (I) and/or the compounds of general formula (I) in the form of a complex with $Mn^{2+}$ and/or the $Mn^{2+}$ complexes of a compound of general formula (I), *supra*.

An activated diamino derivative 1 where AcG represents an activating group, such as for example a 2-nitrobenzenesulfonyl, 4-methylbenzenesulfonyl, or trifluoroacetyl group is cyclized with a pyridine derivative 2 containing two leaving groups (LG) in the benzylic positions where $LG^1$ represents an activated leaving group, such as for example a chloride or bromide, to deliver the 6-oxapyclen compound ((6-oxa-3,9,15-triazabicyclo[9.3.1]pentadeca-1(14),11-dien) 3 under the presence of bases like potassium carbonate or triethylamine. The reaction is carried out in a suitable solvent, such as for example acetonitrile, toluene or THF in a temperature range from room temperature to 110°C.

Cleavage of the activating amine groups of intermediates 3 to yield the intermediates of general formula 4 can be achieved as described in the textbook Greene and Wuts, Protecting groups in Organic Synthesis, fifth edition, page 1091 - 1115. Alkylation of the secondary amine positions at the 6-oxapyclene ring 4 can be achieved with α-bromo, α-methylsulfonyl or α-trifluoromethylsulfonyl esters under the presence of bases like potassium carbonate or triethylamine. Alkylation of 6-oxapyclene 4 with is carried out in a suitable solvent, such as for example acetonitrile, dichloromethane, toluene or tetrahydrofuran, or a mixture thereof, in a temperature range from -20°C up to 80°C, to furnish the intermediates 5. Cleavage of the carboxyl-protecting groups of intermediates 5 to yield the intermediates of general formula 6 can be achieved as described in the textbook Greene and Wuts, Protecting groups in Organic Synthesis, fifth edition, page 699-704. The deprotection is, for example, performed by dissolving and stirring of intermediates 5 in a mixture of water and tetrahydrofuran under the presence sodium hydroxide for 2 to 16 hours at room temperature. The complexation of intermediates of general formula 6 with suitable manganese (II) compounds or salts, such as for example manganese

dichloride tetrahydrate, manganese diacetate, manganese dibromide or manganese dichloride, is known to a person skilled in the art. The intermediates of general formula 6 are dissolved in water and after adding of suitable manganese (II) compound the resulting mixtures are stirred in a temperature range from room temperature up to 100°C at a pH range from 5 to 12 for several hours, to furnish the intermediates of general formula 7.

A route for the preparation of compounds of general formula (I-a) is described in Scheme 2.

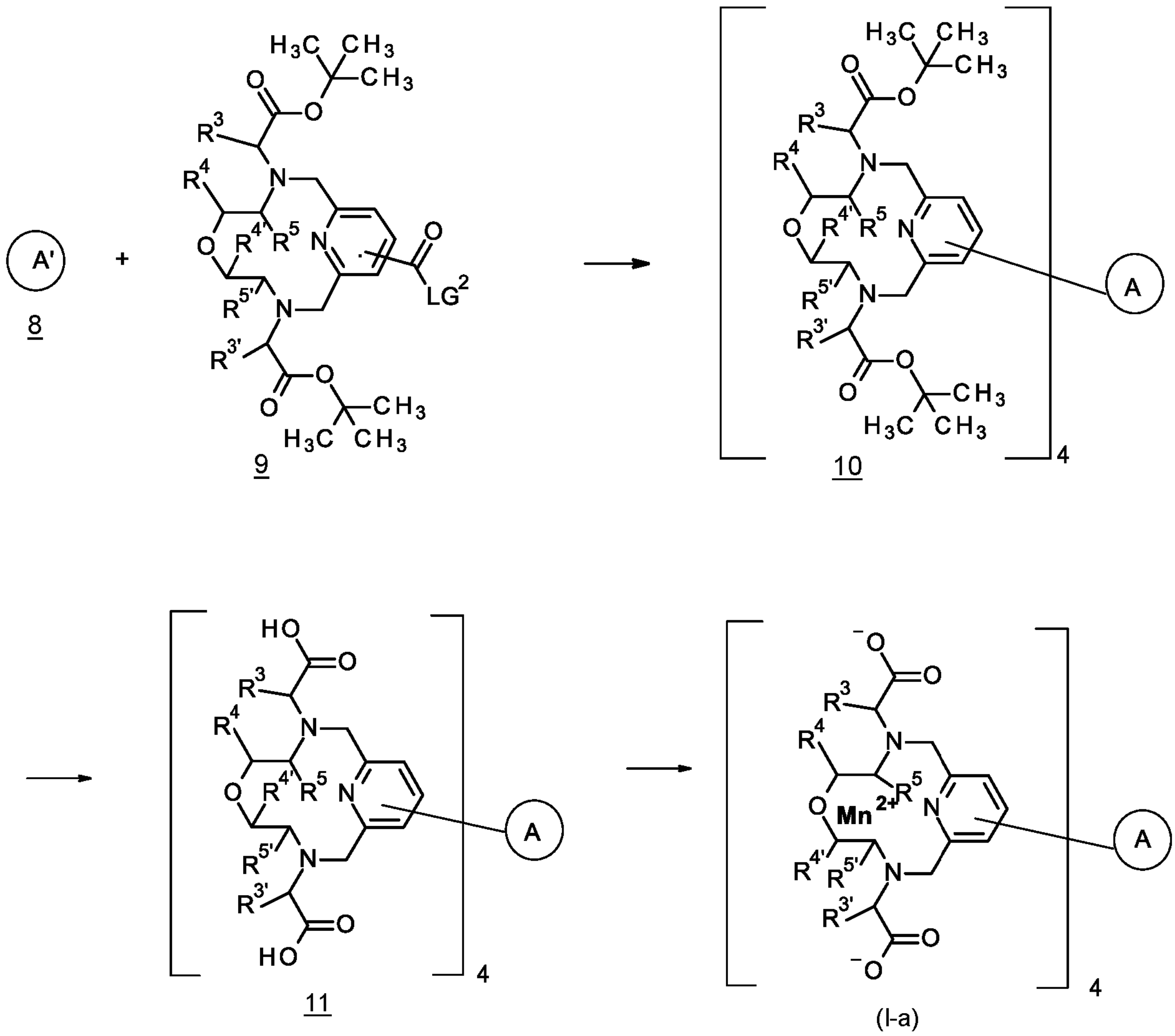

8 9 10 11 (I-a)

**Scheme 2**: Route for the preparation of compounds of general formula (I-a).

According to Scheme 2, A, $R^3$, $R^4$, $R^5$, $R^{3'}$, $R^{4'}$ and $R^{5'}$ have the meaning as given for the compounds of general formula (I) and/or the compounds of general formula (I) in the form of a complex with $Mn^{2+}$ and/or the $Mn^{2+}$ complexes of a compound of general formula (I), *supra,* **A' (8)** represents a tetraamine selected from

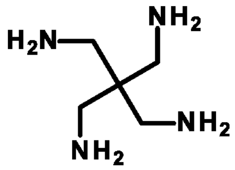

and

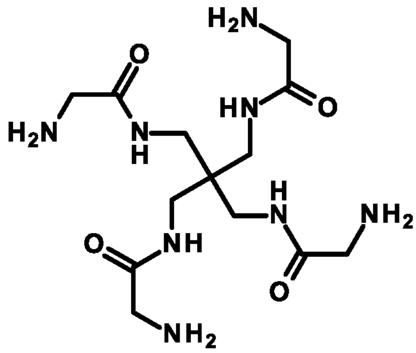

,

and described as Intermediate 4 and Intermediate 6, respectively, and $LG^2$ in compound **9** represents an activating leaving group, such as for example 4-nitrophenol, 2,3,5,6-tetrafluorophenol, pentafluorophenol, 1-hydroxypyrrolidine-2,5-dione, hydroxybenzotriazole or 3H-[1,2,3]triazolo[4,5-b]pyridin-3-ol.

Compound **9** can be generated form a selectively deprotected intermediate **5** (Scheme 1), which has a free carboxylate group at the pyridine ring, by ester forming reaction conditions known to the person skilled in the art. The coupling reaction of polyamines **8** with compound **9** is carried out in a suitable solvent, such as for example *N,N*-dimethylformamide, dichloromethane or dimethyl sulfoxide, or a mixture thereof, in a temperature range from room temperature up to 80°C, to furnish the intermediates **10**. Cleavage of the carboxyl-protecting groups of intermediates **10** to yield the intermediates of general formula **11** can be achieved as described in the textbook Greene and Wuts, Protecting groups in Organic Synthesis, fifth edition, page 699-704. The deprotection is, for example, performed by dissolving and stirring of intermediates **10** in trifluoroacetic acid or formic acid at room temperature up to 80°C for several hours. The complexation of intermediates of general formula **11** with suitable manganese (II) compounds or salts, such as for example manganese dichloride tetrahydrate, manganese diacetate, manganese dibromide or manganese dichloride, is well known to a person skilled in the art. The intermediates of general formula **11** are dissolved in water and after adding of suitable manganese (II) compound the resulting mixtures are stirred in a temperature range from room temperature up to 100°C at a pH range from 5 to 10 for several hours, to furnish the compounds of general formula (**I-a**).

A route for the preparation of compounds of general formula (I-b) is described in Scheme 3.

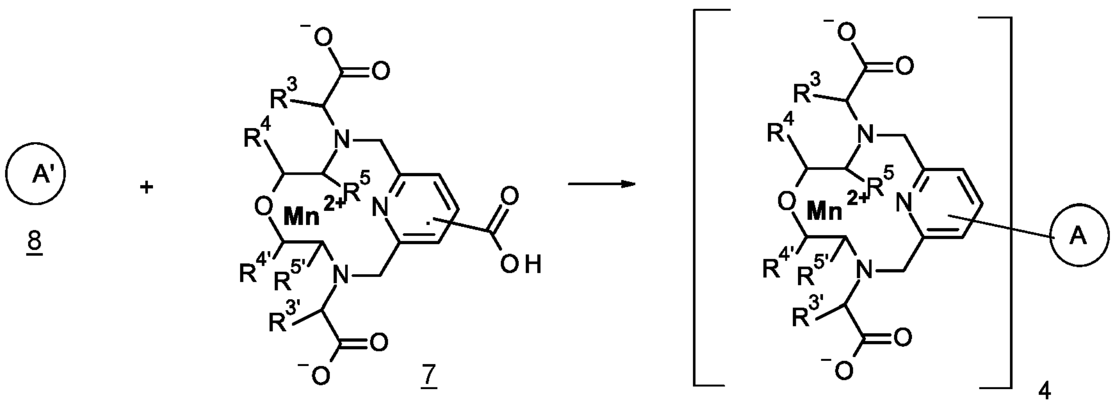

8

7

(I-b)

**Scheme 3**: Route for the preparation of compounds of general formula (I-b).

According to Scheme 3, A, $R^3$, $R^4$, $R^5$, $R^{3'}$, $R^{4'}$ and $R^{5'}$ have the meaning as given for the compounds of general formula (I) and/or the compounds of general formula (I) in the form of a complex with $Mn^{2+}$ and/or the $Mn^{2+}$ complexes of a compound of general formula (I), *supra* and **A'** (**8**) represents a tetraamine selected from

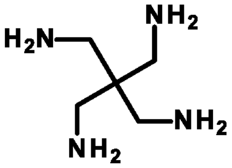

and

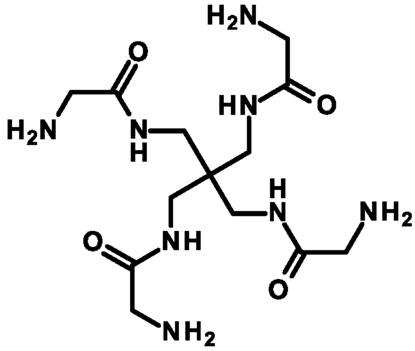

,

and described as Intermediate 4 and Intermediate 6, respectively.

A tetraamine 8 (A') or a salt thereof is reacted with a Mn-complex of the general formula 7 which is activated by a leaving group (LG), such as for example pentafluorophenol, 4-nitrophenol, 1-hydroxypyrrolidine-2,5-dione, hydroxybenzotriazole or 3H-[1,2,3]triazolo[4,5-b]pyridin-3-ol, leading to a compound of the general formula (I-b). The preparation of activated esters is well known to the person skilled in the art or might happen in situ by a peptide coupling reagent. The amid formation is, for example, performed by dissolving and stirring of intermediates 7 in water with a tetraamine 8 under the presence of hydroxybenzotriazole and 1-(3-dimethylaminopropyl)-3-ethylcarbodiimide for 16 to 48 hours at room temperature with a pH at 6.5.

The starting materials 8 are either commercially available tetraamines or salts thereof [for example CAS 4742-00-1] or tetraamines or salts thereof which are known from the literature, or which can be prepared in analogy to compounds which are described in the literature. The preparation of tetraamines

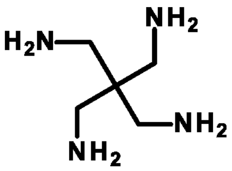

and

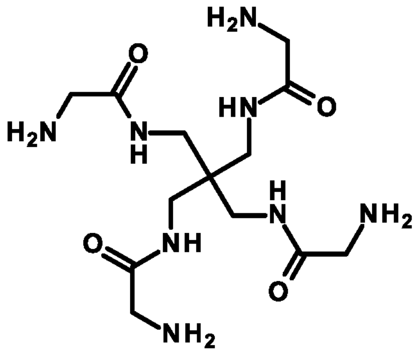

,

are described as Intermediate 4 and Intermediate 6, respectively.

The scheme and procedure described above illustrate synthetic route to the compounds of general formula (I) of the present disclosure and are not intended to be limiting. It would be apparent to the person skilled in the art that the order of transformations as exemplified in the scheme can be modified in various ways. The order of transformations exemplified in the schemes is therefore not intended to be limiting. Appropriate protecting groups and their introduction and cleavage are well-known to the person skilled in the art (see for example T.W. Greene and P.G.M. Wuts in Protective Groups in Organic Synthesis, 5th edition, Wiley 2014). Specific examples are described in the subsequent paragraphs.

## DESCRIPTION OF THE FIGURES

Figure 1. Manganese level determined in rat plasma after intravenous administration of the Manganese chelate of Example 3.

## EXPERIMENTAL SECTION

Chemical names were generated using the ACD/Name software from ACD/Labs. In some cases, generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1:** Abbreviations

| Abbreviation | Meaning |
|---|---|
| AcOH | acetic acid |
| ACN | acetonitrile |
| aq. | aqueous |
| AUC | Area under the curve |
| br | broad signal (NMR) |
| bw | Body weight |
| $C_{Gd}$ | Gadolinium concentration |
| CPMG | Carr-Purcell-Meiboom-Gill (MRI sequence) |
| d | doublet (NMR) |
| DAD | Diode Array Detector |
| DAST | Diethylaminosulfur trifluoride |
| DBU | 1,8-diazabicyclo(5.4.0)undec-7-ene |
| DCM | Dichloromethane |
| dd | doublet of doublet (NMR) |
| DIPEA | diisopropylethylamine |
| DMA | *N,N*-dimethylacetamide |
| DMAP | 4-dimethylaminopyridine |
| DMF | *N,N*-dimethylformamide |
| DMSO | Dimethylsulfoxide |
| EDC.HCl | N-(3-Dimethylaminopropyl)-N'-ethylcarbodiimide hydrochloride salt |
| ELSD | Evaporative light scattering detection |
| ESI | electrospray (ES) ionization |
| EtOAc | ethyl acetate |
| EtOH | ethanol |
| FLASH | Fast Low Angle Shot |
| Gd | Gadolinium |
| h | hour(s) |
| HATU | Hexafluorophosphate azabenzotriazole tetramethyl uronium |
| HCl | hydrogen chloride, hydrochloric acid |
| HPLC | high performance liquid chromatography |
| ICP-MS | inductively coupled plasma mass spectrometry |
| IR | Inversion Recovery |
| LC-MS | liquid chromatography-mass spectrometry |
| m | multiplet (NMR) |
| mCPBA | meta-Chloroperoxybenzoic acid |
| MeCN | Acetonitrile |
| MeOH | Methanol |
| min | minute(s) |
| MS | mass spectrometry |

(continued)

| Abbreviation | Meaning |
|---|---|
| MTBE | Methyl-*tert*-butylether |
| MRI | Magnetic Resonance Imaging |
| MWD | multiple wavelength detector |
| NaCl | sodium chloride |
| NMR | Nuclear Magnetic Resonance spectroscopy : chemical shifts ($\delta$) are given in ppm. The chemical shifts were corrected by setting the DMSO signal to 2.50 ppm using unless otherwise stated. |
| q | quartet (NMR) |
| $r_i$ | relaxivities (where i=1, 2) |
| $r_1$ or r1 | reflect how T1 relaxation rates change as a function of contrast agent concentration (slope T1 versus Gd concentration) |
| $r_2$ or r2 | reflect how T2 relaxation rates change as a function of contrast agent concentration (slope T2 versus Gd concentration) |
| $R_1$ | longitudinal relaxation rate ($1/r_1$ ) |
| $R_2$ | transversal relaxation rate ($1/ r_2$) |
| Rt or RT | room temperature |
| $R_t$, Rt | retention time |
| s | singulet (NMR) |
| sat. | saturated |
| SFC | supercritical fluid chromatography |
| $T_1$ or T1 | longitudinal relaxation time (T1 relaxation time) |
| $T_2$ or T2 | transversal relaxation time (T2 relaxation time) |
| t | triplet (NMR) |
| td | triplet of doublet (NMR) |
| TEA | triethylamine |
| TFA | trifluoroacetic acid |
| THF | tetrahydrofuran |
| $\delta$ | chemical shift |

Other abbreviations have their meanings customary *per se* to the skilled person.

The various aspects of the present disclosure described in this application are illustrated by the following examples which are not meant to limit the present disclosure in any way.

The example testing experiments described herein serve to illustrate the present disclosure and the present disclosure is not limited to the examples given.

**EXPERIMENTAL SECTION - GENERAL PART**

All reagents, for which the synthesis is not described in the experimental part, are either commercially available, or are known compounds or may be formed from known compounds by known methods by a person skilled in the art.

The compounds and intermediates produced according to the methods of the present disclosure may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography, particularly flash column chromatography, using for example prepacked silica gel cartridges, *e.g.* Biotage SNAP cartridges KP-Sil® or KP-NH® in combination with a Biotage autopurifier system (SP4® or Isolera Four®) and eluents such as gradients of hexane/ethyl acetate or DCM/methanol.

In flash column chromatography, unmodified ("regular") silica gel may be used as well as aminophase functionalized silica gel. If reference is made to flash column chromatography or to flash chromatography in the experimental section without specification of a stationary phase, regular silica gel was used.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present disclosure which possess a sufficiently basic or acidic functionality in the form of a salt, such as, in the case of a compound of the present disclosure which is sufficiently basic, a trifluoroacetate or formate salt for example, or, in the case of a compound of the present disclosure which is sufficiently acidic, an ammonium salt for example. A salt of this type can either be transformed into its free base or free acid form, respectively, by various methods known to the person skilled in the art or be used as salts in subsequent biological assays. It is to be understood that the specific form (*e.g.* salt, free base etc.) of a compound of the present disclosure as isolated and as described herein is not necessarily the only form in which said compound can be applied to a biological assay in order to quantify the specific biological activity.

**Analytical LC-MS Method**

Method **1**

Instrument: Waters Acquity UPLC-MS SQD 3001; Column: Acquity UPLC BEH C18 1.7 μm, 50 x 2.1 mm; eluent A: water + 0.2 vol. % aqueous ammonia (32%), Eluent B: acetonitrile; Gradient: 0-1.6 min 1-99% B, 1.6-2.0 min 99% B; Flow rate: 0.8 mL/min; Temperature: 60 °C; Injection: 2 μL; DAD scan: 210-400 nm; ELSD.

Method **2**

Instrument: Waters Acquity UPLC-MS SQD 3001; Column: Acquity UPLC BEH C18 1.7 μm, 50 x 2.1 mm; eluent A: water + 0.1 vol. % formic acid (99%), Eluent B: acetonitrile; Gradient: 0-1.6 min 1-99% B, 1.6-2.0 min 99% B; Flow rate: 0.8 mL/min; Temperature: 60 °C; Injection: 2 μL; DAD scan: 210-400 nm; ELSD.

Method **3**

Instrument: SHIMADZU LCMS-2020 SingleQuad; Column: Chromolith@Flash RP-18E 25 x 2 mm; eluent A: water + 0.0375 vol % trifluoroacetic acid, eluent B: acetonitrile + 0.01875 vol % trifluoroacetic acid; gradient: 0-0.8 min 0-60% B, 0.8-1.2 min 60% B; flow 1.5 ml/min; temperature: 50 °C; PDA: 220 nm & 254 nm.

Method **4**

Instrument: SHIMADZU LCMS-2020 SingleQuad; Column: Chromolith@Flash RP-18E 25 x 2 mm; eluent A: water + 0.0375 vol % trifluoroacetic acid, eluent B: acetonitrile + 0.01875 vol % trifluoroacetic acid; gradient: 0-0.8 min, 5-95% B, 0.8-1.2 min 95% B; flow 1.5 ml/min; temperature: 50 °C; PDA: 220 nm & 254 nm.

**NMR Spectra**

The multiplicities of proton signals in $^1H$ NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of molecular moieties or by interchanging protons - have also been assigned tentatively (often referred to as a broad multiplet or broad singlet) or are not shown

Chemical names were generated using the ACD/Name software from ACD/Labs. In some cases generally accepted names of commercially available reagents were used in place of ACD/Name generated names.

Optical rotations were measured using a JASCO P2000 Polarimeter. Typical, a solution of the compound with a concentration of 1 mg/mL to 15 mg/mL was used for the measurement. The specific rotation $[\alpha]_D$ was calculated according to the following formula:

$$[\alpha]D = \frac{\propto}{\beta \times d}$$

In this equation, $\alpha$ is the measured rotation in degrees; d is the path length in decimetres and $\beta$ is the concentration in g/mL.

**Synthesis of the Intermediates**

**Intermediate 1:**

**2,3,5,6-tetrafluorophenyl 3,9-bis(2-tert-butoxy-2-oxoethyl)-6-oxa-3,9,15-triazabicyclo[9.3.1]pentadeca-1(15),11,13-triene-13-carboxylate**

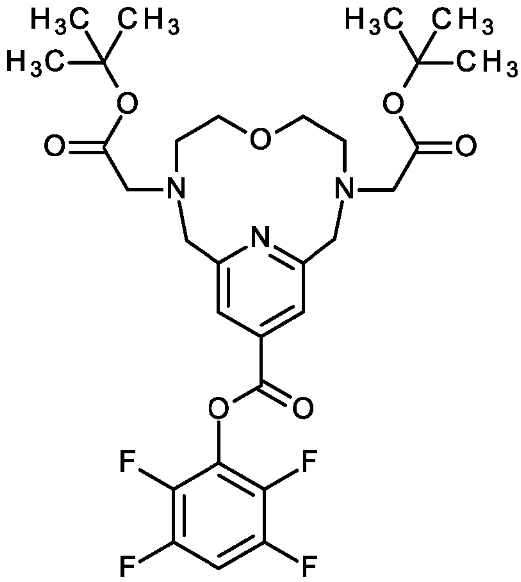

Step 1

methyl 2,6-bis(hydroxymethyl)pyridine-4-carboxylate

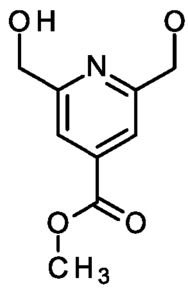

To a solution of methyl pyridine-4-carboxylate (30 g, 219 mmol) in methanol (1.0 l) was added sulfuric acid (5.0 ml, 94 mmol) at room temperature. After refluxing for 0.5 hour, a solution of ammonium peroxodisulphate (499 g, 2.19 mol) in water (1.0 l) was added at 70°C for 1 hour. The mixture was refluxed for 2 hours. The mixture was combined with another identically prepared 30 g batch, the combined mixture was concentrated to remove methanol, quenched with solid sodium bicarbonate and extracted with ethyl acetate. The organic phase was washed with brine, dried over anhydrous sodium sulphate, filtered, and concentrated to give methyl 2,6-bis(hydroxymethyl)isonicotinate (48 g) as a yellow solid.
$^1$H NMR (400 MHz, DMSO-$d_6$): $\delta$ [ppm] = 7.79 (s, 2H), 5.57 (t, 2H), 4.60 (d, 4H), 3.91 (s, 3H).

Step 2

methyl 2,6-bis(bromomethyl)pyridine-4-carboxylate

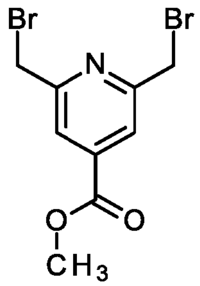

To a solution of methyl 2,6-bis(hydroxymethyl)pyridine-4-carboxylate (48 g, 243 mmol) in N,N-dimethylformamide (1.0 l) was added phosphorus tribromide (49 ml, 510 mmol) at 0 °C. After stirring at room temperature for 2 hours, the mixture was quenched with saturated sodium bicarbonate solution at 0°C. The aqueous layer was extracted with ethyl acetate. The organic phase was washed with brine, dried over anhydrous sodium sulphate, filtered and the filtrate was concentrated to give a residue. The crude product was purified by column chromatography (silica gel 1000 mesh, petroleum ether / ethyl acetate, 50: 1 to 10: 1) to give methyl 2,6-bis(bromomethyl)isonicotinate (42 g, 53% yield) as white solid.

LC-MS (Method 4): $R_t$ = 0.855 min; MS (ESIpos): m/z = 323.9 $[M+H]^+$.

$^1$H NMR (400 MHz, $CDCl_3$): δ [ppm] = 7.94 (s, 2H), 4.60 (s, 4H), 3.99 (s, 3H).

Step 3

*N*,*N*'-[oxydi(ethane-2,1-diyl)]bis(2,2,2-trifluoroacetamide)

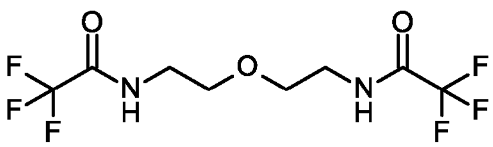

To a solution of 2,2'-oxydi(ethan-1-amine) (25 g, 240 mmol) in dichloromethane (200 ml) was added a solution of ethyl trifluoroacetate (85.3 g, 600 mmol) in dichloromethane (50 ml) at 0 °C. The mixture was stirred at room temperature for 12 hours. The mixture was concentrated to give a residue. The residue was diluted with ethyl acetate and washed with saturated ammonium chloride solution. The organic phase was washed with brine, dried over sodium sulphate, filtered and concentrated under reduced pressure to give *N*,*N*'-(oxydiethane-2,1-diyl)bis(2,2,2-trifluoroacetamide) (62 g, 87% yield) as white solid.

$^1$H NMR (400 MHz, $CDCl_3$): δ [ppm] = 6.91 (br. s, 2H), 3.69-3.50 (m, 8H).

Step 4

methyl 3,9-bis(trifluoroacetyl)-6-oxa-3,9,15-triazabicyclo[9.3.1]pentadeca-1(15), 11,13-triene-13-carboxylate

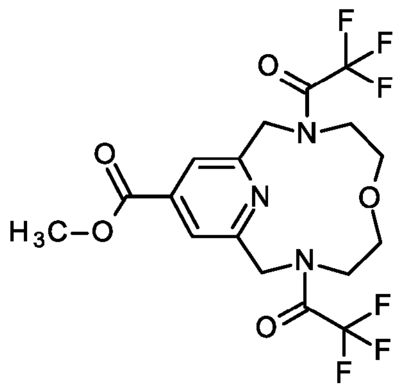

To a mixture of /V,/V'-[oxydi(ethane-2,1-diyl)]bis(2,2,2-trifluoroacetamide) (18 g, 60.8 mmol) and methyl 2,6-bis(bromomethyl)pyridine-4-carboxylate (19.6 g, 60.8 mmol, step 2) in acetonitrile (1.8 l) was added caesium carbonate (79.2 g, 243 mmol) at room temperature. The mixture was stirred at room temperature for 12 hours. The mixture was combined with an identically prepared 18 g batch, the combined batch was filtered, and the filtrate was concentrated to give a residue. The residue was triturated with methanol and filtered. The filter cake was washed with methanol and concentrated to give methyl 3,9-bis(trifluoroacetyl)-6-oxa-3,9,15-triazabicyclo[9.3.1]pentadeca-1(15),11,13-triene-13-carboxylate (46 g) as white solid.

LC-MS (Method 4): $R_t$ = 0.764 min; MS (ESIpos): m/z = 458.1 $[M+H]^+$.

Step 5

methyl 6-oxa-3,9,15-triazabicyclo[9.3.1]pentadeca-1(15), 11,13-triene-13-carboxylate hydrogen chloride (1/2)

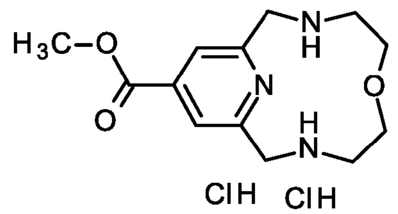

A solution of methyl 3,9-bis(trifluoroacetyl)-6-oxa-3,9,15-triazabicyclo[9.3.1]pentadeca-1 (15), 11, 13-triene-13-carboxylate (50.0 g, 109 mmol) in hydrochloric acid in methanol (4M, 1.0 l, 4.0 mol) was stirred at 60°C for 2 days. The reaction was concentrated to give a methyl 6-oxa-3,9,15-triazabicyclo[9.3.1]pentadeca-1(15),11,13-triene-13-carboxylate dihydrochloride (41 g, crude) as a yellow solid.

LC-MS (Method 3): $R_t$ = 0.119 min; MS (ESIpos): m/z = 266.2 $[M+H]^+$.

Step 6

3,9-di-tert-butyl 13-methyl 6-oxa-3,9,15-triazabicyclo[9.3.1]pentadeca-1(15),11,13-triene-3,9,13-tricarboxylate

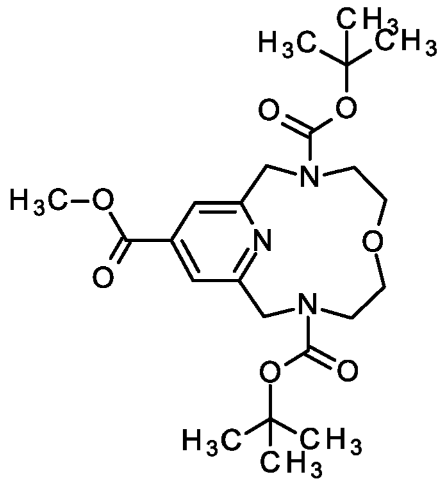

To a solution of methyl 6-oxa-3,9,15-triazabicyclo[9.3.1]pentadeca-1(15),11,13-triene-13-carboxylate - hydrogen chloride (1/2) (41 g, 121 mmol) in tetrahydrofuran (350 ml) and water (350 ml) were added sodium hydrogen carbonate (40.7 g, 485 mmol) and di-tert-butyl dicarbonate (84 ml, 360 mmol) at 0°C. The mixture was stirred at room temperature for 2 hours. The mixture was extracted with ethyl acetate. The organic phase was washed with brine, dried with anhydrous sodium sulphate, filtered and concentrated to give a residue. The residue was purified by column chromatography (silica gel, 1000 mesh, petroleum ether: ethyl acetate, 10: 1 to 1: 1) to give 3,9-di-tert-butyl 13-methyl 6-oxa-3,9,15-triazabicyclo [9.3.1]pentadeca-1(15),11,13-triene-3,9,13-tricarboxylate (37 g, 66% yield) as yellow oil.

LC-MS (Method 4): $R_t$ = 0.549 min; MS (ESIpos): m/z = 466.2 $[M+H]^+$.

$^1$H NMR (400 MHz, $CDCl_3$): δ [ppm] = 7.82-7.67 (m, 2H), 4.68-4.47 (m, 4H), 4.00-3.86 (m, 3H), 3.62-3.41 (m, 8H), 1.50-1.37 (m, 18H).

Step 7

methyl 6-oxa-3,9,15-triazabicyclo[9.3.1]pentadeca-1(15), 11,13-triene-13-carboxylate - hydrogen chloride (1/2)

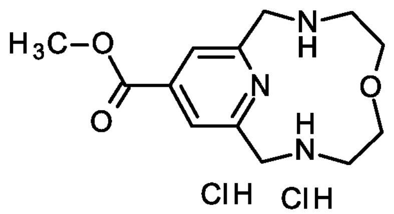

To a solution of 3,9-di-tert-butyl 13-methyl 6-oxa-3,9,15-triazabicyclo[9.3.1]pentadeca-1(15),11,13-triene-3,9,13-tricarboxylate (38.9 g, 83.5 mmol, step 6) in 1,4-dioxane (50 ml) was added hydrochloric acid in dioxane (2M, 500 ml, 1 mol) at 0°C. The mixture was stirred at room temperature for 12 hours. The mixture was concentrated to give methyl 6-oxa-3,9,15-triazabicyclo[9.3.1]pentadeca-1(15),11,13-triene-13-carboxylate dihydrochloride (23 g, 98% purity) as a yellow solid.

LC-MS (Method 3): $R_t$ = 0.237min; MS (ESIpos): m/z = 266.2 $[M+H]^+$.

$^1$H NMR (400 MHz, DMSO-$d_6$): δ [ppm] = 10.7-9.30 (m, 4H), 7.95 (s, 2H), 4.60 (s, 4H), 3.93 (s, 3H), 3.35-3.00 (m, 8H).

Step 8

methyl 3,9-bis(2-tert-butoxy-2-oxoethyl)-6-oxa-3,9,15-triazabicyclo[9.3.1]pentadeca-1(15),11,13-triene-13-carboxylate

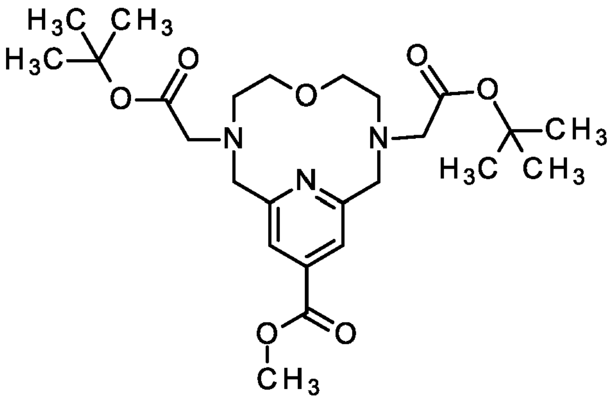

To methyl 6-oxa-3,9,15-triazabicyclo[9.3.1]pentadeca-1(15),11,13-triene-13-carboxylate hydrogen chloride (1/2) (1.5 g, 4.43 mmol, step 7) in acetonitrile (20 ml) was added potassium carbonate (1.84 g, 13.3 mmol) and tert-butyl bromoacetate (1.3 ml, 8.9 mmol). The mixture was stirred at 60°C for 6 h. The mixture was concentrated under reduced pressure and water was added. The mixture was extracted with dichloromethane. The organic phase was washed with brine, dried with anhydrous sodium sulphate, filtered, and concentrated to give 2.5 g of the title compound as raw product, which was used in the next step without further purification.
LC-MS (Method 2): $R_t$ = 0.91 min; MS (ESIpos): m/z = 494 $[M+H]^+$
$^1$H-NMR (400 MHz, DMSO-$d_6$): $\delta$ [ppm] = 7.65 (br s, 2H), 4.04 (br s, 4H), 3.90 (s, 3H), 3.45 (s, 4H), 3.22 (br s, 4H), 2.81 (br s, 4H), 1.45 (s, 18H).

Step 9

3,9-bis(2-tert-butoxy-2-oxoethyl)-6-oxa-3,9,15-triazabicyclo[9.3.1]pentadeca-1(15),11,13-triene-13-carboxylic acid

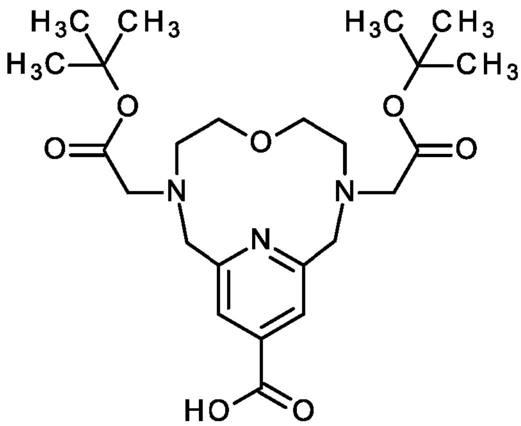

To 3,9-bis(2-tert-butoxy-2-oxoethyl)-6-oxa-3,9,15-triazabicyclo[9.3.1]pentadeca-1(15), 11,13-triene-13-carboxylate (4.1 g, 8.3 mmol) in acetonitrile (100 ml) and water (2 ml) was added lithium bromide (7.2 g, 83 mmol) and triethylamine (3.5 ml, 25 mmol) and the mixture was stirred at RT for 16 h. Water was added and the mixture was extracted with dichloromethane. The organic phase dried over anhydrous sodium sulphate, filtered, and concentrated to give 3.35 g (97% purity, 82% yield) of the title compound as raw product, which was used in the next step without further purification.
LC-MS (Method 2): $R_t$ = 0.96 min; MS (ESIneg): m/z = 478 $[M-H]^-$.

Step 10

2,3,5,6-tetrafluorophenyl 3,9-bis(2-tert-butoxy-2-oxoethyl)-6-oxa-3,9,15-triazabicyclo[9.3.1]pentadeca-1(15),11,13-triene-13-carboxylate

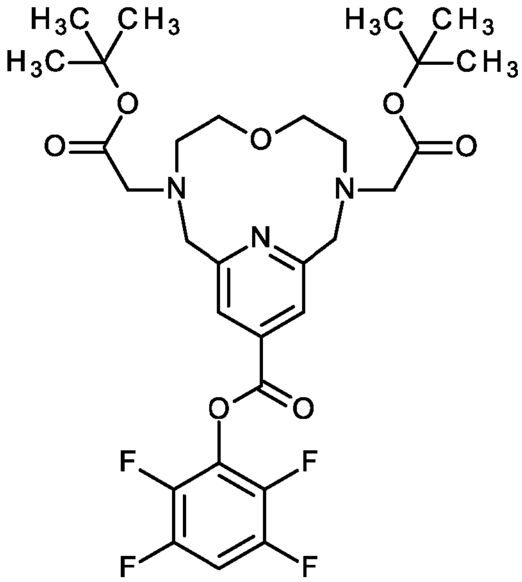

To a solution 3,9-bis(2-tert-butoxy-2-oxoethyl)-6-oxa-3,9,15-triazabicyclo[9.3.1] pentadeca-1(15),11,13-triene-13-carboxylic acid (3.15 g, 6.37 mmol) and 2,3,5,6-tetrafluorophenol (1.69 g, 10.2 mmol) in dichloromethane (50 ml) was added *N*,*N*'-dicyclohexyl methane diimine (2.63 g, 12.7 mmol) and the mixture was stirred at room temperature for 68 hours. The reaction mixture was filtered and concentrated under reduced pressure to give 5.2 g (70% purity, 70% yield) of the title compound as raw product, which was used in the next step (Example 1 and 2) without further purification.

LC-MS (Method 2): $R_t$ = 1.08 min; MS (ESIpos): m/z = 628.6 $[M+H]^+$.

<u>**Intermediate 2**</u>

**manganese(2+) (2R*,2'R*)-2,2'-[13-carboxy-6-oxa-3,9,15-triazabicyclo [9.3.1]pentadeca-1 (15), 11,13-triene-3,9-diyl]dipropanoate**

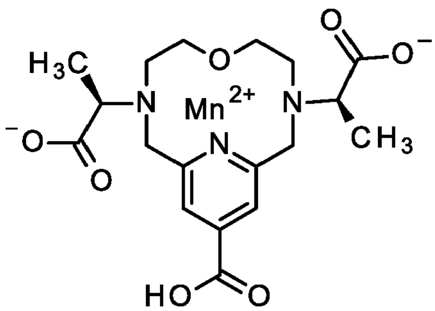

<u>Step 1</u>

methyl 3,9-bis(1-methoxy-1-oxopropan-2-yl]-6-oxa-3,9,15-triazabicyclo[9.3.1] pentadeca-1 (15),11,13-triene-13-carboxylate

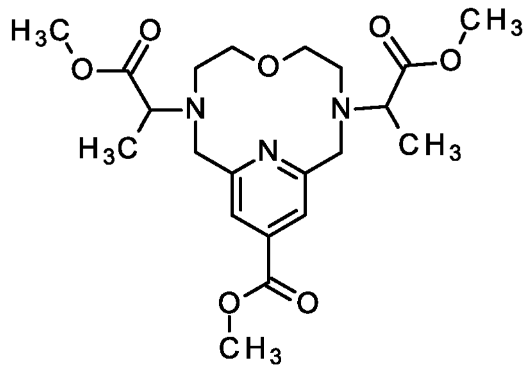

To methyl 6-oxa-3,9,15-triazabicyclo[9.3.1]pentadeca-1(15), 11,13-triene-13-carboxylate hydrogen chloride (1/2) (2.0 g, 5.9 mmol, intermediate 1 step 7) in acetonitrile (40 ml) was added potassium carbonate (2.45 g, 17.7 mmol) and the mixture was stirred at room temperature for 30 minutes. Methyl bromoacetate (1.3 ml, 12 mmol) was added and the mixture was stirred at 60°C for 19 h. The mixture concentrated under reduced pressure and water was added. The mixture was extracted with dichloromethane. The organic phase was dried with anhydrous sodium sulphate, filtered, and concentrated under reduced pressure. The crude product was purified by column chromatography (Interchim puriFlash® PF-15 RP18 AQ F0080, water / acetonitrile, 0 - 40%) to deliver 2.32 g (99% purity, 89% yield) of the title compound as mixture of stereoisomers.

LC-MS (Method 2): $R_t$ = 0.74 and 0.76 min; MS (ESIpos): m/z = 438.3 $[M+H]^+$.

Step 2

3-(1-carboxyethyl)-9-(1-carboxyethyl)-6-oxa-3,9,15-triazabicyclo[9.3.1]pentadeca-1(15), 11,13-triene-13-carboxylic acid

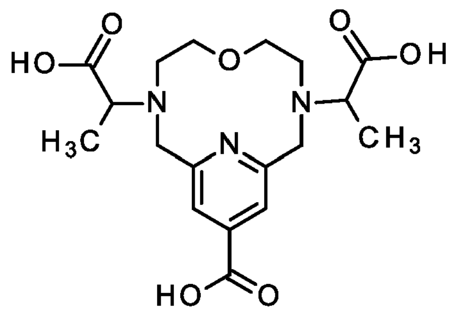

To methyl 3,9-bis(1-methoxy-1-oxopropan-2-yl)-6-oxa-3,9,15-triazabicyclo[9.3.1] pentadeca-1(15),11,13-triene-13-carboxylate (2.32 g, 5.3 mmol) in THF (30 ml) was added an aqueous sodium hydroxide solution (2 M, 11 ml, 22 mmol) and the mixture was stirred at room temperature for 3 h. The mixture was concentrated under reduced pressure to remove the THF and used in the next step without further purification.

LC-MS (Method 2): $R_t$ = 0.22 min; MS (ESIpos): m/z = 395.2 $[M+H]^+$.

Step 3

manganese(2+) (2R*,2'R*)-2,2'-[13-carboxy-6-oxa-3,9,15-triazabicyclo[9.3.1]pentadeca-1(15), 11,13-triene-3,9-diyl]dipropanoate

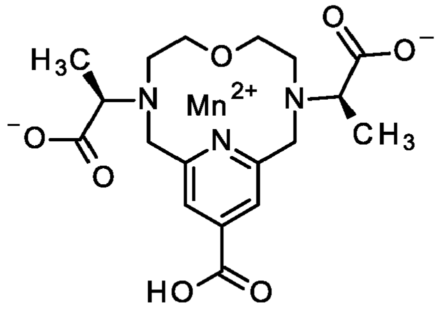

To 3,9-bis(1-carboxyethyl]-6-oxa-3,9,15-triazabicyclo[9.3.1]pentadeca-1(15),11,13-triene-13-carboxylic acid (2.1 g, 5.31 mmol) in an aqueous sodium hydroxide solution (374 mg, 981 pmol) from step 2 was added water (25 ml) and dichloromanganese tetrahydrate (1.16 g, 5.84 mmol). The pH of the suspension was corrected to the value of 6.5 with aqueous hydrochloric acid (2.0 M) and the mixture was stirred at 100°C for 16 h. After cooling to room temperature Chelex®100 was added and the reaction mixture was stirred for 1 h. Chelex®100 was removed by filtration and the pH of the filtrate was corrected to the value of 7.5 with aqueous hydrochloric acid (2M). The aqueous solution was purified by column chromatography (Interchim puriFlash® PF-15 RP18AQ-F00330, water/ acetonitrile, 0 - 20%) to yield 1.3 g (98% purity, 64% yield) of the racemic title compound (intermediate 2) and 787 mg of manganese(2+) (2R)-2-{13-carboxy-9-[(1S)-1-carboxylatoethyl]-6-oxa-3,9,15-triazabicyclo [9.3.1]pentadeca-1(15),11,13-trien-3-yl}propanoate (intermediate 3, meso-compound).

LC-MS (Method 1): $R_t$ = 0.21 min; MS (ESIpos): m/z = 448.3 $[M+H]^+$

<u>**Intermediate 3**</u>

**manganese(2+) (2R)-2-{13-carboxy-9-[(1S)-1-carboxylatoethyl]-6-oxa-3,9,15-triazabicyclo[9.3.1]pentadeca-1(15),11,13-trien-3-yl}propanoate**

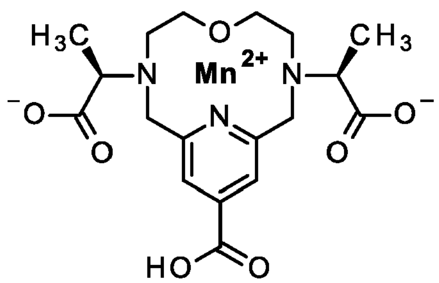

The title compound was isolated as second component (meso-compound) from the synthesis of intermediate 2 in step 3.

LC-MS (Method 1): $R_t$ = 0.24 min; MS (ESIpos): m/z = 448.3 $[M+H]^+$

<u>**Intermediate 4**</u>

**2,2-Bis(aminomethyl)propan-1,3-diamin**

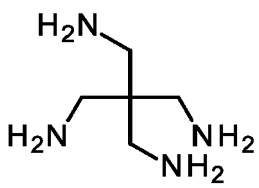

Dowex 1X8, 200-400 mesh was activated by aqueous NaOH (2M) and washed with water to pH 6. 2,2-Bis(aminomethyl)propan-1,3-diamintetrahydrochlorid (10.0 g, 36.0 mmol) in water (120 ml) was filtered through the activated Ion-exchange resin (Dowex 1X8). The basic fractions (pH 13) were collected and concentrated under reduced pressure to yield 4,35 g (90% purity, 82% yield of title compound as free base.

LC-MS: (Method 2): $R_t$ = 0.18 min; MS (ESIpos): m/z = 133 $[M+H]^+$

NMR: $^1H$ NMR (400 MHz, DMSO-$d_6$) $\delta$ [ppm] = 2.33 (s, 8H).

## Intermediate 5

**manganese(2+) 2,2'-[12-carboxy-6-oxa-3,9,15-triazabicyclo[9.3.1]pentadeca-1 (15), 11,13-triene-3,9-diyl]diacetate**

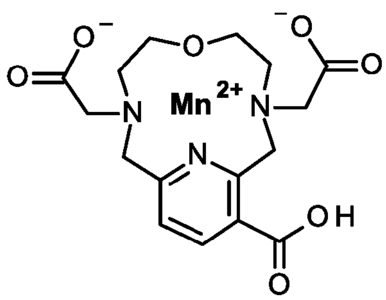

Step 1

methyl 2,6-dimethylpyridine-3-carboxylate

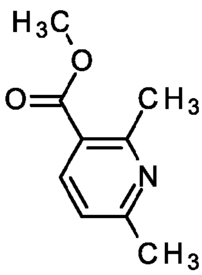

To a solution of 2,6-dimethylnicotinic acid (50 g, 331 mmol) in methanol (250 ml) was added thionyl chloride (25 ml, 469 mmol) in one portion. The reaction mixture was stirred at 60°C for 16 hours. The mixture was concentrated to give a residue. The residue was quenched by saturated sodium hydrogen carbonate and extracted with ethyl acetate. The organic phase was washed with brine, dried with anhydrous sodium sulphate, filtered, and concentrated to give methyl 2,6-dimethylnicotinate (26 g, 48% yield) as a yellow oil.

LC-MS (Method 3): $R_t$ = 0.595 min; MS (ESIpos): m/z = 166.1 $[M+H]^+$.

$^1H$ NMR (400 MHz, DMSO-$d_6$): $\delta$ [ppm] = 8.05 (d, 1H), 7.20 (d, 1H), 3.83 (s, 3H), 2.66 (s, 3H), 2.47 (s, 3H).

Step 2

methyl 2,6-bis(bromomethyl)pyridine-3-carboxylate

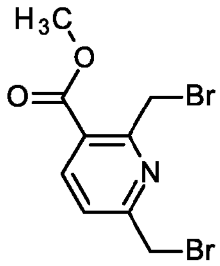

To a solution of methyl 2,6-dimethylnicotinate (25 g, 151 mmol) in 1,2-dichloroethane (500 ml) were added *N*-bromosuccinimide (67.3 g, 378 mmol) and 2,2'-azobis isobutyronitrile (9.94 g, 60.5 mmol) at room temperature. The mixture was stirred at 90°C for 12 hours under nitrogen atmosphere. The mixture was diluted with dichloromethane and washed with saturated sodium hydrogen carbonate solution. The organic phase was washed with brine, dried with anhydrous sodium sulphate, filtered, and concentrated to give a residue. The residue was purified by column chromatography on silica gel (1000 mesh, petroleum ether: ethyl acetate = 100: 1 to 50: 1) to give methyl 2,6-bis(bromomethyl) nicotinate (20.0 g, 41% yield) as yellow oil.
LC-MS (Method 4): $R_t$ = 0.598 min; MS (ESIpos): m/z = 323.8 $[M+H]^+$.

Step 3

*N,N'*-[oxydi(ethane-2,1-diyl)]bis(2-nitrobenzene-1-sulfonamide)

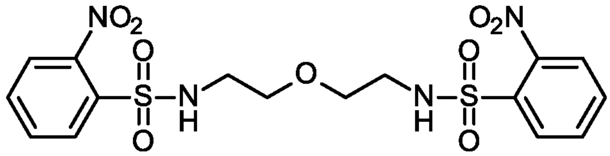

To a solution of 2,2'-oxydiethanamine (45 g, 432 mmol) in tetrahydrofuran (1500 ml) were added a solution of potassium carbonate (239 g, 1.73 mol) in water (500 ml) and 2-nitrobenzenesulfonyl chloride (182 g, 864 mmol) at 0 °C. The mixture was stirred at room temperature for 12 hours. The reaction mixture was combined with an identical 45 g batch, diluted with water, and extracted with ethyl acetate. The organic phase was washed with brine, dried over anhydrous sodium sulphate and concentrated to give a residue. The residue was purified by re-crystallization with ethyl acetate/petroleum ether (1/10) at 0°C to give N,N'-(oxydiethane-2,1-diyl)bis(2-nitrobenzenesulfonamide) (416 g) as white solid.
LC-MS (Method 4): $R_t$ = 0.863 min; MS (ESIpos): m/z = 475.0 $[M+H]^+$.
$^1H$ NMR (400 MHz, DMSO-$d_6$): δ [ppm] = 8.25-8.08 (m, 2H), 8.00-7.92 (m, 4H), 7.85-7.81 (m, 4H), 3.29 (t, 4H), 2.99 (t, 4H).

Step 4

methyl 3,9-bis(2-nitrobenzene-1-sulfonyl)-6-oxa-3,9,15-triazabicyclo[9.3.1]pentadeca-1 (15), 11,13-triene-12-carboxylate

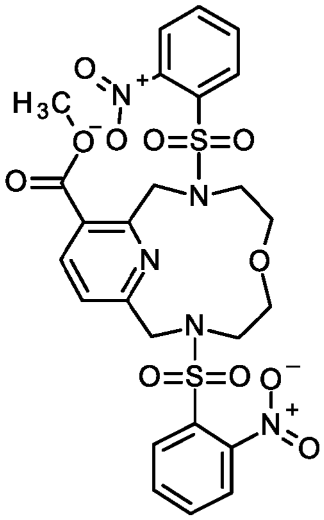

To a solution of /V,/V'-(oxydiethane-2,1-diyl)bis(2-nitrobenzenesulfonamide) (20.0 g, 42.2 mmol, step 3) in acetonitrile (2000 ml) were added methyl 2,6-bis(bromomethyl)nicotinate (13.6 g, 42.2 mmol, step 2) and sodium carbonate (17.9 g, 169 mmol) at room temperature. The mixture was stirred at 80 °C for 4 days and combined with an identically prepared 20 g batch. The combined batch was concentrated to give a residue, then diluted with water and extracted with ethyl acetate. The combined organic layers were washed with brine twice, dried over sodium sulphate, filtered, and concentrated under reduced pressure to give a crude product. The crude product was purified by column chromatography (silica gel, petroleum ether / ethyl acetate, 50 : 1 to 1 : 3) to give methyl 3,9-bis[(2-nitrophenyl)sulfonyl]-6-oxa-3,9,15-triazabicyclo[9.3.1]pentadeca-1(15),11,13-triene-12-carboxylate (9.3 g, 35% yield) as yellow oil.

LC-MS (Method 4): $R_t$ = 0.585 min; MS (ESIpos): m/z = 636.0 $[M+H]^+$.

$^1H$ NMR (400 MHz, $CDCl_3$): δ [ppm] = 8.21 (d, 1H), 8.12-8.10 (m, 1H), 8.07-8.05 (m, 1H), 7.77-7.67 (m, 5H), 7.63-7.60 (m, 1H), 7.46 (d, 1H), 5.08 (s, 2H), 4.55 (s, 2H), 3.94 (s, 3H), 3.56-3.47 (m, 8H).

Step 5

methyl 6-oxa-3,9,15-triazabicyclo[9.3.1]pentadeca-1(15), 11,13-triene-12-carboxylate

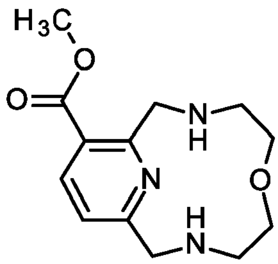

To a solution of methyl 3,9-bis[(4-nitrophenyl)sulfonyl]-6-oxa-3,9,15-triazabicyclo[9.3.1] pentadeca-1(15),11,13-triene-12-carboxylate (9.3 g, 14.6 mmol) in acetonitrile (250 ml) were added potassium carbonate (8.90 g, 64.4 mmol) and 4-methylbenzenethiol (5.45 g, 43.9 mmol) at room temperature. The mixture was stirred at 30 °C for 48 hours. The mixture was concentrated to remove most solvent. The residue was diluted with ethyl acetate and washed with water. Hydrochloric acid (1M) was added and the pH of the aqueous phase was adjusted to pH 2. After phase separation, the aqueous layer was adjusted to pH 9 with solid sodium carbonate. The aqueous phase was used for next step without further purification.

Step 6

3,9-di-tert-butyl 12-methyl 6-oxa-3,9,15-triazabicyclo[9.3.1]pentadeca-1(15),11,13-triene-3,9,12-tricarboxylate

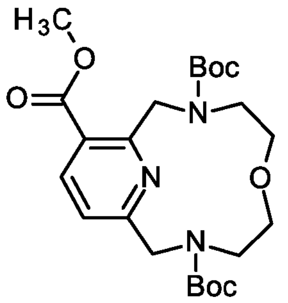

To a mixture of methyl 6-oxa-3,9,15-triazabicyclo[9.3.1]pentadeca-1(15),11,13-triene-12-carboxylate (3.88 g, 14.6 mmol) in water (300 ml) was added a solution of di-*tert*-butyl dicarbonate (6.70 g, 30.7 mmol) in acetonitrile (300 ml) and sodium carbonate (1.55 g, 14.6 mmol). The mixture was stirred at 60°C for 3 hours. The reaction mixture was diluted with water and extracted with ethyl acetate. The combined organic layer was washed with brine twice, dried over sodium sulphate, filtered, and concentrated under reduced pressure to give a residue. The residue was purified by column chromatography (silica gel 100-200 mesh, petroleum ether / ethyl acetate, 50: 1 to 1: 2) to give 3,9-di-*tert*-butyl 12-methyl 6-oxa-3,9,15-triazabicyclo[9.3.1]pentadeca-1(15),11,13-triene-3,9,12-tricarboxylate (4.9 g, 72% yield) as colourless oil.

LC-MS (Method 4): $R_t$ = 0.522 min; MS (ESIpos): m/z = 466.2 $[M+H]^+$.

Step 7

methyl 6-oxa-3,9,15-triazabicyclo[9.3.1]pentadeca-1(15), 11,13-triene-12-carboxylate-hydrogen chloride (1/2)

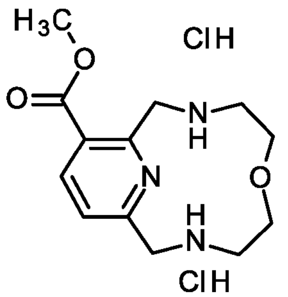

A solution of 3,9-di-tert-butyl 12-methyl 6-oxa-3,9,15-triazabicyclo[9.3.1]pentadeca-1(15),11,13-triene-3,9,12-tricarboxylate (4.90 g, 10.5 mmol, step 5) in hydrochloric acid (2M in ethyl acetate, 100 ml, 200 mmol) was stirred at 25°C for 8 hours. The reaction mixture was concentrated to give methyl 6-oxa-3,9,15-triazabicyclo[9.3.1]pentadeca-1(15),11,13-triene-12-carboxylate dihydrochloride (3.56 g) as white solid.

LC-MS (Method 3): $R_t$ = 0.120 min; MS (ESIpos): m/z = 266.1 $[M+H]^+$.

$^1$H NMR (400 MHz, DMSO): δ [ppm] = 8.54 (d, 1H), 7.60 (d, 1H), 4.97 (s, 2H), 4.71 (s, 2H), 3.95 (s, 3H), 3.65-3.64 (m, 4H),3.45-3.40 (m, 4H).

Step 8

methyl 3,9-bis(2-tert-butoxy-2-oxoethyl)-6-oxa-3,9,15-triazabicyclo[9.3.1]pentadeca-1(15),11,13-triene-12-carboxylate

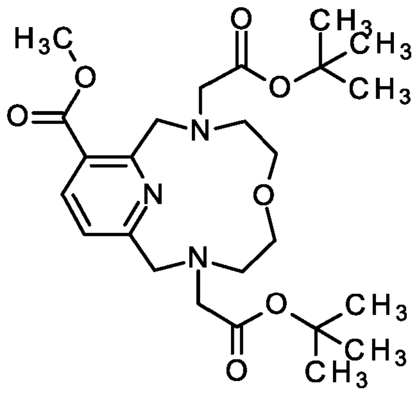

To a solution of methyl 6-oxa-3,9,15-triazabicyclo[9.3.1]pentadeca-1(15),11,13-triene-12-carboxylate dihydrochloride (3.56 g, 10.5 mmol, step 6) in acetonitrile (100 ml) were added tert-butyl bromoacetate (4.52 g, 23.1 mmol) and potassium carbonate (8.73 g, 63.1 mmol) at room temperature. The mixture was stirred at 60°C for 16 hours. The reaction mixture was concentrated to remove most solvent. The residue was diluted with water and extracted with ethyl acetate. The combined organic layers were washed with brine twice, dried over sodium sulphate, filtered, and concentrated under reduced pressure to give a residue. The residue was purified by reversed-phase column chromatography (Instrument: Agela HP1000; Column: Welch Ultimate XB_C18 150*400mm 20/40μm; eluent A: water, eluent B: acetonitrile; gradient: 0-105 min 20 - 85% B; flow 100 ml/min; temperature: room temperature; Detector: UV 220/254 nm). The desired fraction was adjusted to pH 9 with saturated sodium bicarbonate solution and extracted with ethyl acetate. The organic phase was dried over anhydrous sodium sulphate, filtered, and concentrated to give methyl 3,9-bis(2-tert-butoxy-2-oxoethyl)-6-oxa-3,9,15-triazabicyclo[9.3.1]pentadeca-1(15),11,13-triene-12-carboxylate (2.07 g, 40% yield) as yellow oil.

LC-MS (Method 4): $R_t$ = 0.493 min; MS (ESIpos): m/z = 494.2 $[M+H]^+$.

$^1H$ NMR (400 MHz, DMSO): $\delta$ [ppm] = 8.11 (d, 1H), 7.30 (d, 1H), 4.23 (s, 2H), 3.97 (s, 2H), 3.84 (s, 3H), 3.49 (s, 4H), 3.40-3.25 (m, 4H), 2.85-2.65 (m, 4H), 1.45 (s, 18H).

<u>Step 9</u>

3,9-bis(carboxymethyl)-6-oxa-3,9,15-triazabicyclo[9.3.1]pentadeca-1(15),11,13-triene-12-carboxylic acid

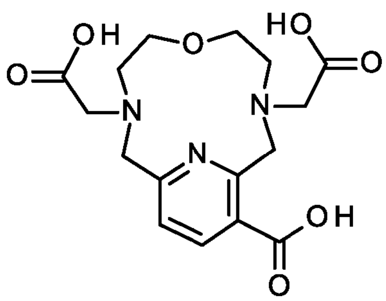

To a solution of 3,9-bis(2-tert-butoxy-2-oxoethyl)-6-oxa-3,9,15-triazabicyclo[9.3.1] pentadeca-1(15),11,13-triene-12-carboxylate (1.40 g, 2.84 mmol, step 7) in THF (34 ml) was added aqueous sodium hydroxide (2.0 M, 5.7 ml, 11 mmol) and the mixture was stirred at room temperature for 72 h. The mixture was concentrated under reduced pressure to remove the THF and used in the next step without further purification.

LC-MS (Method 1): $R_t$ = 0.18 min; MS (ESIpos): m/z = 368.2 $[M+H]^+$.

<u>Step 10</u>

manganese(2+) 2,2'-[12-carboxy-6-oxa-3,9,15-triazabicyclo[9.3.1]pentadeca-1(15), 11,13-triene-3,9-diyl]diacetate

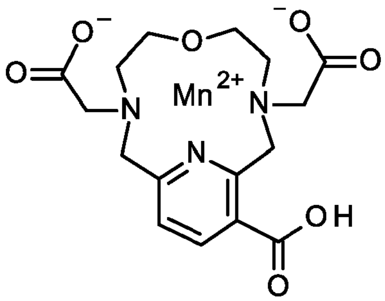

To 3,9-bis(carboxymethyl)-6-oxa-3,9,15-triazabicyclo[9.3.1]pentadeca-1(15),11,13-triene-12-carboxylic acid in an aqueous sodium hydroxide solution (1.04 g, 2.83 mmol) from step 8 was added water (25 ml) and dichloromanganese tetrahydrate (428 mg, 3.4 mmol). The pH of the suspension was corrected to the value of 6.5 with aqueous hydrochloric acid (1.2 ml, 4.0 M, 4.8 mmol) and the mixture was stirred at 95°C for 1h. After cooling to room temperature and Chelex®100 was added and the reaction mixture was stirred for 1h. Chelex®100 was filtered off and purified by column chromatography (Interchim puriflash PF-15RP18AQ-F0040, water/ acetonitrile, 0-20%) to yield 600 mg (97% purity, 49% yield) of the title compound.

LC-MS (Method 1): $R_t$ = 0.20 min; MS (ESIpos): m/z = 421 $[M+H]^+$

## Intermediate 6

**N,N'-{2,2-bis[(2-aminoacetamido)methyl]propane-1,3-diyl}bis(2-aminoacetamide)**

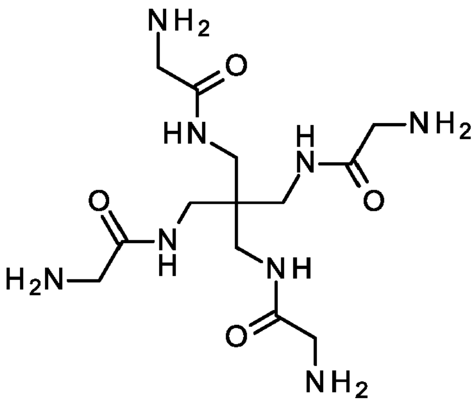

Step 1

*tert*-butyl {10,10-bis[({[(*tert*-butoxycarbonyl)amino]acetyl}amino)methyl]-2,2-dimethyl-4,7,13-trioxo-3-oxa-5,8,12-triazatetradecan-14-yl}carbamate

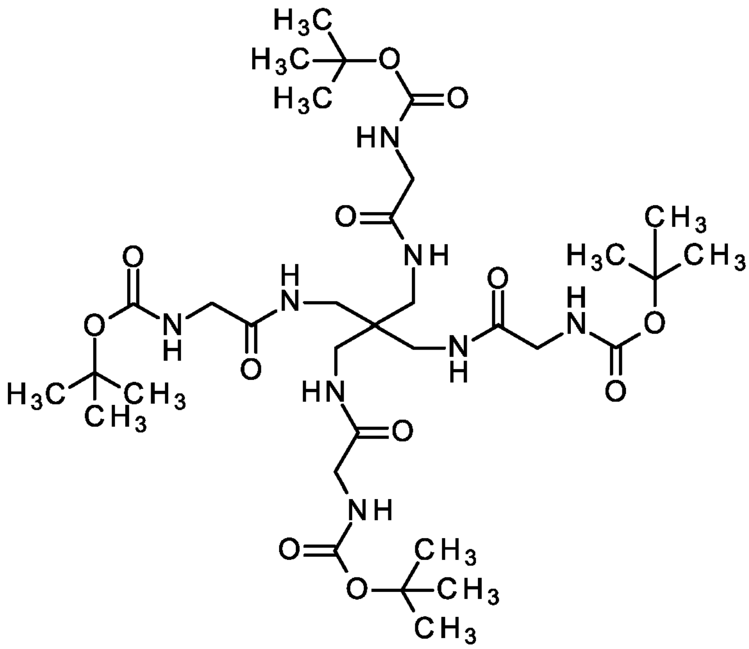

A mixture of 2,2-bis(aminomethyl)propane-1,3-diamine tetrahydrochloride (851 mg, 3.06 mmol, 1 eq.; see W. Hayes et al., Tetrahedron 59 (2003), 7983 - 7996) in dichloromethane (50 mL) was treated with *N*,*N*-diisopropylethylamine (6.00 eq., 3.20 mL, 18.4 mmol) and 2,5-dioxopyrrolidin-1-yl *N*-(*tert*-butoxycarbonyl)glycinate (CAS No. [3392-07-2]; 6.00 eq., 5.00 g, 18.4 mmol) and stirred at room temperature for 2.5 days. The reaction mixture was diluted with water, the formed precipitate filtered off and washed with water and dichloromethane. The precipitated material was subjected to silica gel chromatography (dichloromethane / methanol) to give the title compound (800 mg, 34%).

LC-MS ($ES^+$): m/z = 761.4 $(M + H)^+$; Rt. = 1.16 min.

$^1H$-NMR (400 MHz, DMSO-$d_6$): δ [ppm] = 1.36 (s, br, 36H), 2.74 - 2.76 (m, 8H), 3.48 - 3.50 (m, 8H), 6.96 (s, br, 0.4H*), 7.40 - 7.42 (m, 3.6H*), 7.91 - 8.00 (m, 4H) ppm.

Step 2

2-amino-N-(3-[(aminoacetyl)amino]-2,2-bis{[(aminoacetyl)amino]methyl}propyl)acetamide tetrahydrochloride

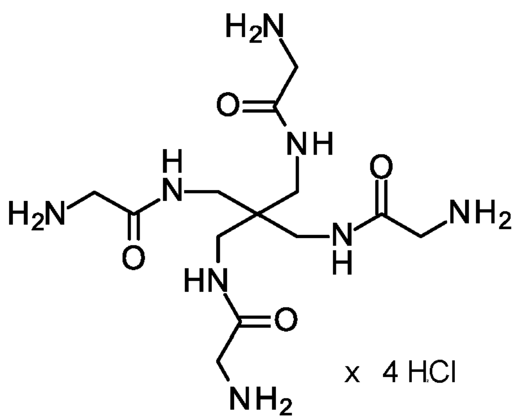

To a suspension of tert-butyl {10,10-bis[({[(*tert*-butoxycarbonyl)amino]acetyl} amino) methyl]-2,2-dimethyl-4,7,13-trioxo-3-oxa-5,8,12-triazatetradecan-14-yl}carbamate (1,76 g 2.31 mmol) in dioxane (22 ml) was added HCL in dioxane (23 ml, 4M, 92 mmol) and the reaction mixture was stirred at room temperature for 4 h. The suspension was concentrated under reduced pressure to yield 1,41g of the crude hydrochloride (quantitative)

LC-MS ($ES^+$): m/z = 361.2 $(M - 3HCl - Cl^-)^+$; Rt. = 0.10 min.

$^1H$-NMR (400 MHz, DMSO-$d_6$): δ [ppm] = 3.17 - 3.18 (m, 8H), 3.59 - 3.61 (m, 8H), 8.21 (s, br, 12H), 8.55 (t, 4H).

Step 3

***N,N'*-{2,2-bis[(2-aminoacetamido)methyl]propane-1,3-diyl}bis(2-aminoacetamide)**

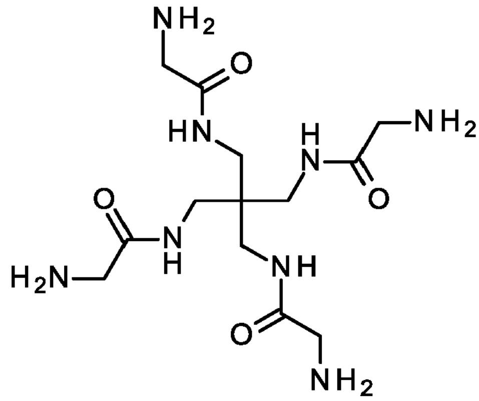

The crude product tert-butyl [10,10-bis({2-[(tert-butoxycarbonyl)amino] acetamido}methyl)-2,2-dimethyl-4,7,13-trioxo-3-oxa-5,8,12-triazatetradecan-14-yl] carbamate (1.41g) was solved in water and filtered through the activated ion-exchange resin column (Dowex 1X8, 100 g, OH-form) and washed with water. The basic fractions (pH13) were collected and concentrated under reduced pressure to give the title compound (0,85 g, 95% purity, 97% yield).

LC-MS (Method 2): $R_t$ = 0.17 min; MS (ESIpos): m/z = 361 [M+H]

$^1$H-NMR (400 MHz, DMSO-$d_6$) $\delta$ [ppm] = 2.82 (br d, 8H) 3.12 - 3.18 (m, 8H) 8.42 (s, 4H).

**Intermediate** 7

**manganese(2+) 2,2'-[13-(carboxymethyl)-6-oxa-3,9,15-triazabicyclo[9.3.1] pentadeca-1 (15),11,13-triene-3,9-diyl]diacetate**

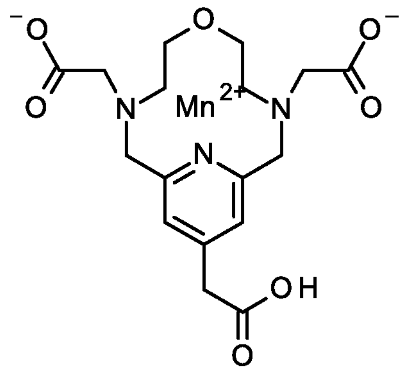

Step 1

4-bromo-2,6-bis(bromomethyl)pyridine

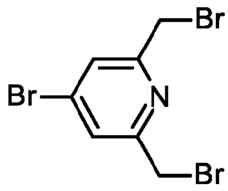

To a solution of 4-bromo-2,6-dimethylpyridine (50 g, 269 mmol) in 1,2-dichloroethane (2.0 l) was added N-bromosuccinimide (191 g, 1.07 mol) and 2,2'-azobisisobutyronitrile (17.7 g, 107 mmol) at room temperature. The mixture was stirred at 90°C for 4 hours under a nitrogen atmosphere. The mixture was diluted with dichloromethane and washed with saturated sodium hydrogen carbonate solution. The organic phase was washed with brine, dried with anhydrous sodium sulphate, filtered, and concentrated in vacuo. The residue was dissolved in tetrahydrofuran (2.0 l). To the mixture was added N,N-diisopropylethylamine (230 ml, 1.3 mol) and diethyl phosphite (170 ml, 1.3 mol) at 0°C. The mixture was stirred at 25°C for 16 hours. The reaction mixture was concentrated to remove most solvent. The residue was diluted with water and extracted with ethyl acetate. The combined organic layers were washed with saturated ammonium chloride solution. The organic layers were washed with brine twice, dried over sodium sulphate, filtered, and concentrated under reduced pressure to give a residue. The residue was purified by column chromatography (petroleum ether/ethyl acetate = 50: 1 to 10: 1) to give a crude product. The crude product was triturated with ethyl acetate, filtered and concentrated to give 4-bromo-2,6-bis(bromomethyl)pyridine (30 g, 32% yield) as a white solid.

LC-MS (Method 4): $R_t$ = 0.920 min, MS (ESIpos): m/z =343.8 $[M+H]^+$.

$^1H$ NMR (400 MHz, $CDCl_3$): δ [ppm] = 7.56 (s, 2H), 4.49 (s, 4H).

Step 2

1,1'-[13-bromo-6-oxa-3,9,15-triazabicyclo[9.3.1]pentadeca-1(15), 11,13-triene-3,9-diyl]bis(trifluoroethan-1-one)

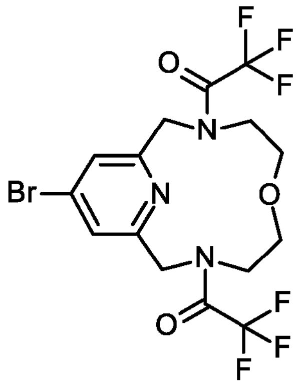

To a mixture of 4-bromo-2,6-bis(bromomethyl)pyridine (27 g, 78.5 mmol) and *N,N'*-[oxydi(ethane-2,1-diyl)]bis(2,2,2-trifluoroacetamide) (23.3 g, 78.5 mmol, intermediate 1 step3) in acetonitrile (2.0 l) was added cesium carbonate (102 g, 314 mmol) at room temperature. The mixture was stirred at room temperature for 12 hours. The mixture was filtered and the filtrate was concentrated to give a crude. The crude product was purified by flash column chromatography (petroleum ether / ethyl acetate, 10% to 100) to give the crude product. The crude product was triturated with a mixture of petroleum ether and ethyl acetate, 5 : 1 as solvent and filtered. The filter cake was dried to give 17.4 g 1,1'-[13-bromo-6-oxa-3,9,15-triazabicyclo[9.3.1]pentadeca-1(15),11,13-triene-3,9-diyl]bis (trifluoroethanone) (46% yield) as white solid.

LC-MS (Method 4): $R_t$ = 0.862 min, MS (ESIpos): m/z =480.1$[M+H]^+$

Step 3

13-bromo-6-oxa-3,9,15-triazabicyclo[9.3.1]pentadeca-1(15),11,13-triene

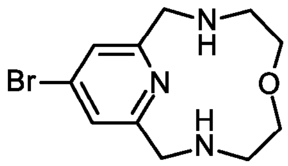

To a solution of 1,1'-[13-bromo-6-oxa-3,9,15-triazabicyclo[9.3.1]pentadeca-1(15),11,13-triene-3,9-diyl]bis(trifluoroethan-1-one) (10.0 g, 20.9 mmol) in methanol (67 ml), tetrahydrofuran (270 ml) and water (170 ml) was added sodium carbonate (22.2 g, 209 mmol) at room temperature. The mixture was stirred at room temperature for 12 hour and concentrated under reduced pressure to remove methanol and THF. The aqueous residue was used directly in the next step.

LC-MS (Method 3): $R_t$ = 0.166 min, MS (ESIpos): m/z =286.1$[M+H]^+$.

Step 4

di-tert-butyl 13-bromo-6-oxa-3,9,15-triazabicyclo[9.3.1]pentadeca-1(15),11,13-triene-3,9-dicarboxylate

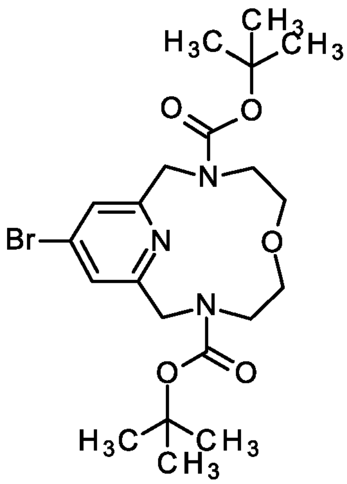

To a solution of 13-bromo-6-oxa-3,9,15-triazabicyclo[9.3.1]pentadeca-1(15),11,13-triene (7.78 g, 27.2 mmol) in tetrahydrofuran (350 ml) and water (220 ml) was added di-tert-butyl dicarbonate(16 ml, 68 mmol) and sodium carbonate (72.0 g, 680 mmol) at 0 °C. The mixture was stirred at room temperature for 12 hours. The mixture was concentrated to give a residue. The residue was diluted with water and extracted with ethyl acetate. The organic phase was washed with brine, dried over anhydrous sodium sulphate and concentrated to give a residue. The crude product was purified by flash column chromatography (petroleum ether/ethyl acetate = 10: 1 to 0: 1) to give di-tert-butyl 13-bromo-6-oxa-3,9,15-triazabicyclo [9.3.1]pentadeca-1(15),11,13-triene-3,9-dicarboxylate (10.8 g, 99% purity, 81% yield) as a colorless oil.

$^1H$ NMR (400 MHz, $CDCl_3$): δ [ppm] = 7.42 (s, 1H), 7.31 (s, 1H), 4.57-4.30 (m, 4H), 3.66-3.38 (m,8H), 1.53-1.32 (m,18H).

Step 5

di-*tert*-butyl 13-(1,3-diethoxy-1,3-dioxopropan-2-yl)-6-oxa-3,9,15-triazabicyclo[9.3.1]pentadeca-1(15),11,13-triene-3,9-dicarboxylate

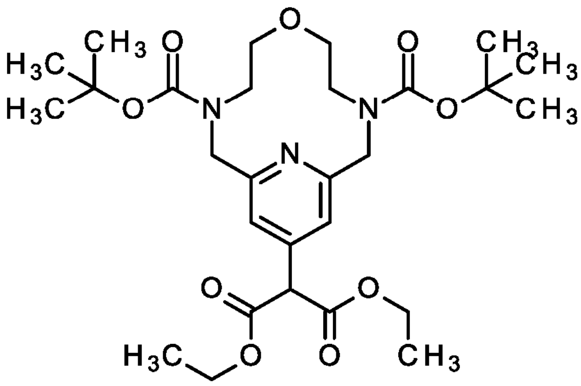

To a solution of di-tert-butyl 13-bromo-6-oxa-3,9,15-triazabicyclo[9.3.1]pentadeca-1(15),11,13-triene-3,9-dicarboxylate (7.60 g, 15.6 mmol), diethyl propanedioate (3.6 ml, 23 mmol), cesium carbonate (7.64 g, 23.4 mmol) in toluene (150 ml) was added methanesulfonato-2-dicyclohexylphosphino-2-(N,N-dimethylamino)biphenyl(2-amino-1,1-biphenyl-2-yl)palladium(II) (1.19 g, 1.56 mmol) at room temperature. The reaction mixture was stirred at 90°C under nitrogen atmosphere. The reaction mixture was diluted with water and extracted with ethyl acetate. The organic phase was washed with water and brine, dried over anhydrous sodium sulphate and concentrated to give a residue. The residue was purified by flash column chromatography (petroleum ether: ethyl acetate = 20: 1 to 3: 2) to give a crude product (6 g). The crude product was purified by reversed phase chromatography (column: spherical C18, 40-60 μm, 120 A ; eluent A: 0.05% hydrochloric acid in water, eluent B: acetonitrile; gradient: 0-40 min 15-40% B; flow 100 ml/min; temperature: room temperature; detector: UV 220/254 nm). The fraction was adjust to pH = 7-8 with saturated sodium bicarbonate solution, then extracted with ethyl acetate and dried under reduced pressure to give di-*tert*-butyl 13-(1,3-diethoxy-1,3-dioxopropan-2-yl)-6-oxa-3,9,15-triazabicyclo[9.3.1]pentadeca-1(15),11,13-triene-3,9-dicarboxylate (3.50 g, 90% purity, 40% yield) as a colourless oil.

LC-MS (Method 4): $R_t$ = 0.509 min; MS (ESIpos): m/z = 566.3 $[M+H]^+$.

$^1H$ NMR (400 MHz, $CD_3OD$): δ [ppm] = 7.27-7.15 (m, 2H), 4.56-4.43 (m, 5H), 4.30-4.10 (m, 4H), 3.65-3.35 (m, 8H), 1.43-1.37 (m, 18H), 1.24 (t, 18H).

Step 6

diethyl [6-oxa-3,9,15-triazabicyclo[9.3.1]pentadeca-1(15), 11,13-trien-13-yl]propanedioate

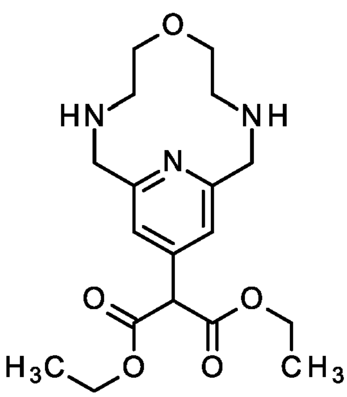

To a solution of di-tert-butyl 13-(1,3-diethoxy-1,3-dioxopropan-2-yl)-6-oxa-3,9,15-triazabicyclo [9.3.1]pentadeca-1(15),11,13-triene-3,9-dicarboxylate (1.7 g, 3.0 mmol) in dioxane (20 ml) was added HCl in dioxane (4M, 3 ml, 12 mmol) and the mixture was stirred at room temperature for 20 h. Additional HCl in dioxane (4M, 1.5 ml, 6 mmol) was added and the mixture was stirred at 50°C for 2 h. HCL in dioxane (4M, 1.5 ml, 6 mmol) addition was repeated and stirring at 50°C was continued for 1 h. The mixture was concentrated under reduced pressure and co-distilled one time with toluene to yield 1.1 g of the title compound as hydrochloride salt.

LC-MS (Method 2): $R_t$ = 0.50 min; MS (ESIpos): m/z = 366.5 $[M+H]^+$.

Step 7

diethyl [3,9-bis(2-methoxy-2-oxoethyl)-6-oxa-3,9,15-triazabicyclo[9.3.1]pentadeca-1(15), 11,13-trien-13-yl]propanedioate

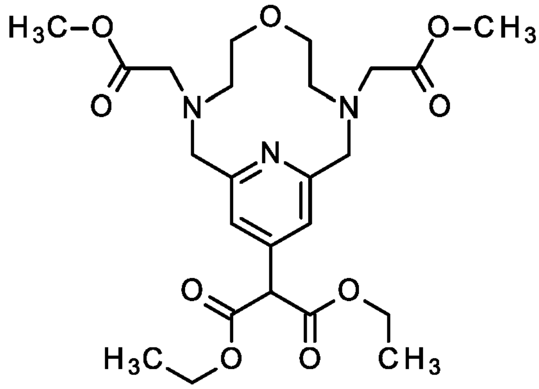

To diethyl [6-oxa-3,9,15-triazabicyclo[9.3.1]pentadeca-1(15),11,13-trien-13-yl] propanedioate (1.10 g, 3.01 mmol) in acetonitrile (65 ml) was added potassium carbonate (2.5 g, 18.1 mmol) and the mixture was stirred at room temperature for 1h. Methyl bromoacetate (840 µl, 9.0 mmol) was added and stirring at room temperature was continued for 21 h. The mixture was filtered, and the filtrate was purified by column chromatography (Biotage® Sfär KP-Amino D 55 g, DCM / MeOH, 0 - 10%) to yield 0.5 g of the title compound.
LC-MS (Method 2): $R_t$ = 0.80 min; MS (ESIpos): m/z = 510 $[M+H]^+$.
$^1H$ NMR (400 MHz, DMSO-$d_6$): δ [ppm] = 1.16 (t, 6H) 2.82 (t, 4H) 3.26 (t, 4H) 3.64 (s, 6H) 3.66 (s, 4H) 3.91 (s, 4H) 4.01 (q, 4H) 7.14 (s, 2H) 11.32 (s, 1H).

Step 8

[3,9-bis(carboxymethyl)-6-oxa-3,9,15-triazabicyclo[9.3.1]pentadeca-1(15), 11,13-trien-13-yl]propanedioic acid

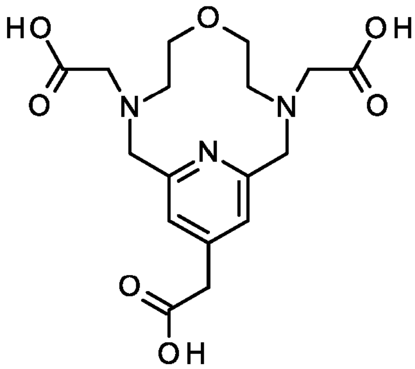

To a suspension of [3,9-bis(2-methoxy-2-oxoethyl)-6-oxa-3,9,15-triazabicyclo[9.3.1] pentadeca-1(15),11,13-trien-13-yl]propanedioate (500 mg, 981 µmol) in THF (12 ml) was added an aqueous sodium hydroxide solution (2M, 2.0 ml, 4.0 mmol) and the mixture was stirred at room temperature for 25 h. The mixture was concentrated under reduced pressure to remove the THF and used in the next step without further purification.
LC-MS (Method 2): $R_t$ = 0.23 min; MS (ESIpos): m/z = 382.2 $[M+H]^+$.

Step 9

manganese(2+) 2,2'-[13-(carboxymethyl)-6-oxa-3,9,15-triazabicyclo[9.3.1] pentadeca-1(15),11,13-triene-3,9-diyl]diacetate

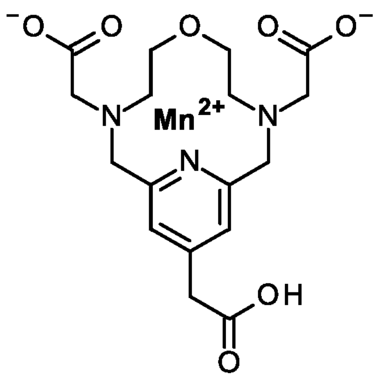

To 2,2',2"-[6-oxa-3,9,15-triazabicyclo[9.3.1]pentadeca-1(15), 11,13-triene-3,9,13-triyl] triacetic acid in an aqueous sodium hydroxide solution (374 mg, 981 μmol) from step 8 was added water (12 ml) and dichloromanganese tetrahydrate (194 mg, 981 μmol). The pH of the mixture was corrected to the value of 6.5 with aqueous hydrochloric acid (250 μl, 4.0 M, 1 mmol) and the mixture was stirred at 95°C for 1h. After cooling to room temperature Chelex®100 was added and the reaction mixture was stirred for 1h. Chelex®100 was removed by filtration and the filtrate was concentrated by lyophilisation. The crude product (537 mg) was purified by column chromatography (Interchim purifFash® PF-15 RP18AQ-F0040, water / acetonitrile, 0 - 20%) to yield 277 mg (98% purity, 64% yield) of the title compound.

LC-MS (Method 1): $R_t$ = 0.21 min; MS (ESIpos): m/z = 435.2 $[M+H]^+$.

<u>**Intermediate 8**</u>

**manganese(2+) (2R,2'S)-2,2'-[13-(carboxymethyl)-6-oxa-3,9,15-triazabicyclo[9.3.1]pentadeca-1(15),11,13-triene-3,9-diyl]dipropanoate**

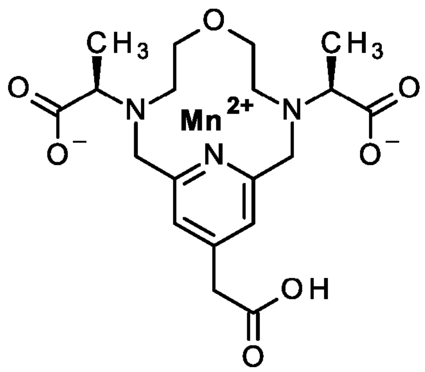

<u>Step 1</u>

diethyl [3,9-bis(1-methoxy-1-oxopropan-2-yl)-6-oxa-3,9,15-triazabicyclo[9.3.1] pentadeca-1(15),11,13-trien-13-yl]propanedioate

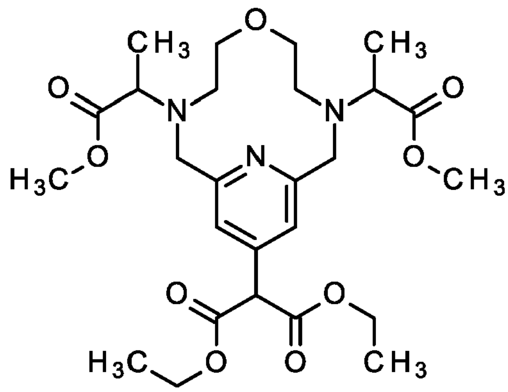

To diethyl [6-oxa-3,9,15-triazabicyclo[9.3.1]pentadeca-1(15),11,13-trien-13-yl]propane dioate hydrogen chloride (1/2) (2.61 g, 5.95 mmol, intermediate 7 step 6) in acetonitrile (60 ml) was added potassium carbonate (4.94 g, 35.7 mmol) and the mixture was stirred at room temperature for 30 minutes. Methyl 2-bromopropanoate (2.7 ml, 24 mmol) was added and stirring at room temperature was continued for 16 h. The mixture was filtered, and the filtrate was purified by column chromatography (Biotage® Sfär KP-Amino D 110 g, DCM / MeOH, 0 - 10%) to yield 3.0 g (98% purity, 92% yield) of the title compound.

LC-MS (Method 2): $R_t$ = 0.92 min; MS (ESIpos): m/z = 538.6 $[M+H]^+$.

$^1$H NMR (400 MHz, DMSO-$d_6$): δ [ppm] = 11.38 (d, 1H), 7.16 (d, 2H), 4.00 (q, 4H), 3.91 (d, 1H), 3.71 -3.82 (m, 5H), 3.65 (s, 3H), 3.63 (s, 3H), 3.20 - 3.50 (m, 4H), 2.74 - 2.87 (m, 4H), 1.25 (dd, 6H), 1.15 (t, 6H).

Step 2

2,2'-[13-(carboxymethyl)-6-oxa-3,9,15-triazabicyclo[9.3.1]pentadeca-1(15),11,13-triene-3,9-diyl]dipropanoic acid

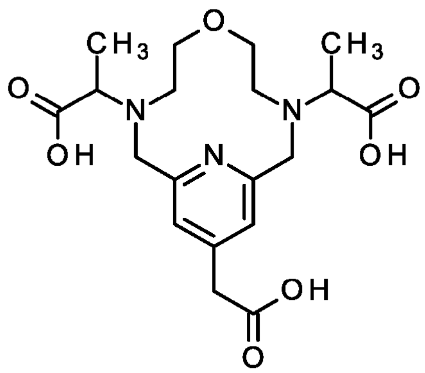

To a suspension of diethyl [3,9-bis(1-methoxy-1-oxopropan-2-yl)-6-oxa-3,9,15-triazabicyclo[9.3.1] pentadeca-1(15),11,13-trien-13-yl]propanedioate (3.00 g, 5.58 mmol) in THF (35 ml) was added an aqueous sodium hydroxide solution (2M, 11 ml, 22 mmol) and the mixture was stirred at room temperature for 18 h. Additional aqueous sodium hydroxide solution (2M, 3.0 ml, 6 mmol) was added and the mixture was stirred at room temperature for additional 22 h The mixture was concentrated under reduced pressure to remove the THF and used in the next step without further purification.

LC-MS (Method 1): $R_t$ = 0.39 min; MS (ESIneg): m/z = 408.2 $[M-H]^-$.

Step 3

**manganese(2+) (2R,2'S)-2,2'-[13-(carboxymethyl)-6-oxa-3,9,15-triazabicyclo[9.3.1]pentadeca-1 (15),11,13-triene-3,9-diyl]dipropanoate**

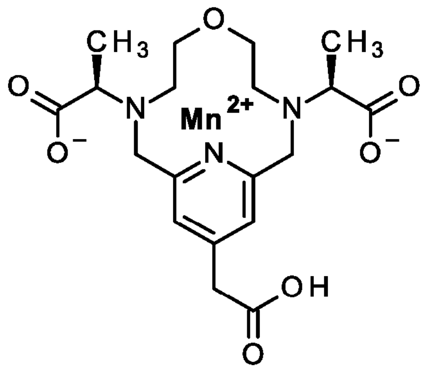

To 2,2'-[13-(carboxymethyl)-6-oxa-3,9,15-triazabicyclo[9.3.1]pentadeca-1(15),11,13-triene-3,9-diyl]dipropanoic acid in an aqueous sodium hydroxide solution (2.28 g, 5.57 mmol) from step 2 was added water (15 ml) and dichloromanganese tetrahydrate (1.10 g, 5.57 mmol). The pH of the mixture was corrected to the value of pH 8 with aqueous hydrochloric acid (2M) and the mixture was stirred at 95°C for 3h. After cooling to room temperature Chelex®100 was added and the reaction mixture was stirred for 1h. Chelex®100 was removed by filtration and the pH of the filtrate was adjusted pH 8. The crude mixture of isomers in water was purified by column chromatography (Interchim purifFash® PF-15 RP18 AQ-F0330, water / acetonitrile, 0 - 20%) to yield 218 mg (98% purity, 8% yield) of the title compound (meso-compound, 98% purity, 8% yield) and 948 mg (98% purity, 36% yield) of manganese(2+) (2R*,2'R*)-2,2'-[13-(carboxymethyl)-6-oxa-3,9,15-triazabicyclo[9.3.1]pentadeca-1(15),11,13-triene-3,9-diyl]dipropanoate (intermediate 9).

LC-MS (Method 1): $R_t$ = 0.21 min; MS (ESIpos): m/z = 435.2 $[M+H]^+$.

__Intermediate 9__

**manganese(2+) (2R*,2'R*)-2,2'-[13-(carboxymethyl)-6-oxa-3,9,15-triazabicyclo[9.3.1]pentadeca-1(15),11,13-triene-3,9-diyl]dipropanoate**

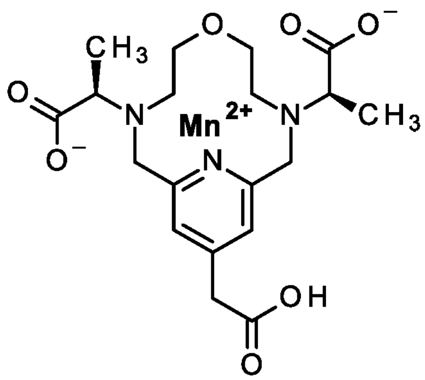

The racemic title compound was isolated as second component from the synthesis of intermediate 8 in step 3.

LC-MS (Method 1): $R_t$ = 0.24 min; MS (ESIpos): m/z = 448.3 $[M+H]^+$

__Intermediate 10__

**manganese(2+) (2R*,2'R*)-2,2'-[12-carboxy-6-oxa-3,9,15-triazabicyclo[9.3.1]pentadeca-1 (15), 11,13-triene-3,9-diyl]dipropanoate**

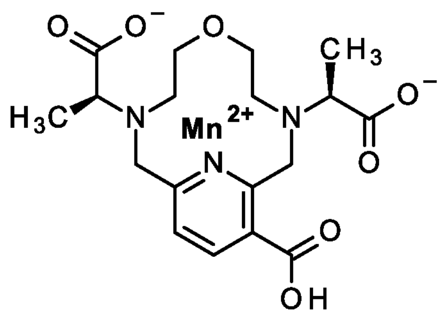

Step 1

methyl 3,9-bis(1-methoxy-1-oxopropan-2-yl)-6-oxa-3,9,15-triazabicyclo[9.3.1] pentadeca-1 (15),11,13-triene-12-carboxylate

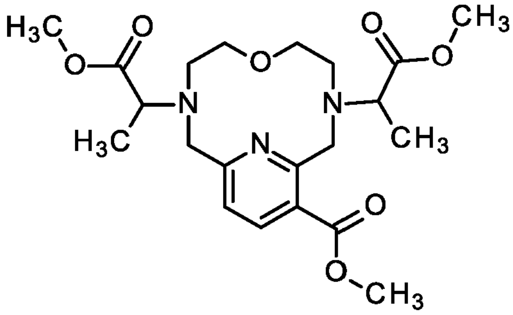

To methyl 6-oxa-3,9,15-triazabicyclo[9.3.1]pentadeca-1(15), 11,13-triene-12-carboxylate hydrogen chloride (1/2) (2.5 g, 5.9 mmol, intermediate 5 step 7) in acetonitrile (50 ml) was added potassium carbonate (4.09 g, 29.6 mmol) and the mixture was stirred at room temperature for 30 minutes. Methyl bromoacetate (2.72 g, 16.3 mmol) was added and the mixture was stirred at room temperature for 19 h. The mixture filtered and the filtrate concentrated under reduced pressure. Water was added and the mixture was extracted with dichloromethane. The organic phase was washed with brine, dried over anhydrous sodium sulphate, filtered, and concentrated under reduced pressure. The crude product (3.23 g, quant.) was used without further purification for the next step.

LC-MS (Method 2): $R_t$ = 0.75 and 0.76 min; MS (ESIpos): m/z = 438.4 $[M+H]^+$.

Step 2

3,9-bis(1-carboxyethyl)-3-(1-carboxyethyl)-6-oxa-3,9,15-triazabicyclo[9.3.1]pentadeca-1(15), 11,13-triene-12-carboxylic acid

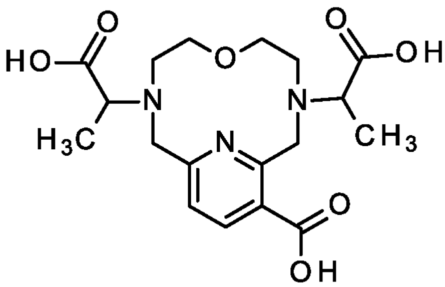

To methyl 3,9-bis(1-methoxy-1-oxopropan-2-yl)-6-oxa-3,9,15-triazabicyclo[9.3.1] pentadeca-1(15),11,13-triene-12-carboxylate (3.23 g, 7.38 mmol) in THF (50 ml) was added an aqueous sodium hydroxide solution (2.5 M, 15 ml, 37.5 mmol) and the mixture was stirred at room temperature for 1 h. The mixture was concentrated under reduced

pressure to remove the THF and used in the next step without further purification.

LC-MS (Method 1): $R_t$ = 0.22 min; MS (ESIpos): m/z = 396.3 $[M+H]^+$.

Step 3

manganese(2+) (2*R**,2'*R**)-2,2'-[12-carboxy-6-oxa-3,9,15-triazabicyclo[9.3.1]pentadeca-1(15), 11,13-triene-3,9-diyl]dipropanoate

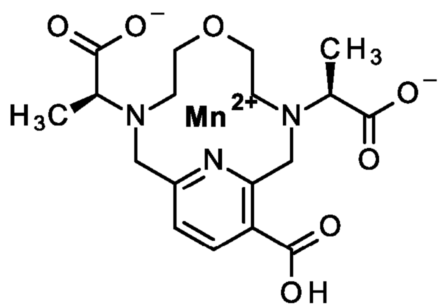

To 3,9-bis(1-carboxyethyl]-6-oxa-3,9,15-triazabicyclo[9.3.1]pentadeca-1(15),11,13-triene-13-carboxylic acid (2.92 g, 7.38 mmol) in an aqueous sodium hydroxide solution from step 2 was added water (50 ml) and manganese(2+) dichloride (1.02 g, 8.1 mmol). The pH of the suspension was corrected to the value of 7 with aqueous sodium hydroxide (2.0 M) and the mixture was stirred at 100°C for 10 h. After cooling to room temperature Chelex®100 was added and the reaction mixture was stirred for 1 h. Chelex®100 was removed by filtration and the aqueous solution was purified by column chromatography (Interchim puriFlash® PF-15 RP18AQ-F00330, water/ acetonitrile, 0 - 20%) to yield 1.18 g (95% purity, 34% yield) of the racemic title compound (intermediate 10) and 1.55 g of manganese(2+) (2R*,2'S*)-2,2'-[12-carboxy-6-oxa-3,9,15-triazabicyclo[9.3.1]pentadeca-1(15),11,13-triene-3,9-diyl]dipropanoate (intermediate 11).

LC-MS (Method 1): $R_t$ = 0.25 min; MS (ESIpos): m/z = 449.2 $[M+H]^+$.

**Intermediate 11**

**manganese(2+) (2R*,2'S*)-2,2'-[12-carboxy-6-oxa-3,9,15-triazabicyclo[9.3.1]pentadeca-1 (15), 11,13-triene-3,9-diyl]dipropanoate**

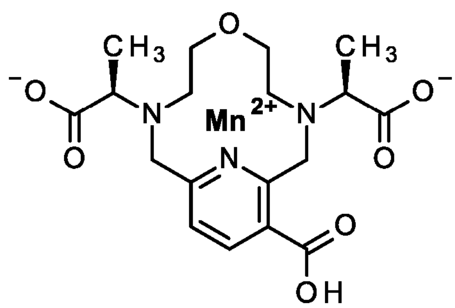

The racemic title compound was isolated as second component from the synthesis of intermediate 10 in step 3.

LC-MS (Method 1): $R_t$ = 0.26 min; MS (ESIpos): m/z = 449.2 $[M+H]^+$ .

**Intermediate 12**

**manganese(2+) 2,2'-[13-(2-carboxyethyl)-6-oxa-3,9,15-triazabicyclo [9.3.1]pentadeca-1 (15), 11,13-triene-3,9-diyl]dipropanoate**

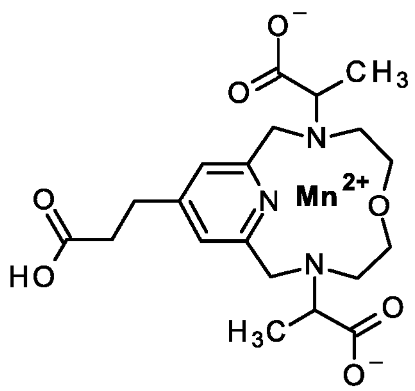

Step 1

di-tert-butyl 13-[(1E)-3-methoxy-3-oxoprop-1-en-1-yl]-6-oxa-3,9,15-triazabicyclo[9.3.1] pentadeca-1(15), 11,13-triene-3,9-dicarboxylate

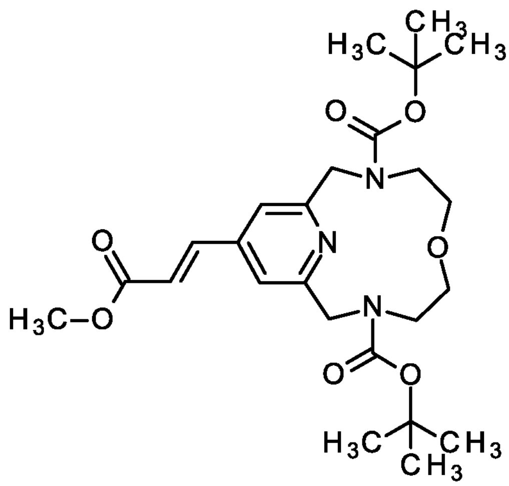

To a solution of di-tert-butyl 13-bromo-6-oxa-3,9,15-triazabicyclo[9.3.1]pentadeca-1(15),11,13-triene-3,9-dicarboxylate (9.5 g, 19.5 mmol, intermediate 7 step 4) and methyl prop-2-enoate (8.8 ml, 98 mmol) in *N*,*N*-dimethylacetamide (150 ml) were added palladium(II) acetate (219 mg, 977 μmol), tri-2-tolylphosphine (594 mg, 1.95 mmol) and N,N-diisopropylethylamine (10 ml, 59 mmol) at room temperature. The mixture was stirred at 130 °C for 12 hours under nitrogen atmosphere. The mixture was poured into water and extracted with ethyl acetate. The organic phase was washed with brine, dried over anhydrous sodium sulphate, filtered and concentrated to give a residue. The residue was purified by column chromatography (1000 mesh, petroleum ether: ethyl acetate = 4: 1 to 1: 2) and reversed phase column (Instrument: Agela HP1000; Column: Welch Ultimate XB_C18 150*400mm 20/40μm; eluent A: 0.1% formic acid in water, eluent B: acetonitrile; gradient: 0-40 min 30-80% B; flow 100 ml/min; temperature: room temperature; Detector: UV 220/254 nm) to give di-tert-butyl 13-[(1E)-3-methoxy-3-oxoprop-1-en-1-yl]-6-oxa-3,9,15-triazabicyclo[9.3.1]pentadeca-1(15),11,13-triene-3,9-dicarboxylate (8.1 g, 84% yield) as yellow oil.

LC-MS (Method 4): $R_t$ = 0.792 min; MS (ESIpos): m/z = 492.3 $[M+H]^+$.

Step 2

di-tert-butyl 13-(3-methoxy-3-oxopropyl)-6-oxa-3,9,15-triazabicyclo[9.3.1]pentadeca-1(15), 11,13-triene-3,9-dicarboxylate

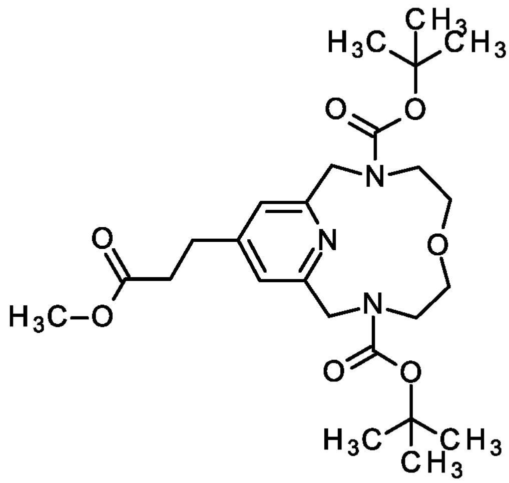

To a solution of di-tert-butyl 13-[(1E)-3-methoxy-3-oxoprop-1-en-1-yl]-6-oxa-3,9,15-triazabicyclo[9.3.1]pentadeca-1(15),11,13-triene-3,9-dicarboxylate (8.1 g, 16.5 mmol) in methanol (100 ml) were added palladium on carbon (1.0 g, 10%) at room temperature. The mixture was stirred at room temperature for 12 hours under hydrogen atmosphere (30 psi). The mixture was filtered and The filtrate was concentrated to give di-tert-butyl 13-(3-methoxy-3-oxopropyl)-6-oxa-3,9,15-triazabicyclo[9.3.1]pentadeca-1(15),11,13-triene-3,9-dicarboxylate (6.78 g, 92% purity, 77% yield) as yellow oil.

LC-MS (Method 4): $R_t$ = 0.788 min; MS (ESIpos): m/z = 494.3 $[M+H]^+$.

$^1H$ NMR (400 MHz, DMSO-$d_6$): δ [ppm] = 7.14-6.94 (m, 2H), 4.42 (d, 2H), 4.34 (d, 2H), 3.75-3.50 (m, 7H), 3.46-3.33 (m, 4H), 2.84 (t, 2H), 2.66 (t, 2H), 1.45-1.32 (m, 9H), 1.27 (s, 9H).

Step 3

methyl 3-[6-oxa-3,9,15-triazabicyclo[9.3.1]pentadeca-1(15),11,13-trien-13-yl]propanoate

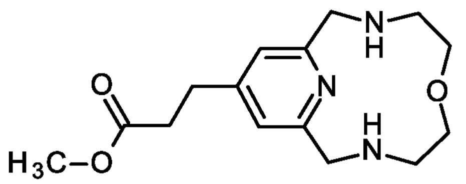

To a solution of di-tert-butyl 13-(3-methoxy-3-oxopropyl)-6-oxa-3,9,15-triazabicyclo [9.3.1]pentadeca-1(15),11,13-triene-3,9-dicarboxylate (1.81 g, 3.67 mmol) in 1,4-dioxane (24 ml) was added hydrochloric acid in dioxane (4.0 M, 3.7 ml, 15 mmol and the mixture was stirred at room temperature for 3 days. The mixture was concentrated under reduced pressure, co-distilled one time with toluene, and used in the next step without further purification.

LC-MS (Method 2): $R_t$ = 0.24 min; MS (ESIpos): m/z = 294.2 $[M+H]^+$.

Step 4

dimethyl 2,2'-[13-(3-methoxy-3-oxopropyl)-6-oxa-3,9,15-triazabicyclo[9.3.1]pentadeca-1(15), 11,13-triene-3,9-diyl]dipropanoate

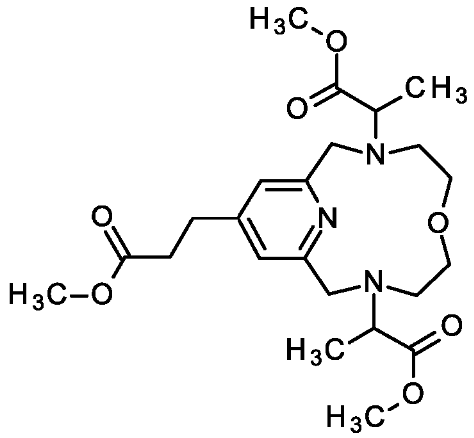

To a suspension of methyl 3-[6-oxa-3,9,15-triazabicyclo[9.3.1]pentadeca-1(15), 11,13-trien-13-yl]propanoate (crude product, 1.08 g, 3.67 mmol) in acetonitrile (25 ml) was added potassium carbonate (3.05 g, 22.1 mmol) and the mixture was stirred for 1 h at room temperature. Methyl 2-bromopropanoate (1.0 ml, 9.2 mmol) was added and the mixture was stirred at room for 40 h. Additional methyl 2-bromopropanoate (205 μl, 1.9 mmol) was added and stirring at room temperature was continued for 20 h. Potassium carbonate was filtered off and the filtrate was concentrated under reduced pressure. The residue was dissolved in DCM and washed with water and brine. The organic phase was dried by filtration through a water repellent filter and concentrated under reduced pressure to give the crude title compound (1.78 g) as mixture of isomers.

LC-MS (Method 2): $R_t$ = 0.79 min; MS (ESIpos): m/z = 466.4 $[M+H]^+$.

$^1H$ NMR (400 MHz, DMSO-$d_6$): δ [ppm] = 1.30 (dd, 3H), 1.34 (dd, 3H), 2.61 - 2.79 (m, 4H), 2.68 (t, 2H), 2.84 (t, 2H), 3.56 (s, 3H) 3.50 - 3.92 (m, 6H) 3.71 (s, 3H), 3.73 (s, 3H), 7.08-7.12 (m, 2H).

Step 5

2,2'-[13-(2-carboxyethyl)-6-oxa-3,9,15-triazabicyclo[9.3.1]pentadeca-1(15), 11,13-triene-3,9-diyl]dipropanoic acid

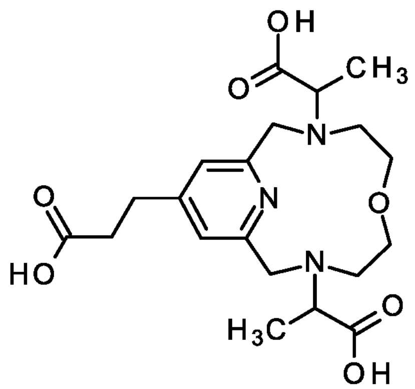

To a suspension of dimethyl 2,2'-[13-(3-methoxy-3-oxopropyl)-6-oxa-3,9,15-triazabicyclo [9.3.1]pentadeca-1(15),11,13-triene-3,9-diyl]dipropanoate (crude product, 1.78 g, 3.67 mmol) in THF (16.5 ml) was added aqueous sodium hydroxide solution (2M, 7.5 ml, 15 mmol) and the mixture was stirred at room temperature for 24 h. The mixture was concentrated under reduced pressure to remove the THF and used in the next step without further purification.

LC-MS (Method 2): $R_t$ = 0.24 min; MS (ESIpos): m/z = 424.3 $[M+H]^+$.

Step 6

manganese(2+) 2,2'-[13-(2-carboxyethyl)-6-oxa-3,9,15-triazabicyclo[9.3.1]pentadeca-1(15),11,13-triene-3,9-diyl]dipropanoate (only one group of and/or stereo is supported now.)

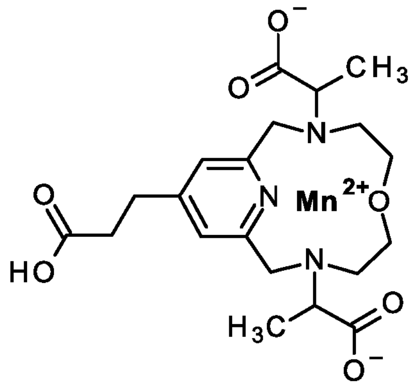

To 2,2'-[13-(2-carboxyethyl)-6-oxa-3,9,15-triazabicyclo[9.3.1]pentadeca-1(15),11,13-triene-3,9-diyl]dipropanoic acid (1.55 g, 3.83 mmol) in an aqueous sodium hydroxide solution from step 5 was added water. (16 ml) and manganese(II)chloride tetrahydrate (833 mg, 4.21 mmol). The pH was adjusted to pH6.5 by addition of aqueous hydrochloric acid (4M, 1.25 ml, 5 mmol) and the solution was stirred at 100°C. for 1 h. The mixture was cooled to room temperature and Chelex®100 was added and the mixture was stirred at room temperature for 1h. Chelex®100 was filtered off and the filtrate was concentrated under reduced pressure. The crude product was purified by column chromatography (Interchim puriflash PF-15RP18AQ-F0330, water/ acetonitrile, 0 - 20%) to yield 720mg of the title compound (98% purity, 39% yield).

LC-MS (Method 1): $R_t$ = 0.39 min; MS (ESIpos): m/z = 477 $[M+H]^+$.

## Intermediate 13

**manganese(2+) 2,2'-(13-carboxy-4-methyl-6-oxa-3,9,15-triazabicyclo [9.3.1]pentadeca-1 (15), 11,13-triene-3,9-diyl)diacetate**

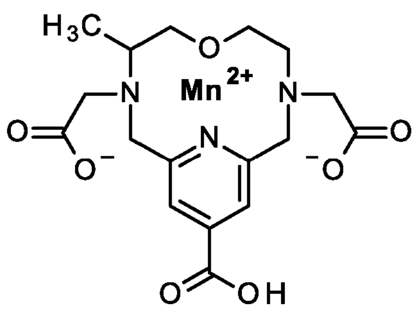

Step 1

1-[2-(benzyloxy)ethoxy]propan-2-ol

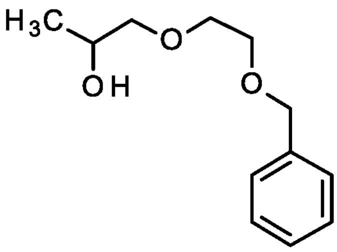

To a mixture of 2-(benzyloxy)ethan-1-ol (210 g, 1.38 mol) and boron trifluoride diethyl etherate (3.5 ml, 28 mmol) in dichloromethane (2000 ml) was dropwise added a solution of 2-methyloxirane (64.1 g, 1.10 mol) in dichloromethane

(100 ml) at 0 °C. The reaction mixture was stirred at 0 °C for 1 hour. The reaction mixture was stirred at room temperature for 16 hour. The reaction mixture was concentrated to give a crude product. The crude product was purified by reversed phase column (Welch Ultimate XB_C18, 20-40 µm, 120 A ; eluent A: 0.1% formic acid in water, eluent B: acetonitrile; gradient: 0-60 min 35-50% B; flow 400 ml/min; temperature: room temperature; Detector: UV 220/254 nm) to give to give 1-[2-(benzyloxy)ethoxy]propan-2-ol (84.0 g, 29% yield) as colourless oil.

$^1$H NMR (400 MHz, $CDCl_3$): δ [ppm] = 7.40-7.27 (m, 5H), 4.59 (s, 2H), 4.07-3.92 (m, 1H), 3.89-3.42 (m, 6H), 3.35-3.22 (m, 1H), 1.23-1.03 (m, 3H).

Step 2

1-(2-hydroxyethoxy)propan-2-ol

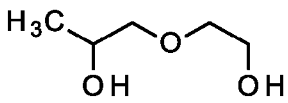

Flow chemistry: 1-[2-(Benzyloxy)ethoxy]propan-2-ol (84 g, 399 mmol) in methanol (2000 ml) was stirred at 25°C until it formed a clear solution.. The clear solution was pumped through a fixed bed reactor made of stainless-steel tubing with the dimensions 60 cm x ½ inch filled completely with 5% palladium on activated carbon and palladium on activated carbon/Aluminium oxide. The hydrogen back pressure regulator of the reactor was adjusted to 2.5 MPa and the flow rate of hydrogen was 100 ml/min while the reactor was heated to 70°C by an oil bath. The solution was pumped into the reactor at a flow rate of 2.5 ml/min. After the reaction was finished, the tubing was washed with methanol (500 ml). The reaction and wash solution was combined and concentrated to give 1-(2-hydroxyethoxy)propan-2-ol (45 g, 94% yield) as colourless oil.

$^1$H NMR (400 MHz, $CDCl_3$): δ [ppm] = 4.09-3.92 (m, 1H), 3.85-3.43 (m, 6H), 3.41-3.17 (m, 2H), 1.24-1.02 (m, 3H).

Step 3

1-{2-[(4-methylbenzene-1-sulfonyl)oxy]ethoxy}propan-2-yl 4-methylbenzene-1-sulfonate

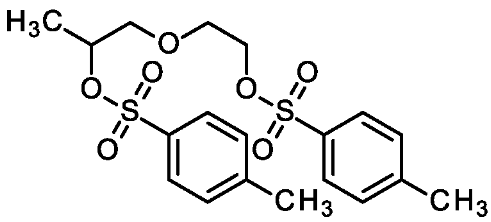

To a solution of 1-(2-hydroxyethoxy)propan-2-ol (42 g, 350 mmol) in dichloromethane (2.8 l) was added 4-(dimethylamino)pyridine (4.27 g, 35 mmol), pyridine (169 ml, 2.1 mol) and 4-toluenesulfonyl chloride (266 g, 1.4 mol) at 0°C. The mixture was stirred at room temperature for 16 hours. Then to the mixture was added pyridine (85 ml, 1.05 mol) and 4-toluenesulfonyl chloride (133 g, 699 mmol) at 0°C. After stirring at room temperature for 16 hour and 25 °C for 16 hours, the reaction mixture was diluted with dichloromethane (1.0 l) and washed with saturated ammonium chloride solution and brine. The organic layer was concentrated to give a crude product. The crude product was purified by flash column chromatography (petroleum ether: ethyl acetate = 20: 1 to 1: 1) to give 123 g of the title compound (82% yield) as yellow oil.

$^1$H NMR (400 MHz, $CDCl_3$): δ [ppm] = 7.88-7.69 (m, 4H), 7.46-7.29 (m, 4H), 4.09-3.98 (m, 2H), 3.93-3.83 (m, 1H), 3.70-3.34 (m, 4H), 2.45 (s, 6H), 1.24-1.02 (m, 3H).

Step 4

2-{1-[2-(1, 3-dioxo-1, 3-dihydro-2H-isoindol-2-yl)ethoxy]propan-2-yl}-1H-isoindole-1, 3 (2H)-dione

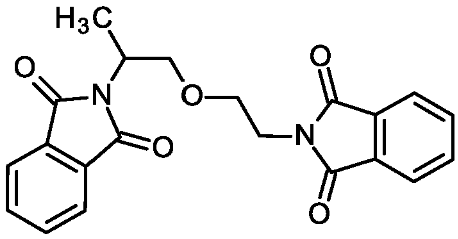

A mixture of 1-{2-[(4-methylbenzene-1-sulfonyl)oxy]ethoxy}propan-2-yl 4-methylbenzene-1-sulfonate (123 g, 287 mmol) and potassium 1, 3-dioxo-1, 3-dihydroisoindol-2-ide (117 g, 631 mmol) in *N*,*N*-dimethylformamide (2.5 l) was stirred at 100°C for 16 hours The mixture was diluted with ethyl acetate (500 ml) and washed with brine (500 ml). The organic phase was dried with anhydrous sodium sulphate, filtered and concentrated to give a residue. The residue was purified by column chromatography on silica gel (1000 mesh, petroleum ether: ethyl acetate = 10: 1 to 1: 1) to give a crude, the crude was concentrated and triturated with a mixture solvent (30 ml) (petroleum ether: ethyl acetate = 1: 3) and filtered to give 2-{1-[2-(1, 3-dioxo-1, 3-dihydro-2H-isoindol-2-yl)ethoxy]propan-2-yl}-1H-isoindole-1, 3 (2H)-dione (52 g, 48% yield) as a white solid.

LC-MS (Method 4): $R_t$ = 0.853 min; MS (ESIpos): m/z = 379.2 $[M+H]^+$.

$^1$H NMR (400 MHz, DMSO-$d_6$): δ [ppm] = 7.80-7.70 (m, 4H), 7.66-7.56 (m, 4H), 3.84 (t, 1H), 3.75-3.42 (m, 6H), 1.26 (d, 3H).

Step 5

1-(2-aminoethoxy)propan-2-amine - hydrogen chloride (1/2)

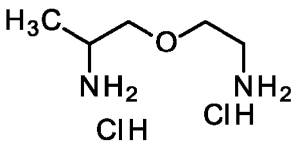

A solution of 2-{1-[2-(1, 3-dioxo-1, 3-dihydro-2H-isoindol-2-yl)ethoxy]propan-2-yl}-1H-isoindole-1, 3 (2H)-dione (52 g, 137 mmol) in dioxane (300 ml) and hydrochloric acid (400 ml, 12 M in dioxane) was stirred at 100 °C for 4 days. The mixture was concentrated to remove dioxane and diluted with water (150 ml). The mixture was washed with ethyl acetate. The aqueous layer was after phase separation adjusted to pH 9 with sodium carbonate (solid) and used directly in the next step.

Step 6

2-nitro-*N*-[1-{2-[(2-nitrobenzene-1-sulfonyl)amino]ethoxy}propan-2-yl]benzene-1-sulfonamide

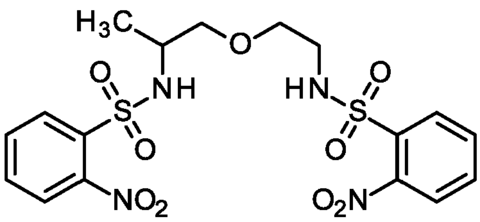

To a solution of 1-(2-aminoethoxy)propan-2-amine - hydrogen chloride (1/2) (26.3 g, 137 mmol) in tetrahydrofuran (150 ml) and water (150 ml) were added sodium carbonate (58 g, 0.55 mol) and 2-nitrobenzene-1-sulfonyl chloride (82.3 g, 372 mmol) at 0°C. The mixture was stirred at room temperature for 12 hours. The mixture was diluted with water and extracted with ethyl acetate. The organic phase was washed with brine (300 ml), dried with anhydrous sodium sulphate, filtered, and concentrated to give a residue. The residue was purified by column chromatography on silica gel (1000 mesh, petroleum ether: ethyl acetate = 8: 1 to 1: 1) to give a crude. Then the crude was triturated with a mixture

solvent (petroleum ether: ethyl acetate = 1: 1) and filtered to give 2-nitro-*N*-[1-(2-{[(2-nitrophenyl)sulfonyl]amino}ethoxy) propan-2-yl]benzenesulfonamide (42 g) as a white solid. The mother liquor was concentrated and purified by reversed phase column (Welch Ultimate XB_C18 20-40μm; 120 A; eluent A: 0.1% formic acid in water, eluent B: acetonitrile; gradient: 0-60 min 35-45% B; flow 200 ml/min; temperature: room temperature; Detector: UV 220/254 nm) to give additional 2-nitro-/V-[1-(2-{[(2-nitrophenyl)sulfonyl]amino}ethoxy)propan-2-yl]benzene-1-sulfonamide (17 g) as colourless oil.

$^1$H NMR (400 MHz, $CDCl_3$): δ [ppm] = 8.26-8.20 (m, 2H), 7.97-7.63 (m, 6H), 5.78-5.44 (m, 2H), 3.71-3.11 (m, 6H), 3.00-2.77 (m, 1H), 1.19-0.95 (m, 3H).

Step 7

methyl 4-methyl-3,9-bis(2-nitrobenzene-1-sulfonyl)-6-oxa-3, 9, 15-triazabicyclo[9.3.1] pentadeca-1 (15), 11, 13-triene-13-carboxylate

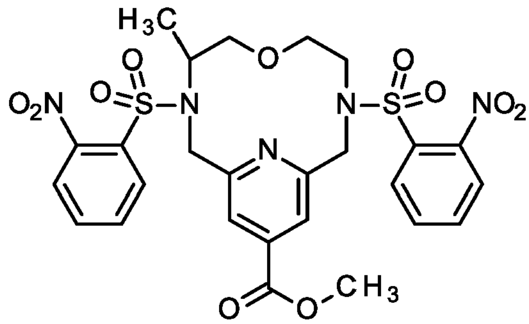

To a solution of 2-nitro-*N*-[1-{2-[(2-nitrobenzene-1-sulfonyl)amino]ethoxy}propan-2-yl]benzene-1-sulfonamide (21 g, 43 mmol) in acetonitrile (2.1 l) were added sodium carbonate (18 g, 172 mmol) and methyl 2, 6-bis (bromomethyl) pyridine-4-carboxylate (13.9 g, 43.0 mmol, intermediate 1 step 2) at room temperature. The mixture was stirred at 80°C for 48 hours. The mixture was combined with an identically prepared 21 g batch was filtered and the filtrate was concentrated to give a residue. The residue was purified by column chromatography on silica gel (1000 mesh, petroleum ether: ethyl acetate = 8: 1 to 1: 1) to give a crude. The crude was trituration with methanol (50 ml) and filtered to give the methyl 4-methyl-3, 9-bis[ (2-nitrophenyl)sulfonyl]-6-oxa-3,9,15-triazabicyclo [9.3.1]pentadeca-1 (15), 11, 13-triene-13-carboxylate (31 g) as a white solid.

LC-MS (Method 4): $R_t$ = 0.976 min; MS (ESIpos): m/z = 650.2 $[M+H]^+$.

$^1$H NMR (400 MHz, $CDCl_3$): δ [ppm] = 8.18 (t, 2H), 7.88-7.63 (m, 8H), 4.83-4.69 (m, 2H), 4.65-4.45 (m, 2H), 4.04-3.90 (m, 3H), 3.75-3.55 (m, 2H), 3.54-3.18 (m, 4H), 3.15-2.88 (m, 2H), 0.95 (d, 3H).

Step 8

Methyl 4-methyl-6-oxa-3, 9, 15-triazabicyclo[9.3.1]pentadeca-1 (15),11,13-triene-13-carboxylate

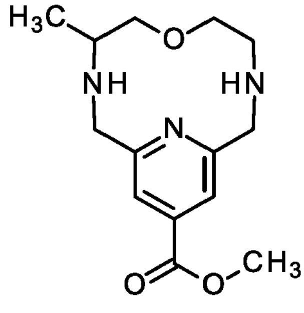

To a solution of methyl 4-methyl-3, 9-bis(2-nitrobenzene-1-sulfonyl)-6-oxa-3, 9, 15-triazabicyclo[9.3.1]pentadeca-1 (15), 11, 13-triene-13-carboxylate (31 g, 47.7 mmol) in acetonitrile (350 ml) were added potassium carbonate (39.6 g, 286 mmol) and 4-methoxybenzene-1-thiol (26.65 g, 191 mmol) at room temperature. The mixture was stirred at 40°C for 12 hours. Additional 4-methoxybenzene-1-thiol (26.65 g, 191 mmol) was added at room temperature and stirring at 40°C was continued for another 16 hours. The mixture was filtered, and the filtrate was concentrated to give a residue. The residue was diluted with water and adjust pH~2 with hydrochloric acid (2 M). The mixture was extracted with ethyl acetate (200 ml). After phase separation the aqueous layer was adjusted to pH 8 with sodium hydrogen carbonate at 0°C and used directly in the next step (13.3 g, 100% theoretical yield).

LC-MS (Method 3): $R_t$ = 0.131 min; MS (ESIpos): m/z = 280.2 $[M+H]^+$.

Step 9

3,9-di-*tert*-butyl 13-methyl 4-methyl-6-oxa-3,9,15-triazabicyclo[9.3.1]pentadeca-1(15),11,13-triene-3, 9, 13-tricarboxylate

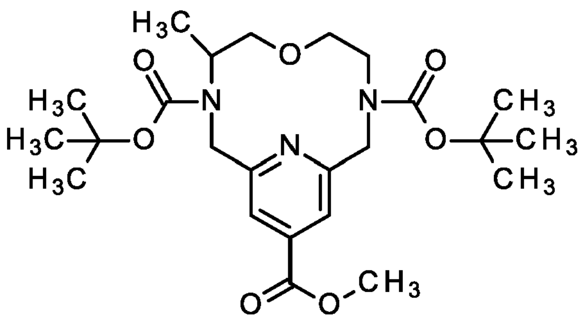

To a solution of methyl 4-methyl-6-oxa-3, 9, 15-triazabicyclo[9.3.1]pentadeca-1 (15), 11, 13-triene-13-carboxylate (13.3 g, 47.6 mmol, raw from step 8) in tetrahydrofuran (170 ml) and water (170 ml) were added sodium hydrogen carbonate (16 g, 190 mmol) and di-tert-butyl dicarbonate (22 ml, 95 mmol) at 0 °C. The mixture was stirred at room temperature for 2 hours. The mixture was diluted with water and extracted with ethyl acetate. The organic phase was washed with brine, dried with anhydrous sodium sulphate, filtered and concentrated to give a residue. The residue was purified by column chromatography on silica gel (100-200 mesh, petroleum ether: ethyl acetate = 10: 1 to 1: 1) to give 3, 9-di-tert-butyl 13-methyl 4-methyl-6-oxa-3, 9, 15-triazabicyclo[9.3.1]pentadeca-1 (15), 11, 13-triene-3, 9, 13-tricarboxylate (7.60 g, 33% yield) as colourless oil.

LC-MS (Method 3): $R_t$ = 0.974 min; MS (ESIpos): m/z = 480.4 $[M+H]^+$.

$^1$H NMR (400 MHz, $CDCl_3$): $\delta$ [ppm] = 8.02-7.55 (m, 2H), 4.85-4.35 (m, 4H), 4.05-3.90 (m, 3H), 3.86-3.16 (m, 7H), 1.80-1.37 (m, 18H), 1.22-0.84 (m, 3H).

Step 10

methyl 4-methyl-6-oxa-3, 9, 15-triazabicyclo[9.3.1]pentadeca-1 (15), 11, 13-triene-13-carboxylate - hydrogen chloride (1/2)

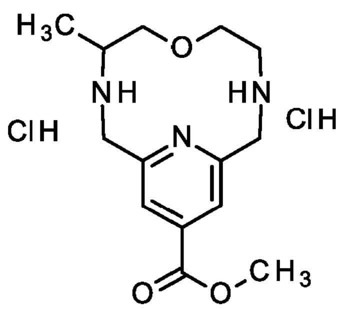

A solution of 3,9-di-tert-butyl 13-methyl 4-methyl-6-oxa-3, 9, 15-triazabicyclo[9.3.1] pentadeca-1 (15), 11, 13-triene-3, 9, 13-tricarboxylate (8.40 g, 17.5 mmol) in hydrochloric acid (150 ml, 2M in dioxane) was stirred at room temperature for 12 hours. The reaction mixture was concentrated to give methyl -4-methyl-6-oxa-3, 9, 15-triazabicyclo [9.3.1] pentadeca-1 (15), 11, 13-triene-13-carboxylate - hydrogen chloride (1/2) (7.8 g) and used directly in the next step.

LC-MS (Method 3): $R_t$ = 0.124 min; MS (ESIpos): m/z = 280.2 $[M+H]^+$.

Step 11

methyl 3, 9-bis (2-tert-butoxy-2-oxoethyl)-4-methyl-6-oxa-3, 9, 15-triazabicyclo[9.3.1]pentadeca -1 (15), 11, 13-triene-13-carboxylate

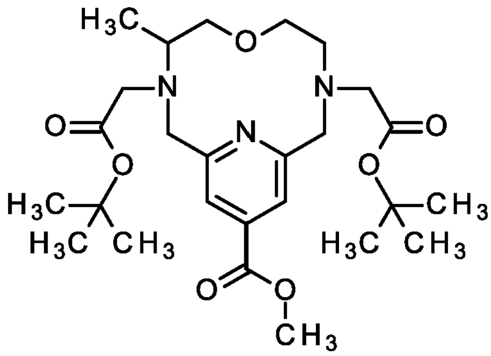

To a solution of methyl 4-methyl-6-oxa-3, 9, 15-triazabicyclo[9.3.1]pentadeca-1 (15), 11, 13-triene-13-carboxylate - hydrogen chloride (1/2) (7.80 g, 22.1 mmol) in acetonitrile (200 ml) was added potassium carbonate (30.6 g, 221 mmol) at room temperature. The mixture was stirred at room temperature for 0.5 hour. Then to the mixture was added tert-butyl bromoacetate (10.8 g, 55.4 mmol) at room temperature. The mixture was stirred at room temperature for 12 hours. The mixture was filtered, and the filtrate was concentrated to give a residue. The residue was purified by reversed phase chromatography (Column: Welch Ultimate XB-C18 20-40 μm 120 A, 100 mm x 400 mm; eluent A: water (0.225% formic acid), eluent B: acetonitrile; gradient: 0-30 min 30-50% B; flow 200 ml/min; temperature: room temperature; detector: UV 220/254 nm) to give methyl 3,9-bis (2-tert-butoxy-2-oxoethyl)-4-methyl-6-oxa-3, 9, 15-triazabicyclo[9.3.1]pentadeca-1 (15),11,13-triene-13-carboxylate (4.42 g, 37% yield) as yellow oil.

LC-MS (Method 4): $R_t$ = 0.525 min; MS (ESIpos): m/z = 508.2 $[M+H]^+$.

$^1H$ NMR (400 MHz, $CDCl_3$): δ [ppm] = 7.70-7.50 (m, 2H), 4.43-4.11 (m, 3H), 4.06-3.85 (m, 4H), 3.73-3.25 (m, 7H), 3.25-3.08 (m, 1H), 3.07-2.92 (m, 1H), 2.83-2.71 (m, 1H), 2.68-2.54 (m, 1H), 1.54-1.47 (m, 18H), 1.13-0.90 (m, 3H).

Step 12

2,2'-[13-(methoxycarbonyl)-4-methyl-6-oxa-3,9,15-triazabicyclo[9.3.1]pentadeca-1(15),11,13-triene-3,9-diyl]diacetic acid

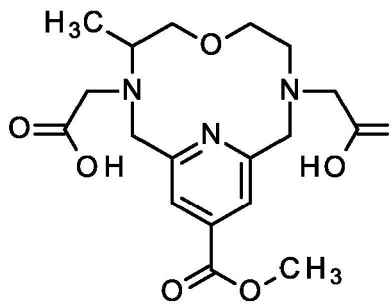

To a solution of methyl 3,9-bis(2-tert-butoxy-2-oxoethyl)-4-methyl-6-oxa-3,9,15-triazabicyclo[9.3.1]pentadeca-1(15),11,13-triene-13-carboxylate (2.74 g, 5.40 mmol) in dioxane (17 ml) was added HCL in dioxane: (4M, 5.5 ml, 22 mmol) and the mixture was stirred at 70°C for 3 h. HCL in dioxane (4M, 0.83 ml, 3.3 mmol) addition was repeated and

stirring at 70°C was continued for 1 h. The mixture was concentrated under reduced pressure and used in the next step without further purification (2.13 g, 100% theoretical yield).

LC-MS (Method 2): $R_t$ = 0.35 min; MS (ESIpos): m/z = 396.3 $[M+H]^+$.

Step 13

3,9-bis(carboxymethyl)-4-methyl-6-oxa-3,9,15-triazabicyclo[9.3.1]pentadeca-1(15),11,13-triene-13-carboxylic acid

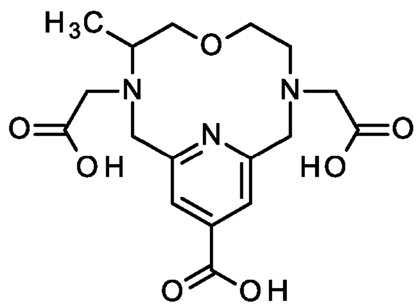

To a solution of 2,2'-(-13-(methoxycarbonyl)-4-methyl-6-oxa-3,9,15-triazabicyclo[9.3.1] pentadeca-1(15),11,13-triene-3,9-diyl)diacetic acid (2.13 g, 5.39 mmol, raw from step 12) in water (33 ml) was added sodium hydroxide solution (2M, 13,5ml, 27 mmol) and the mixture was stirred at room temperature. After one hour the mixture was used in the next step without further purification (2.05 g, 100% theoretical yield).

LC-MS (Method 2): $R_t$ = 0.25 min; MS (ESIpos): m/z = 382.5 $[M+H]^+$.

Step 14

manganese(2+) 2,2'-(13-carboxy-4-methyl-6-oxa-3,9,15-triazabicyclo[9.3.1]pentadeca-1(15), 11,13-triene-3,9-diyl)diacetate

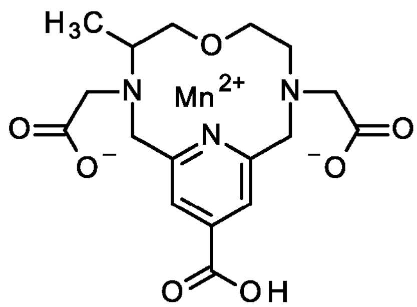

To a crude solution of 3,9-bis(carboxymethyl)-4-methyl-6-oxa-3,9,15-triazabicyclo[9.3.1] pentadeca-1(15),11,13-triene-13-carboxylic acid (2.05 g, 5.38 mmol) in water (45 ml) was added dichloromanganese tetrahydrate (1.17 g, 5.38 mmol) and the pH of the mixture was adjusted to pH 6.5 by addition of aqueous HCl (2M, 2.1 ml). This solution was stirred at 95°C for 16 h. Chelex® 100 was added and the reaction mixture was stirred for 2 h. Chelex®100 was removed by filtration and the pH of the filtrate was adjusted to pH 8. The crude mixture of isomers in water was purified by column chromatography (Interchim purifFash®, PF-30RP18AQ-F0800, water / acetonitrile, 0 - 25%) to yield 1.94 g (83% yield) of the title compound.

LC-MS (Method 1): $R_t$ = 0.23 min; MS (ESIpos): m/z = 435 $[M+H]^+$.

**Intermediate 14**

**manganese(2+) 2,2'-(13-carboxy-4-ethyl-6-oxa-3,9,15-triazabicyclo[9.3.1]pentadeca-1 (15), 11,13-triene-3,9-diyl)diacetate**

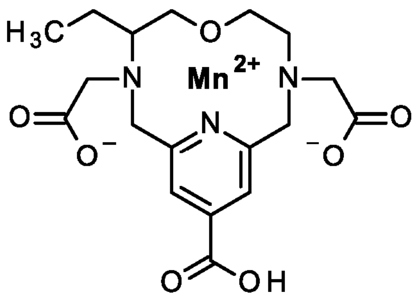

Step 1

1-[2-(benzyloxy)ethoxy]butan-2-ol

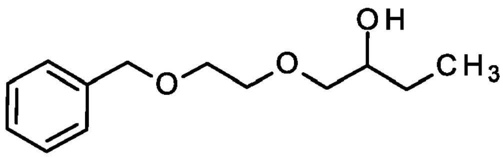

To a mixture of 2-(benzyloxy)ethan-1-ol (150 g, 986 mmol) and boron trifluoride diethyl etherate (2.5 ml, 20 mmol) in dichloromethane (1500 ml) was dropwise added 2-ethyloxirane (56.9 g, 788 mmol) in dichloromethane (300 ml) at 0 °C. The reaction mixture was stirred at 0 °C for 1 hour. The reaction mixture was furtherly stirred at room temperature for 16 hours. The reaction mixture was concentrated to give a crude product. The crude product was purified by reversed phase column (Column: Spherical C18, 40-60 µm, 120 A ; eluent A: 0.1% formic acid in water, eluent B: acetonitrile; gradient: 0-30 min 0-50% B; flow 100 ml/min; temperature: room temperature; Detector: UV 220/254 nm) to give 1-[2-(benzyloxy)ethoxy] butan-2-ol (75.0 g, 34% yield) as colourless oil.

LC-MS (Method 4): $R_t$ = 0.533 min; MS (ESIpos): m/z = 247.2 $[M+Na]^+$.

$^1$H NMR (400 MHz, $CDCl_3$): δ [ppm] = 7.31-7.25 (m, 4H), 7.25-7.17 (m, 1H), 4.50 (s, 2H), 3.80-3.50 (m, 5H), 3.50-3.37 (m, 1H), 3.34-3.21 (m, 1H), 2.70-2.60 (m, 1H), 1.55-1.30 (m, 2H), 0.95-0.80 (m, 3H).

Step 2

1-(2-hydroxyethoxy)butan-2-ol

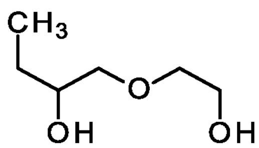

To a solution of 1-[2-(benzyloxy)ethoxy]butan-2-ol (74.5 g, 346 mmol) in methanol (50 ml) were added palladium (1.00 g, 10% on activated carbon) and palladium hydroxide (1.00 g , 20% on activated carbon) at room temperature. The reaction mixture was stirred at 50°C for 16 hours under hydrogen atmosphere (45 psi). The reaction mixture was filtered. The filtrate was concentrated to give 1-(2-hydroxyethoxy)butan-2-ol (39.6 g, 91% yield) as colourless oil.

$^1$H NMR (400 MHz, $CDCl_3$): δ [ppm] =3.80-3.52 (m, 6H), 3.49 (s, 2H), 3.40-3.30 (m, 1H), 1.65-1.40 (m, 2H), 1.00-0.85 (m, 3H).

Step 3

2-{2-[(4-methylbenzene-1-sulfonyl)oxy]butoxy}ethyl 4-methylbenzene-1-sulfonate

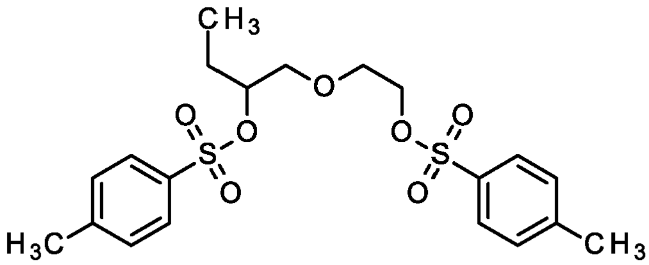

To a solution of 1-(2-hydroxyethoxy)butan-2-ol (34.8 g, 259 mmol) in dichloromethane (1.2 l) were added 4-(dimethylamino)pyridine (3.17 g, 25.9 mmol), pyridine (126 ml, 1.55 mol) and 4-methylbenzene-1-sulfonyl chloride (198 g, 1.56 mol) at 0°C. The mixture was stirred at room temperature for 16 hours. To the mixture was added pyridine (63 ml, 0.78 mmol), 4-methylbenzene-1-sulfonyl chloride (99 g, 0.78 mol) at 0 °C. After stirring at room temperature for anothere48 hours, the reaction mixture was diluted with dichloromethane, washed with dilute hydrochloric acid aqueous solution (1M) and saturated sodium bicarbonate aqueous solution, brine, dried over anhydrous sodium sulfate and concentrated to give a crude product. The crude product was purified by flash column chromatography (petroleum ether / ethyl acetate, 20:1 to 1:1) to give 2-{[2-{[(4-methylphenyl)sulfonyl]oxy}butyl]oxy}ethyl 4-methylbenzenesulfonate (104 g, 91% yield) as colourless oil.

$^1$H NMR (400 MHz, $CDCl_3$): δ [ppm] = 7.85-7.70 (m, 4H), 7.40-7.27 (m, 4H), 4.18-3.93 (m, 3H), 3.90-3.37 (m, 4H), 2.55-2.35 (m, 6H), 1.67-1.56 (m, 1H), 1.50-1.36 (m, 1H), 0.83-0.78 (m, 3H).

Step 4

2-{2-[2-(1,3-dioxo-1,3-dihydro-2H-isoindol-2-yl)butoxy]ethyl}-1H-isoindole-1,3(2H)-dione

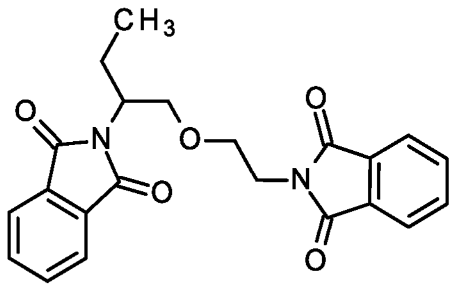

A mixture of 2-{2-[(4-methylbenzene-1-sulfonyl)oxy]butoxy}ethyl 4-methylbenzene-1-sulfonate (104 g, 235 mmol) and potassium 1,3-dioxo-1,3-dihydroisoindol-2-ide (131 g, 705 mmol) in *N*,*N*-dimethylformamide (2.0 l) was stirred at 100 °C for 16 hours. The reaction mixture was diluted with water and extracted with ethyl acetate. The organic phase was washed with water and brine, dried over anhydrous sodium sulphate and concentrated to give a residue. The residue was purified by flash column chromatography (petroleum ether / ethyl acetate, 20:1 to 3:1) to give 2-(2-{[2-(1,3-dioxo-1,3-dihydro-2H-isoindol-2-yl)butyl]oxy}ethyl)-1H-isoindole-1,3(2H)-dione (63.0 g, 68% yield) as colourless oil.

$^1$H NMR (400 MHz, $CDCl_3$): δ [ppm] = 7.86-7.60 (m, 8H), 4.35-4.25 (m, 0.5H), 4.03-3.95 (m, 0.5H), 3.86-3.50 (m, 6H), 1.99-1.62 (m, 1H), 1.55-1.40 (m, 1H), 0.91-0.79 (m, 3H).

Step 5

1-(2-aminoethoxy)butan-2-amine - hydrogen chloride (1/2)

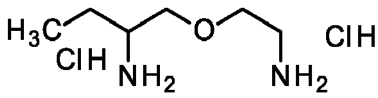

To a solution of 2-{2-[2-(1,3-dioxo-1,3-dihydro-2H-isoindol-2-yl)butoxy]ethyl}-1H-isoindole-1,3(2H)-dione (63.0 g, 161 mmol) in 1,4-dioxane (100 ml) was added hydrochloric acid (1.0 l, 12 M in water, 12 mol) at room temperature. The reaction mixture was refluxed for 4 days. The reaction mixture was concentrated to remove dioxane and filtered. The filtrate was adjusted pH to 7 with sodium carbonate powder and used in the next step directly without further purification.

Step 6

2-nitro-/V-(2-{2-[(2-nitrobenzene-1-sulfonyl)amino]butoxy}ethyl)benzene-1-sulfonamide

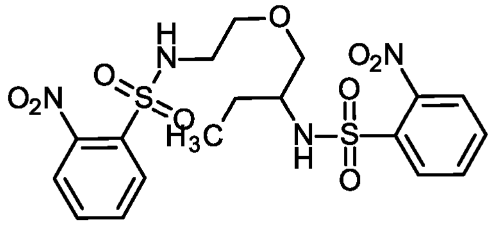

To a solution of 1-(2-aminoethoxy)butan-2-amine - hydrogen chloride (1/2) (32.0 g, 156 mmol) in a mixture of tetrahydrofuran (310 ml) and water (310 ml) were added sodium carbonate (49.6 g, 468 mmol) and 2-nitrobenzene-1-sulfonyl chloride (76.1 g, 343 mmol) at room temperature. The reaction mixture was stirred at room temperature for 16 hours. The reaction mixture was concentrated to remove tetrahydrofuran, diluted with water and extracted with ethyl acetate. The organic phase was washed with water and brine, dried over anhydrous sodium sulphate and concentrated to give a residue. The residue was purified by flash column chromatography (petroleum ether / ethyl acetate, 20:1 to 1:1) to give 2-nitro-N-(2-{[2-{[(2-nitrophenyl)sulfonyl]amino}butyl]oxy}ethyl)benzenesulphone amide (56 g, 71% yield) as a colourless oil.

LC-MS (Method 4): $R_t$ = 0.554 min; MS (ESIpos): m/z = 503.1 $[M+H]^+$.

Step 7

methyl 4-ethyl-3,9-bis(2-nitrobenzene-1-sulfonyl)-6-oxa-3,9,15-triazabicyclo[9.3.1] pentadeca-1(15), 11,13-triene-13-carboxylate

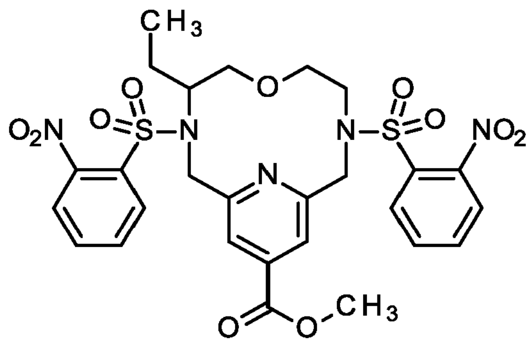

To a solution of 2-nitro-N-(2-{2-[(2-nitrobenzene-1-sulfonyl)amino]butoxy}ethyl)benzene-1-sulfonamide (27 g, 53.7 mmol) in acetonitrile (2.7 l) were added sodium carbonate (22.8 g, 215 mmol) and methyl 2,6-bis(bromomethyl) pyridine-4-carboxylate (17.4 g, 53.7 mmol, intermediate 1 step 2) at room temperature. The reaction mixture was stirred at 80 °C for 64 hours under nitrogen atmosphere. The mixture was combined with an identical 27 g batch and filtered. The filtrate was purified by flash column chromatography (petroleum ether / ethyl acetate, 20:1 to 1:1) and purified by reversed phase column (Instrument: ACSWH-GX-C; Column: Xtimate C18 150*40mm*10 μm; eluent A: 0.225% formic acid in

water, eluent B: acetonitrile; gradient: 0-60 min 20-80% B; flow 80 ml/min; temperature: room temperature; Detector: UV 220/254 nm) to give methyl 4-ethyl-3,9-bis[(2-nitrophenyl)sulfonyl]-6-oxa-3,9,15-triazabicyclo[9.3.1]pentadeca-1(15),11,13-triene-13-carboxylate (34 g, 47% yield) as a yellow solid.

LC-MS (Method 4): $R_t$ = 0.624 min; MS (ESIpos): m/z = 664.2 $[M+H]^+$.

Step 8

methyl 4-ethyl-6-oxa-3,9,15-triazabicyclo[9.3.1]pentadeca-1(15), 11,13-triene-13-carboxylate

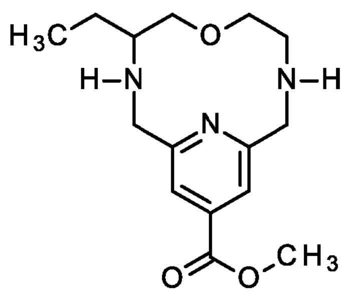

To a solution of methyl 4-ethyl-3,9-bis(4-nitrobenzene-1-sulfonyl)-6-oxa-3,9,15-triaza bicyclo[9.3.1]pentadeca-1(15),11,13-triene-13-carboxylate (32 g, 48 mmol) in acetonitrile (320 ml) were added potassium carbonate (40 g, 289 mmol) and 4-methoxybenzene-1-thiol (25 ml, 190 mmol) at room temperature. The mixture was stirred at 40 °C for 12 hours. The mixture was filtered, and the filtrate was concentrated to give a residue. The residue was diluted with water and the pH value was adjusted to pH2 with hydrochloric acid (2M). The mixture was extracted with ethyl acetate. The phases were separated, the pH value of the aqueous layer was adjusted to pH 8 with sodium hydrogen carbonate at 0°C, and used directly in the next step.

LC-MS (Method 3): $R_t$ = 0.239 min; MS (ESIpos): m/z = 294.2 $[M+H]^+$.

Step 9

3,9-di-tert-butyl 13-methyl 4-ethyl-6-oxa-3,9,15-triazabicyclo[9.3.1]pentadeca-1(15),11,13-triene-3,9,13-tricarboxylate

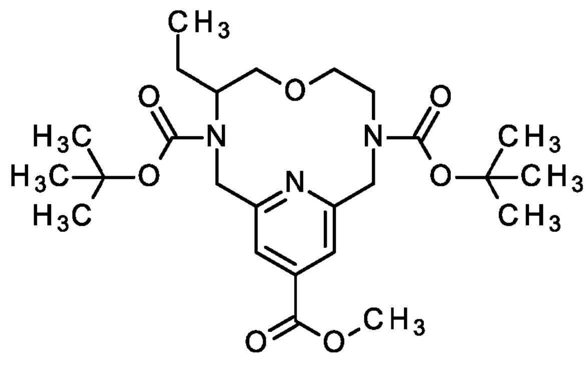

To a solution of methyl 4-ethyl-6-oxa-3,9,15-triazabicyclo[9.3.1]pentadeca-1(15),11,13-triene-13-carboxylate (14.1 g, 48.2 mmol) in tetrahydrofuran (300 ml) and water (500 ml) were added sodium hydrogen carbonate (16.2 g, 193 mmol) and di-tert-butyl dicarbonate (22 ml, 96 mmol) at 0 °C. The mixture was stirred at room temperature for 2 hours. The reaction mixture was concentrated, diluted with water and extracted with ethyl acetate. The organic phase was washed with water and brine, dried over anhydrous sodium sulfate and concentrated to give a residue. The residue was purified by flash column chromatography (petroleum ether: ethyl acetate = 20: 1 to 3: 1) to give 3,9-di-tert-butyl 13-methyl 4-ethyl-6-oxa-3,9,15-triazabicyclo[9.3.1]pentadeca-1(15),11,13-triene-3,9,13-tricarboxylate (10.0 g, 42% yield) as colourless oil.

LC-MS (Method 4): $R_t$ = 0.556 min; MS (ESIpos): m/z = 494.3 $[M+H]^+$.

$^1H$ NMR (400 MHz, $CDCl_3$): δ [ppm] =7.80-7.65 (m, 2H), 4.87-4.27 (m, 4H), 4.00-3.90 (m, 3H), 3.75-3.05 (m, 7H),

1.50-1.30 (m, 20H), 0.93-0.77 (m, 3H).

Step 10

methyl 4-ethyl-6-oxa-3,9,15-triazabicyclo[9.3.1]pentadeca-1(15), 11,13-triene-13-carboxylate -hydrogen chloride (1/2)

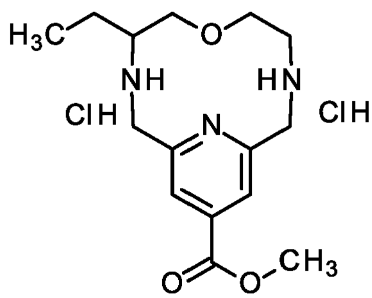

A solution of 3,9-di-tert-butyl 13-methyl 4-ethyl-6-oxa-3,9,15-triazabicyclo[9.3.1]pentadeca-1(15),11,13-triene-3,9,13-tricarboxylate (10 g, 20.3 mmol) in hydrochloric acid (100 ml, 2M in dioxane) was stirred at room temperature for 16 hours. The mixture was concentrated to give methyl 4-ethyl-6-oxa-3,9,15-triazabicyclo[9.3.1] pentadeca-1(15), 11,13-triene-13-carboxylate dihydrochloride (7.42 g) as white solid.
LC-MS (Method 3): $R_t$ = 0.399 min; MS (ESIpos): m/z = 294.2 $[M+H]^+$.

Step 11

methyl 3,9-bis(2-tert-butoxy-2-oxoethyl)-4-ethyl-6-oxa-3,9,15-triazabicyclo[9.3.1] pentadeca-1(15),11,13-triene-13-carboxylate

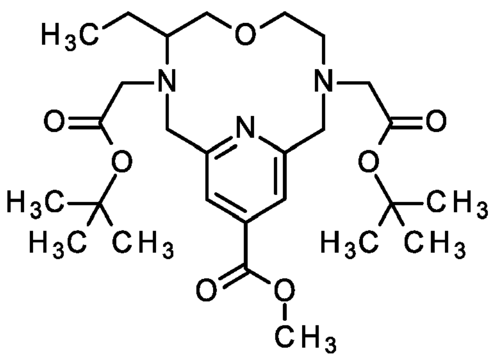

To a solution of methyl 4-ethyl-6-oxa-3,9,15-triazabicyclo[9.3.1]pentadeca-1(15),11,13-triene-13-carboxylate -hydrogen chloride (1/2) (1.0 g, 2.73 mmol) in acetonitrile (8.1 ml) was added potassium carbonate (2.26 g, 16.4 mmol) at room temperature. The mixture was stirred at room temperature for 30 minutes. Then to the mixture was added tert-butyl bromoacetate (0.99 ml, 6.8 mmol) at room temperature. The mixture was stirred at room temperature for 12 hours. The reaction mixture was concentrated to give a crude product. The crude product was purified by reversed phase column (Instrument: ACSWH-GX-C; Column: Xtimate C18 150*40mm*10 μm; eluent A: 0.225% formic acid in water, eluent B: acetonitrile; gradient: 0-50 min 10-40% B; flow 80 ml/min; temperature: room temperature; Detector: UV 220/254 nm) to give methyl 3,9-bis(2-tert-butoxy-2-oxoethyl)-4-ethyl-6-oxa-3,9,15-triazabicyclo[9.3.1]pentadeca-1(15),11,13-triene-13-carboxylate (1 g, 96% purity, 67% yield) as a yellow oil.
LC-MS (Method 4): $R_t$ = 0.503 min; MS (ESIpos): m/z = 522.3 $[M+H]^+$.
$^1H$ NMR (400 MHz, DMSO-$d_6$): δ [ppm] = 7.66 (s, 2H), 4.10-4.01 (m, 4H), 3.98 (s, 3H), 3.55-3.43 (m, 5H), 3.39-3.33 (m, 1H), 2.87-2.65 (m, 4H), 2.44-2.41 (m, 1H), 1.45 (s, 18H), 1.31-1.03 (m, 2H), 0.69 (t, 3H).

Step 12

2,2'-[4-ethyl-13-(methoxycarbonyl)-6-oxa-3,9,15-triazabicyclo[9.3.1]pentadeca-1(15), 11,13-triene-3,9-diyl]diacetic acid

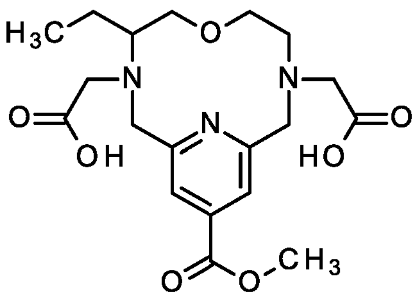

To a solution of methyl 3,9-bis(2-tert-butoxy-2-oxoethyl)-4-ethyl-6-oxa-3,9,15-triazabicyclo[9.3.1]pentadeca-1(15),11,13-triene-13-carboxylate (3.54 g, 6.79 mmol) in dioxane (23 ml) was added HCL in dioxane (4M, 6.8 ml, 27 mmol) and the mixture was stirred at 70°C for 3h. The mixture was concentrated under reduced pressure and used in the next step without further purification.
LC-MS (Method 2): $R_t$ = 0.44 min; MS (ESIpos): m/z = 410.3 $[M+H]^+$.

Step 13

3,9-bis(carboxymethyl)-4-ethyl-6-oxa-3,9,15-triazabicyclo[9.3.1]pentadeca-1(15), 11,13-triene-13-carboxylic acid

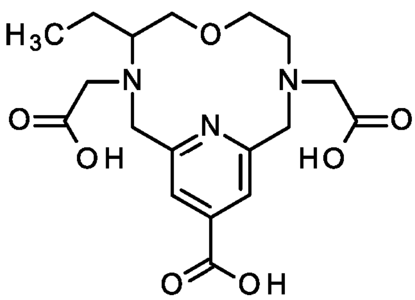

To crude 2,2'-[4-ethyl-13-(methoxycarbonyl)-6-oxa-3,9,15-triazabicyclo[9.3.1]pentadeca-1(15),11,13-triene-3,9-diyl]diacetic acid (2,78 g, 6.79 mmol, raw from step) in water (41 ml) was added sodium hydroxide solution (2M, 17 ml, 34 mmol) and the mixture was stirred at room temperature. The mixture was used in the next step without further purification.
LC-MS (Method 2): $R_t$ = 0.40 min; MS (ESIpos): m/z = 396.4 $[M+H]^+$.

Step 14

manganese(2+) 2,2'-(13-carboxy-4-ethyl-6-oxa-3,9,15-triazabicyclo[9.3.1]pentadeca-1(15), 11,13-triene-3,9-diyl)diacetate

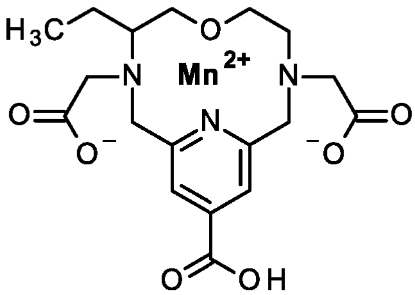

To a crude solution of 3,9-bis(carboxymethyl)-4-ethyl-6-oxa-3,9,15-triazabicyclo[9.3.1] pentadeca-1(15),11,13-triene-13-carboxylic acid (2.68 g, 6.78 mmol) in water (57 ml) was added dichloromanganese tetrahydrate (1.48 g, 7.46 mmol) and the pH of the mixture was adjusted to pH 6.5 by addition of aqueous HCl (2M, 0.5 ml). This solution was stirred at 95°C for 16 h. Chelex®100 was added and the reaction mixture was stirred for 2 h. Chelex®100 was removed by filtration and the pH of the filtrate was adjusted to pH 8. The crude mixture of isomers in water was purified by column chromatography (Interchim puriFlash®, PF-30RP18AQ-F0800, water / acetonitrile, 0 - 25%) to yield 2.07 g (68% yield) of the title compound.

LC-MS (Method 1): $R_t$ = 0.30 min; MS (ESIpos): m/z = 449 $[M+H]^+$.

__Intermediate 15__

**manganese(2+) 2,2'-(13-carboxy-5-methyl-6-oxa-3,9,15-triazabicyclo[9.3.1]pentadeca-1 (15), 11,13-triene-3,9-diyl)diacetate**

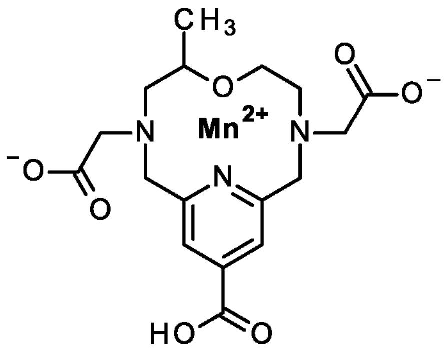

__Step 1__

1-(benzyloxy)propan-2-yl 4-methylbenzene-1-sulfonate

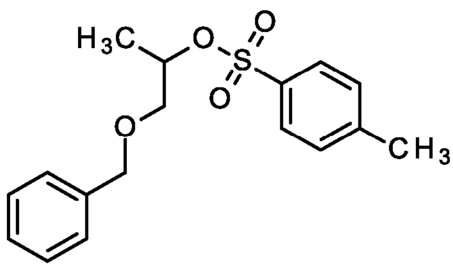

To a solution of 1-(benzyloxy)propan-2-ol (950 g, 572 mmol) in dichloromethane (1.9 l) was added 4-(dimethylamino)pyridine (7.0 g, 57.2 mmol), pyridine (90.4 g, 1.14 mol) and 4-toluenesulfonyl chloride (119.9 g, 686 mmol) at 0 °C. The mixture was stirred at 30°C for 16 hours. To the mixture was added pyridine (90.4 g, 1.14 mol) and 4-toluenesulfonyl chloride (109.0 g, 571 mmol) at 0 °C. After stirring at 30°C for 16 hours, the mixture was diluted with dichloromethane. The

organic phase was washed with hydrochloric acid (1M in water), saturated sodium bicarbonate solution and brine. The organic phase was dried over anhydrous sodium sulphate and concentrated to give a crude product. The crude product was diluted with petroleum ether and then ethyl acetate was added (until the solution was clear). The solution was placed at -20 °C for 30 minutes and the solid was separated out. The suspension was filtered. The solid cake was concentrated to give 1-(benzyloxy)propan-2-yl 4-methylbenzenesulfonate (110 g, 60% yield) as a white solid. The filtrate was purified by column chromatography on silica gel (1000 mesh, petroleum ether: ethyl acetate = 20: 1 to 10: 1) to give 1-(benzyloxy) propan-2-yl 4-methylbenzenesulfonate (40 g, 22% yield) as a white solid.

LC-MS (Method 4): $R_t$ = 0.646 min; MS (ESlpos): m/z =343.1 $[M+Na]^+$.

$^1H$ NMR (400 MHz, $CDCl_3$): δ [ppm] = 7.81 (d, 2H), 7.40-7.19 (m, 7H), 4.87-4.70 (m, 1H), 4.54-4.37 (m, 2H), 3.63-3.34 (m, 2H), 2.44 (s, 3H), 1.34 (d, 3H).

Step 2

({2-[2-(benzyloxy)ethoxy]propoxy}methyl)benzene

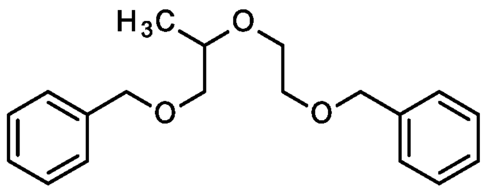

To a suspension of 2-(benzyloxy)ethan-1-ol (142 g, 936 mmol) and potassium hydroxide (52.5 g, 936 mmol) in 1,4-dioxane (1.5 l) was added 1-(benzyloxy)propan-2-yl 4-methylbenzene-1-sulfonate (150 g, 468 mmol) at room temperature. After stirring at 100°C for 16 hours, the reaction mixture was diluted with water, concentrated to removed 1,4-dioxane and extracted with ethyl acetate. The organic phase was washed with water and brine, dried over anhydrous sodium sulphate and concentrated to give a residue. The residue was purified by flash column chromatography (petroleum ether: ethyl acetate = 40: 1 to 10: 1) and reversed phase column (Column: Spherical C18, 40-60 μm, 120 A ; eluent A: 0.1% formic acid in water, eluent B: acetonitrile; gradient: 0-30 min 50-80% B; flow 100 ml/min; temperature: room temperature; Detector: UV 220/254 nm) to give [({2-[2-(benzyloxy)ethoxy]propyl}oxy)methyl]benzene (65 g, 46% yield) as colourless oil.

LC-MS (Method 4): $R_t$ = 0.576 min; MS (ESlpos): m/z =323.3 $[M+Na]^+$.

$^1H$ NMR (400 MHz, $CDCl_3$): δ [ppm] = 7.41-7.28 (m, 10H), 4.64-4.53 (m, 4H), 3.76-3.67 (m, 3H), 3.66-3.61 (m, 2H), 3.56-3.50 (m, 1H), 3.47-3.40 (m, 1H), 1.20 (d, 3H).

Step 3

2-(2-hydroxyethoxy)propan-1-ol

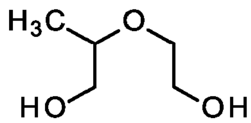

Flow chemistry: ({2-[2-(Benzyloxy)ethoxy]propoxy}methyl)benzene (65 g, 216 mmol) in methanol (1300 ml) was stirred at 20°C until it formed a clear solution. The clear solution was pumped through a fixed bed reactor made of stainless steel tubing with the dimensions 60 cm x ½ inch filled completely with 5% Palladium(II) hydroxide on aluminium oxide. The hydrogen back pressure regulator of the reactor was adjusted to 0.5 MPa and the flow rate of hydrogen was 95 ml/min while the reactor was heated to 40°C by an oil bath. The solution was pumped into the reactor by a piston pump at a flow rate of 3 ml/min. The reaction mixture was collected from the reactor outlet after a retention time of 3.33 min in the reactor zone, and the tubing was washed with methanol after completion. The mixture was concentrated to give 2-(2-hydroxyethoxy) propan-1-ol (21 g, 81% yield) as yellow oil.

$^1H$ NMR (400 MHz, $CDCl_3$): δ [ppm] = 3.85-3.73 (m, 3H), 3.67-3.57 (m, 2H), 3.51-3.38 (m, 2H), 3.25-3.11 (m, 2H), 1.12 (d, 3H).

Step 4

2-{2-[(4-methylbenzene-1-sulfonyl)oxy]ethoxy}propyl 4-methylbenzene-1-sulfonate

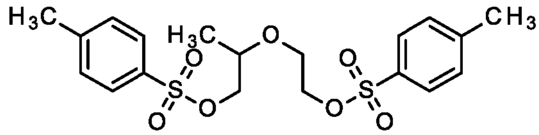

To a solution of 2-(2-hydroxyethoxy)propan-1-ol (21.0 g, 175 mmol) in dichloromethane (840 ml) were added trimethylamine (120 ml, 870 mmol), 4-(dimethylamino)pyridine (6.41 g, 52.4 mmol) and 4-toluenesulfonyl chloride (100 g, 524 mmol) at 0 °C. After stirring at room temperature for 16 hours, the mixture was diluted with saturated aqueous ammonium chloride solution. The mixture was extracted with dichloromethane. The organic phase was washed with brine, dried with anhydrous sodium sulphate, filtered and concentrated to give a residue. The residue was purified by column chromatography on silica gel (1000 mesh, petroleum ether: ethyl acetate = 20: 1 to 10: 1) and reverse phase column (Column: Spherical C18, 40-60 μm, 120 A ; eluent A: 0.1% formic acid in water, eluent B: acetonitrile; gradient: 0-30 min 50-100% B; flow 100 ml/min; temperature: room temperature; Detector: UV 220/254 nm) to give 2-(2-{[(4-methylphenyl) sulfonyl]oxy}ethoxy)propyl 4-methylbenzenesulfonate (72.0 g, 96% yield) a white solid.

$^1$H NMR (400 MHz, $CDCl_3$): δ [ppm] = 7.80-7.75 (m, 4H), 7.37-7.32 (m, 4H), 4.09-4.05 (m, 2H), 3.93-3.85 (m, 2H), 3.68-3.57 (m, 3H), 2.45 (s, 6H), 1.06 (d, *J* = 6.4 Hz, 3H).

Step 5

2-{2-[2-(1,3-dioxo-1,3-dihydro-2H-isoindol-2-yl)ethoxy]propyl}-1H-isoindole-1,3(2H)-dione

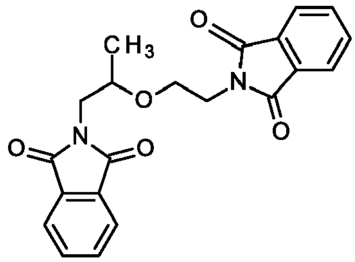

A mixture of 2-{2-[(4-methylbenzene-1-sulfonyl)oxy]ethoxy}propyl 4-methylbenzene-1-sulfonate (72 g, 168 mmol) and potassium 1,3-dioxo-1,3-dihydroisoindol-2-ide (93.4 g, 504 mmol) in N,N-dimethylformamide (1.4 l) was stirred at 100 °C for 16 hours. After adding to water (7.5 l) under stirring, the mixture was filtered. The solid cake was concentrated by oil pump to give 2-{2-[2-(1,3-dioxo-1,3-dihydro-2H-isoindol-2-yl)ethoxy]propyl}-1H-isoindole-1,3(2H)-dione (43.0 g, 68% yield) as a white solid.

LC-MS (Method 4): $R_t$ = 0.496 min; MS (ESIpos): m/z =379.2 $[M+H]^+$.

$^1$H NMR (400 MHz, $CDCl_3$): δ [ppm] = 7.80-7.61 (m, 8H), 3.94-3.66 (m, 6H), 3.64-3.54 (m, 1H), 1.16 (d. *J* = 6.4 Hz, 3H).

Step 6

2-(2-aminoethoxy)propan-1-amine - hydrogen chloride (1/2)

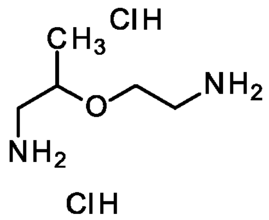

To a solution of 2-{2-[2-(1,3-dioxo-1,3-dihydro-2H-isoindol-2-yl)ethoxy]propyl}-1H-isoindole-1,3(2H)-dione (43 g, 114 mmol,) in 1,4-dioxane (300 ml) was added hydrochloric acid (1.2 l) at room temperature. After refluxing for 3 days, the reaction mixture was concentrated and filtered. The filtrate was adjusted to pH 7 with sodium carbonate powder and used in the next step directly without further purification (Theor. Yield: 21.7 g).

Step 7

2-nitro-*N*-[2-{2-[(2-nitrobenzene-1-sulfonyl)amino]ethoxy}propyl]benzene-1-sulfonamide

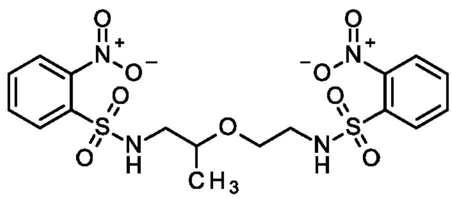

To a solution of 2-(2-aminoethoxy)propan-1-amine - hydrogen chloride (1/2) (21.7 g, 114 mmol) in a mixture of water (450 ml) and tetrahydrofuran (450 ml) was added sodium carbonate (30.1 g, 284 mmol) and 2-nitrobenzene-1-sulfonyl chloride (52.9 g, 239 mmol) at room temperature. After stirring at room temperature for 16 hours, the reaction mixture was diluted with water and extracted with ethyl acetate. The organic phase was washed with water and brine, dried over anhydrous sodium sulphate and concentrated to give a residue. The residue was purified by flash column chromatography (petroleum ether: ethyl acetate = 20: 1 to 0: 1) to give 2-nitro-*N*-[2-(2-{[(2-nitrophenyl)sulfonyl]amino}ethoxy)propyl]benzene sulfonamide (19.5 g, 36% yield) as yellow oil.

LC-MS (Method 4): $R_t$ = 0.503 min; MS (ESIpos): m/z =489.1 $[M+H]^+$.

$^1H$ NMR (400 MHz, $CDCl_3$): $\delta$ [ppm] = 8.18-8.09 (m, 2H), 7.95-7.85 (m, 2H), 7.80-7.70 (m, 4H), 5.78-5.51 (m, 2H), 3.67-3.59 (m, 1H), 3.57-3.48 (m, 1H), 3.38-3.14 (m, 4H), 2.97-2.84 (m, 1H), 1.08 (d, 3H).

Step 8

methyl 5-methyl-3,9-bis(2-nitrobenzene-1-sulfonyl)-6-oxa-3,9,15-triazabicyclo[9.3.1]pentadeca-1(15),11,13-triene-13-carboxylate

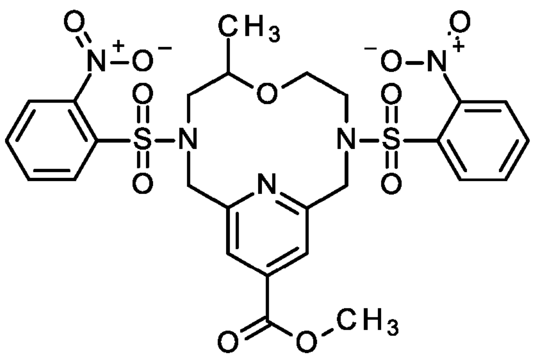

To a solution of 2-nitro-*N*-[2-{2-[(2-nitrobenzene-1-sulfonyl)amino]ethoxy}propyl] benzene-1-sulfonamide (7.0

g, 14.3 mmol), methyl 2,6-bis(hydroxymethyl)pyridine-4-carboxylate (3.39 g, 17.2 mmol, intermediate 1 step) and tri-n butylphosphine (14 ml, 57 mmol) in tetrahydrofuran (700 ml) was added *N*,*N*,*N*,*N*-tetramethylazodicarboxamide (9.87 g, 57.3 mmol) at 0 °C. After stirring at room temperature for 16 hours under nitrogen atmosphere the reaction mixture was combined with a batch generated from 12.5 g 2-nitro-*N*-[2-{2-[(2-nitrobenzene-1-sulfonyl)amino]ethoxy}propyl]benzene-1-sulfonamide (25.6 mmol) and methyl 2,6-bis(hydroxymethyl)pyridine-4-carboxylate (25.6 mmol) in by identical reaction with tri-n butylphosphine (20.7 ml, 102 mmol) and *N,N,N,N*-tetramethylazodicarboxamide (17.6 g, 102 mmol) in tetrahydrofuran (1200 ml). The combined reaction mixture was diluted with water and extracted with ethyl acetate. The organic phase was washed with water and brine, dried over anhydrous sodium sulphate and concentrated to give a residue. The residue was purified by reversed phase column three times (Column: Spherical C18, 40-60 μm, 120 A ; eluent A: 0.1% formic acid in water, eluent B: acetonitrile; gradient: 0-30 min 20-80% B; flow 100 ml/min; temperature: room temperature; Detector: UV 220/254 nm) to give methyl 5-methyl-3,9-bis[(2-nitrophenyl)sulfonyl]-6-oxa-3,9,15-triazabicyclo[9.3.1]pentadeca-1(15),11,13-triene-13-carboxylate (4.50 g) as a brown solid.

LC-MS (Method 4): $R_t$ = 0.523 min; MS (ESIpos): m/z =650.1 $[M+H]^+$.

$^1H$ NMR (400 MHz, $CDCl_3$): δ [ppm] = 8.25-8.13 (m, 2H), 7.87-7.84 (m, 1H), 7.82-7.71 (m, 5H), 7.70-7.65 (m, 2H), 4.81-4.70 (m, 2H), 4.60-4.49 (m, 2H), 3.97 (s, 3H), 3.70- 3.63 (m, 1H), 3.54-3.47 (m, 1H), 3.42-3.30 (m, 3H), 3.10-2.95 (m, 2H), 0.95 (d, *J* = 6.4 Hz, 3H).

Step 9

methyl 5-methyl-6-oxa-3,9,15-triazabicyclo[9.3.1]pentadeca-1(15),11,13-triene-13-carboxylate

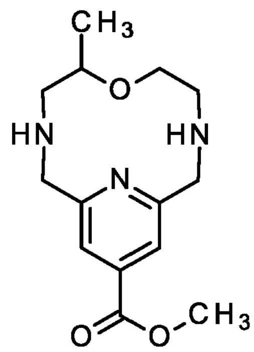

To a solution of methyl 5-methyl-3,9-bis(2-nitrobenzene-1-sulfonyl)-6-oxa-3,9,15-triazabicyclo [9.3.1]pentadeca-1(15),11,13-triene-13-carboxylate (4.5 g, 6.93 mmol) in acetonitrile (90 ml) was added potassium carbonate (4.79 g, 34.6 mmol) and 4-methoxybenzenethiol (3.88 g, 27.7 mmol) at room temperature. After stirring at 40 °C for 16 hours followed by stirring at 50 °C for 16 hours, the mixture was combined with a batch generated from, 0.4 g methyl 5-methyl-3,9-bis(2-nitrobenzene-1-sulfonyl)-6-oxa-3,9,15-triazabicyclo[9.3.1]pentadeca-1(15),11,13-triene-13-carboxylate in an identical way. The combined reaction mixture was concentrated to give a residue. The residue was adjusted to pH 7 by addition of hydrochloric acid (1M) and then adjusted to pH 5 by addition of formic acid. The mixture was washed with ethyl acetate. After phase separation, the aqueous layer was adjusted to pH 8 with sodium hydrogen carbonate at 0 °C and used directly in the next step (theor. yield: 2.1 g).

Step 10

3,9-di-tert-butyl 13-methyl 5-methyl-6-oxa-3,9,15-triazabicyclo[9.3.1]pentadeca-1(15),11,13-triene-3,9,13-tricarboxylate

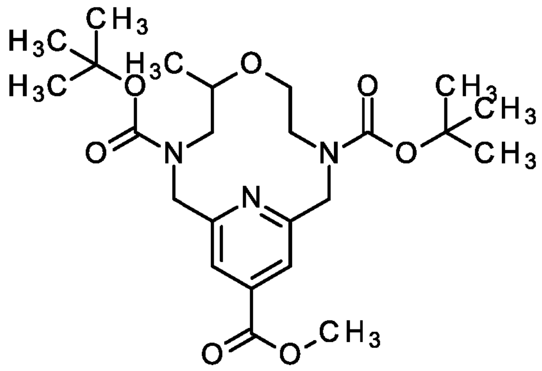

To a solution of methyl 5-methyl-6-oxa-3,9,15-triazabicyclo[9.3.1]pentadeca-1(15),11,13-triene-13-carboxylate (2.1 g, 7.52 mmol) in water (20 ml) which had been adjusted to pH 8 in step 9 with sodium hydrogen carbonate, was added THF (20 ml) and di-tert-butyl dicarbonate (4.8 ml, 21 mmol). After stirring at room temperature for 1 hour, the reaction mixture was concentrated to remove the THF, diluted with water and extracted with ethyl acetate. The organic phase was washed with water and brine, dried over anhydrous sodium sulphate and concentrated to give a residue. The residue was purified by reversed phase column (Column: Spherical C18, 40-60 μm, 120 A ; eluent A: water, eluent B: acetonitrile; gradient: 0-30 min 30-80% B; flow 60 ml/min; temperature: room temperature; Detector: UV 220/254 nm) to give 3,9-di-tert-butyl 13-methyl 5-methyl-6-oxa-3,9,15-triazabicyclo[9.3.1]pentadeca-1(15),11,13-triene-3,9,13-tricarboxylate (1.50 g, 42% yield) as brown oil.

LC-MS (Method 4): $R_t$ = 0.462 min; MS (ESIpos): m/z =480.3 $[M+H]^+$.

$^1H$ NMR (400 MHz, $CDCl_3$): δ [ppm] = 7.84-7.63 (m, 2H), 4.85-4.33 (m, 4H), 4.01-3.88 (m, 3H), 3.76-3.12 (m, 7H), 1.55-1.35 (m, 18H), 1.08-0.91 (m, 3H).

Step 11

methyl 5-methyl-6-oxa-3,9,15-triazabicyclo[9.3.1]pentadeca-1(15),11,13-triene-13-carboxylate - hydrogen chloride (1/2)

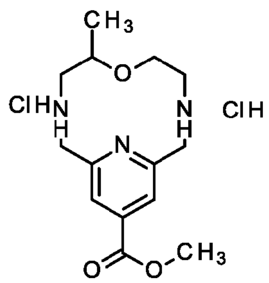

To a solution of 3,9-di-tert-butyl 13-methyl 5-methyl-6-oxa-3,9,15-triazabicyclo[9.3.1] pentadeca-1(15),11,13-triene-3,9,13-tricarboxylate (1.36 g, 2.84 mmol) in methanol (5.0 ml) was added hydrochloric acid (30 ml, 2 M in methanol) at room temperature. After stirring at room temperature for 3 hours, the reaction mixture was concentrated to give methyl 5-methyl-6-oxa-3,9,15-triazabicyclo[9.3.1]pentadeca-1(15),11,13-triene-13-carboxylate dihydrochloride (980 mg, 98% yield) as a white solid.

LC-MS (Method 3): $R_t$ = 0.118 min; MS (ESIpos): m/z =280.1 $[M+H]^+$.

Step 12

formic acid - methyl 3,9-bis(2-methoxy-2-oxoethyl)-5-methyl-6-oxa-3,9,15-triazabicyclo[9.3.1] pentadeca-1(15), 11,13-triene-13-carboxylate (1/1)

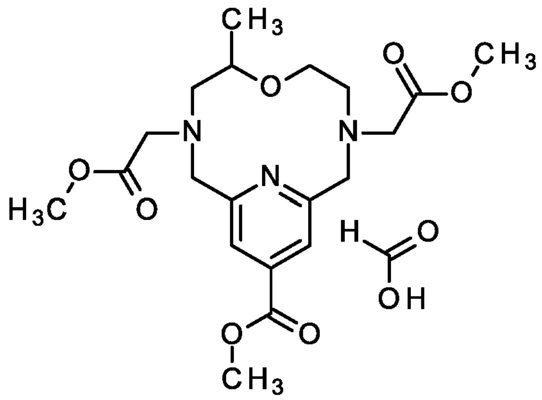

To a solution of methyl 5-methyl-6-oxa-3,9,15-triazabicyclo[9.3.1]pentadeca-1(15),11,13-triene-13-carboxylate - hydrogen chloride (1/2) (980 mg, 2.78 mmol) in acetonitrile (40 ml) were added *N,N*-diisopropylethylamine (2.9 ml, 17 mmol) and methyl bromoacetate (1.06 g, 6.96 mmol) at room temperature. After stirring at room temperature for 16 hours, the reaction mixture was concentrated to give a residue. The residue was purified by reversed phase column twice (Column: Spherical C18, 40-60 μm, 120 A ; eluent A: water, eluent B: acetonitrile; gradient: 0-30 min 0-40% B; flow 60 ml/min; temperature: room temperature; Detector: UV 220/254 nm) to give methyl 3,9-bis(2-methoxy-2-oxoethyl)-5-methyl-6-oxa-3,9,15-triazabicyclo[9.3.1]pentadeca-1(15),11,13-triene-13-carboxylate as brown oil. The product was diluted with a mixture of water and acetonitrile and lyophilized to give formic acid - methyl 3,9-bis(2-methoxy-2-oxoethyl)-5-methyl-6-oxa-3,9,15-triazabicyclo[9.3.1]pentadeca-1(15),11,13-triene-13-carboxylate (1:1) (820 mg, 88% purity) as brown oil.

LC-MS (Method 4): $R_t$ = 0.369 min; MS (ESIpos): m/z =424.2 $[M+H]^+$.

$^1H$ NMR (400 MHz, $CDCl_3$): δ [ppm] = 8.35 (s, 1H), 7.84-7.78 (m, 2H), 4.40-4.32 (m, 2H), 4.28-4.23 (m, 1H), 4.22-4.16 (m, 1H), 3.98 (s, 3H), 3.76-3.72 (m, 10H), 3.49-3.41 (m, 1H), 3.34-3.25 (m, 1H), 3.19-3.10 (m, 1H), 3.09-3.01 (m, 1H), 2.86-2.79 (m, 1H), 2.75-2.65 (m, 1H), 2.58-2.50 (m, 1H), 0.91 (d, 3H).

Step 13

3,9-bis(carboxymethyl)-5-methyl-6-oxa-3,9,15-triazabicyclo[9.3.1]pentadeca-1(15),11,13-triene-13-carboxylic acid

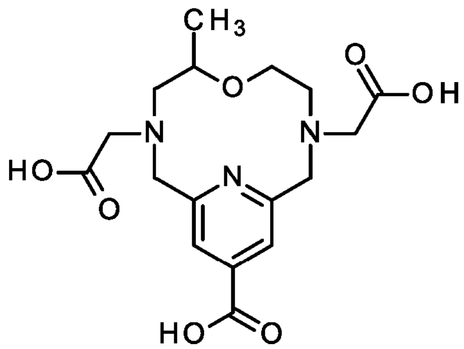

To a solution of methyl 3,9-bis(2-methoxy-2-oxoethyl)-5-methyl-6-oxa-3,9,15-triazabicyclo [9.3.1]pentadeca-1(15),11,13-triene-13-carboxylate formic acid salt (1/1) (820 mg, 1.75 mmol) in THF (7,5ml) was added aqueous sodium hydroxide solution (2M, 3.5 ml, 7.0 mmol) and the mixture was stirred at room temperature for 18 h. The mixture was concentrated under reduced pressure to remove the THF and used in the next step without further purification.

LC-MS (Method 2): $R_t$ = 0.25 min; MS (ESIpos): m/z = 382.3 $[M+H]^+$.

Step 14

manganese(2+) 2,2'-(13-carboxy-5-methyl-6-oxa-3,9,15-triazabicyclo[9.3.1]pentadeca-1(15),11,13-triene-3,9-diyl)diacetate

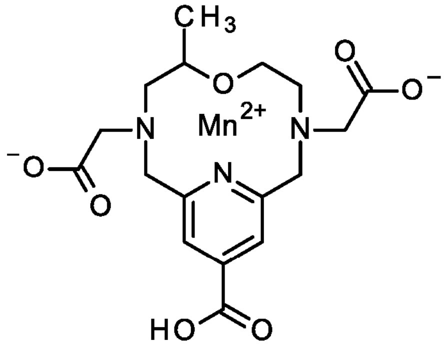

To 3,9-bis(carboxymethyl)-5-methyl-6-oxa-3,9,15-triazabicyclo[9.3.1]pentadeca-1(15), 11,13-triene-13-carboxylic acid in an aqueous sodium hydroxide solution (666 mg, 1.75 mmol) from step was added water (16 ml) and manganese(II)chloride tetrahydrate 380 mg, 1.92 mmol). The pH was adjusted to pH 6.5 by addition of aqueous HCl (2M, 1.6 ml). The solution was stirred at 95°C for 6 h. Then Chelex®100 was added and the mixture was stirred for 2 h at room temperature. Chelex®100 was removed by filtration and the crude product was purified by column chromatography (Interchim puriFlash® PF-30 RP18AQ-F120, water/ acetonitrile, 0 - 25%) to yield 547 g (100 % purity, 72 % yield) of the racemic title compound.

LC-MS (method 1): $R_t$ = 0.22 min; MS (ESIpos): m/z = 435.2 $[M+H]^+$ .

<u>**Intermediate 16**</u>

**manganese(2+) (2*R*,2'*S*)-2,2'-[13-carboxy-6-oxa-3,9,15-triazabicyclo[9.3.1] pentadeca-1(15),11,13-triene-3,9-diyl]dibutanoate**

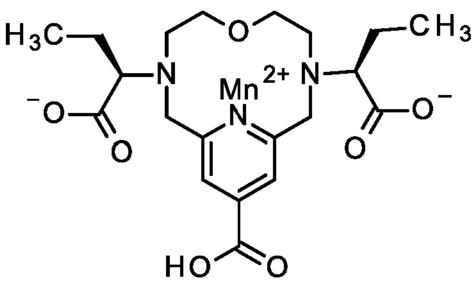

<u>Step 1</u>

methyl 3,9-bis(1-methoxy-1-oxobutan-2-yl)-6-oxa-3,9,15-triazabicyclo[9.3.1]pentadeca-1 (15), 11,13-triene-13-carboxylate

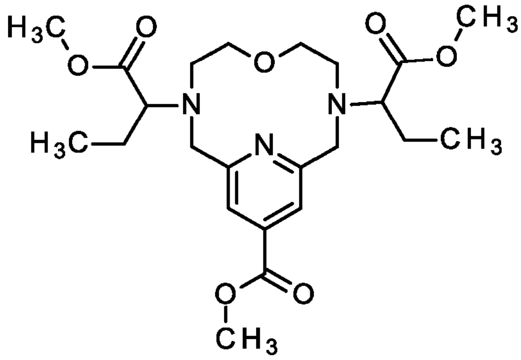

To methyl 6-oxa-3,9,15-triazabicyclo[9.3.1]pentadeca-1(15),11,13-triene-13-carboxylate hydrogen chloride (1/2) (1.0 g, 2.96 mmol, intermediate 1 step 7) in acetonitrile (100 ml) was added potassium carbonate (1,63 g, 11.8 mmol) and methyl 2-bromobutanoate (710 μl, 6.2 mmol). The mixture was stirred at RT for 1 day followed by one additional day of stirring at 60°C. The mixture was filtered after cooling to RT, the filtrate was concentrated under reduced pressure and dichloromethane was added. The mixture was washed with water, dried over anhydrous sodium sulphate, filtered, and concentrated to give 1.33 g of the title compound as raw product, which was used in the next step without further purification.

LC-MS (Method 2): $R_t$ = 0.87 min; MS (ESIpos): m/z = 467.1 $[M+H]^+$.

Step 2

3,9-bis (1-carboxypropyl)-6-oxa-3,9,15-triazabicyclo[9.3.1]pentadeca-1(15),11,13-triene-13-carboxylic acid

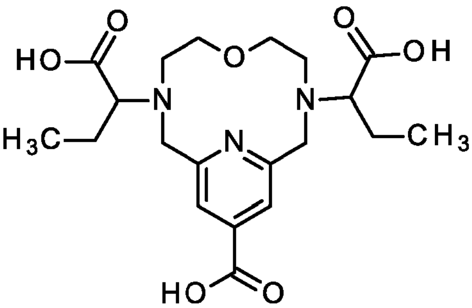

To methyl 3,9-bis(1-methoxy-1-oxobutan-2-yl)-9-(1-methoxy-1-oxobutan-2-yl)-6-oxa-3,9,15-triazabicyclo [9.3.1]pentadeca-1(15),11,13-triene-3-carboxylate (1.33 g crude product from step 1, 2.86 mmol) in THF (15 ml) was added an aqueous sodium hydroxide solution (2M, 5.7 ml, 11.4 mmol) and the mixture was stirred at room temperature for 20 h. The mixture was concentrated under reduced pressure to remove the THF and used in the next step without further purification.

LC-MS (Method 1): $R_t$ = 0.29 min; MS (ESIpos): m/z = 424.2 $[M+H]^+$.

Step 3

manganese(2+) (2*R*,2'*S*)-2,2'-[13-carboxy-6-oxa-3,9,15-triazabicyclo[9.3.1]pentadeca-1(15), 11,13-triene-3,9-diyl]di-butanoate

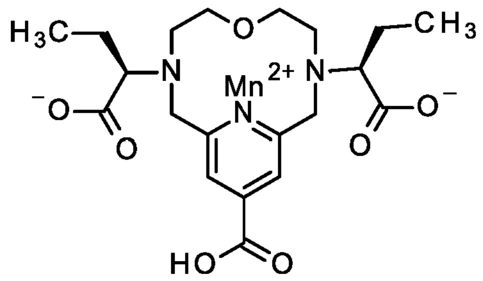

To 3,9-bis (1-carboxypropyl)-9-(1-carboxypropyl)-6-oxa-3,9,15-triazabicyclo[9.3.1] pentadeca-1(15),11,13-triene-13-carboxylic acid (1.21 g, 2.86 mmol) in an aqueous sodium hydroxide solution from step 2 was added water (10 ml) and manganese dichloride tetrahydrate (622 mg, 3.14 mmol). The pH of the suspension was corrected to the value of 7 with aqueous hydrochloric acid (2M) and the mixture was stirred at 100°C for 4 h. After cooling to room temperature Chelex®100 was added and the reaction mixture was stirred for 1 h. Chelex®100 was removed by filtration and the aqueous filtrate was purified by column chromatography (Interchim puriFlash® PF-15 RP18AQ-F00330, water/ acetonitrile, 0 - 15%) to yield 140 mg (98% purity, 10% yield) of the title compound.

LC-MS (Method 1): $R_t$ = 0.39 min; MS (ESIpos): m/z = 477.2 $[M+H]^+$.

## Intermediate 17

**manganese(2+) rac(2*R**,2'*S**)-2,2'-[12-carboxy-6-oxa-3,9,15-triazabicyclo[9.3.1]pentadeca-1(15),11,13-triene-3,9-diyl]dibutanoate**

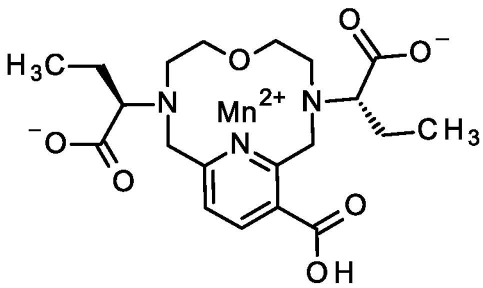

Step 1

methyl 3,9-bis(1-methoxy-1-oxobutan-2-yl)-6-oxa-3,9,15-triazabicyclo[9.3.1]pentadeca-1(15),11,13-triene-12-carboxylate

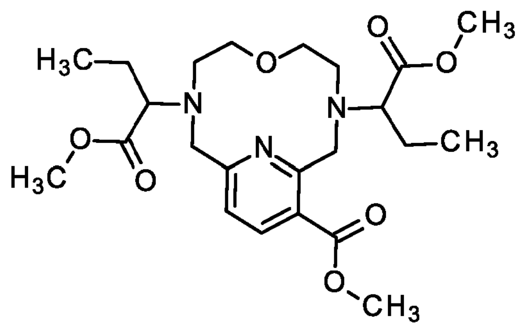

To methyl 6-oxa-3,9,15-triazabicyclo[9.3.1]pentadeca-1(15), 11,13-triene-12-carboxylate hydrogen chloride (1/2) (2.0 g, 5.9 mmol, intermediate 5 step 7) in acetonitrile (100 ml) was added potassium carbonate (4,17 g, 30.2 mmol) and methyl 2-bromobutanoate (3.28 g, 18.1 mmol). The mixture was stirred at RT for 4 days. The mixture was

filtered, and the filtrate concentrated under reduced pressure and dichloromethane was added. The mixture was washed with water, dried with anhydrous sodium sulphate, filtered, and concentrated to give 3.1 g of the title compound as raw product, which was used in the next step without further purification.

LC-MS (Method 2): $R_t$ = 0.86 min; MS (ESIpos): m/z = 466.4 $[M+H]^+$.

Step 2

3,9-bis(1-carboxypropyl)-6-oxa-3,9,15-triazabicyclo[9.3.1]pentadeca-1(15),11,13-triene-12-carboxylic acid

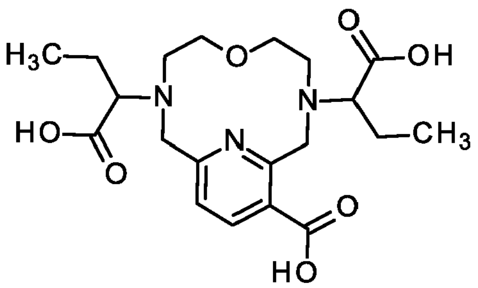

To methyl 3,9-bis(1-methoxy-1-oxobutan-2-yl)-6-oxa-3,9,15-triazabicyclo[9.3.1] pentadeca-1(15),11,13-triene-12-carboxylate (3.1 g crude product from step 1, 5.9 mmol) in THF (100 ml) was added an aqueous sodium hydroxide solution (2.5M, 14 ml, 35 mmol) and the mixture was stirred at room temperature for 3 h. The mixture was concentrated under reduced pressure to remove the THF and used in the next step without further purification.

LC-MS (Method 1): $R_t$ = 0.29 min; MS (ESIpos): m/z = 424.2 $[M+H]^+$.

Step 3

manganese(2+) (2*R**,2'*S**)-2,2'-[12-carboxy-6-oxa-3,9,15-triazabicyclo[9.3.1]pentadeca-1(15),11,13-triene-3,9-diyl]dibutanoate

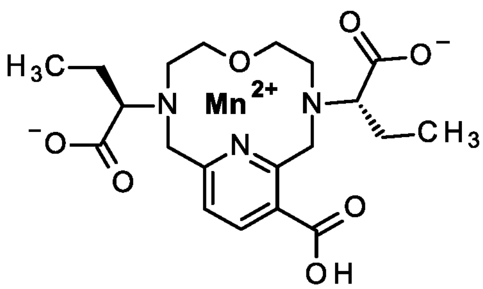

To 3-(1-carboxypropyl)-9-(1-carboxypropyl)-6-oxa-3,9,15-triazabicyclo[9.3.1]pentadeca-1(15),11,13-triene-12-carboxylic acid (2.5 g, 5.9 mmol) in an aqueous sodium hydroxide solution from step 2 was added water (15 ml) and manganese dichloride (892 mg, 7.08 mmol). The pH of the suspension was corrected to the value of 7 with aqueous hydrochloric acid (2M) and the mixture was stirred at 100°C for 10 h. After cooling to room temperature Chelex®100 was added and the reaction mixture was stirred for 1 h. Chelex®100 was removed by filtration and the aqueous filtrate was purified by column chromatography (Interchim puriFlash® PF-15 RP18AQ-F00330, water/ acetonitrile, 0 - 15%) to yield 450 mg (98% purity, 16% yield) of the racemic title compound.

LC-MS (Method 1): $R_t$ = 0.39 min; MS (ESIpos): m/z = 477.3 $[M+H]^+$.

**Intermediate 18**

**manganese(2+) 2-[13-carboxy-9-(carboxylatomethyl)-6-oxa-3,9,15-triazabicyclo[9.3.1]pentadeca-1(15),11,13-trien-3-yl]propanoate**

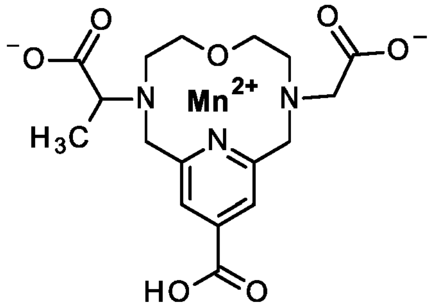

Step 1

methyl 3-(2-methoxy-2-oxoethyl)-9-(1-methoxy-1-oxopropan-2-yl)-6-oxa-3,9,15-triazabicyclo[9.3.1]pentadeca-1(15),11,13-triene-13-carboxylate

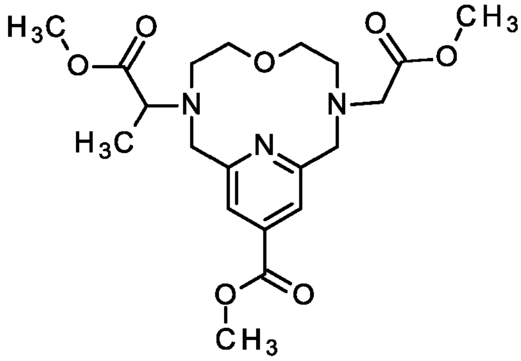

To methyl 6-oxa-3,9,15-triazabicyclo[9.3.1]pentadeca-1(15),11,13-triene-12-carboxylate hydrogen chloride (1/2) (1.0 g, 2.96 mmol, intermediate 1 step 7) in acetonitrile (150 ml) was added potassium carbonate (2.04 g, 14.8 mmol), methyl bromoacetate (452 mg, 2.96 mmol) and methyl 2-bromopropanoate (592 mg, 3.55 mmol). The mixture was stirred at 60°C for 22 h. The mixture was filtered, the filtrate concentrated under reduced pressure, and dichloromethane was added. The mixture was washed with water, dried over anhydrous sodium sulphate, filtered, and concentrated to give 0.7 g of the title compound as raw product in a mixture with other alkylation products, which was used in the next step without further purification.

LC-MS (Method 2): $R_t$ = 0.65 min; MS (ESIpos): m/z = 424.3 $[M+H]^+$.

Step 2

3-(1-carboxyethyl)-9-(carboxymethyl)-6-oxa-3,9,15-triazabicyclo[9.3.1]pentadeca-1(15),11,13-triene-13-carboxylic acid

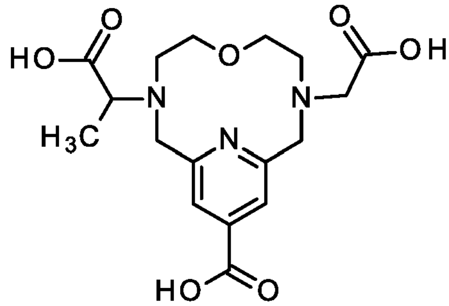

To methyl 3-(2-methoxy-2-oxoethyl)-9-(1-methoxy-1-oxopropan-2-yl)-6-oxa-3,9,15-triazabicyclo[9.3.1]pentadeca-1(15),11,13-triene-13-carboxylate (700 mg, 1.65 mmol, crude product from step 1) in THF (30 ml) was added an aqueous sodium hydroxide solution (2.6 ml, 2.5 M, 6.5 mmol) and the mixture was stirred at room temperature for 18 h. The mixture was concentrated under reduced pressure to remove the THF and used in the next step without further purification.
LC-MS (Method 1): $R_t$ = 0.23 min; MS (ESIpos): m/z = 382.3 $[M+H]^+$.

Step 3

manganese(2+) 2-[13-carboxy-9-(carboxylatomethyl)-6-oxa-3,9,15-triazabicyclo[9.3.1]pentadeca-1(15),11,13-trien-3-yl]propanoate

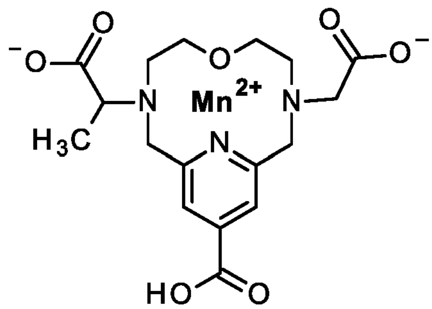

To 3-(1-carboxyethyl)-9-(carboxymethyl)-6-oxa-3,9,15-triazabicyclo[9.3.1]pentadeca-1(15), 11,13-triene-13-carboxylic acid (630 mg, 1.65 mmol) in an aqueous sodium hydroxide solution from step 2 was added water (20 ml) and manganese dichloride (250 mg, 1.98 mmol). The pH of the suspension was corrected to the value of 6.5 with aqueous hydrochloric acid (2M) and the mixture was stirred at 100°C for 1 h. After cooling to room temperature Chelex®100 was added and the reaction mixture was stirred for 1 h. Chelex®100 was removed by filtration and the pH of the filtrate was corrected to the value of 7.5 with aqueous hydrochloric acid (2M).The aqueous solution was purified by column chromatography (Interchim puriFlash® PF-15 RP18AQ-F00120, water/ acetonitrile, 0 - 20%) to yield 405 mg (99% purity, 56% yield) of the racemic title compound.
LC-MS (Method 1): $R_t$ = 0.21 min; MS (ESIpos): m/z = 435.3 $[M+H]^+$.

**Intermediate 19**

**manganese(2+) 2,2'-(14-carboxy-4-methyl-6-oxa-3,9,15-triazabicyclo[9.3.1]pentadeca-1(15),11,13-triene-3,9-diyl)diacetate**

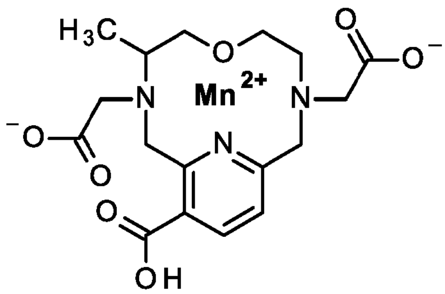

Step 1

ethyl 5-bromo-6-methylpyridine-2-carboxylate

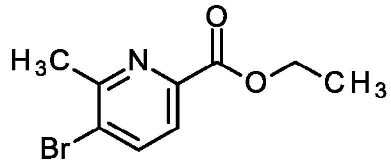

To a solution of 5-bromo-6-methylpyridine-2-carboxylic acid (20.0 g, 92.6 mmol) in ethanol (200 ml) was added sulfuric acid (20 ml, 380 mmol) at room temperature. The mixture was stirred at 80 °C for 12 hours. The mixture was concentrated to give a residue. The residue was diluted with water and quenched with saturated sodium bicarbonate solution at 0 °C. The mixture was extracted with ethyl acetate. After separated, the organic phase was washed with brine, dried with anhydrous sodium sulphate, filtered and concentrated to give ethyl 3-bromo-6-methylpyridine-2-carboxylate (20 g, 95% purity, 84% yield) as yellow solid.

LC-MS (Method 4): $R_t$ = 0.702 min; MS (ESIpos): m/z = 246.3 $[M+1]^+$.

$^1H$ NMR (400 MHz, $CDCl_3$): δ [ppm] = 8.19 (d, 1H), 7.77 (d, 1H), 4.33 (q, 2H), 2.69 (s, 3H), 1.30 (t, 2H).

Step 2

ethyl 5-bromo-6-(bromomethyl)pyridine-2-carboxylate

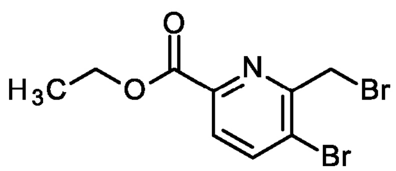

To a solution of ethyl 5-bromo-6-methylpyridine-2-carboxylate (20 g, 99% purity, 81.1 mmol) in 1,2-dichloroethane (500 ml) were added *N*-bromosuccinimide (42 ml, 240 mmol) and 2,2'-azobisisobutyronitrile (6.66 g, 40.6 mmol) at room temperature. The mixture was stirred at 90°C for 12 hours under nitrogen atmosphere in dark condition. The reaction was diluted with dichloromethane and washed with saturated sodium bicarbonate solution. After phase separation, the organic phase was washed with brine, dried with anhydrous sodium sulphate, filtered and concentrated in vacuo. The residue was dissolved in tetrahydrofuran (700 ml). To the mixture was added *N,N*-diisopropylethylamine (42.4 ml, 243 mmol) and diethyl phosphite (31 ml, 243 mmol) at 0°C. The mixture was stirred at 25 °C for 16 hours. The reaction mixture was concentrated to remove most solvent. The residue was diluted with water and extracted with ethyl acetate. The combined organic phase was washed with ammonium chloride solution. The organic layers were washed with brine, dried over sodium sulphate, filtered and concentrated under reduced pressure to give a residue. The residue was purified by column chromatography on silica gel (petroleum ether/ethyl acetate = 50 : 1 to 10 : 1) to give ethyl 5-bromo-6-(bromomethyl)pyridine-2-carboxylate (16 g, 65% purity, 40% yield) as yellow solid.

LC-MS (Method 4): $R_t$ = 0.802 min; MS (ESIpos): m/z = 323.9 $[M+1]^+$.

Step 3

*tert*-butyl {2-[(2-methylprop-2-en-1-yl)oxy]ethyl}carbamate

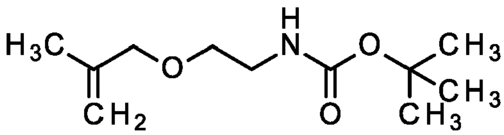

To a solution of tert-butyl (2-hydroxyethyl)carbamate (84.9 g, 527 mmol) in dichloromethane (1.0 l) were added 3-bromo-2-methylprop-1-ene (78.2 g, 579 mmol), *N,N,N*-tributylbutan-1-aminium bromide (119 g, 369 mmol)and sodium hydroxide (510 ml, 10 M in water, 5.1 mol) at 0 °C. The mixture was stirred at room temperature for 12 hours. The mixture was diluted with water extracted with dichloromethane. The organic phase was washed with saturated aqueous ammonium chloride solution and brine, dried over anhydrous sodium sulphate, filtered and concentrated under reduced pressure. The residue was purified by column chromatography on silica gel (1000 mesh, petroleum ether: ethyl acetate = 1: 0 to 3: 1) to give *tert*-butyl {2-[(2-methylprop-2-en-1-yl)oxy]ethyl}carbamate (100 g) as a colourless oil.

$^1H$ NMR (400 MHz, $CDCl_3$): δ [ppm] = 4.96-4.75 (m, 3H), 3.88 (s, 2H), 3.50-3.40 (m, 2H), 3.35-3.25 (m, 2H), 1.72 (s, 3H), 1.44 (s, 9H).

Step 4

*tert*-butyl [2-(2-oxopropoxy)ethyl]carbamate

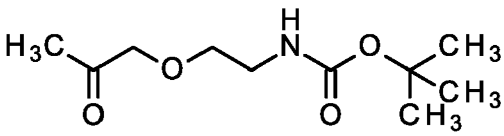

To a solution of *tert*-butyl {2-[(2-methylprop-2-en-1-yl)oxy]ethyl}carbamate (50 g, 232 mmol) in tetrahydrofuran (1.0 l) and water (1.0 l) were added dipotassium osmate (3.86 g, 11.6 mmol) and sodium periodate (124 g, 581 mmol) at room temperature. The mixture was stirred at room temperature for 2 hours and was combined with an identically treated 50 g batch. The combined mixture was diluted with water and extracted with ethyl acetate. The organic phase was washed with brine, dried with anhydrous sodium sulfate, filtered and concentrated to give a residue. The residue was purified by column chromatography on silica gel (1000 mesh, petroleum ether: ethyl acetate, 10: 1 to 0: 1) to give *tert*-butyl [2-(2-oxopropoxy)ethyl]carbamate (75 g) as brown oil.

$^1H$ NMR (400 MHz, $CDCl_3$): δ [ppm] =5.20-5.00 (m, 1H), 4.07 (s, 2H), 3.60-3.48 (m, 2H), 3.35-3.25 (m, 2H), 2.09 (s, 3H), 1.42 (s, 9H).

Step 5

*tert*-butyl {2-[2-(benzylamino)propoxy]ethyl}carbamate

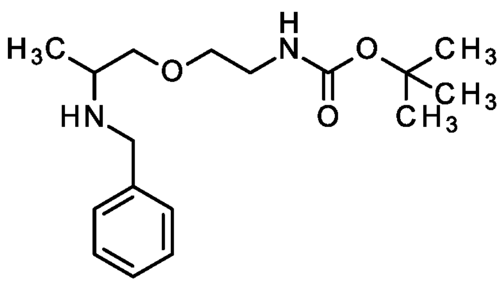

To a solution of *tert*-butyl [2-(2-oxopropoxy)ethyl]carbamate (37.5 g, 173 mmol) in methanol (1.5 l) were added 1-phenylmethanamine (19 ml, 170 mmol) and sodium cyanoborohydride (21.7 g, 345 mmol) at room temperature. The mixture was stirred at room temperature for 12 hours. Then the mixture was stirred at 50 °C for 16 hours. The mixture was combined with a batch generated from, 37.5 g *tert*-butyl [2-(2-oxopropoxy)ethyl]carbamate in an identical way and the combined mixture was concentrated to give a residue. The residue was diluted with water and extracted with ethyl acetate. The organic phase was dried over anhydrous sodium sulphate, filtered and concentrated to give a residue. The residue was purified by reversed phase HPLC (instrument: Agela HP1000; column: Phenomenex Luna C18 15 μm 100 Å, 150 x 500 mm; eluent A: water, eluent B: acetonitrile; gradient: 0-90 min 30-80% B; flow 300 ml/min; temperature: room temperature; detector: UV 220/254 nm) to give tert-butyl (2-{[2-(benzylamino)propyl]oxy}ethyl)carbamate (80 g) as yellow oil.

LC-MS (Method 3): $R_t$ = 0.520 min; MS (ESIpos): m/z = 309.2 $[M+1]^+$.

Step 6

ethyl 6-[2-benzyl-3,11,11-trimethyl-9-oxo-5,10-dioxa-2,8-diazadodecan-1-yl]-5-bromopyridine-2-carboxylate

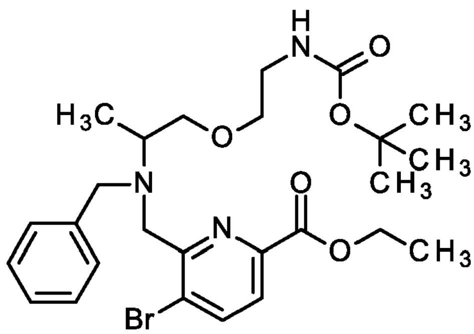

To a solution of tert-butyl {2-[2-(benzylamino)propoxy]ethyl}carbamate (15.3 g, 49.5 mmol, step 5) in acetonitrile (200 ml) were added *N,N*-diisopropylethylamine (17 ml, 99 mmol) and ethyl 5-bromo-6-(bromomethyl)pyridine-2-carboxylate (16 g, 49.5 mmol, step 2) at room temperature. The mixture was stirred at room temperature for 12 hours. The mixture was concentrated to give a residue. The residue was reversed phase column (instrument: Agela HP1000; column: Welch Ultimate XB_C18 20 μm, 150 x 400 mm; eluent A: 0.225% formic acid in water, eluent B: acetonitrile; gradient: 0-25 min 20 - 80% B; flow 100 ml/min; temperature: room temperature; detector: UV 220/254 nm) to give ethyl 6-[2-benzyl-3,11,11-trimethyl-9-oxo-5,10-dioxa-2,8-diazadodec-1-yl]-5-bromo pyridine-2-carboxylate (19.5 g, 72% yield) as yellow oil.

LC-MS (Method 4): $R_t$ = 0.510 min; MS (ESIpos): m/z = 552.2 $[M+1]^+$.

$^1H$ NMR (400 MHz, $CDCl_3$): δ [ppm] = 7.95-7.85 (m, 1H), 7.80-7.70 (m, 1H), 7.35-7.27 (m, 2H) 7.25-7.05 (m, 3H), 5.20-4.92 (m, 1H), 4.56-4.34 (m, 2H), 4.25-4.05 (m, 2H), 3.96-3.61 (m, 3H), 3.54-3.36 (m, 3H), 3.35-3.05 (m, 3H), 1.50-1.35 (m, 12H), 1.25-1.08 (m, 3H).

Step 7

6-({[1-(2-aminoethoxy)propan-2-yl](benzyl)amino}methyl)-5-bromopyridine-2-carboxylic acid - hydrogen chloride (1/1)

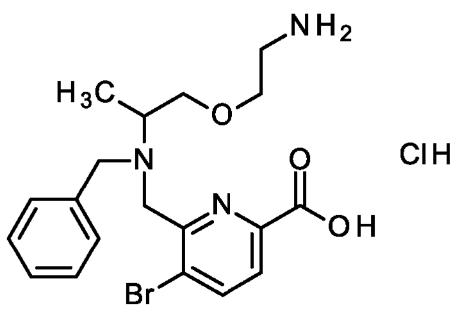

To a solution of ethyl 6-[2-benzyl-3,11,11-trimethyl-9-oxo-5,10-dioxa-2,8-diazadodecan-1-yl]-5-bromopyridine-2-carboxylate (19.5 g, 35.4 mmol) in 1,4-dioxane (10 ml) was added hydrochloric acid (200 ml, 12M) at room temperature. The reaction mixture was stirred at 50°C for 16 hours. The reaction mixture was concentrated to give 6-({[1-(2-aminoethoxy)propan-2-yl](benzyl)amino}methyl)-5-bromopyridine-2-carboxylic acid - hydrogen chloride (1/1) (16.25 g, crude) and directly used in the next step without further purification.
LC-MS (Method 4): $R_t$ = 0.362 min; MS (ESIpos): m/z = 424.1 $[M+1]^+$.

Step 8

9-benzyl-12-bromo-8-methyl-6-oxa-3,9,15-triazabicyclo[9.3.1]pentadeca-1(15),11,13-trien-2-one

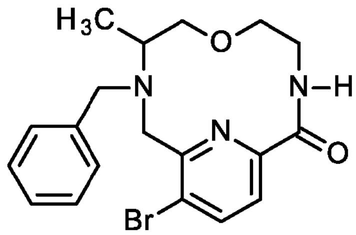

To a solution of 6-({[1-(2-aminoethoxy)propan-2-yl](benzyl)amino}methyl)-5-bromopyridine-2-carboxylic acid - hydrogen chloride (1/1) (16.3 g, 35.4 mmol) in acetonitrile (1.6 l) was added *N,N*-diisopropylethylamine (19 ml, 110 mmol) and 2,4,6-tributyl-1,3,5,2,4,6-trioxatriphosphorinane-2,4,6-trioxide (30.4 g, 50% in ethyl acetate, 42 mmol) at room temperature. The mixture was stirred at room temperature for 12 hours. The reaction mixture was concentrated, diluted with ethyl acetate and washed with water. The organic phase was dried over anhydrous sodium sulphate and concentrated to give a residue. The residue was purified by preparative reversed phase HPLC (column: Welch Xtimate® C18, 10 μm, 40 x 150 mm; eluent A: 0.225% formic acid in water, eluent B: acetonitrile; gradient: 0-10 min 10-40% B; flow 80 ml/min; temperature: room temperature; detector: UV 220/254 nm) to give 9-benzyl-12-bromo-8-methyl-6-oxa-3,9,15-triazabicyclo[9.3.1] pentadeca-1(15),11,13-trien-2-one (11.4 g, 80% yield) as a yellow solid.
LC-MS (Method 4): $R_t$ = 0.436 min; MS (ESIpos): m/z = 404.0 $[M+1]^+$.

Step 9

3-benzyl-14-bromo-4-methyl-6-oxa-3,9,15-triazabicyclo[9.3.1]pentadeca-1(15),11,13-triene

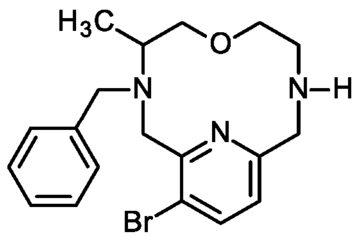

To a solution of 9-benzyl-12-bromo-8-methyl-6-oxa-3,9,15-triazabicyclo[9.3.1] pentadeca-1(15),11,13-trien-2-one (10.4 g, 25.7 mmol) in tetrahydrofuran (100 ml) were added sodium tetrahydroborate (2.1 g, 56.6 mmol) and then dropwise boron trifluoride diethyl etherate (7.2 ml, 57 mmol) at 0 °C under nitrogen. The mixture was stirred at room temperature for 16 hours under nitrogen. The reaction mixture was then directly used in the next step reaction without further purification.
LC-MS (Method 4): $R_t$ = 0.346 min; MS (ESIpos): m/z = 392.2 $[M+1]^+$.

Step 10

*tert*-butyl 9-benzyl-12-bromo-8-methyl-6-oxa-3,9,15-triazabicyclo[9.3.1]pentadeca-1(15), 11, 13-triene-3-carboxylate

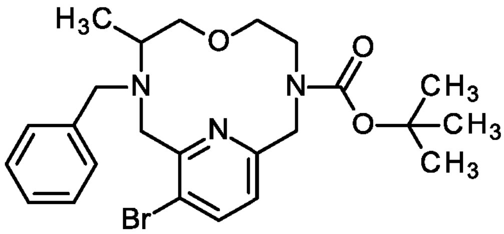

To a solution of 3-benzyl-14-bromo-4-methyl-6-oxa-3,9,15-triazabicyclo[9.3.1] pentadeca-1(15),11,13-triene (10 g, 25.6 mmol) in water (100 ml) and tetrahydrofuran (100 ml) was added sodium hydrogen carbonate (6.46 g, 76.9 mmol) and di-tert-butyl dicarbonate (8.8 ml, 38 mmol) at room temperature. The reaction mixture was stirred at room temperature for 16 hours. The reaction mixture was diluted with water and extracted with ethyl acetate. The organic phase was washed with water and brine, dried over anhydrous sodium sulphate and concentrated to give a residue. The residue was purified by preparative reversed phase HPLC (column: Welch Xtimate® C18, 10 μm, 40 x 150 mm; eluent A: 0.225% formic acid in water, eluent B: acetonitrile; gradient: 0-10 min 10-40% B; flow 80 ml/min; temperature: room temperature; Detector: UV 220/254 nm) to give *tert*-butyl 9-benzyl-12-bromo-8-methyl-6-oxa-3,9,15-triazabicyclo[9.3.1]pentadeca-1(15),11,13-triene-3-carboxylate (3.20 g, 25% yield) as a yellow solid.

LC-MS (Method 4): $R_t$ = 0.535 min; MS (ESIpos): m/z = 490.1 $[M+1]^+$.

Step 11

3-*tert*-butyl 12-methyl 9-benzyl-8-methyl-6-oxa-3,9,15-triazabicyclo[9.3.1]pentadeca-1(15),11,13-triene-3,12-dicarboxylate

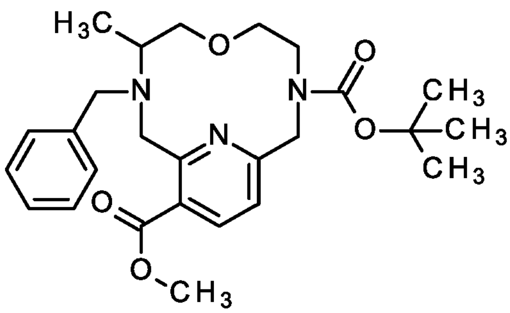

To a solution of *tert*-butyl 9-benzyl-12-bromo-8-methyl-6-oxa-3,9,15-triazabicyclo[9.3.1]pentadeca-1(15),11,13-triene-3-carboxylate (3.20 g, 6.52 mmol) in methanol (32 ml) added triethylamine (1.8 ml, 13 mmol) and [1,1-bis(diphenylphosphino)ferrocene]palladium(II) dichloride (477 mg, 652 μmol) at room temperature. The reaction mixture was stirred at 80°C for 48 hours under carbon monoxide atmosphere (45 psi). The reaction mixture was concentrated to give a crude product. The crude product was purified by preparative reversed phase HPLC (column: Welch Xtimate® C18, 10 μm, 40 x 150 mm; eluent A: 0.225% formic acid in water, eluent B: acetonitrile; gradient: 0-10 min 10-40% B; flow 80 ml/min; temperature: room temperature; detector: UV 220/254 nm) to give 3-*tert*-butyl 12-methyl 9-benzyl-8-methyl-6-oxa-3,9,15-triazabicyclo[9.3.1]pentadeca-1(15),11,13-triene-3,12-dicarboxylate (3.00 g, 98% yield) as a yellow oil.

LC-MS (Method 4): $R_t$ = 0.518 min; MS (ESIpos): m/z = 470.2 $[M+1]^+$.

Step 12

3-*tert*-butyl 12-methyl 8-methyl-6-oxa-3,9,15-triazabicyclo[9.3.1]pentadeca-1(15),11,13-triene-3,12-dicarboxylate

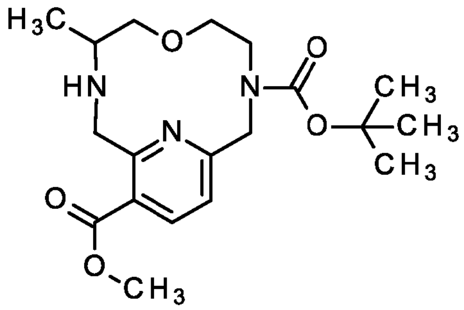

To a solution of 3-*tert*-butyl 12-methyl 9-benzyl-8-methyl-6-oxa-3,9,15-triazabicyclo [9.3.1]pentadeca-1(15),11,13-triene-3,12-dicarboxylate (3.0 g, 6.39 mmol) in methanol (30 ml) was added palladium (500 mg, 10% on activated carbon), palladium(II) hydroxide (500 mg, 20% on activated carbon) under nitrogen atmosphere. The mixture was stirred at 25 °C for 16 hours under hydrogen (45 psi) atmosphere. The reaction mixture was filtered and the filtrate was concentrated to give 3-*tert*-butyl 12-methyl 8-methyl-6-oxa-3,9,15-triazabicyclo [9.3.1]pentadeca-1(15),11,13-triene-3,12-dicarboxylate (2.4 g, crude) and used directly for the next step without further purification.

LC-MS (Method 4): $R_t$ = 0.496 min; MS (ESIpos): m/z = 380.3 $[M+1]^+$.

Step 13

methyl 8-methyl-6-oxa-3,9,15-triazabicyclo[9.3.1]pentadeca-1(15),11,13-triene-12-carboxylate - hydrogen chloride (1/2)

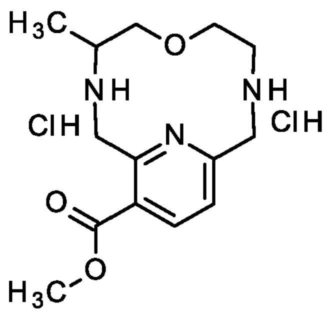

To a solution of 3-tert-butyl 12-methyl 8-methyl-6-oxa-3,9,15-triazabicyclo [9.3.1]pentadeca-1(15),11,13-triene-3,12-dicarboxylate (2.4 g, 6.32 mmol) in 1,4-dioxane (5.0 ml) was added hydrochloric acid (30 ml, 2 M in 1,4-dioxane) at room temperature. The reaction mixture was stirred at room temperature for 2 hours. The reaction mixture was concentrated to give 3-tert-butyl 12-methyl 8-methyl-6-oxa-3,9,15-triazabicyclo[9.3.1]pentadeca-1(15),11,13-triene-3,12-dicarboxylate (2.3 g) as a yellow solid.

LC-MS (Method 3): $R_t$ = 0.156 min; MS (ESIpos): m/z = 280.3 $[M+1]^+$.

Step 14

formic acid - methyl 3,9-bis(2-methoxy-2-oxoethyl)-8-methyl-6-oxa-3,9,15-triazabicyclo[9.3.1] pentadeca-1(15),11,13-triene-12-carboxylate (1/1)

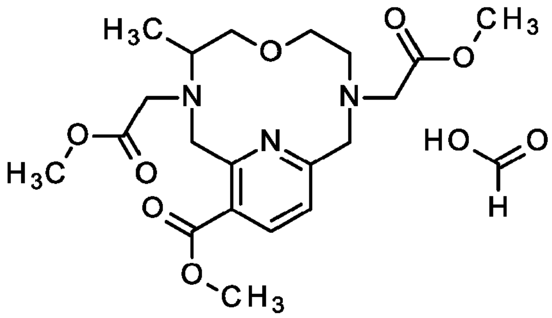

To a solution of methyl 8-methyl-6-oxa-3,9,15-triazabicyclo[9.3.1]pentadeca-1(15),11,13-triene-12-carboxylate - hydrogen chloride (1/2) (2.30 g, 6.53 mmol) in acetonitrile (45 ml) was added *N,N*-diisopropylethylamine (6.8 ml, 39 mmol) and methyl bromoacetate (2.50 g, 16.3 mmol) at room temperature. The reaction mixture was stirred at room temperature for 16 hours. The reaction mixture was concentrated to give a crude product. The crude product was purified by reversed phase chromatography (Instrument: ACSWH-GX-C; Column: Xtimate C18, 10 μm, 40 x 150 mm; eluent A: 0.225% formic acid in water, eluent B: acetonitrile; gradient: 0-10 min 0-20% B; flow 80 ml/min; temperature: room temperature; Detector: UV 220/254 nm) to give methyl 3,9-bis(2-methoxy-2-oxoethyl)-8-methyl-6-oxa-3,9,15-triazabicyclo[9.3.1]pentadeca-1(15),11,13-triene-12-carboxylate (700 mg, 86% purity, 20% yield) as a yellow oil.
LC-MS (Method 4): $R_t$ = 0.437 min; MS (ESIpos): m/z = 424.1 $[M+1]^+$.
$^1H$ NMR (400 MHz, $CDCl_3$): δ [ppm] =8.43 (d, 1H), 8.39 (s, 1H), 7.46 (d, 1H), 4.65-4.50 (m, 2H) 4.35 (s, 2H), 3.95-3.90 (m, 3H), 3.75-3.68 (m, 6H), 3.66 (s, 3H), 3.61-3.53 (m, 2H), 3.39-3.31 (m, 2H), 3.30-3.20 (m, 1H), 3.19-3.08 (m, 2H), 2.99-2.93 (m, 1H), 1.09 (d, 3H).

Step 15

3,9-bis(carboxymethyl)-8-methyl-6-oxa-3,9,15-triazabicyclo[9.3.1]pentadeca-1(15),11,13-triene-12-carboxylic acid

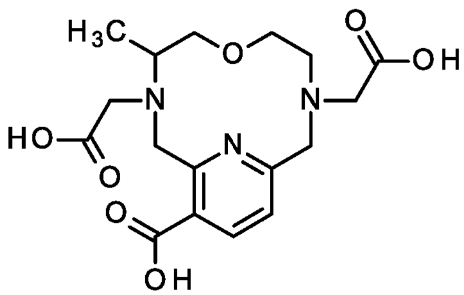

To methyl 3,9-bis(2-methoxy-2-oxoethyl)-8-methyl-6-oxa-3,9,15-triazabicyclo[9.3.1] pentadeca-1(15),11,13-triene-12-carboxylate formic acid salt (1/1) (700 mg, 1.49 mmol) in THF (30 ml) was added an aqueous sodium hydroxide solution (3.6 ml, 2.5 M, 8.9 mmol) and the mixture was stirred at room temperature for 3 h. The mixture was concentrated under reduced pressure to remove the THF and used in the next step without further purification.
LC-MS (Method 2): $R_t$ = 0.20 min; MS (ESIpos): m/z = 382.3 $[M+H]^+$.

Step 16

manganese(2+) 2,2'-(14-carboxy-4-methyl-6-oxa-3,9,15-triazabicyclo[9.3.1]pentadeca-1(15),11,13-triene-3,9-diyl)diacetate

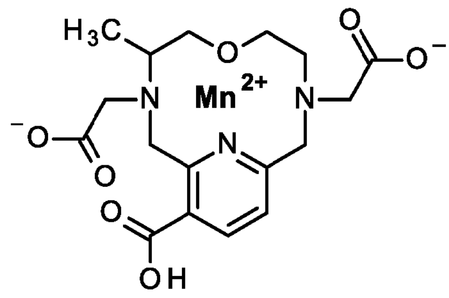

To 3,9-bis(carboxymethyl)-8-methyl-6-oxa-3,9,15-triazabicyclo[9.3.1]pentadeca-1(15), 11,13-triene-12-carboxylic acid (569 mg, 1.49 mmol) in an aqueous sodium hydroxide solution from step 15 was added water (20 ml) and dichloromanganese tetrahydrate (225 mg, 1.79 mmol). The pH of the suspension was corrected to the value of 6.5 with aqueous hydrochloric acid (2M) and the mixture was stirred at 100°C for 10 h. After cooling to room temperature Chelex®100 was added and the reaction mixture was stirred for 1 h. Chelex®100 was removed by filtration and the pH of the filtrate was corrected to the value of 7.5 with aqueous hydrochloric acid (2M).The aqueous solution was purified by column chromatography (Interchim puriFlash® PF-15 RP18AQ-F00330, water/ acetonitrile, 0 - 20%) to yield 250 mg (98% purity, 38% yield) of the racemic title compound.

LC-MS (Method 1): $R_t$ = 0.23 min; MS (ESIpos): m/z = 435.3 $[M+H]^+$.

<u>**Intermediate 20**</u>

**manganese(2+) (2*S*,2'*S*)-2,2'-[13-carboxy-6-oxa-3,9,15-triazabicyclo[9.3.1] pentadeca-1(15),11,13-triene-3,9-diyl]dipropanoate**

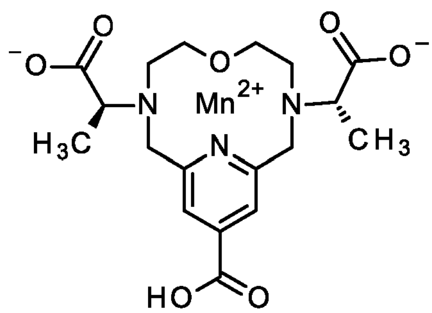

<u>Step 1</u>

methyl 6-oxa-3,9,15-triazabicyclo[9.3.1]pentadeca-1(15), 11,13-triene-13-carboxylate

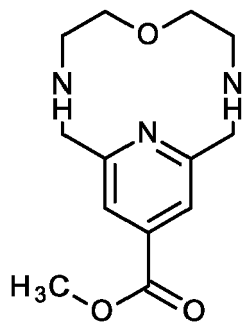

Methyl 6-oxa-3,9,15-triazabicyclo[9.3.1]pentadeca-1(15),11,13-triene-13-carboxylate-hydrogen chloride (10 g, 29.6 mmol, intermediate 1 step 7) was stirred with DCM (500 ml). NaOH solution (200 ml 10M) was added, and the mixture

stirred for 30 minutes. The phases were separated, and the aqueous layer extracted with DCM (2 x) The combined DCM phases were passed through a water excluding filter and concentrated under reduced pressure yielding the title compound (6.05 g, 77% yield).

LC-MS (Method 2): $R_t$ = 0.23 min; MS (ESIpos): m/z = 266.4 $[M+H]^+$.

$^1H$ NMR (DMSO-d6, 400 MHz): δ (ppm) 7.58 (s, 2H), 3.88 (s, 7H), 2.78-2.91 (m, 4H), 2.55-2.65 (m, 4H).

Step 2

methyl 3,9-bis[(2*S*)-1-ethoxy-1-oxopropan-2-yl]-6-oxa-3,9,15-triazabicyclo[9.3.1]pentadeca-1(15),11,13-triene-13-carboxylate

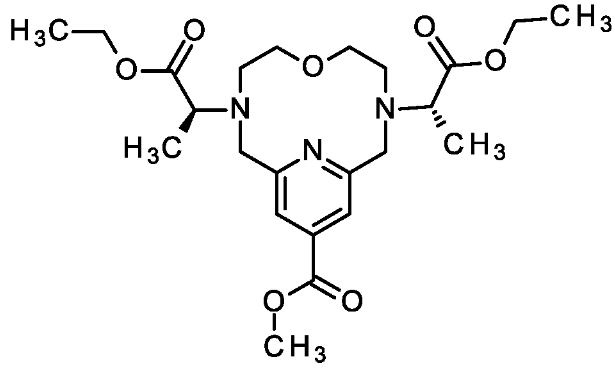

To a mixture of methyl 6-oxa-3,9,15-triazabicyclo[9.3.1]pentadeca-1(15),11,13-triene-13-carboxylate (615 mg, 2.32 mmol) in acetonitrile (20 ml) and potassium carbonate (1.28 g, 9.27 mmol), ethyl (2*R*)-2-[(methanesulfonyl)oxy] propanoate (1.0 g, 5.10 mmol, CAS-RN:[68331-44-2]) was slowly added, and the mixture was stirred at 50°C for 96 h. The reaction mixture was filtered, the filtrate was concentrated under reduced pressure. The residue was treated with DCM/water and was stirred for 5 minutes. The layers were separated, the organic layer was dried over sodium sulphate and concentrated under reduced pressure, yielding the crude title compound (1080 mg, 100% theoretical yield), which was used without further purification in the following step.

LC-MS (Method 2): $R_t$ = 1.02 min; MS (ESIpos): m/z = 466.7 $[M+H]^+$ .

Step 3

3,9-bis[(1*S*)-1-carboxyethyl]-6-oxa-3,9,15-triazabicyclo[9.3.1]pentadeca-1(15),11,13-triene-13-carboxylic acid

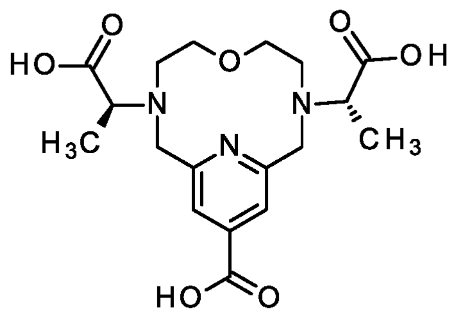

Methyl 3,9-bis[(2*S*)-1-ethoxy-1-oxopropan-2-yl]-6-oxa-3,9,15-triazabicyclo[9.3.1] pentadeca-1(15),11,13-triene-13-carboxylate (1.08 g, 2.32 mmol) was dissolved in aqueous sodium hydroxide solution (3.5 ml, 2.0 M, 7.0 mmol) and THF (10 ml) was added. The mixture was stirred at RT for 48h, the mixture was concentrated under reduced pressure used in the following step without further purification.

LC-MS (Method 2): $R_t$ = 0.28 min; MS (ESIpos): m/z = 396.4 $[M+H]^+$ .

Step 4

manganese(2+) (2*S*,2'*S*)-2,2'-[13-carboxy-6-oxa-3,9,15-triazabicyclo[9.3.1]pentadeca-1(15), 11,13-triene-3,9-diyl]dipropanoate

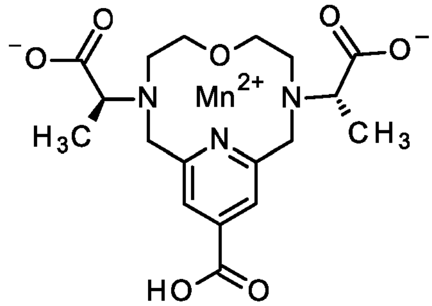

To the crude mixture of 3,9-bis[(1S)-1-carboxyethyl]-6-oxa-3,9,15-triazabicyclo [9.3.1]pentadeca-1(15),11,13-triene-13-carboxylic acid (theoretically 920 mg presuming 100% yield over 2 steps, 2.33 mmol) was added water (7.9 ml) and $MnCl_2.4H_2O$ (322 mg, 2.56 mmol; CAS-RN:[7773-01-5]), then pH was set to 6 - 7 with 2M HCl. The mixture was stirred at RT for 16h. The title compound was purified by reverse phase medium pressure preparative HPLC (Interchim puriFlash® PF-15 RP18AQ-F00330, water / acetonitrile, 0 - 40%, 127 ml/minute flow rate). The product containing fractions were concentrated and freeze dried yielding the title compound as an off white solid (500 mg, 48% yield).

LC-MS (Method 1): $R_t$ = 0.20 min; MS (ESIpos): m/z = 449.4 $[M+H]^+$

**Intermediate 21**

**manganese(2+) sodium 3,9-bis[(1*R*)-1-carboxylatoethyl]-6-oxa-3,9,15-triazabicyclo[9.3.1]pentadeca-1(15),11,13-triene-13-carboxylate (1/1/1)**

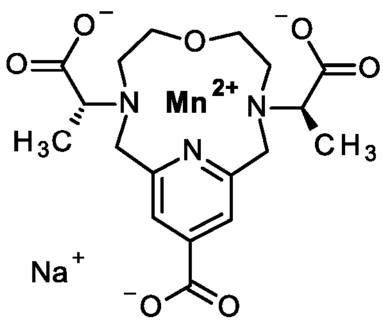

Step 1

methyl 3,9-bis[(1*R*)-2-ethoxy-1-methyl-2-oxo-ethyl]-6-oxa-3,9,15-triazabicyclo[9.3.1]pentadeca-1(14),11(15),12-triene-13-carboxylate

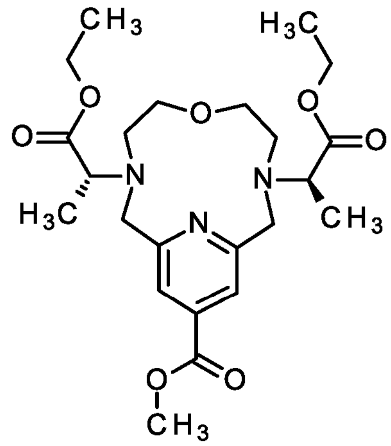

Methyl 6-oxa-3,9, 15-triazabicyclo[9.3.1]pentadeca-1 (15), 11, 13-triene-13-carboxylate (5.0 g, 18.8 mmol, intermediate 20 step1), potassium carbonate (7.81 g, 56.5 mmol) were stirred in acetonitrile (120 ml), ethyl (2S)-2-[(trifluoromethanesulfonyl)oxy]propanoate (4.48 g, 17.9 mmol, CAS-RN:[84028-88-6]), dissolved in acetonitrile (5 ml), was slowly added and the mixture stirred overnight at RT. The reaction mixture was filtered and concentrated under reduced pressure. The crude material was absorbed to Isolute® HM - N, and the mixture purified by preparative medium pressure liquid chromatography (Biotage® Sfär C18 400 g column, water with 0.1% formic acid / acetonitrile, 0 - 50% ,65 ml/min flow rate). The fraction containing the title compound were neutralized with ammonia, concentrated under reduced pressure and lyophilized yielding the title compound (2.06 g, 47% yield).
LC-MS (Method 2): $R_t$ = 0.90 min; MS (ESIpos): m/z = 466.5 $[M+H]^+$
LC-MS (Standard Agilent): Rt = 0.83 min (100% DAD); MS (ESIpos): m/z = 234 [M + 2H]++, 466 [M + H]+.
HRMS (ESI, $[M+H]^+$): calcd for C23H36N3O7, 466.2553; found. 466.2544 (-1.9 ppm).
Optical rotation: $[\alpha]_D$ = +5.64° +/-0.23° (c = 5.2 mg/ml, MeOH).

<u>Step 2</u>

manganese(2+) sodium 3,9-bis[(1*R*)-1-carboxylatoethyl]-6-oxa-3,9,15-triazabicyclo[9.3.1]pentadeca-1(15),11,13-triene-13-carboxylate (1/1/1)

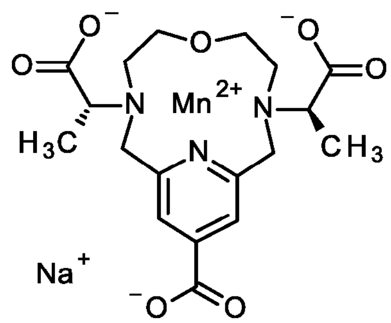

Methyl 3,9-bis[(1*R*)-2-ethoxy-1-methyl-2-oxo-ethyl]-6-oxa-3,9,15-triazabicyclo[9.3.1] pentadeca-1(14),11(15),12-triene-13-carboxylate was added to a mixture of water and DCM. The pH of the aqueous phase was set to 10 using NaOH (10M, aqueous) and the layers stirred together. The layers were separated, and the aqueous layer was washed with a further portion of DCM. Aqueous layer was concentrated under reduced pressure yielding the triacid intermediate (1.80 g, 4.55 mmol). The material was taken up in water (50 ml), dichloromanganese tetrahydrate (901 mg, 4.55 mmol) was added and the pH adjusted (using 1 M HCl) from pH 11 to pH=7 and the mixture was stirred overnight. Chelex® 100 was added and the mixture stirred for 1 h. The mixture was then absorbed to Isolute® HM-N and directly subjected to column chromatography (Interchim puriFlash® PF-15 RP18AQ-F00120, water/ acetonitrile, 0 - 20%, 20 ml/minute flow rate) for purification. The fraction containing the title compound were combined and lyophilised to yield 426 mg of the title compound (99% purity, 20% yield) and 145 mg in 96% purity (6% yield).
LC-MS (Method 1): $R_t$ = 0.23 min; MS (ESIpos): m/z = 449.4 $[M+H]^+$
HRMS (ESI, $[M+H]^+$): calcd for C18H24N3O7Mn, 449.0990; found: 449.0996 (+1.4 ppm).

**Synthesis of the Examples**

**Example 1**

**tetramanganese (2+) 2,2',2'',2'''-{[2,2-bis({[3,9-bis(carboxylatomethyl)-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo [9.3.1]pentadeca-1(15),11,13-triene-13,3,9-triyl]}tetraacetate**

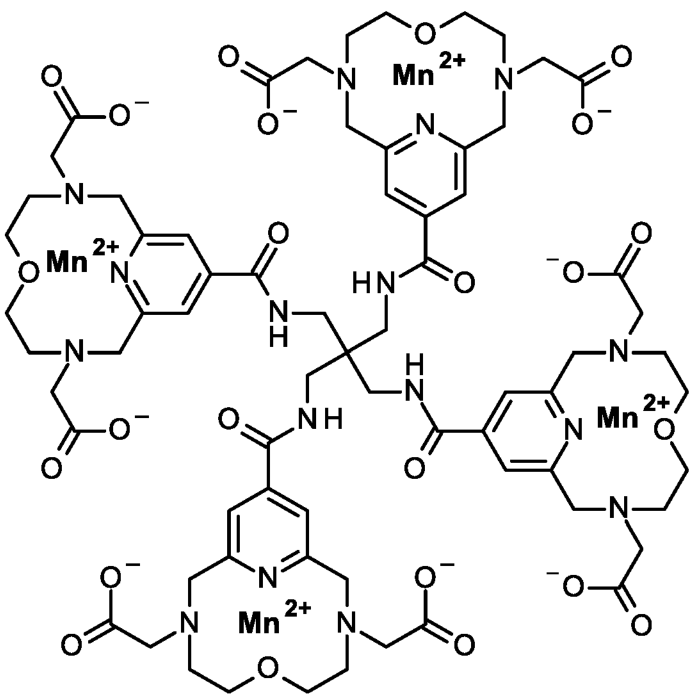

Step 1

tetra-*tert*-butyl 2,2',2'',2'''-{[2,2-bis({[3,9-bis(2-tert-butoxy-2-oxoethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1]pentadeca-1(15),11,13-triene-13,3,9-triyl]}tetraacetate

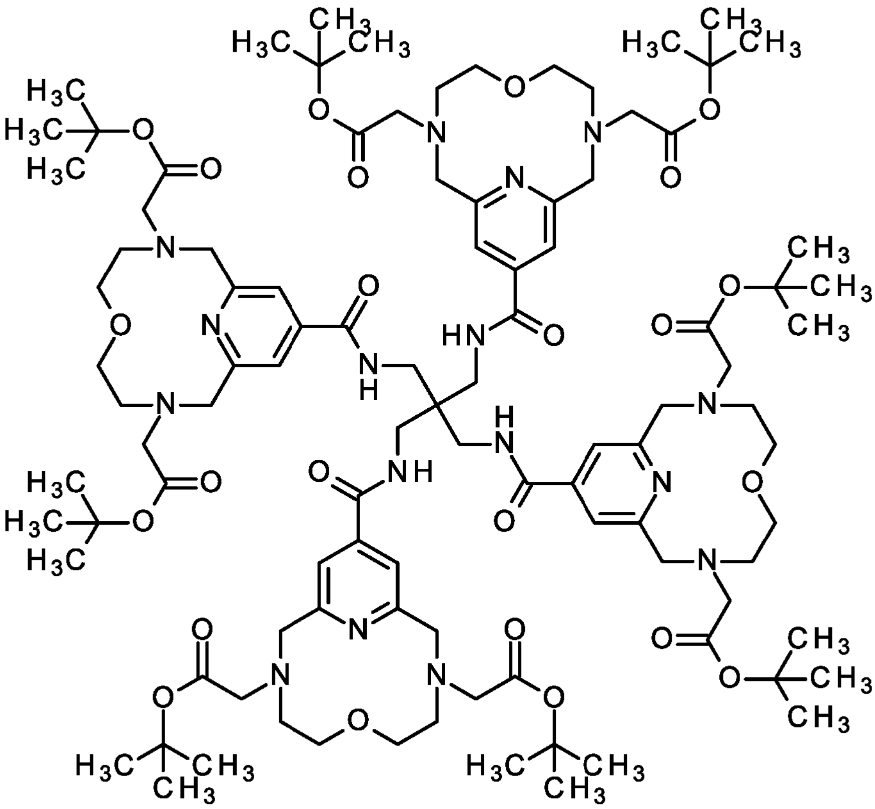

A solution of 2,3,5,6-tetrafluorophenyl 3,9-bis(2-tert-butoxy-2-oxoethyl)-6-oxa-3,9,15-triazabicyclo[9.3.1]pentadeca-1(15),11,13-triene-13-carboxylate (1.80 g, 70% purity, 1.43 mmol, Intermediate 1) in dichloromethane (16 mL) was added to a solution of 2,2-bis(aminomethyl)propane-1,3-diamine hydrogen chloride (1/4) (90.6 mg, 326 μmol) and N,N-diisopropylethylamine (850 μl, 4.9 mmol) in DMF, which was stirred for 10 minutes before addition. The reaction mixture was stirred at room temperature for 19 h. The reaction mixture was diluted with saturated aqueous sodium carbonate and extracted with ethyl acetate. The combined organic layer was washed with brine, dried over anhydrous sodium sulphate and concentrated under reduced pressure. The crude product was purified by preparative HPLC (Biotage Sfär C18D, acetonitrile / water +0,1% formic acid) to give 713 mg of the title compound (81% purity, 90% yield).

LC-MS (Method 2): $R_t$ = 0.95 min; MS (ESIpos): m/z = 990.4 $[M+2H]^{2+}$, 660.6 $[M+3H]^{3+}$.

Step 2

2,2',2",2'''-{[2,2-bis({[3,9-bis(carboxymethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1]pentadeca-1(15),11,13-triene-13,3,9-triyl]}tetraacetic acid

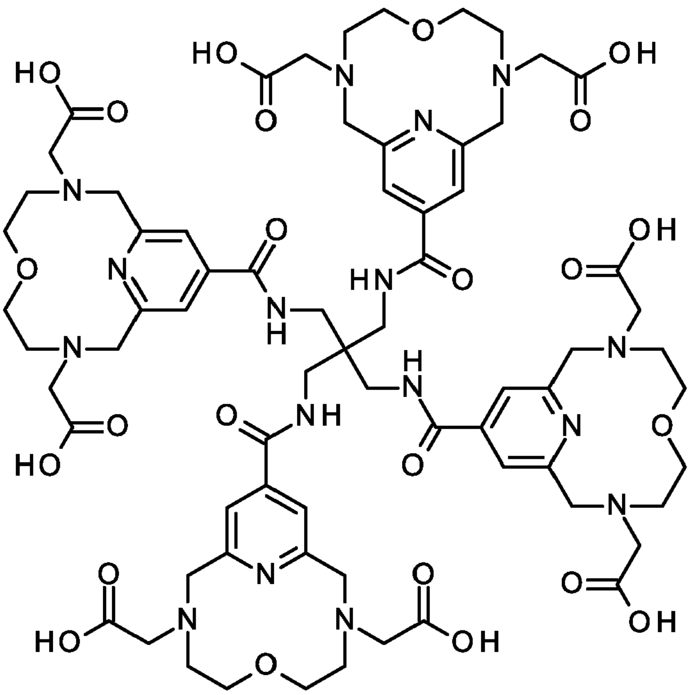

To tetra-*tert*-butyl 2,2',2",2'''-{[2,2-bis({[3,9-bis(2-tert-butoxy-2-oxoethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1] pentadeca-1(15), 11,13-triene-13,3,9-triyl]}tetraacetate (713 mg, 81% purity, 292 μmol) was added formic acid (8.0 ml, 212 mmol) and the resulting mixture was stirred at 70°C for 2 hours. The reaction mixture was concentrated under reduced pressure. The obtained crude material was distilled with toluene two times and used in the next step without further purification.

LC-MS (Method 2): $R_t$ = 0.21 min; MS (ESIpos): m/z = 765.6 $[M+2H]^{2+}$, 510.8 $[M+3H]^{3+}$.

Step 3

**tetramanganese (2+) 2,2',2",2'''{[2,2-bis({[3,9-bis(carboxylatomethyl)-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo [9.3.1]pentadeca-1(15),11,13-triene-13,3,9-triyl]}tetraacetate**

The pH of a solution of 2,2',2",2'''-{[2,2-bis({[3,9-bis(carboxymethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1]pentadeca-1(15), 11,13-triene-13,3,9-triyl]}tetraacetic acid (447 mg, 292 μmol) and dichloromanganese tetrahydrate (231 mg, 1.17 mmol) in water (10 ml). was adjusted from pH 2 to pH 8 by addition of aqueous sodium hydroxide (2M) and the mixture was stirred for 20 h at 100°C. After cooling the mixture was diluted with water and stirred in the presence of Chelex®100 for 2 h. Chelex®100 was removed by filtration, the filtrate was adjusted to pH 7.5 by addition of aqueous hydrochloric acid (1M) and purified by column chromatography (Interchim puriFlash® PF-15 RP18 AQ F0080, water/ acetonitrile, 0 - 20%) to deliver 298 mg (100% purity, 59% yield) of the title compound LC-MS (Method 1): $R_t$ = 0.42 min; MS (ESIpos): m/z = 871.7 $[M+2H]^{2+}$, 581.2 $[M+3H]^{3+}$.

**Example 2**

**tetramanganese(2+) 2,2',2",2'''-({2,2-bis[(2-{[3,9-bis(carboxylatomethyl)-6-oxa-3,9,15-triazabicyclo[9.3.1]pentadeca-1(15),11,13-triene-13-carbonyl]amino} acetamido)methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-13,3,9-triyl])tetraacetate**

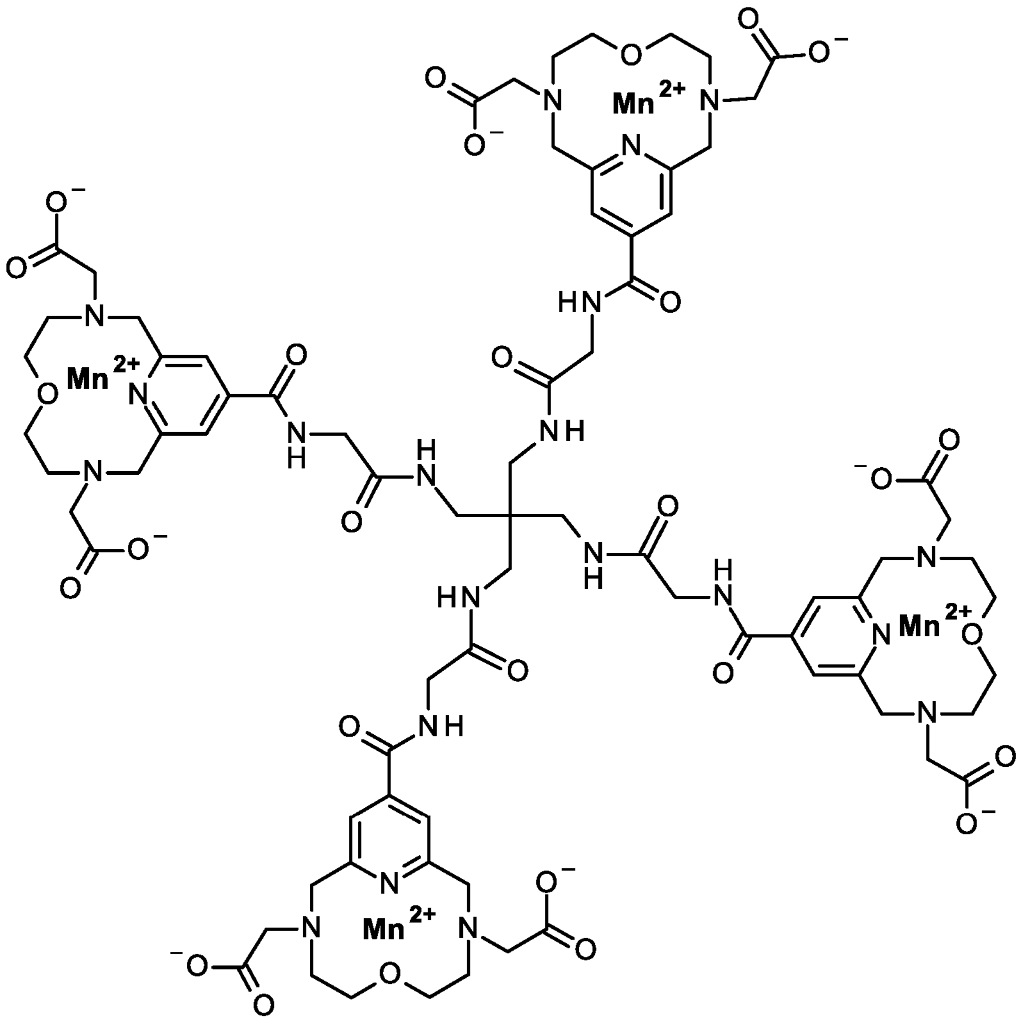

Step 1

tetra-*tert*-butyl 2,2',2'',2'''-({2,2-bis[(2-{[3,9-bis(2-tert-butoxy-2-oxoethyl)-6-oxa-3,9,15-triazabicyclo[9.3.1]pentadeca-1 (15), 11, 13-triene-13-carbonyl]amino}acetamido)methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-13,3,9-triyl])tetraacetate

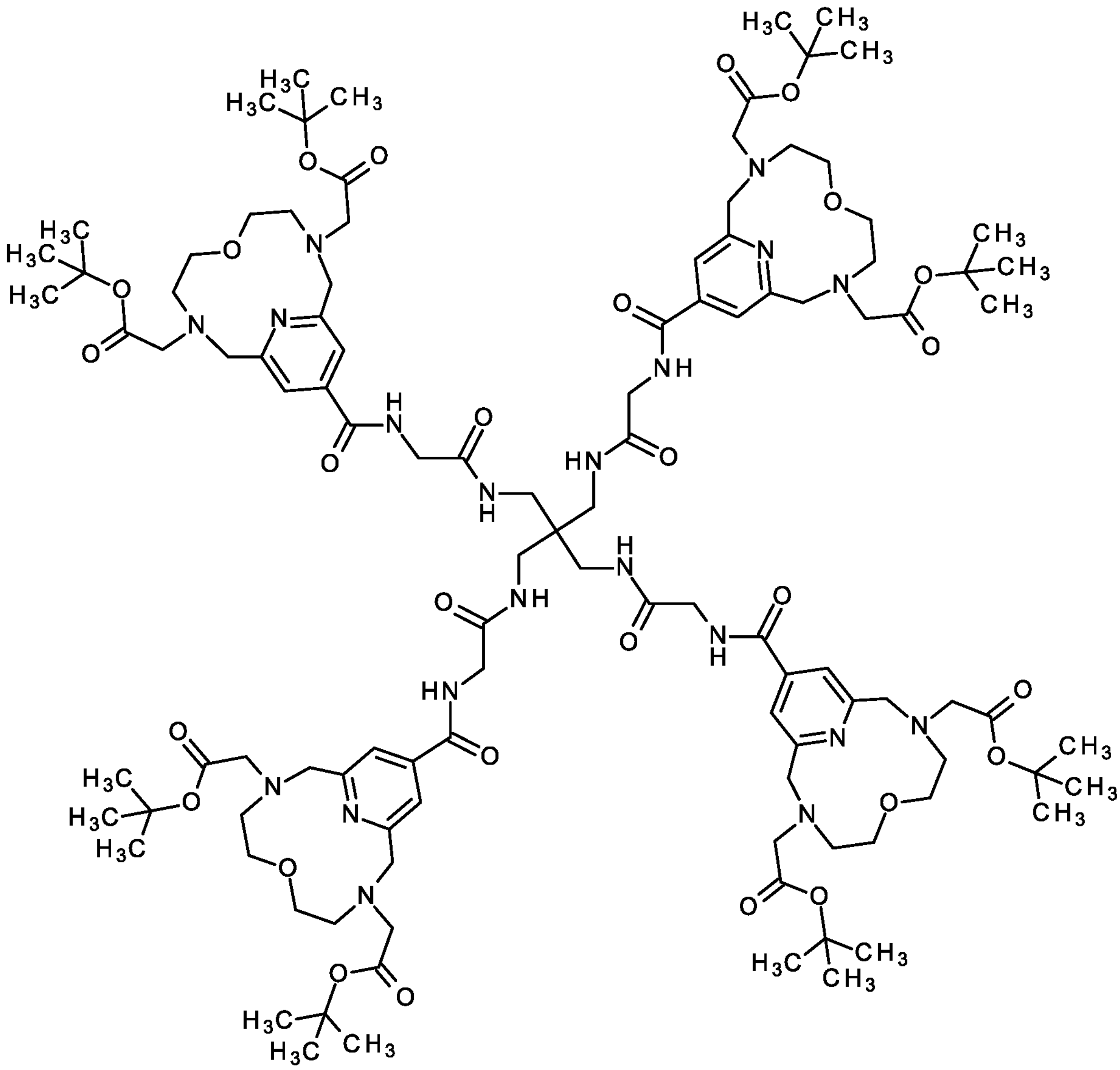

A solution of 2,3,5,6-tetrafluorophenyl 3,9-bis(2-tert-butoxy-2-oxoethyl)-6-oxa-3,9,15-triazabicyclo[9.3.1]pentadeca-1(15),11,13-triene-13-carboxylate (1.80 g, 70% purity, 1.43 mmol, Intermediate 1) in dichloromethane (16 mL) was added to a solution of N,N'-{2,2-bis[(2-aminoacetamido)methyl]propane-1,3-diyl}bis(2-aminoacetamide) (124 mg, 326 μmol, intermediate 6) and *N,N*-diisopropylethylamine (850 μl, 4.9 mmol) in DMF, which was stirred for 10 minutes before addition. The reaction mixture was stirred at room temperature for 19 h. The reaction mixture was filtered and the filtrate concentrated under reduced pressure. The crude product was purified by preparative HPLC (instrument: PuriFlash 5.250; column: Chromatorex RP C-18 10 μm, 125 x 30mm; eluent A: water + 0.1% formic acid; eluent B: acetonitrile; gradient: 0 - 1 min 10% B, 1.01 - 7 min 10 - 60%B, 7.01 - 8 min 60%B; flow: 150 ml/min;) to give 488 mg of the title compound (95% purity, 64% yield).

LC-MS (Method 2): $R_t$ = 0.97 min; MS (ESIpos): m/z = 1104.6 $[M+2H]^{2+}$, 736.8 $[M+3H]^{3+}$.

1H-NMR (400MHz, DMSO-d6): δ [ppm]= 9.35 (br t, 4H), 8.15 (s, 4H), 7.87 (br t, 4H), 7.58 (s, 8H), 3.96 (br s, 16H), 3.70 - 3.84 (m, 8H), 3.46 (s, 16H), 3.24 - 3.43 (m, 40H), 3.17 (br t, 16H), 2.79 (br t, 16H), 2.68 - 2.78 (m, 8H), 1.43 (s, 72H).

Step 2

2,2',2'',2'''-({2,2-bis[(2-{[3,9-bis(carboxymethyl)-6-oxa-3,9,15-triazabicyclo[9.3.1]pentadeca-1 (15),11,13-triene-13-carbonyl]amino}acetamido)methyl]propane-1,3-diyl}bis[azanediyl(2-oxoethane-2, 1-diyl)carbamoyl-6-oxa-3,9, 15-triazabicyclo[9.3.1]pentadeca-1 (15), 11, 13-triene-13,3,9-triyl])tetraacetic acid

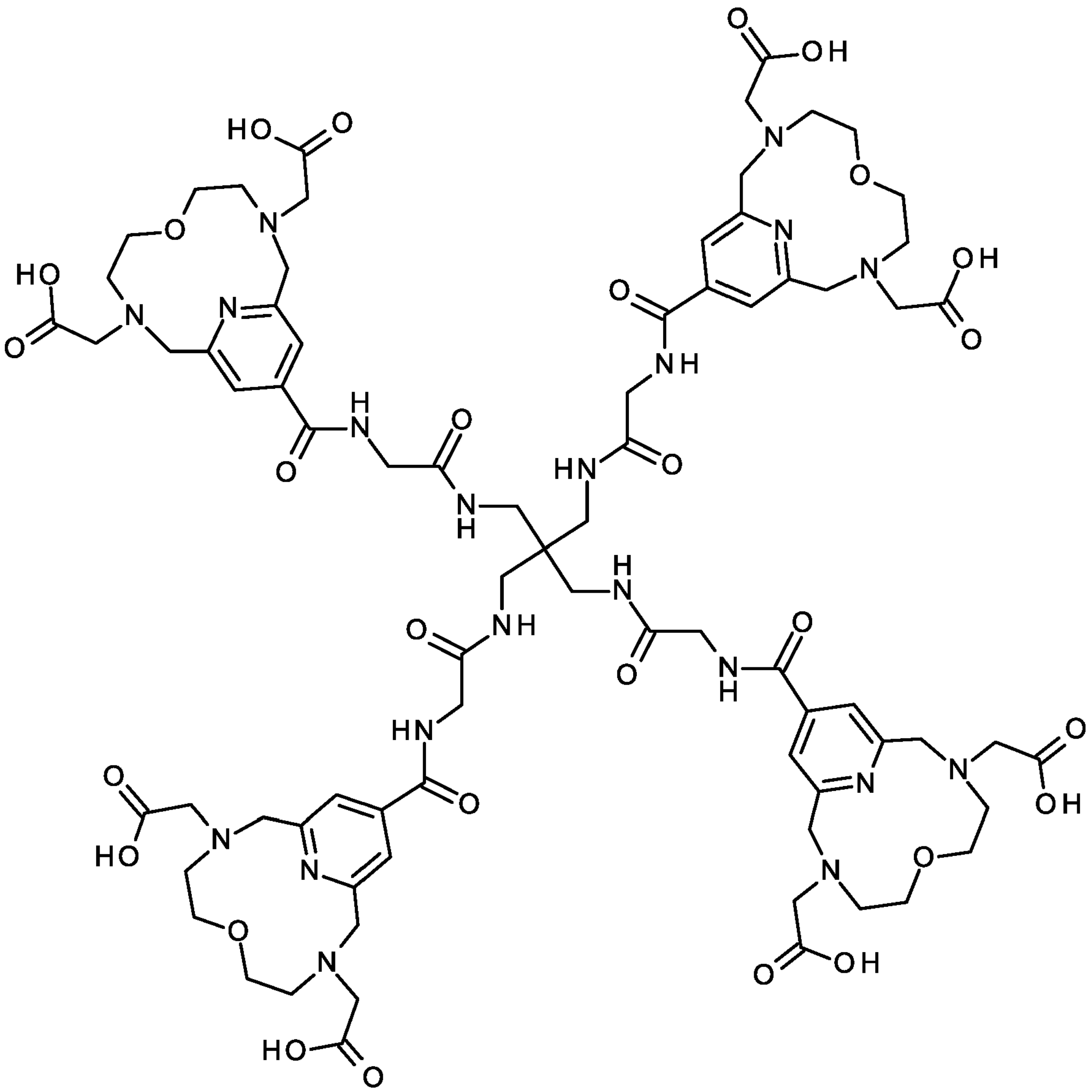

To tetra-*tert*-butyl 2,2',2",2‴-({2,2-bis[(2-{[3,9-bis(2-tert-butoxy-2-oxoethyl)-6-oxa-3,9,15-triazabicyclo[9.3.1] pentadeca-1(15),11,13-triene-13-carbonyl]amino}acetamido)methyl] propane-1,3-diyl}bis[azanediyl(2-oxoethane-2,1-diyl)carbamoyl-6-oxa-3,9,15-triaza bicyclo [9.3.1]pentadeca-1(15),11,13-triene-13,3,9-triyl])tetraacetate (488 mg, 95% purity, 210 μmol) was added formic acid (6.0 ml, 159 mmol) and the resulting mixture was stirred at 70°C for 4 hours. The reaction mixture was concentrated under reduced pressure. The obtained crude material was distilled with toluene two times and used in the next step without further purification.

LC-MS (Method 2): $R_t$ = 0.21 min; MS (ESIpos): m/z = 879.8 $[M+2H]^{2+}$, 586.8 $[M+3H]^{3+}$.

Step 3

**tetramanganese(2+) 2,2',2",2'"-({2,2-bis[(2-{[3,9-bis(carboxylatomethyl)-6-oxa-3,9,15-triazabicyclo[9.3.1]pentadeca-1(15),11,13-triene-13-carbonyl]amino} acetamido)methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-13,3,9-triyl])tetraacetate**

The pH of a solution of 2,2',2",2‴-({2,2-bis[(2-{[3,9-bis(carboxymethyl)-6-oxa-3,9,15-triazabicyclo[9.3.1]pentadeca-1(15),11,13-triene-13-carbonyl]amino}acetamido)methyl]propane-1,3-diyl}bis[azanediyl(2-oxoethane-2,1-diyl) carbamoyl-6-oxa-3,9,15-triazabicyclo[9.3.1]pentadeca-1(15),11,13-triene-13,3,9-triyl])tetraacetic acid (369 mg, 210 μmol) and dichloromanganese tetrahydrate 166 mg, 840 μmol) in water (10 ml). was adjusted from pH 2 to pH 8 by addition of aqueous sodium hydroxide (2M) and the mixture was stirred for 20 h at 100°C. After cooling the mixture was diluted with water and stirred in the presence of Chelex®100 for 1 hour. Chelex®100 was removed by filtration, the filtrate

was adjusted to pH 7.5 by addition of aqueous hydrochloric acid (1M) and purified by column chromatography (Interchim puriFlash® PF-15 RP18 AQ F0080, water / acetonitrile, 0 - 20%) to deliver 342 mg (100 % purity, 83 % yield) of the title compound.

LC-MS (Method 1): $R_t$ = 0.95 min; MS (ESIpos): m/z = 990.4 $[M+2H]^{2+}$., 660.6 $[M+3H]^{3+}$.

## Example 3

**tetramanganese(2+) (2*R*,2'*S*,2"*R*,2'''*S*)-2,2',2",2'''-{(2,2-bis[({3-[(1*R*)-1-carboxylato ethyl]-9-[(1S)1-carboxylatoethyl]-6-oxa-3,9,15-triazabicyclo[9.3.1]pentadeca-1(15), 11,13-triene-13-carbonyl}amino)methyl] propane-1,3-diyl)bis[carbamoyl-6-oxa-3,9,15-triazabicyclo[9.3.1]pentadeca-1(15),11,13-triene-13,3,9-triyl]}tetrapropanoate.**

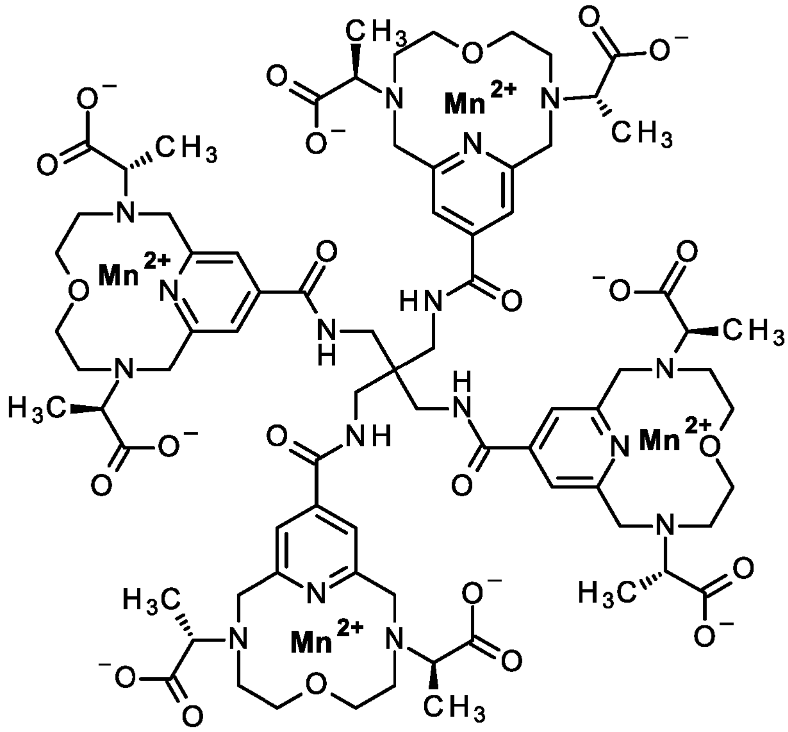

A solution of 2,2-bis(aminomethyl)propane-1,3-diamine hydrogen chloride (1/4) (13.6 mg, 49 µmol) in water (3 ml) was adjusted from pH 3 to pH 6 by addition of aqueous sodium hydroxide (1M). Manganese(2+) (2*R*)-2-{13-carboxy-9-[(1*S*)-1-carboxylatoethyl]-6-oxa-3, 9,15-triazabicyclo[9.3.1]pentadeca-1(15),11,13-trien-3-yl}propanoate (100 mg, 223 µmol, intermediate ), 1-hydroxy-1*H*-benzotriazol hydrate (17.1 mg, 112 µmol) and 1-(3-dimethylaminopropyl)-3-ethylcarbodiimid hydrochloride (85.5 mg, 446 µmol) were added and the mixture was stirred at room temperature for 4 days. Ion-exchange resin AmberChrome® 1X2 (chloride form, 1 g) and Amberlite® IRA120 (sodium form, 4 g) were added and the mixture was stirred for 2 h at room temperature. Ion-exchange resins were removed by filtration and the filtrate was concentrated under reduced pressure. The crude product was purified by column chromatography (Interchim puriFlash® PF-15 C18 AQ F0025, water / acetonitrile, 0 - 25%) to deliver 57.5 mg (99% purity, 63% yield) of the title compound.

LC-MS (Method 1): $R_t$ = 0.47 min; MS (ESIpos): m/z = 928.0 $[M+2H]^{2+}$., 619.0 $[M+3H]^{3+}$.

## Example 4

**tetramanganese(2+) 2,2',2",2'''-{[2,2-bis({[3,9-bis(1-carboxy)atoethy))-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo [9.3.1]pentadeca-1(15),11,13-triene-13,3,9-triyl]}tetrapropanoate.**

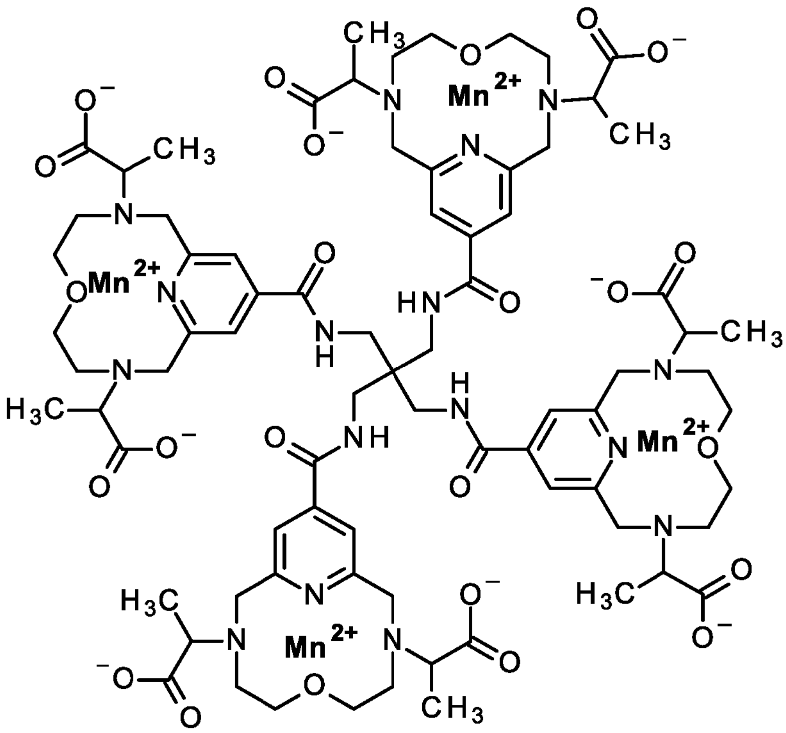

A solution of 2,2-bis(aminomethyl)propane-1,3-diamine hydrogen chloride (1/4) (13.6 mg, 49 μmol) in water (3 ml) was adjusted from pH 3 to pH 6 by addition of aqueous sodium hydroxide (1M). Manganese(2+) (2*R**,2'*R**)-2,2'-[13-carboxy-6-oxa-3,9,15-triazabicyclo [9.3.1] pentadeca-1(15),11,13-triene-3,9-diyl]dipropanoate (100 mg, 223 μmol, intermediate 2), 1-hydroxy-1H-benzotriazol hydrate (17.6 mg, 115 μmol) and 1-(3-dimethylaminopropyl)-3-ethylcarbodiimid hydrochloride (86 mg, 446 μmol) were added and the mixture was stirred at room temperature for 18 h. Ion-exchange resin Amberlite® IRA400 (chloride form, 0.5 g) and Amberlite® IRA120 (sodium form, 2 g) were added and the mixture was stirred for 2 h at room temperature. Ion-exchange resins were removed by filtration and the filtrate was purified by column chromatography (Interchim puriFlash® PF-15 RP18 AQ F0025, water / acetonitrile, 0 - 25%) to deliver 29 mg (80% purity, 26% yield) of the title compound.

LC-MS (Method 1): $R_t$ = 0.46 min; MS (ESIpos): m/z = 928.0 $[M+2H]^{2+}$., 619.0 $[M+3H]^{3+}$.

## Example 5

**tetramanganese(2+)(2*R*,2'*S*,2"*R*,2'''*S*)-2,2',2",2'''-((2,2-bis([2-((3-[(1R)-1-carboxylato ethyl]-9-[(1*S*)-1-carboxylatoethyl]-6-oxa-3,9,15-triazabicyclo[9.3.1]pentadeca-1(15),11,13-triene-13-carbonyl}amino)acetamido]methyl} propane-1,3-diyl)bis[azanediyl(2-oxoethane-2,1-diyl)carbamoyl{6-oxa-3,9,15-triazabicyclo[9.3.1] pentadeca-1(15),11,13-triene-13,3,9-triyl}]}tetrapropanoate**

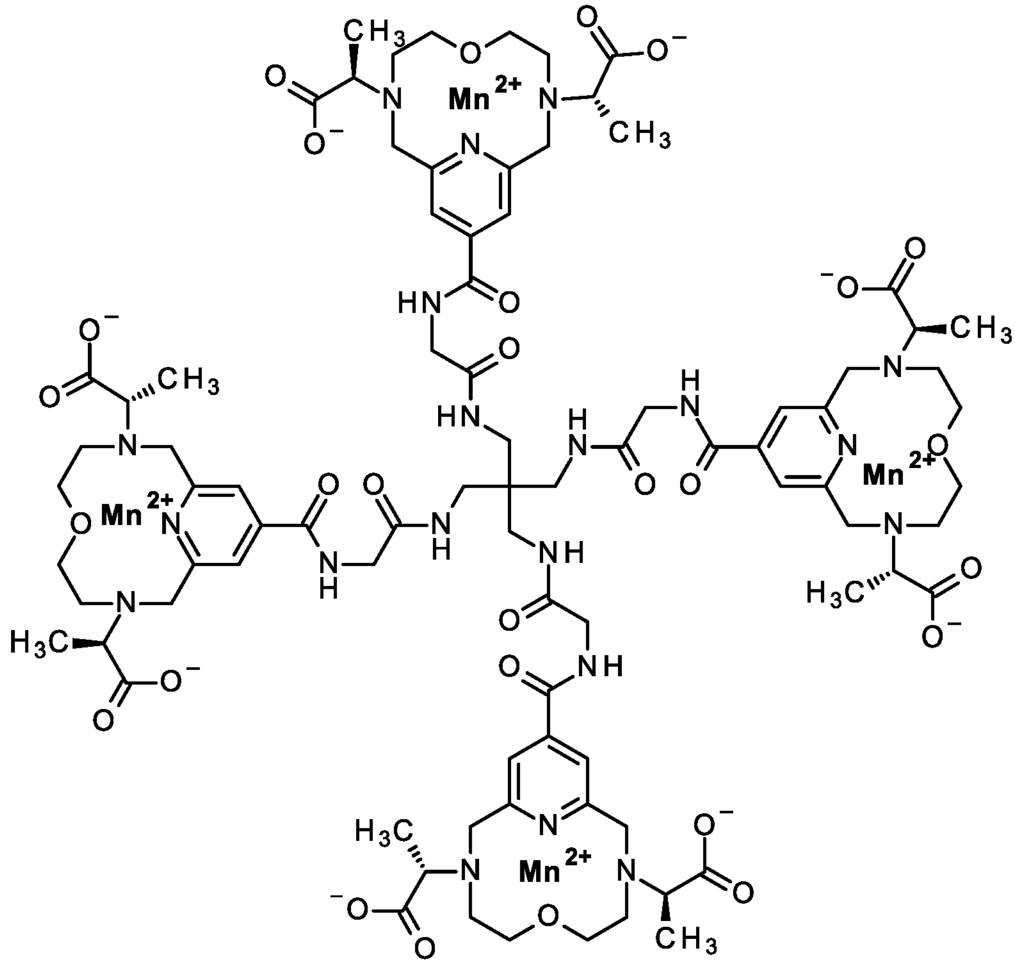

A solution of *N,N'*-{2,2-bis[(2-aminoacetamido)methyl]propane-1,3-diyl}bis(2-aminoacetamide) hydrogen chloride (1/4) (73.6 mg, 145 µmol) in water (5 ml) was adjusted from pH 3 to pH 6 by addition of aqueous sodium hydroxide (1M). Manganese(2+) (2R)-2-{13-carboxy-9-[(1S)-1-carboxylatoethyl]-6-oxa-3,9,15-triaza bicyclo[9.3.1]pentadeca-1(15),11,13-trien-3-yl}propanoate (300 mg, 669 µmol, intermediate 3), 1-hydroxy-1*H*-benzotriazol hydrate (22.3 mg, 145 µmol) and 1-(3-dimethylaminopropyl)-3-ethylcarbodiimid hydrochloride (112 mg, 582 µmol) were added and the mixture was stirred at room temperature for 3 days. Additional 1-(3-dimethylaminopropyl)-3-ethylcarbodiimid hydrochloride (55.8 mg, 291 µmol) was added and stirring was continued for 1 day. Additional manganese(2+) (2R)-2-{13-carboxy-9-[(1S)-1-carboxylatoethyl]-6-oxa-3,9,15-triaza bicyclo[9.3.1]pentadeca-1(15),11,13-trien-3-yl}propanoate (33 mg, 73 µmol) and 1-(3-dimethylaminopropyl)-3-ethylcarbodiimid hydrochloride (28 mg, 145 µmol) was added, and stirring was continued for 1 day. Ion-exchange resin AmberChrome® 1X2 (chloride form, 1 g) and Amberlite® IRA120 (sodium form, 4 g) were added, and the mixture was stirred for 2 h at room temperature. Ion-exchange resins were removed by filtration and the filtrate was purified by column chromatography (Interchim puriFlash® PF-15 C18 AQ F0040, water / acetonitrile, 0 - 25%) to deliver 70 mg (99% purity, 23% yield) of the title compound.

LC-MS (Method1): $R_t$ = 0.48 min; MS (ESIpos): m/z = 1042.6 $[M+2H]^{2+}$, 713.9 $[M+3H]^{3+}$.

## Example 6

**tetramanganese(2+) 2,2',2",2'"-{[2,2-bis({[3,9-bis(carboxy)atomethy))-6-oxa-3,9,15-triazabicyclo[9.3.1]pentadeca-1(15),11,13-triene-12-carbonyl]amino}methyl) propane-1,3-diyl]bis[carbamoyl-6-oxa-3,9,15-triazabicyclo [9.3.1]pentadeca-1(15),11,13-triene-12,3,9-triyl]}tetraacetate**

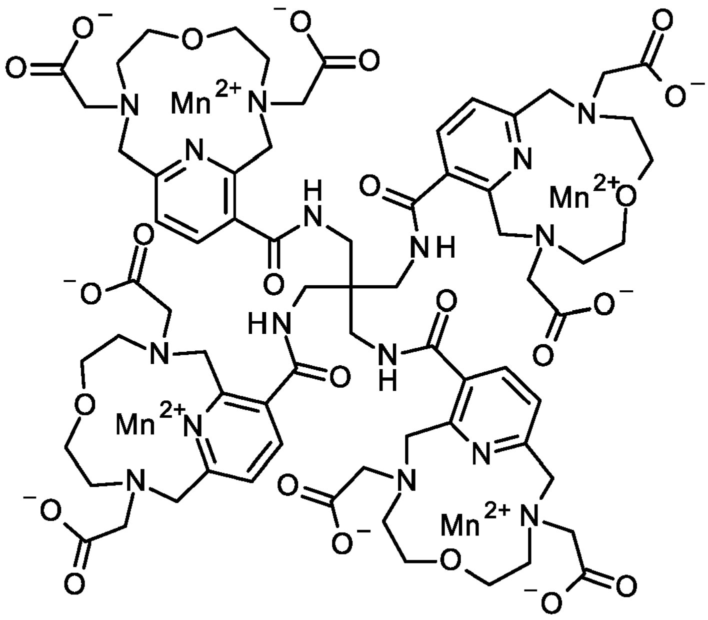

A solution of 2,2-bis(aminomethyl)propane-1,3-diamine hydrogen chloride (1/4) (36.8 mg, 132 μmol) in water (9 ml) was adjusted from pH 3 to pH 6 by addition of aqueous sodium hydroxide (2N, 100 μl, 200 μmol). manganese(2+) 2,2'-[12-carboxy-6-oxa-3,9,15-triazabicyclo[9.3.1]pentadeca-1(15),11,13-triene-3,9-diyl]diacetate (500 mg, 50% purity, 595 μmol) in water (3 ml), 1-hydroxy-1H-benzotriazol hydrate (10.1 mg, 66.1 μmol) and 1-(3-dimethylaminopropyl)-3-ethylcarbodiimid hydrochloride (51 mg, 264 μmol) were added and the mixture was stirred at room temperature for 40 h. The addition of 1-(3-dimethylaminopropyl)-3-ethylcarbodiimid hydrochloride (51 mg, 264 μmol) with additional stirring for 20 h was repeated twice. Ion exchange resins Amberlite IRA 120 (Na+ form, 4 g) and AmberChrome 1X2 (chloride form, 1g) were added and the mixture was stirred for 2 h. This mixture was purified by column chromatography (Interchim puriflash PF-15RP18 AQ-F0040, with water/ acetonitrile, 0 - 20%) to give 108 mg (90% purity, 42% yield) of the title compound.

LC-MS (Method1): $R_t$ = 0.41 min; MS (ESIpos): m/z = 871.6 $[M+2H]^{2+}$, 581.4 $[M+3H]^{3+}$.

## Example 7

**tetramanganese(2+) 2,2',2",2'''-({2,2-bis[(2-{[3,9-bis(carboxylatomethyl)-6-oxa-3,9,15-triazabicyclo[9.3.1]pentadeca-1(15),11,13-triene-12-carbonyl]amino} acetamido)methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-12,3,9-triyl])tetraacetate**

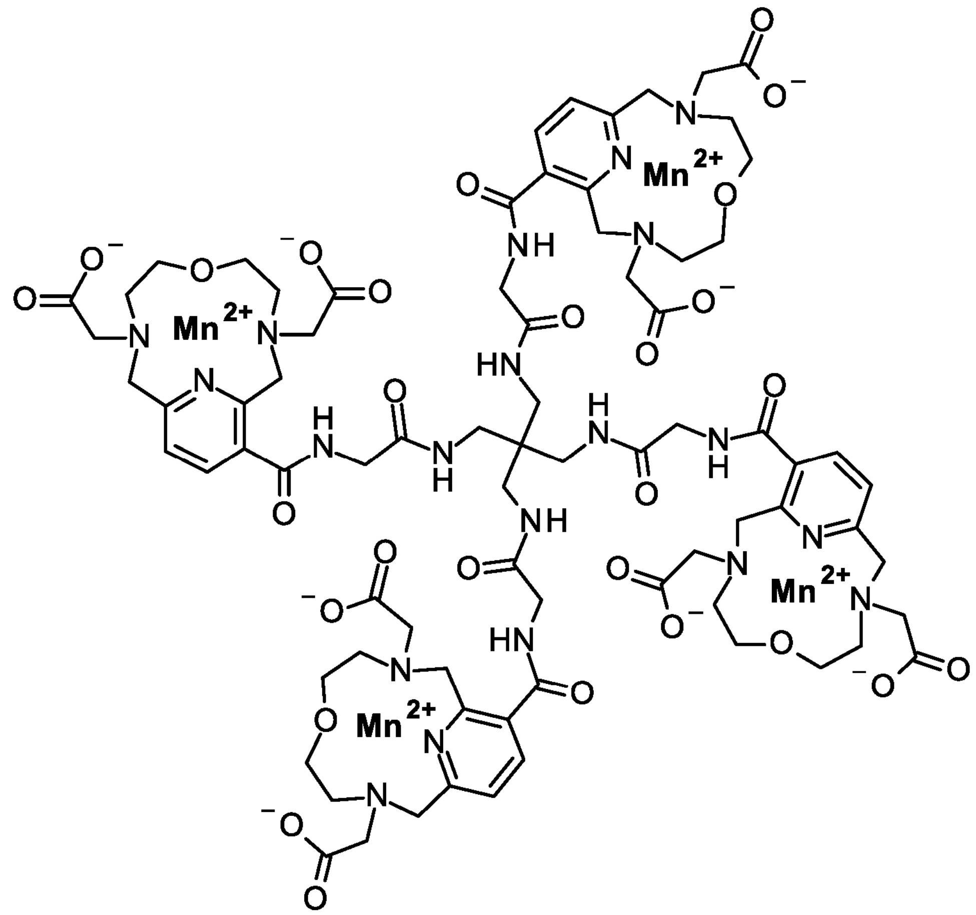

To a solution of *N,N'*-{2,2-bis[(2-aminoacetamido)methyl]propane-1,3-diyl}bis(2-amino acetamide) (97.5 mg, 270 µmol, intermediate 6) in water (5 ml) were added manganese(2+) 2,2'-[12-carboxy-6-oxa-3,9,15-triazabicyclo[9.3.1] pentadeca-1(15),11, 13-triene-3,9-diyl]diacetate (500 mg, 1.19 mmol, intermediate 4), 1-hydroxy-1H-benzotriazol hydrate (20.7 mg, 135 µmol) and 1-(3-dimethylaminopropyl)-3-ethylcarbodiimid hydrochloride (104 mg, 541 µmol) and the mixture was stirred at room temperature for 68 h. The addition of 1-(3-dimethylaminopropyl)-3-ethylcarbodiimid hydrochloride (104 mg, 541 µmol) with additional stirring for 22 h was repeated twice. Ion-exchange resin Amberlite IRA 400 (chloride form, 1 g) and Amberlite IRA 120 (sodium form, 4 g) were added, and the mixture was stirred for 2 h at room temperature. Ion-exchange resins were removed by filtration and the filtrate was concentrated under reduced pressure. The crude product was purified by column chromatography (Interchim puriflash PF-15RP18 AQ-F0012, water / acetonitrile, 0 - 20%) to deliver 85.2 mg (96% purity, 15% yield) of the title compound.

LC-MS (Method 1): $R_t$ = 0.43 min; MS (ESIpos): m/z = = 985.6 $[M+2H]^{2+}$, 657.6 $[M+3H]^{3+}$.

## Example 8

**tetramanganese(2+) 2,2',2",2'''-{[2,2-bis({2-[3,9-bis(carboxy)atomethy))-6-oxa-3,9,15-triazabicyclo[9.3.1]pentadeca-1(15),11,13-trien-13-yl]acetamido}methyl)propane-1,3-diyl]bis[azanediyl(2-oxoethane-2,1-diyl)-6-oxa-3,9,15-triazabicyclo[9.3.1]pentadeca-1(15),11,13-triene-13,3,9-triyl]}tetraacetate**

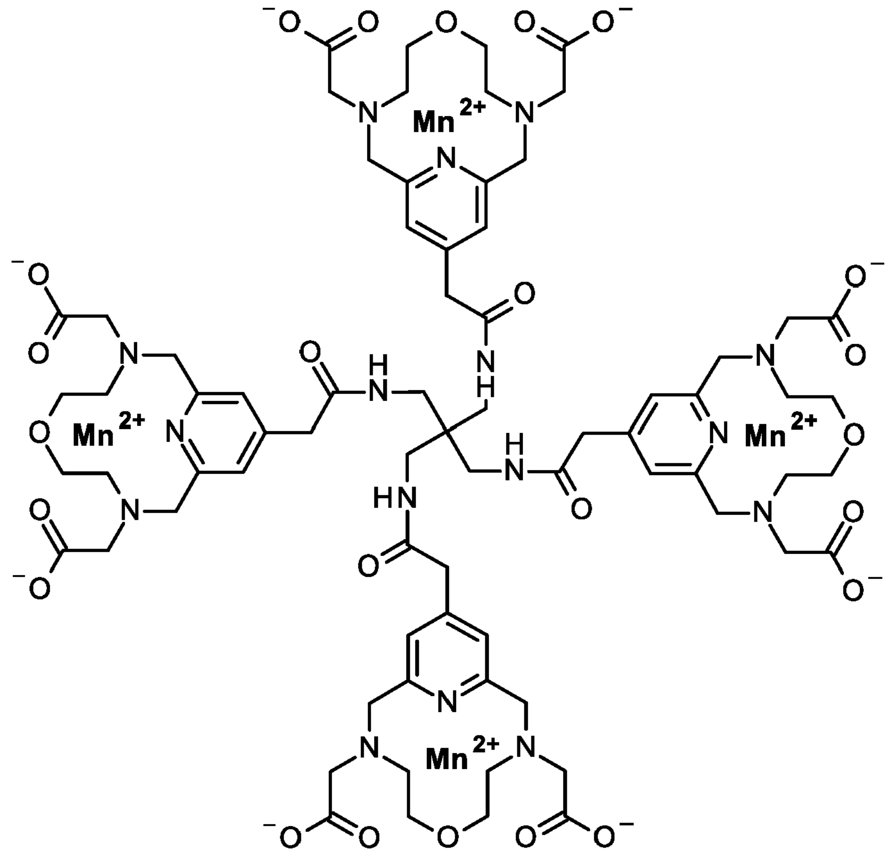

A solution of 2,2-bis(aminomethyl)propane-1,3-diamine hydrogen chloride (1/4) (14.1 mg, 50.7 μmol) in water (3 ml) was adjusted from pH 3 to pH 6 by addition of aqueous sodium hydroxide (1N, 45μl, 45 μmol). Manganese(2+) 2,2'-[13-(carboxymethyl)-6-oxa-3,9,15-triazabicyclo[9.3.1]pentadeca-1(15),11,13-triene-3,9-diyl]diacetate (100 mg, 230 μmol, intermediate 7), 1-hydroxy-1H-benzotriazol hydrate (17.6 mg, 115 μmol) and 1-(3-dimethylaminopropyl)-3-ethylcarbodiimid hydrochloride (88.3 mg, 460 μmol) were added and the mixture was stirred at room temperature for 18h. Ion-exchange resin Amberlite® IRA400 (chloride form, 0.5 g) and Amberlite® IRA120 (sodium form, 2 g) were added and the mixture was stirred for 2 h at room temperature. Ion-exchange resins were removed by filtration and the filtrate was concentrated under reduced pressure. The crude product was purified by column chromatography (Interchim puriFlash® PF-15 RP18 AQ F0012, water / acetonitrile, 0 - 20%) to deliver 66.9 mg (96% purity, 71% yield) of the title compound.

LC-MS (Method 1): $R_t$ = 0.44 min; MS (ESIpos): m/z = 899.7 $[M+2H]^{2+}$., 600.0 $[M+3H]^{3+}$.

## Example 9

**tetramanganese(2+)(2*R*,2'*S*,2"*R*,2'''*S*)-2,2',2",2'''-({2,2-bis[(2-{3-[(1*R*)-1-carboxylato ethyl]-9-[(1*S*)-1-carboxylatoethyl]-6-oxa-3,9,15-triazabicyclo[9.3.1]pentadeca-1(15),11, 13-trien-13-yl}acetamido)methyl]propane-1,3-diyl}bis[azanediyl(2-oxoethane-2,1-diyl) {6-oxa-3,9,15-triazabicyclo[9.3.1]pentadeca-1(15),11,13-triene-13,3,9-triyl}])tetra propanoate**

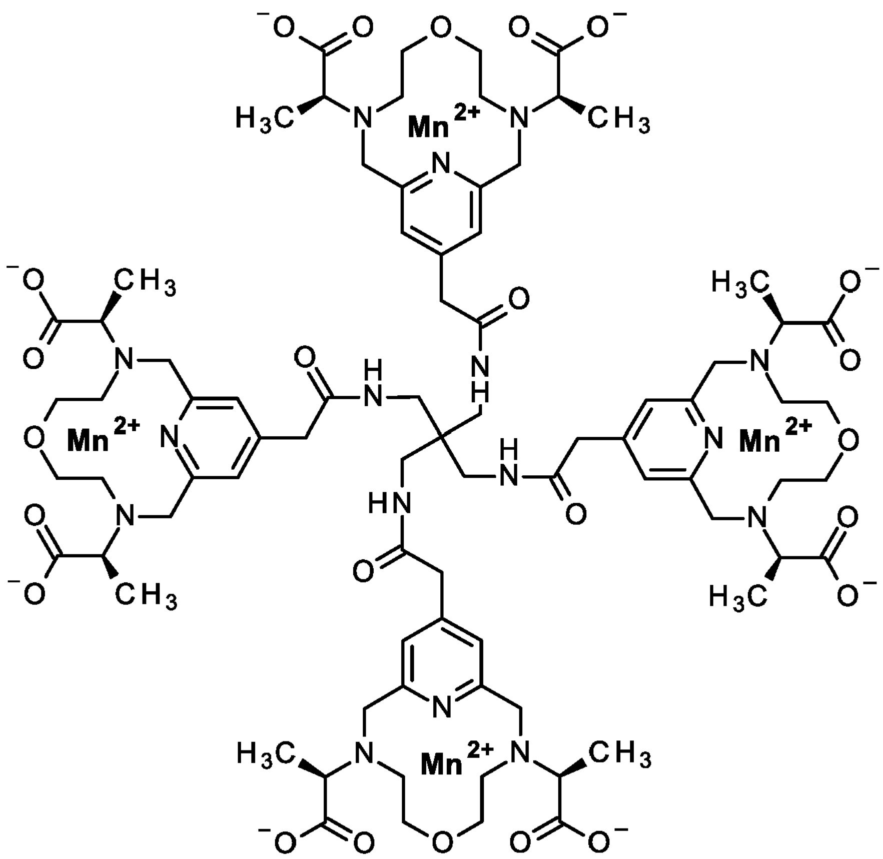

A solution of 2,2-bis(aminomethyl)propane-1,3-diamine hydrogen chloride (1/4) (28.5 mg, 102 μmol) in water (3 ml) was adjusted from pH 3 to pH 6 by addition of aqueous sodium hydroxide (1M). Manganese(2+) (2*R*)-2-{9-[(1*S*)-1-carboxylatoethyl]-13-(carboxymethyl)-6-oxa-3,9,15-triazabicyclo[9.3.1]pentadeca-1(15),11,13-trien-3-yl} propanoate (218 mg, 471 μmol, intermediate 8) in water (5 ml), 1-hydroxy-1*H*-benzotriazol hydrate (15.7 mg, 102 μmol) and 1-(3-dimethylaminopropyl)-3-ethyl carbodiimid hydrochloride (79 mg, 410 μmol) were added and the mixture was stirred at room temperature. Additional 1-(3-dimethylaminopropyl)-3-ethylcarbodiimid hydrochloride (19 mg, 100 μmol) was added after 16 h and 40 h while stirring was continued for 5 days overall. Ion-exchange resin AmberChrome® 1X2 (chloride form, 0.5 g) and Amberlite® IRA120 (sodium form, 2 g) were added and the mixture was stirred for 2 h at room temperature. Ion-exchange resins were removed by filtration and the filtrate was concentrated under reduced pressure. The crude product was purified by column chromatography (Interchim puriFlash® PF-15 C18 AQ F0080, water / acetonitrile, 0 - 20%) to deliver 85.8 mg (97% purity, 43% yield) of the title compound.

LC-MS (Method 1): $R_t$ = 0.46 min; MS (ESIpos): m/z = 956.0 $[M+2H]^{2+}$., 637.7 $[M+3H]^{2+}$.

**Example 10**

**tetramanganese(2+) (2*R**,2'*S**,2"*RS*,2'''*SR*)-2,2',2",2'''-{(2,2-bis{[({3-[(1*RS*)-1-carboxylato ethyl]-9-[(1*SR*)-1-carboxylatoethyl]-6-oxa-3,9,15-triazabicyclo[9.3.1] pentadeca-1(15),11, 13-trien-12-yl}carbonyl)amino]methyl}propane-1,3-diyl)bis[carbamoyl-6-oxa-3,9,15-triazabicyclo[9.3.1]pentadeca-1(15),11,13-triene-12,3,9-triyl]}tetrapropanoate**

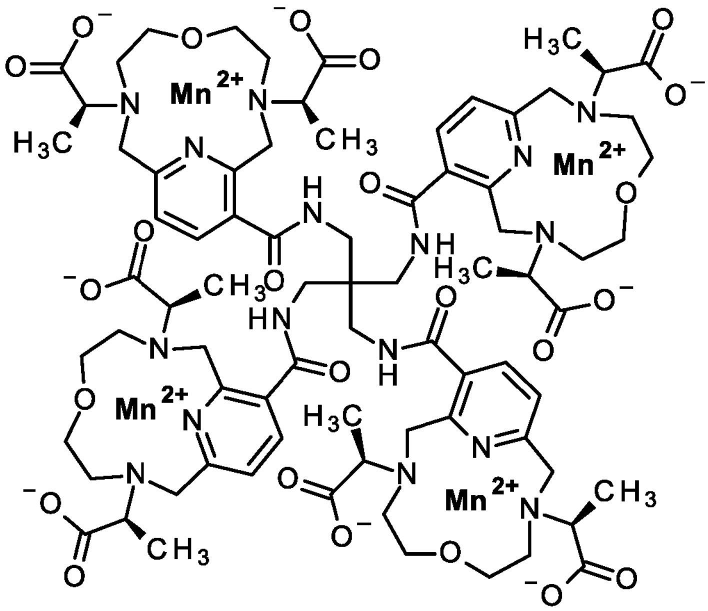

A solution of 2,2-bis(aminomethyl)propane-1,3-diamine hydrogen chloride (1/4) (209 mg, 752 µmol) in water (50 ml) was adjusted from pH 3 to pH 6 by addition of aqueous sodium hydroxide (1M). Manganese(2+) (2*R**,2'*S**)-2,2'-[12-carboxy-6-oxa-3,9,15-triazabicyclo[9.3.1]penta deca-1(15),11,13-triene-3,9-diyl]dipropanoate (1.55 g, 3.46 mmol, intermediate 11), 1-hydroxy-1*H*-benzotriazol hydrate (115 mg, 752 µmol) and 1-(3-dimethylaminopropyl)-3-ethylcarbodiimid hydrochloride (576 mg, 3.0 mmol) were added and the mixture was stirred at room temperature for 20 h. Additional 1-(3-dimethylaminopropyl)-3-ethylcarbodiimid hydrochloride (144 mg, 752 µmol) was added and stirring was continued for 20 h. Ion-exchange resin AmberChrom® 1X2 (chloride form, 0.5 g) and Amberlite® IRA120 (sodium form, 2 g) were added and the mixture was stirred for 2 h at room temperature. Ion-exchange resins were removed by filtration and the filtrate was concentrated under reduced pressure. The crude product was purified by column chromatography (Interchim puriFlash® PF-15C RP18 AQ F0330, water / acetonitrile, 0 - 25%) to deliver 615 mg (99% purity, 44% yield) of the title compound as a mixture of diastereomers.

LC-MS (Method 1): $R_t$ = 0.47 min; MS (ESIpos): m/z = 927.8 $[M+2H]^{2+}$., 618.8 $[M+3H]^{3+}$.

## Example 11

**tetramanganese(2+) (2*R**,2'*R**,2"*RS*,2'''*RS*)-2,2',2",2'''-({2,2-bis[({3,9-bis[(1*RS*)-1-carboxylatoethyl]-6-oxa-3,9,15-triazabicyclo[9.3.1]pentadeca-1(15),11,13-triene-12-carbonyl}amino)methyl]propane-1,3-diyl}bis[carbamoyl-6-oxa-3,9,15-triazabicyclo [9.3.1]pentadeca-1(15),11,13-triene-12,3,9-triyl])tetrapropanoate**

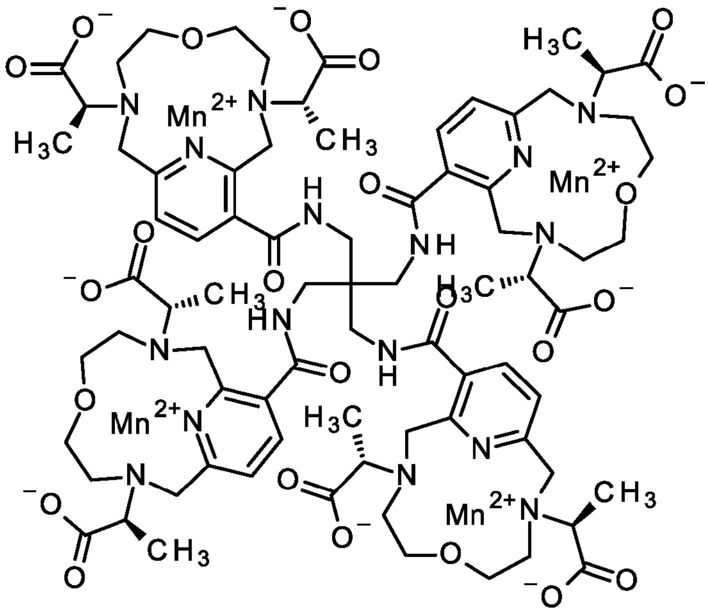

A solution of 2,2-bis(aminomethyl)propane-1,3-diamine hydrogen chloride (1/4) (159 mg, 572 μmol) in water (50 ml) was adjusted from pH 3 to pH 6 by addition of aqueous sodium hydroxide (1M). Manganese(2+) (2R*,2'R*)-2,2'-[12-carboxy-6-oxa-3,9,15-triazabicyclo [9.3.1]pentadeca-1(15),11,13-triene-3,9-diyl]dipropanoate (1.18 g, 2.63 mmol, intermediate 10), 1-hydroxy-1*H*-benzotriazol hydrate (88 mg, 572 μmol) and 1-(3-dimethyl aminopropyl)-3-ethylcarbodiimid hydrochloride (439 mg, 3.29 mmol) were added and the mixture was stirred at room temperature for 20 h. Additional 1-(3-dimethylaminopropyl)-3-ethylcarbodiimid hydrochloride (110 mg, 572 μmol) was added and stirring was continued for 20 h. Ion-exchange resin AmberChrom® 1X2 (chloride form, 0.5 g) and Amberlite® IRA120 (sodium form, 2 g) were added and the mixture was stirred for 1.5 h at room temperature. Ion-exchange resins were removed by filtration and the filtrate was concentrated under reduced pressure. The crude product was purified by column chromatography (Interchim puriFlash® PF-15 RP18 AQ F0330, water / acetonitrile, 0 - 25%) to deliver 364 mg (99% purity, 34% yield) of the title compound as a mixture of diastereomers.

LC-MS (Method 1): $R_t$ = 0.45 min; MS (ESIpos): m/z = 927.7 $[M+2H]^{2+}$., 618.8 $[M+3H]^{3+}$.

## Example 12

**tetramanganese(2+) 2,2',2",2'''-{[2,2-bis({3-[3,9-bis(1-carboxy)atoethy))-6-oxa-3,9,15-triazabicyclo[9.3.1]pentadeca-1(15),11,13-trien-13-yl]propanamido} methyl)propane-1,3-diyl]bis[azanediyl(3-oxopropane-3,1-diyl)-6-oxa-3,9,15-triazabicyclo[9.3.1] pentadeca-1(15),11,13-triene-13,3,9-triyl]}tetrapropanoate**

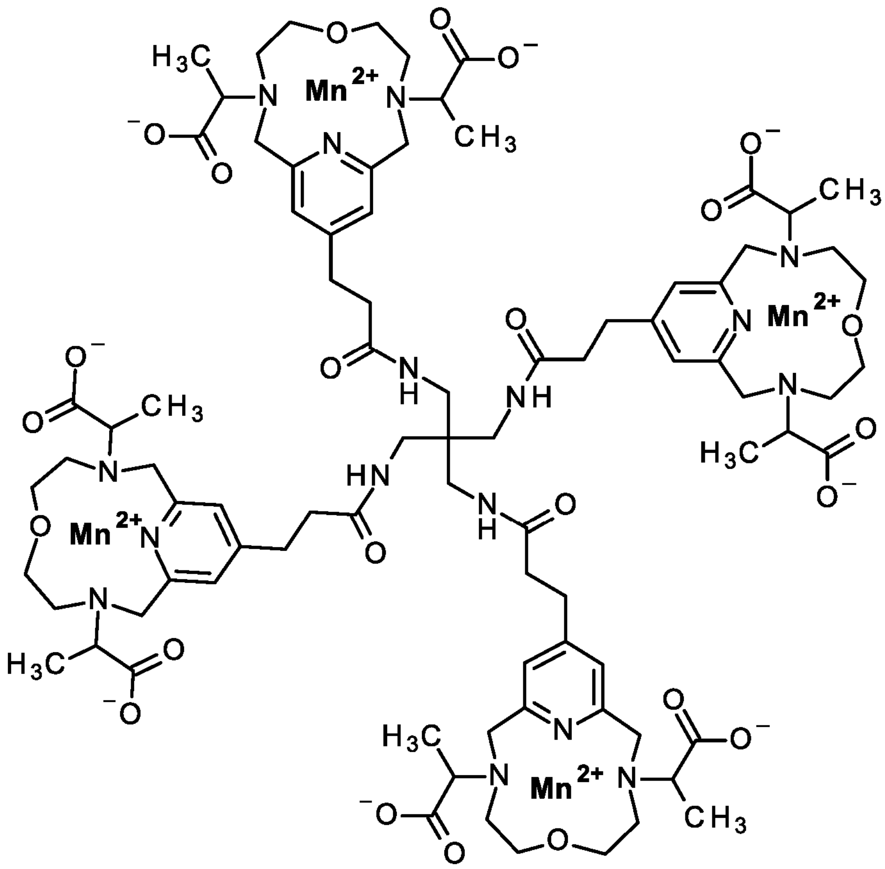

A solution of 2,2-bis(aminomethyl)propane-1,3-diamine hydrogen chloride (1/4) (38 mg, 137 µmol) in water (3,5 ml) was adjusted from pH 3 to pH 7 by addition of addition of aqueous sodium hydroxide (2M, 100µl, 200µmol). Manganese(2+) 2,2'-[13-(2-carboxyethyl)-6-oxa-3,9,15-triazabicyclo[9.3.1]pentadeca-1(15),11,13-triene-3,9-diyl] dipropanoate (300 mg, 630 µmol, intermediate 12) solved in water (3.7 ml), 1-hydroxy-1H-benzotriazol hydrate (21 mg, 137 µmol) and 1-(3-dimethylaminopropyl)-3-ethylcarbodiimid hydrochloride (105 mg, 548 µmol) were added and the mixture was stirred at room temperature for 40 h. Ion-exchange resin AmberChrom® 1X2 (chloride form, 1 g) and Amberlite® IRA120 (sodium form, 4 g) were added and the mixture was stirred for 2 h at room temperature. Additional AmberChrom® 1X2 (chloride form, 1 g) and Amberlite® IRA120 (sodium form, 4 g) were added and stirring was continued for 2 h. Ion-exchange resins were removed by filtration and the filtrate was concentrated under reduced pressure. The crude product was purified by column chromatography (Interchim puriFlash® PF-15 C18 AQ-F0040, water / acetonitrile, 0 - 25%). To give 137 mg (98% purity, 50% yield) of the title compound.

LC-MS (Method 2): $R_t$ = 0.55 min; MS (ESIpos): m/z = 983.7 $[M+2H]^{2+}$., 656.2 $[M+3H]^{3+}$.

**Example 13**

tetramanganese(2+) 2,2',2",2'''-([2,2-bis({[3,9-bis(carboxylatomethyl)-4-methyl-6-oxa-3,9,15-triazabicyclo[9.3.1]pentadeca-1(15),11,13-triene-13-carbonyl]amino}methyl) propane-1,3-diyl]bis(carbamoyl[4-methyl-6-oxa-3,9,15-triazabicyclo[9.3.1]pentadeca-1(15),11,13-triene-13,3,9-triyl]})tetraacetate

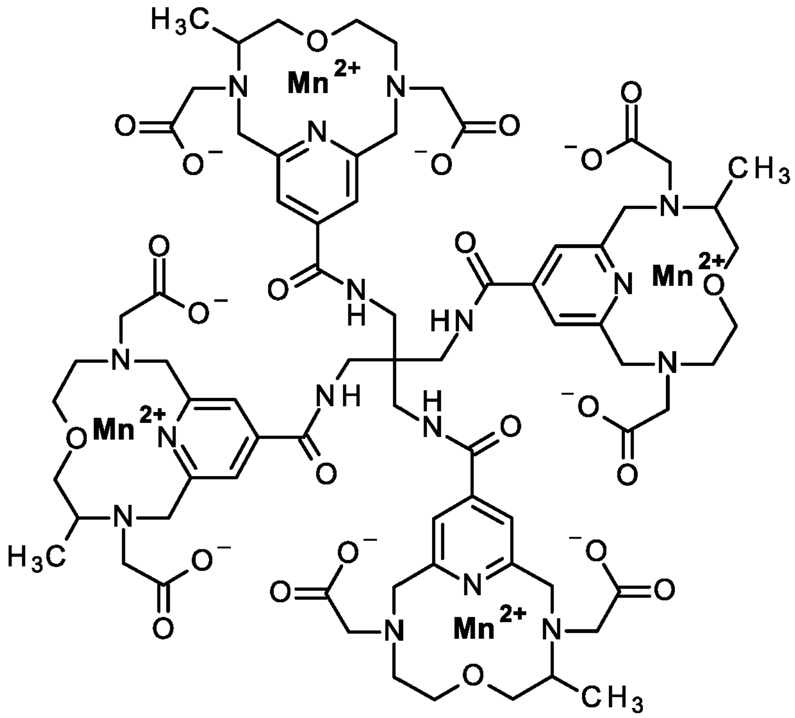

A solution of 2,2-bis(aminomethyl)propane-1,3-diamine hydrogen chloride (1/4) (181 mg, 651 µmol) in water (20 ml) was adjusted from pH 3 to pH 7 by addition of addition of aqueous sodium hydroxide (2M, 700µl, 1.4mol). Manganese(2+) 2,2'-(13-carboxy-4-methyl-6-oxa-3,9,15-triazabicyclo[9.3.1]pentadeca-1(15),11,13-triene-3,9-diyl)diacetate (1.3 g, 3.0 mmol, intermediate 13) solved in water (23 ml), 1-hydroxy-1*H*-benzotriazol hydrate (100 mg, 0.65 mmol) and 1-(3-dimethylaminopropyl)-3-ethylcarbodiimid hydrochloride (500 mg, 2.6 mmol) were added and the mixture was stirred at room temperature for 22 h. Additional 1-(3-dimethylaminopropyl)-3-ethylcarbodiimidhydrochlorid (125 mg, 651 µmol) was added and stirring was continued for 22h. The addition of 1-(3-dimethylaminopropyl)-3-ethylcarbodiimidhydrochlorid (125 mg, 651 µmol) followed by stirring for 22 h was repeated two times, then ion-exchange resins Dowex® 1X2 (chloride form, 2 g) and Amberlite® IRA120 (sodium form, 8 g) were added, and the mixture was stirred for 2 h at room temperature. Ion-exchange resins were removed by filtration and the filtrate was concentrated under reduced pressure. The crude product was purified by column chromatography (Interchim puriFlash® PF-15RP18AQ-F120, water / acetonitrile, 0 - 30%). To give 309 mg (98% purity, 26% yield) of the title compound.

LC-MS (Method 2): $R_t$ = 0.47 min; MS (ESIpos): m/z = 899.7 $[M+2H]^{2+}$, 600.0 $[M+3H]^{3+}$.

## Example 14

**tetramanganese(2+) 2,2',2",2'''-([2,2-bis({[3,9-bis(carboxylatomethyl)-4-ethyl-6-oxa-3,9,15-triazabicyclo[9.3.1] pentadeca-1(15),11,13-triene-13-carbonyl]amino}methyl) propane-1,3-diyl]bis{carbamoyl[4-ethyl-6-oxa-3,9,15-triazabicyclo[9.3.1]pentadeca-1(15),11,13-triene-13,3,9-triyl]})tetraacetate**

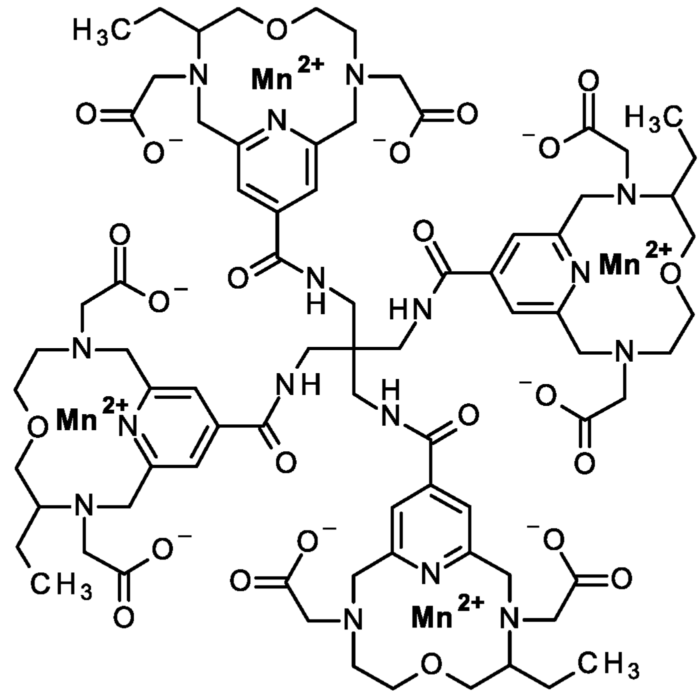

A solution of 2,2-bis(aminomethyl)propane-1,3-diamine hydrogen chloride (1/4) (86 mg, 310 µm) in water (10 ml) was adjusted from pH 3 to pH 7 by addition of addition of aqueous sodium hydroxide (2M). Manganese(2+) 2,2'-(13-carboxy-4-ethyl-6-oxa-3,9,15-triazabicyclo[9.3.1]pentadeca-1(15),11,13-triene-3,9-diyl)diacetate (640 mg, 1.43 mmol, intermediate 14) solved in water (11 ml), 1-hydroxy-1*H*-benzotriazol hydrate (47.5 mg, 310 µmol) and 1-(3-dimethylaminopropyl)-3-ethylcarbodiimid hydrochloride (238 mg, 1.24 mmol) were added and the mixture was stirred at room temperature for 22 h. Additional 1-(3-dimethylaminopropyl)-3-ethylcarbodiimidhydrochlorid (60 mg, 310 µmol) was added and stirring was continued for 22 h. The addition of 1-(3-dimethylaminopropyl)-3-ethylcarbodiimidhydrochlorid (60 mg, 310 µmol) followed by stirring for 22 h was repeated two times, then ion-exchange resins Dowex® 1X2 (chloride form, 1.3 g) and Amberlite® IRA120 (sodium form, 5 g) were added, and the mixture was stirred for 2 h at room temperature. Ion-exchange resins were removed by filtration and the filtrate was concentrated under reduced pressure. The crude product was purified by column chromatography (Interchim puriFlash® PF-15 RP18 AQ-F120, water / acetonitrile, 0 - 30%) to give 203 mg (100% purity, 35% yield) of the title compound.

LC-MS (Method 2): $R_t$ = 0.53 min; MS (ESIpos): m/z = 927 $[M+2H]^{2+}$.

## Example 15

tetramanganese(2+) 2,2',2'',2'''-{[2,2-bis({[3,9-bis(carboxylatomethyl)-5-methyl-6-oxa-3,9,15-triazabicyclo[9.3.1]pentadeca-1(15),11,13-triene-13-carbonyl]amino}methyl) propane-1,3-diyl]bis[carbamoyl(5-methyl-6-oxa-3,9,15-triazabicyclo[9.3.1]pentadeca-1(15),11,13-triene-13,3,9-triyl)]}tetraacetate

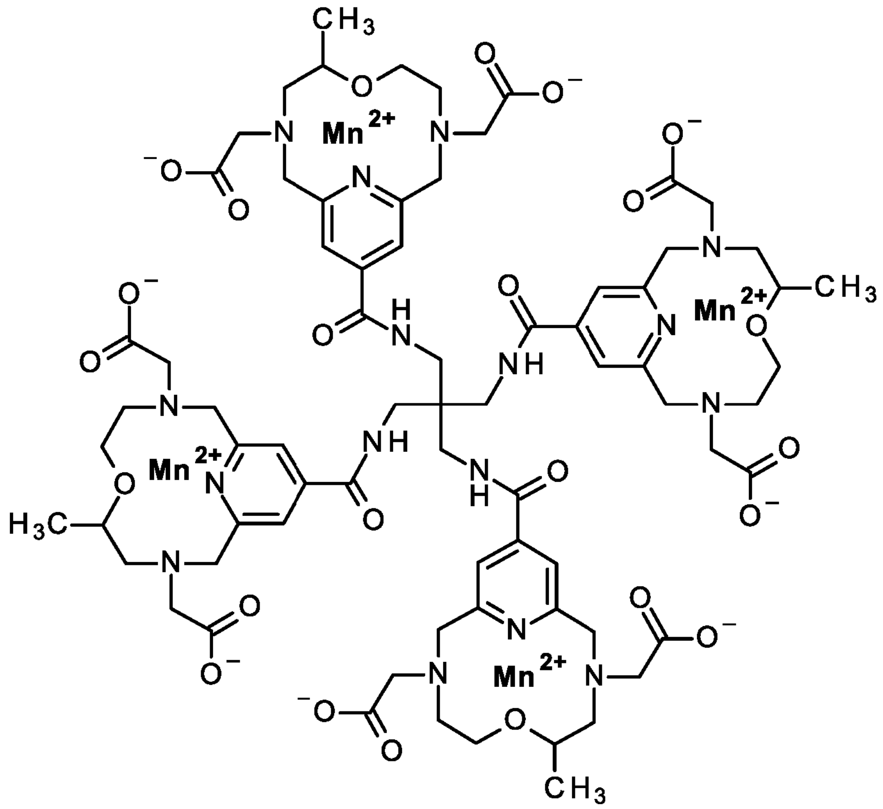

A solution of N,N-{2,2-bis[(2-aminoacetamido)methyl]propane-1,3-diyl}bis(2-amino acetamide) hydrogen chloride (1/4) (38.3 mg, 250 μmol) in water (7 ml) was adjusted from pH 3 to pH 6 by addition of aqueous sodium hydroxide (2M). Manganese(2+) 2,2'-(13-carboxy-5-methyl-6-oxa-3,9,15-triazabicyclo[9.3.1]pentadeca-1(15),11, 13-triene-3, 9-diyl]diacetate (500 mg, 1.15 mmol, intermediate 15) in water (7 ml), 1-hydroxy-1H-benzotriazol hydrate (38.3 mg, 1.5 mmol) and 1-(3-dimethylaminopropyl)-3-ethylcarbodiimid hydrochloride (288 mg, 582 μmol) were added and the mixture was stirred at room temperature for 3 days. Ion-exchange resin AmberChrome® 1X2 (chloride form, 1.5 g) and Amberlite® IRA120 (sodium form, 2.5 g) were added, and the mixture was stirred for 2 h at room temperature. Ion-exchange resins were removed by filtration and the filtrate was purified by column chromatography (Interchim puriFlash® PF-15 C18 AQ F0120, water / acetonitrile, 0 - 25%) to deliver 81.3 mg (97% purity, 18% yield) of the title compound.

LC-MS (Method 1): $R_t$ = 0.49 min; MS (ESIpos): m/z = 899.6 $[M+2H]^{2+}$, 600.2 $[M+3H]^{3+}$.

Example 16

tetramanganese(2+) (2*R*,2'*S*,2"*R*,2‴*S*)-2,2',2",2‴-{(2,2-bis[({3-[(1*R*)1-carboxylato propyl]-9-[(1S)-1-carboxylatopropyl]-6-oxa-3,9,15-triazabicyclo[9.3.1] pentadeca-1(15), 11,13-triene-13-carbonyl}amino)methyl] propane-1,3-diyl) bis [carbamoyl-6-oxa-3,9,15-triazabicyclo[9.3.1]pentadeca-1(15),11,13-triene-13,3,9-triyl]}tetrabutanoate

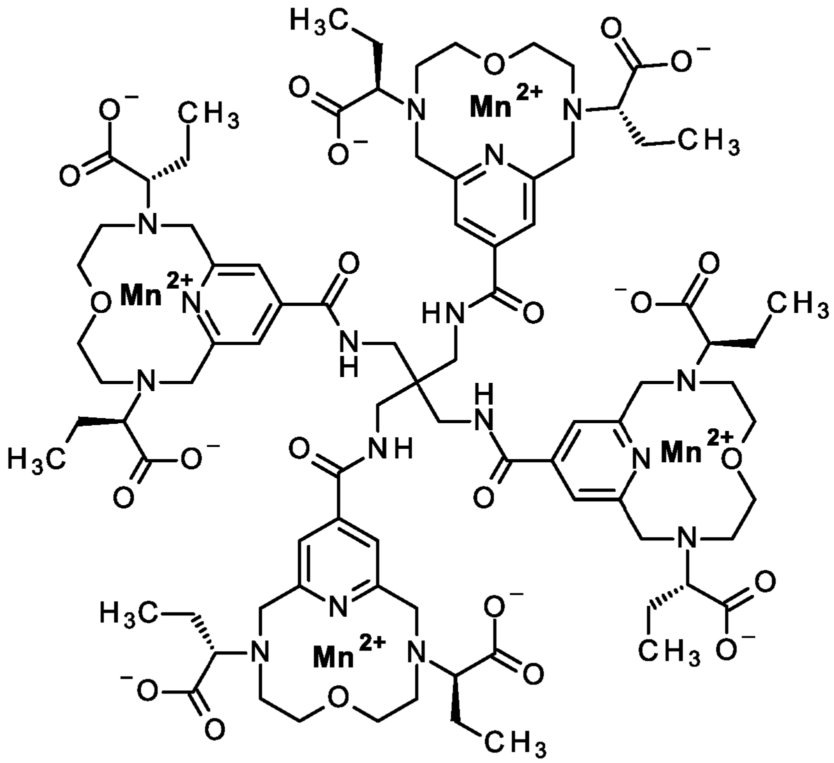

A solution of *N,N'*-{2,2-bis[(2-aminoacetamido)methyl]propane-1,3-diyl}bis(2-amino acetamide) hydrogen chloride (1/4) (17.7 mg, 63.7 µmol) in water (3 ml) was adjusted from pH 3 to pH 6 by addition of aqueous sodium hydroxide (1M). Manganese(2+) (2*R*)-2-{13-carboxy-9-[(1*S*)-1-carboxylatopropyl]-6-oxa-3,9,15-triazabicyclo[9.3.1]pentadeca-1(15),11,13-trien-3-yl}butanoate (138 mg, 290 µmol, intermediate 16), 1-hydroxy-1*H*-benzotriazol hydrate (22 mg, 145 µmol) and 1-(3-dimethylaminopropyl)-3-ethylcarbodiimid hydrochloride (111 mg, 579 µmol) were added and the mixture was stirred at room temperature for 22 hours. Additional 1-(3-dimethylaminopropyl)-3-ethylcarbodiimid hydrochloride (28 mg, 145 µmol) was added and stirring was continued for 3 days. Ion-exchange resin AmberChrome® 1X2 (chloride form, 1.5 g) and Amberlite® IRA120 (sodium form, 2.5 g) were added, and the mixture was stirred for 2 h at room temperature. Ion-exchange resins were removed by filtration and the filtrate was purified by column chromatography (Interchim puriFlash® PF-15 C18 AQ F0040, water / acetonitrile, 0 - 25%) to deliver 23 mg (99% purity, 18% yield) of the title compound.

LC-MS (Method 1): $R_t$ = 0.59 min; MS (ESIpos): m/z = 983.9 $[M+2H]^{2+}$, 656.3 $[M+3H]^{3+}$.

## Example 17

**tetramanganese(2+) (2*R**,2'*S**,2"*RS*,2'''*SR*)-2,2',2",2'''-((2,2-bis([((3-[(1*RS*)-1-carboxylatopropyl]-9-[(1*SR*)-1-carboxylatopropyl]-6-oxa-3,9,15-triazabicyclo[9.3.1] pentadeca-1(15),11,13-trien-13-yl}carbonyl)amino]methyl} propane-1,3-diyl)bis [carbamoyl-6-oxa-3,9,15-triazabicyclo[9.3.1]pentadeca-1(15),11,13-triene-13,3,9-triyl]} tetrabutanoate**

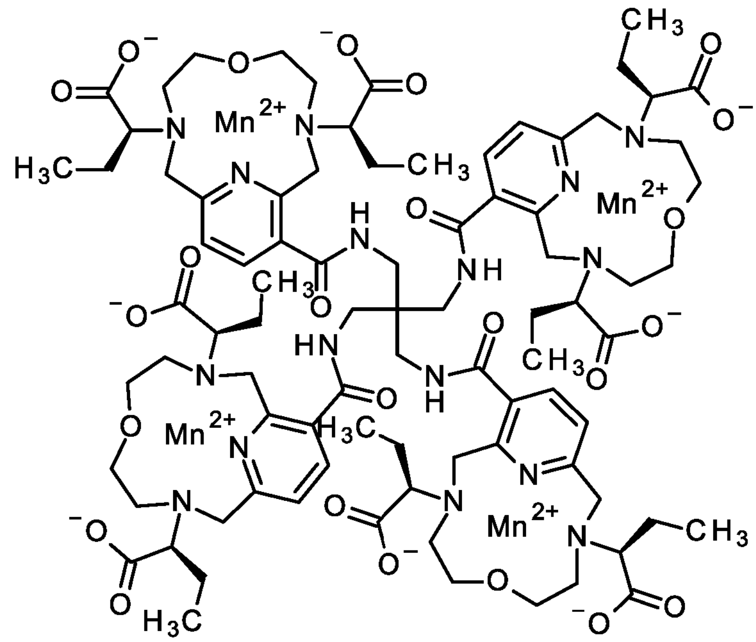

A solution of 2,2-bis(aminomethyl)propane-1,3-diamine hydrogen chloride (1/4) (27.9 mg, 100 μmol) in water (30 ml) was adjusted from pH 3 to pH 6 by addition of aqueous sodium hydroxide (1M). Manganese(2+) (2*R**,2'*S**)-2,2'-[12-carboxy-6-oxa-3,9,15-triazabicyclo[9.3.1] pentadeca-1(15),11,13-triene-3,9-diyl]dibutanoate (220 mg, 462 μmol, intermediate 17), 1-hydroxy-1*H*-benzotriazol hydrate (15.4 mg, 100 μmol) and 1-(3-dimethylaminopropyl)-3-ethylcarbodiimid hydrochloride (77 mg, 402 μmol) were added and the mixture was stirred at room temperature for 22 h. Ion-exchange resin AmberChrom® 1X2 (chloride form, 0.5 g) and Amberlite® IRA120 (sodium form, 2 g) were added and the mixture was stirred for 1.5 h at room temperature. Ion-exchange resins were removed by filtration and the filtrate was concentrated under reduced pressure. The crude product was purified by column chromatography (Interchim puriFlash® PF-15C RP18 AQ F0040, water/ acetonitrile, 0 - 20%) to deliver 70 mg (98% purity, 35% yield) of the title compound as mixture of diastereomers.

LC-MS (Method 1): $R_t$ = 0.59 min; MS (ESIpos): m/z = 1967.6 $[M+1H]^+$, 983.7 $[M+2H]^{2+}$, 656.0 $[M+3H]^{3+}$.

## Example 18

**tetramanganese(2+) 2,2'-({2,2-bis({[3-(1-carboxylatoethyl)-9-(carboxylatomethyl)-6-oxa-3,9,15-triazabicyclo [9.3.1]pentadeca-1(15),11,13-triene-13-carbonyl]amino} methyl) propane-1,3-diyl}bis{carbamoyl[9-(carboxylatomethyl)-6-oxa-3,9,15-triazabicyclo[9.3.1] pentadeca-1(15), 11, 13-triene-13,3-diyl]})dipropanoate**

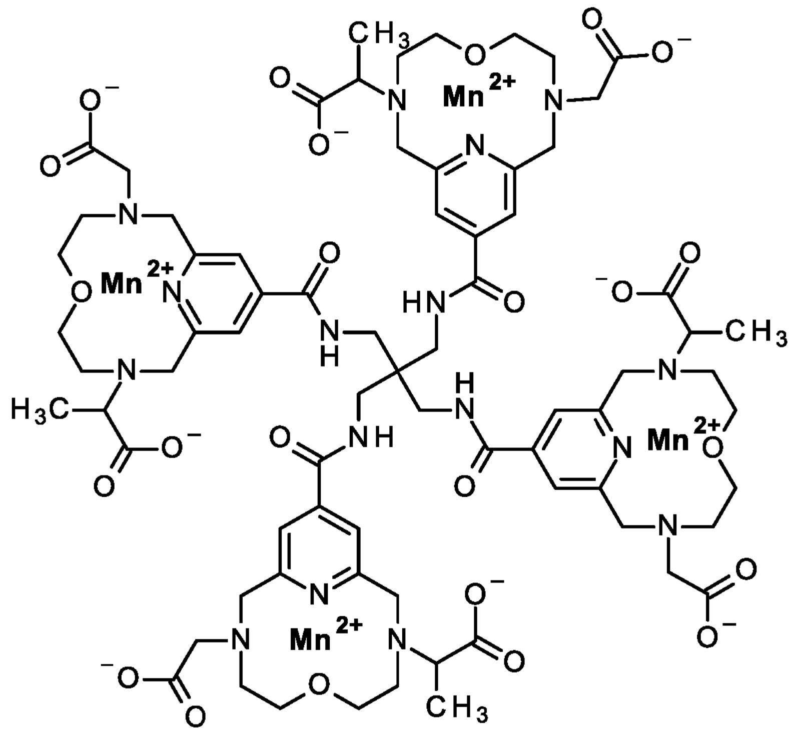

A solution of 2,2-bis(aminomethyl)propane-1,3-diamine hydrogen chloride (1/4) (48.7 mg, 175 μmol) in water (10 ml) was adjusted from pH 3 to pH 6 by addition of aqueous sodium hydroxide (1M). Manganese(2+) 2-[13-carboxy-9-(carboxylatomethyl)-6-oxa-3,9,15-triazabicyclo[9.3.1] pentadeca-1(15),11,13-trien-3-yl]propanoate (365 mg, 840 μmol, intermediate 18), 1-hydroxy-1*H*-benzotriazol hydrate (26.8 mg, 175 μmol) and 1-(3-dimethylaminopropyl)-3-ethylcarbodiimid hydrochloride (101 mg, 525 μmol) were added and the mixture was stirred at room temperature for 20 h. Ion-exchange resin AmberChrom® 1X2 (chloride form, 0.5 g) and Amberlite® IRA120 (sodium form, 2 g) were added and the mixture was stirred for 1.5 h at room temperature. Ion-exchange resins were removed by filtration and the filtrate was concentrated under reduced pressure. The crude product was purified by column chromatography (Interchim puriFlash® PF-15C RP18 AQ F0040, water / acetonitrile, 0 - 20%) to deliver 99.6 mg (99% purity, 31% yield) of the title compound.

LC-MS (Method 1): $R_t$ = 0.44 min; MS (ESIpos): m/z = 899.6 $[M+2H]^{2+}$, 600.1 $[M+3H]^{3+}$.:

__Example 19__

**tetramanganese(2+) 2,2',2",2'''-{[2,2-bis({[3,9-bis(carboxylatomethyl)-8-methyl-6-oxa-3,9,15-triazabicyclo[9.3.1] pentadeca-1(15),11,13-triene-12-carbonyl]amino} methyl) propane-1,3-diyl]bis[carbamoyl(8-methyl-6-oxa-3,9,15-triazabicyclo[9.3.1]pentadeca-1(15),11,13-triene-12,3,9-triyl)]}tetraacetate**

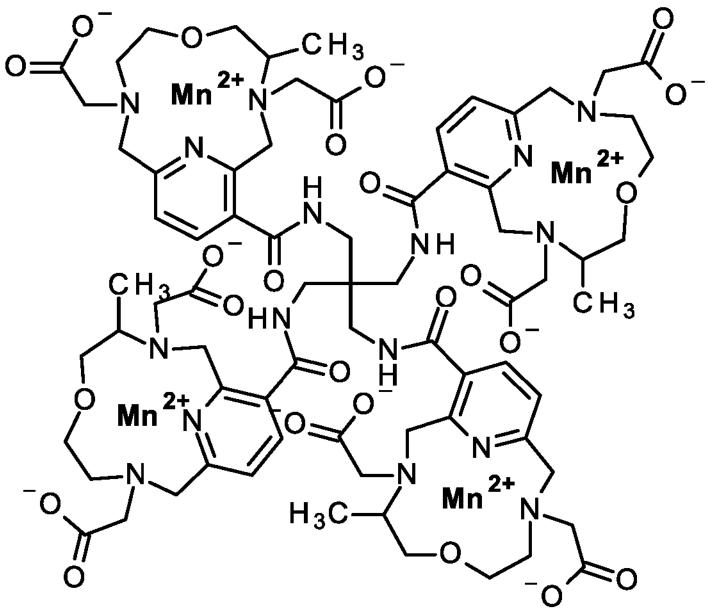

A solution of 2,2-bis(aminomethyl)propane-1,3-diamine hydrogen chloride (1/4) (29.2 mg, 105 μmol) in water (10 ml) was adjusted to pH 6 by addition of aqueous sodium hydroxide (1M). Manganese(2+) 2,2'-(14-carboxy-4-methyl-6-oxa-3,9,15-triazabicyclo[9.3.1] pentadeca-1(15),11,13-triene-3,9-diyl)diacetate (219 mg, 504 μmol, intermediate 19), 1-hydroxy-1*H*-benzotriazol hydrate (16 mg, 105 μmol) and 1-(3-dimethylaminopropyl)-3-ethylcarbodiimid hydrochloride (80.6 mg, 420 μmol) were added and the mixture was stirred at room temperature for 20 h. Additional 1-(3-dimethylaminopropyl)-3-ethylcarbodiimid hydrochloride (40 mg, 210 μmol) was added and stirring was continued for one day. Ion-exchange resin AmberChrom® 1X2 (chloride form, 0.5 g) and Amberlite® IRA120 (sodium form, 2 g) were added, and the mixture was stirred for 1.5 h at room temperature. Ion-exchange resins were removed by filtration and the filtrate was concentrated under reduced pressure. The crude product was purified by column chromatography (Interchim puriFlash® PF-15C RP18 AQ F0080, water / acetonitrile, 0 - 20%) to deliver 28.9 mg (99% purity, 15% yield) of the title compound.

LC-MS (Method 1): $R_t$ = 0.49 min; MS (ESIpos): m/z = 899.8 $[M+2H]^{2+}$, 600.1 $[M+3H]^{3+}$.

**Example 20**

**tetramanganese (2+) (2S,2'S,2"S,2‴S)-2,2',2",2‴-{(2,2-bis[({3,9-bis[(1*S*)-1-carboxylato ethyl]-6-oxa-3,9,15-triazabicyclo[9.3.1]pentadeca-1(15),11,13-triene-13-carbonyl}amino) methyl]propane-1,3-diyl)bis[carbamoyl-6-oxa-3,9,15-triazabicyclo[9.3.1]pentadeca-1(15),11,13-triene-13,3,9-triyl]}tetrapropanoate**

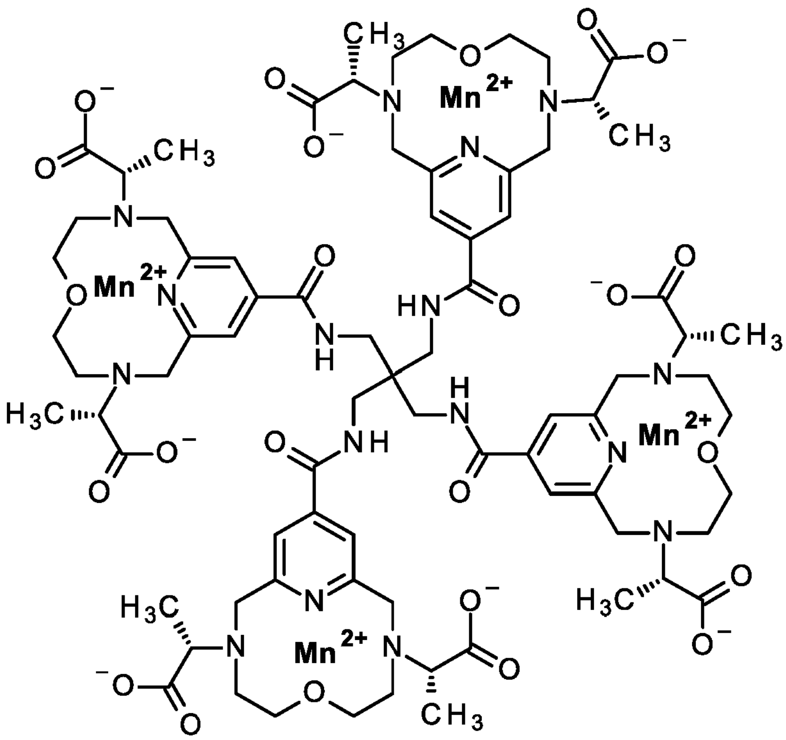

2,2-bis(aminomethyl)propane-1,3-diamine-hydrogen chloride (1/4) (28 mg, 101 μmol) was dissolved in water (7 ml) and the pH level was set to 6 using sodium hydroxide (1M aqueous solution), then manganese(2+) (2S,2'S)-2,2'-[13-carboxy-6-oxa-3,9,15-triazabicyclo[9.3.1] pentadeca-1(15), 11,13-triene-3,9-diyl]dipropanoate (200 mg, 0.45 mmol, intermediate 20) was added. 1-Hydroxy-1*H*-benzotriazol hydrate (15.5 mg, 101 μmol) and EDCI (78 mg, 0.41 mmol) were added, the pH level was again readjusted to 6 using a mixture of HCl (1M, aqueous) and NaOH (1M aqueous solution), and the mixture was stirred at RT for 16h. An additional quantity of EDCI (20 mg, 0.1 mmol) was added and the mixture was stirred for another 24h. The mixture was then purified by reverse phase HPLC (Interchim puriFlash® PF-15C RP18 AQ F0025, water / acetonitrile, 0 - 30%, 46 ml/minute flow rate). The product containing fractions were combined and subjected to a second purification using the same preparative method, yielding the title compound (42 mg, 22% yield).

LC-MS (Method 1): $R_t$ = 0.45 min; MS (ESIpos): m/z = 618.9 $[M+3H]^{+3}$

**Example 21**

**tetramanganese (2+) (2R,2'R,2"R,2‴S)-2,2',2",2‴-{(2,2-bis[({3,9-bis[(1R)-1-carboxylato ethyl]-6-oxa-3,9,15-triazabicyclo[9.3.1]pentadeca-1(15), 11,13-triene-13-carbonyl} amino)methyl] propane-1,3-diyl)bis[carbamoyl-6-oxa-3,9,15-triazabicyclo[9.3.1] pentadeca-1(15),11,13-triene-13,3,9-triyl]}tetra-2-propanoate**

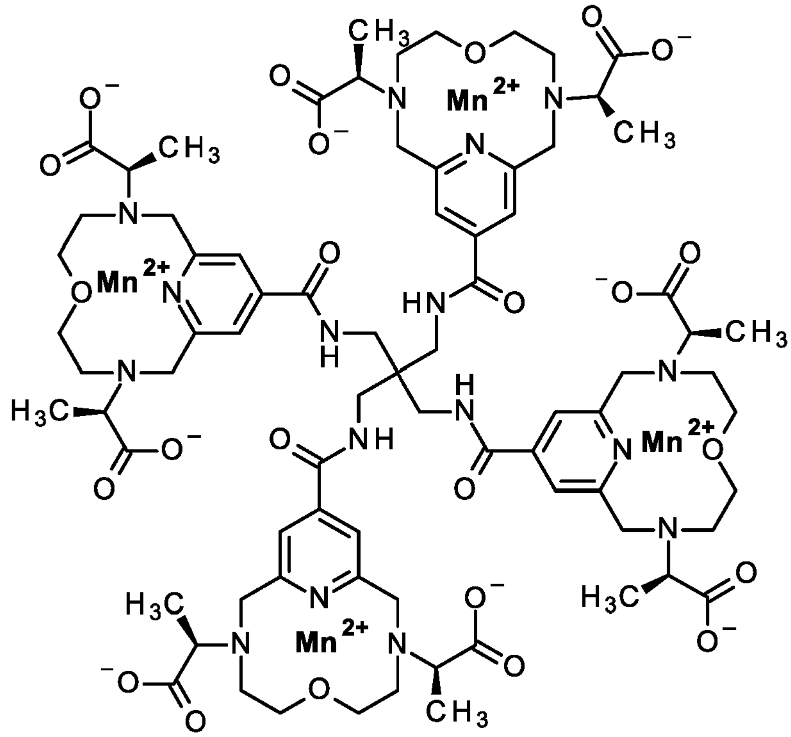

2,2-bis(aminomethyl)propane-1,3-diamine-hydrogen chloride (1/4) (59.9 mg, 215 μmol) was dissolved in water (15 ml) and the pH level was set to 6 using sodium hydroxide (1M aqueous solution), then manganese(2+) (2R,2'R)-2,2'-[13-carboxy-6-oxa-3,9,15-triazabicyclo[9.3.1] pentadeca-1(15),11,13-triene-3,9-diyl]dipropanoate (425 mg, 948 μmol) was added. HOBt (33 mg, 215 μmol) and 1-(3-dimethylaminopropyl)-3-ethylcarbodiimid hydrochloride (165 mg, 0.87 mmol) were added and the pH level was again adjusted to 6 using a mixture of HCl (1M, aqueous) and NaOH (1M aqueous solution) and the mixture was stirred at RT for 16h. An additional quantity of 1-(3-dimethylaminopropyl)-3-ethylcarbodiimid hydrochloride (42 mg, 0.22 mmol) was added and the mixture was stirred for another 24h. The mixture was then purified by reverse phase HPLC (Interchim puriFlash® PF-15C RP18 AQ F0025, water / acetonitrile, 0 - 30%, 46 ml/minute flow rate). Fraction containing the desired compound were combined, lyophilised, dissolved in water (2 ml) and heated at 120°C for 2 hours, followed by a second purification using the same conditions described above, yielding the title compound (127 mg, 32% yield).

LC-MS (Method 1): $R_t$ = 0.45 min; MS (ESIpos): m/z = 927.9 $[M+2H]^{+2}$ 618.9 $[M+3H]^{+3}$.

**EXPERIMENTAL SECTION - PHYSCOCHEMICAL AND BIOLOGICAL ASSAYS**

**In vitro and in vivo characterization of example compounds**

Examples were tested in selected assays one or more times. When tested more than once, data are reported as either average values or as median values, wherein

- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from assays represent average values or median values calculated utilizing data sets obtained from testing of one or more synthetic batch.

**Example A - Water solubility**

The exploratory water solubility of the compounds is determined at room temperature (20°C) in buffer solution (10

mM Tris-HCl, pH 7.4). The solid compounds were added stepwise to the buffer solution. More substance is added once all the suspended material is dissolved until an equilibrium between undissolved and dissolved substance is reached. The suspension was mixed using a shaker (Heidolph Reax 2000) and treated 5 min in an ultrasound bath (Bandelin, Sonorex Super RK255H). The manganese concentration of the test substance in the clear solution is measured by ICP-MS.

**Example B - Relaxivity measurements at 1.4 T**

T1 and T2 relaxation time were measured at 60 MHz (1.41 T) and 37°C using a Bruker mq60 minispec contrast agent analyzer. Manganese containing chelates were dissolved in different media at and each relaxivity measurement was performed using three different Mn concentrations. Measurement of r1 and r2 relaxivities were performed using concentrations of 0, 0.25, 0.5 and 1mM Mn in water and human plasma containing heparin as anticoagulant. The relaxivities $r_i$ (where i=1, 2) were calculated based on the measured relaxation rates $R_i$ in water and plasma:

$$r_i = (R_i - R_{i(0)}) / C_{Mn}$$

where $R_{i(0)}$ represent the relaxation rate of the respective solvent and $C_{Mn}$ the concentration of the compound normalized to Manganese. The Manganese concentrations of the investigated solutions were verified by Inductively Coupled Plasma Mass Spectrometry (ICP-MS Agilent 7500a, Waldbronn, Germany). The determined relaxivity values are summarized in Table 1.

**Table 1:** Relaxivities (normalized to Mn) in water and human plasma at 1.41 T and 37°C [L $mmol^{-1}$ $s^{-1}$]

| Example No | $r_1$ water* | $r_1$ human plasma* | $r_2$ water* | $r_2$ human plasma* |
|---|---|---|---|---|
| 1 | 7.0 | 8.8 | 14.8 | 19.5 |
| 2 | 7.4 | 8.5 | 16.1 | 18.2 |
| 3 | 8.2 | 10.1 | 18.5 | 22.8 |
| 4 | n.d. | n.d. | n.d. | n.d |
| 5 | 6.8 | 8.8 | 16.1 | 20.7 |
| 6 | n.d. | n.d. | n.d. | n.d |
| 7 | 7.1 | 8.4 | 15.4 | 18.5 |
| 8 | 5.1 | 6.9 | 11.6 | 15.7 |
| 9 | 6.0 | 8.4 | 17.3 | 23.2 |
| 10 | 8.2 | 10.1 | 18.9 | 22.5 |
| 11 | 8.4 | 10.5 | 18.8 | 25.0 |
| 12 | 7.4 | 9.2 | 16.1 | 21.3 |
| 13 | 6.4 | 8.1 | 13.6 | 19.2 |
| 14 | 6.7 | 8.4 | 14.6 | 20.2 |
| 15 | 7.3 | 9.9 | 15.9 | 21.8 |
| 16 | 10.5 | 12.2 | 22.9 | 30.1 |
| 17 | 8.8 | 10.8 | 19.1 | 25.3 |
| 18 | 7.3 | 9.0 | 17.5 | 22.6 |
| * arithmetic mean, values are depicted in L $mmol^{-1}$ $s^{-1}$. n.d.: not determined | | | | |

**Example C - Chemical stability (PhysChem)**

Examples were separately dissolved in appropriate media/buffer.

The stability of the Mn-chelate was tested using different conditions:

a) Heat: The Mn chelate was dissolved in 10 mM Tris-HCl buffer, pH 7.4 at a final concentration of 1 mmol Mn/L. The

solution was autoclaved three times at 1 bar, 121°C for 20 min.

b) Oxidation: Assessment of stability of the test item after addition of oxidants such as $H_2O_2$ and O2.

c) pH: Assessment of stability of the test item in solutions at different pH values which can be performed at different temperatures, for example at 25°C and 121°C.

d) Light: Assessment of stability after exposure of the test item to light.

Aliquots of the Mn-chelate during the tests were removed, frozen at -20°C for later analytical analyses by HPLC-ICP-MS or HPLC-ESI-MS to determine the integrity of the compound.

Examples of used HPLC methods: Agilent 1290 Infinity II LC, Agilent ICP-MS 7900, Column: Waters BEH Acquity C18 UPLC, 1.7 μm, 2.1 x 50 mm. Solvent A: 50 mmol/L NH4HCO3 pH 8.0. Solvent B: MeOH. Gradient from 0% B to 0.7% B within 6.5 min, flow 0.8 mL/min. Detection by ICP-MS or ESI-MS. The chromatograms, displaying the intensity of the detected Mn were compared.

The chemical stability of isomers and isomer conversion were compared.

**Example D - Complex stability (Transmetallation)**

The stability of the complex against other metals, for example Zn, was assessed using relaxivty as a readout. Mn chelates were dissolved in 100 mM BIS-TRIS Buffer at pH 7. T2 relaxation rates were measured at 37°C using a Bruker mq60 minispec contrast agent analyzer. T2 relaxation rates were periodically monitored after addition of a ZnCl2 solution resulting in a Mn:Zn ratio of 1:18. If test compounds are instable under these conditions, transmetallation during this test would lead to the formation of the $Zn^{2+}$ complex of the test item, while $Mn^{2+}$ is released and present in the solution as $[Mn(H_2O)_6]^{2+}$. Since $[Mn(H_2O)_6]^2$ influences the T2 relaxation time, this ongoing exchange reaction would lead to decreasing T2 relaxation times of the solution over time (measured at 1.41 T).

The stability of the Mn chelate was determined based on the measurements of the relaxation rate of the Mn-chelate and calculated with the known Mn concentration in the assay and the known relaxivity of $[Mn(H_2O)_6]^{2+}$ (r2 = 57 $mM^{-1}s^{-1}$) and the known relaxation rate of the buffer system.

$$Stability\ (\%) = (C_{Mn,\ total} - C_{Mn}(t)) / C_{Mn,\ total}$$

and

$$C_{Mn} = \frac{R_2 - R_{2,0} - r_{2,MnL} \times C_{Mn,total}}{r_{2,Mn} - r_{2,MnL}}$$

Values in % are summarized in Table 2.

Table 2: Transmetallation stability [%] of Mn chelates over 168 hours (18 equivalents of Zinc at pH 7.4)

| Example No | Stability [%] | | | |
|---|---|---|---|---|
| | 1h | 24h | 168h | 336h |
| 1 | 97 | 88 | 57 | 37 |
| 2 | 98 | 83 | 42 | 20 |
| 3 | 99 | 97 | 90 | 86 |
| 4 | 99 | 92 | 78 | 65 |
| 5 | 100 | 99 | 91 | 85 |
| 6 | 87 | 80 | 53 | 34 |
| 7 | 96 | 86 | 51 | 30 |
| 8 | 84 | 51 | 15 | 5 |
| 9 | 100 | 98 | 91 | 86 |
| 10 | 100 | 99 | 98 | 96 |
| 11 | 100 | 97 | 90 | 84 |

(continued)

| Example No | Stability [%] | | | |
|---|---|---|---|---|
| | 1h | 24h | 168h | 336h |
| 12 | 100 | 99 | 89 | 81 |
| 13 | 100 | 98 | 89 | 79 |
| 14 | 100 | 98 | 90 | 80 |
| 15 | 96 | 89 | 81 | 69 |
| 16 | 98 | 86 | 54 | 35 |
| 17 | 100 | 100 | 92 | 82 |
| 18 | 98 | 92 | 70 | 55 |
| 19 | 96 | 94 | 86 | 80 |
| 20 | n.d. | n.d. | n.d. | n.d. |
| 21 | n.d. | n.d. | n.d. | n.d. |
| n.d.: not determined | | | | |

**Example E - Complex stability (Plasma)**

Measurement of stability in human plasma. A buffered solution of Mn-chelate was added human plasma containing heparin as an anticoagulant. The concentration of the Mn-chelate was 100 μM Mn in the plasma sample. T2 relaxation rates were measured at 37°C using a Bruker mq60 minispec contrast agent analyzer. T2 relaxation rates of the mixture were periodically monitored. If test compounds are instable under these conditions, de-chelation during this test would lead to release $Mn^{2+}$, which binds to plasma proteins and/or low molecular weight species in the plasma sample. Proteinbinding of $Mn^{2+}$ influences the T2 relaxation time, this ongoing exchange reaction would lead to decreasing T2 relaxation times of the solution over time (measured at 1.41 T).

As described in Example D, the stability of the Mn chelate in plasma was determined based on the measurements of the relaxation of the Mn-chelate in plasma and calculated together with the known Mn concentration in the assay and the known relaxivity of unchelated $Mn^{2+}$ ($MnCl_2$) in plasma ($r2 = 76\ mM^{-1}s^{-1}$) and the known relaxation rate of the pure plasma sample. Figure 2 shows percent intact chelate over time measured by change in the relaxation rate T2.

**Example F - Cell Toxicity (SH-SY5Y cells)**

Measurement of cell viability after exposure to different concentrations of the test item. Undifferentiated adherent SH-SY5Y cells were exposed to Mn-chelate at concentrations 1 and 5 mM for 24h in cell culture medium. Cell viability was measured using CellTiter Glo (Promega) following the manufacturers instructions. The luminescence was measured with a photometer (Spectramax, Molecular Devices). Viability is determined by comparing the luminescence recorded for Mn chelate and the reference compound (Gadovist, Bayer AG) in percent. Figure 3 shows the viablility percent of the Mn chelate.

**Example G - Partition coefficient (logP butanol/buffer)**

The partition coefficient P (log P= $\log(C_{butanol}/C_{buffer})$) was determined in buffered aqueous solution (10 μM of compound in 0.5 mL 50 mM Tris-HCl saturated with Butanol, pH 7.4) and 1-butanol 1+1 and the mixture was shaken for 2h at room temperature (n=3). The Mn-concentrations in each phase. $C_{butanol}$ and $C_{buffer}$ were measured by ICP-MS (The Chemistry of Contrast Agents in Medical Magnetic Resonance Imaging" 2nd Ed. Merbach AS, Helm L, Toth E, eds. Hoboken, NJ: Wiley 2013).

Table 4: Partition coefficient P (log P = log(butanol/buffer, pH 7.4)

| Example No | Log P |
|---|---|
| 1 | -4.05 |
| 2 | -4.08 |

(continued)

| Example No | Log P |
|---|---|
| 3 | < - 4.0 |

**Example H - NOAEL**

The no-observed-adverse-effect level (NOAEL) was tested in mice using a modified 4-level up procedure with increasing doses of the test items. The observed NOAEL denotes the level of exposure, at which no severe adverse effects were observed (Dorato et al. Regul Toxicol Pharmacol. 2005 Aug;42(3):265-74). The test items were applicated as intravenous bolus injection via the tail vein. The starting dose was 0.5 mmol/kg bw (5-fold of the clinical dose of example 2), and the dose for the next level was increased to 1.0 mmol Mn/kg bw if the animals showed no adverse effects and decreased if the mice showed any adverse effects. The next level was 2.5 mmol Mn/kg bw. At the highest dose level of 5.0 mmol Gd/kg bw 3 animals were injected. Symptoms were observed continuously during the whole experiment (2h after contrast agent administration). Animals that survived were euthanized in deep anesthesia 7 days post injection (xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin). Macroscopic analysis was performed in all mice immediately after death. Blood was collected by puncture of vena cava and ALT, AST, GGT, GLDH, creatinine and BUN were analyzed (300 μL serum).

**Example I - Plasma protein binding**

The binding of example 2 to plasma proteins of different species (human, dog, monkey, rabbit, mouse and rat) was investigated *in vitro* by using equilibrium dialysis in reusable 96-well Micro-Equilibrium Dialysis Devices (ht dialysis) (Banker MJ et al. J Pharm Sci . 2003 May;92(5):967-74).

**Example J - In vivo Pharmacokinetics Following i.v. Administration in Rat**

Pharmacokinetic parameters of Example 2 determined in male rats (Han-Wistar, n=3). The compounds were administered as a sterile aqueous solution into the tail vein as bolus of 100 μmol Mn/kg bodyweight. Plasma was sampled before and 1, 3, 5, 10, 15, 30, 60, 120, 240, 360 and 1440 min post administration and the Mn concentration was determined by Inductively Coupled Plasma Mass Spectrometry (ICP-MS Agilent 7500a, Waldbronn, Germany). The samples were digested using strong acidic and oxidizing conditions at elevated temperature following dilution in 1% nitric acid to bring the samples in the quantification range of the method. The lower limit of quantitation amounts to 1 nmol/L for all elements and the upper limit to 1000 nmol/L in the diluted sample.

The fit of the obtained data to a three-compartment model (Phoenix, WinNonlin 6.4, 3- compartment mode) yielded the pharmacokinetic parameters.

The Mn level determined in plasma after intravenous administration of Mn chelate example 3 are shown in Figure 1.

**Example K - Biliary elimination in bile duct cannulated (BDC) rats**

The biliary excretions of compounds were investigated in bile duct cannulated (BDC) rats (Burden N et al. Lab Anim. 2017 Oct; 51(5): 457-464). All animals are initially anesthetized using a body weight-adjusted intramuscular injection of a mixture (1+2) of xylazine hydrochloride (20 mg/mL, Rompun 2%, Bayer Vital GmbH, Leverkusen) and ketamine hydrochloride (100 mg/mL, Ketavet, Pfizer, Pharmacia GmbH, Berlin) using 1 mUkg body weight. The continuous anesthesia of the animals is achieved by the intravenous injection of 1.5 mL per hour (Braun perfusor infusion of 1+2, 1:20 saline, xylazine hydrochloride 20 mg/mL, Rompun 2%, Bayer Vital GmbH, Leverkusen and ketamine hydrochloride 100 mg/mL, Ketavet, Pfizer, Pharmacia GmbH, Berlin) via tail vein (Introcan, 24G, yellow). The animals received 0.1 mmol Mn/kg bw as intravenous bolus injection via tail vein (50 mmol Mn/L formulation). Bile was sampled 0-0.5h, 0.5-1h, 1-2h, 2-3h und 3-4h post injection. The manganese concentrations in the bile fractions were determined by Inductively Coupled Plasma Mass Spectrometry (ICP-MS Agilent 7500a, Waldbronn, Germany). The bile fractions (n=3 times 10 μL) were dried for 2h at 90°C and 50 μL 65% nitric acid ($HNO_3$, Fa. Fisher, Optima Grade #1219010) and 30 μL hydrogen peroxide 30% (H2O2 Fa VWR Chemicals, AnalaR NORMAPUR, #18J024022) were added and processed in microwave oven (5 cycles: 5min 40W, 5min 120W, 10min 200W, 10min 320W and 25min 400W, Fa. CEM, Mars5 Xpress). After the samples have cooled down to room temperature 920 μL of ICP diluent were added (1% HNO3 + 0.01% Triton Tx100 and internal standard 50 μM Terbium), samples were adequately diluted, and the manganese concentrations were measured via ICP-MS.

**Example L - Excretion study in rats (5 days) and residual Manganese organ distribution (7 days)**

The excretion and organ distribution of the test item was determined in male rats (Han-Wistar, n=3). The compound was administered as a sterile aqueous solution as a bolus in the tail vein of the animals at an appropriate dose. Urine was collected in the following time periods 0-1h, 1-3h, 3-6h, 6-24h, 1-2d and 2-5d post injection and feces 0-1h, 1-3h, 3-6h, 6-24h, 1-2d and 2-5d post injection and feces 0-1d, 1-2d and 2-5d post injection. As a control, 3 animals were treated in the same way with saline. On day 7 the animals were sacrificed, and the following organs were excised: blood, liver, kidney, spleen, heart, lung, brain, muscle, skin, stomach, gut, bone and bone marrow. The remaining carcass was freeze dried and ground to a fine powder. The Mn concentration in the organs and the carcass was determined by ICP-MS (ICP-MS Agilent 7500a, Waldbronn, Germany). The results of the organ distribution of investigated examples and saline control animals are summarized.

**Example M -Redox stability assay of Mn(II) complexes.**

Electrochemical experiments were carried out using an Autolab PGSTAT101 with a 2 mm glass-carbon working electrode (Metrohm 61204600, platinum wire counter electrode (Metrohm 6.0726.100), a Ag/AgCl (Metrohm 6.0726.100) reference electrode, and K/KCl as supporting electrolyte (0.1 M Millipore $H_2O$). All samples were prepared at 1.0 mM (pH 7.0, millipore $H_2O$) and obtained values are referenced to the $K_3[Fe(CN)_6]$ redox couple as an internal reference. Voltammograms were cycled three times at scan rate 50.0 mV/s.

## Claims

1. A compound of general formula (I),

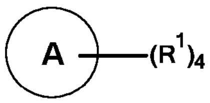

**(I)**

wherein

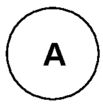

represents a group selected from

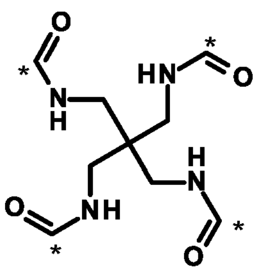

,

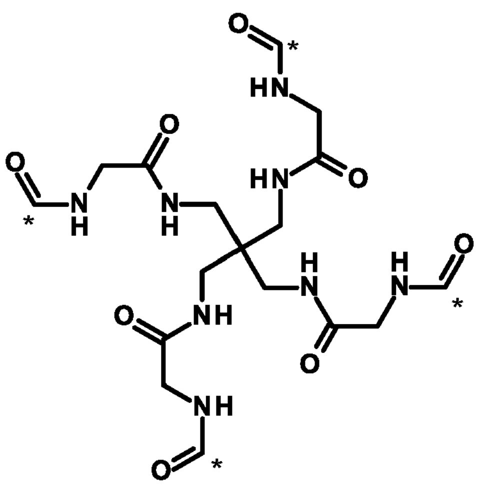

,

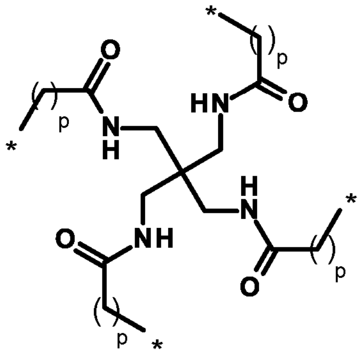

and

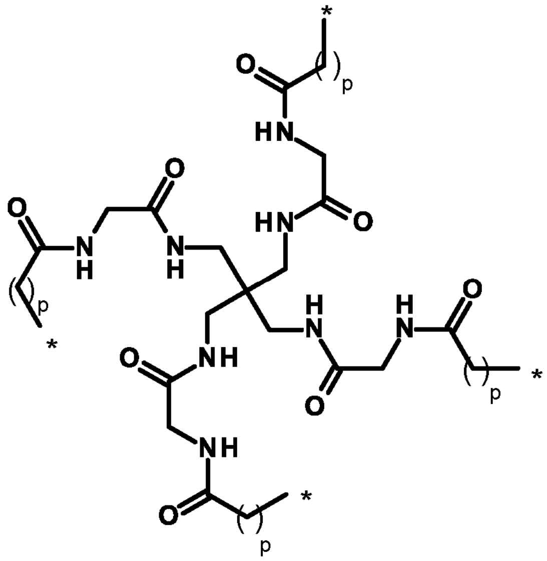

in which * represents the point of attachment to $R^1$ and p is an integer selected from 1 and 2,

$R^1$ represents a group selected from

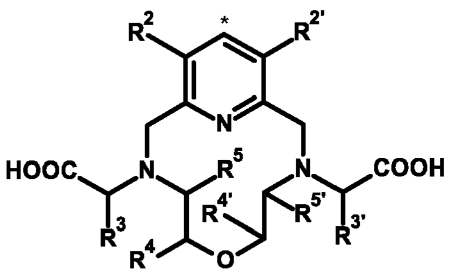

and

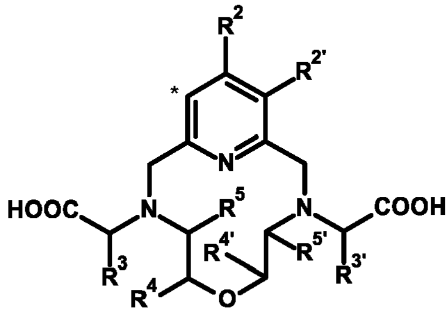

,

in which * represents the point of attachment to A,
$R^2$ and $R^{2'}$ are each independently selected from a hydrogen atom and a methyl group;
$R^3$ and $R^{3'}$ are each independently selected from a hydrogen atom, a $C_1$-$C_3$ alkyl group and a $-(CH_2)_m-(C=O)(NH)-(CH_2)-(CH(OH))_n-CH_2OH$ group, wherein m is an integer of from 1 to 3 and n is an integer of from 0 to 5;
$R^4$, $R^{4'}$, $R^5$ and $R^{5'}$ are each independently selected from a hydrogen atom and a $C_1$-$C_3$ alkyl group;
and stereoisomers, tautomers, N-oxides, hydrates, solvates, or salts thereof, or mixtures of same.

**2.** The compound of formula (I) according to claim 1, wherein $R^3$ and $R^{3'}$ are each independently selected from a hydrogen atom and a $C_1$-$C_3$ alkyl group and stereoisomers, tautomers, N-oxides, hydrates, solvates, or salts thereof, or mixtures of same.

**3.** The compound of formula (I) according to any of claims 1 or 2, wherein at least one, preferably wherein at least two of $R^3$, $R^{3'}$, $R^4$, $R^{4'}$, $R^5$ and $R^{5'}$ are not a hydrogen atom and stereoisomers, tautomers, N-oxides, hydrates, solvates, or salts thereof, or mixtures of same.

**4.** The compound of formula (I) according to any of claims 1 or 2, wherein at least one, preferably wherein at least two of $R^3$, $R^{3'}$, $R^4$, $R^{4'}$, $R^5$ and $R^{5'}$ are a $C_1$-$C_3$ alkyl group and stereoisomers, tautomers, N-oxides, hydrates, solvates, or salts thereof, or mixtures of same.

**5.** The compound of formula (I) according to any of claims 1 or 2 wherein at least one, preferably wherein at least two of $R^3$, $R^{3'}$, $R^4$, $R^{4'}$, $R^5$ and $R^{5'}$ are each independently selected from a methyl group and an ethyl group and stereoisomers, tautomers, N-oxides, hydrates, solvates, or salts thereof, or mixtures of same.

6. The compound of formula (I) according to any of claims 1 to 5, wherein

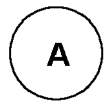

represents a group selected from

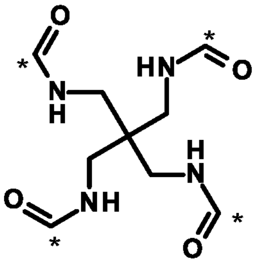

and

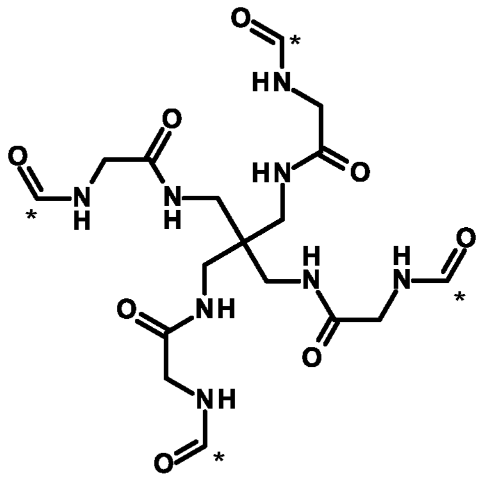

and stereoisomers, tautomers, N-oxides, hydrates, solvates, or salts thereof, or mixtures of same.

7. The compound of formula (I) according to any of claims 1 to 6 selected from the group consisting of

2,2',2",2"'-{[2,2-bis({[3,9-bis(carboxymethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo[9.3.1]pentadeca-1(15),11,13-triene-13,3,9-triyl]}tetraacetic acid,
2,2',2",2'"-({2,2-bis[(2-{[3,9-bis(carboxymethyl)-6-oxa-3,9,15-triazabicyclo[9.3.1]pentadeca-1(15),11,13-triene-13-carbonyl]amino}acetamido)methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-13,3,9-triyl])tetraacetic acid,
(2R,2'S,2"R,2'"S)-2,2',2",2'"-{(2,2-bis[({3-[(1*R*)-1-carboxyethyl]-9-[(1S)1-carboxyethyl]-6-oxa-3,9,15-triazabicyclo[9.3.1]pentadeca-1(15),11,13-triene-13-carbonyl}amino)methyl]propane-1,3-diyl)bis[carbamoyl-6-oxa-3,9,15-triazabicyclo [9.3.1]pentadeca-1(15),11,13-triene-13,3,9-triyl]}tetrapropanoic acid,
2,2',2",2'"-{[2,2-bis({[3,9-bis(1-carboxyethyl)-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo[9.3.1]pentadeca-1(15),11,13-triene-13,3,9-triyl]}tetrapropanoic acid,
(2*R*,2'*S*,2"*R*,2'"*S*)-2,2',2",2'"-{(2,2-bis{[2-({3-[(1*R*)-1-carboxyethyl]-9-[(1*S*)-1-carboxyethyl]-6-oxa-3,9,15-triazabicyclo[9.3.1]pentadeca-1(15),11,13-triene-13-carbonyl}amino)acetamido] methyl}propane-1,3-diyl)bis[azanediyl(2-oxoethane-2,1-diyl)carbamoyl{6-oxa-3,9,15-triazabicyclo[9.3.1] pentadeca-1(15),11,13-triene-13,3,9-triyl}]}tetrapropanoic acid,
2,2',2",2'"-{[2,2-bis({[3,9-bis(carboxymethyl)-6-oxa-3,9,15-triazabicyclo[9.3.1]pentadeca-1(15),11,13-triene-12-carbonyl]amino}methyl)propane-1,3-diyl]bis[carbamoyl-6-oxa-3,9,15-triazabicyclo[9.3.1]pentadeca-1(15), 11, 13-triene-12,3,9-triyl]}tetraacetic acid,
2,2',2",2'"-({2,2-bis[(2-{[3,9-bis(carboxymethyl)-6-oxa-3,9,15-triazabicyclo[9.3.1]pentadeca-1 (15), 11, 13-triene-12-carbonyl]amino}acetamido) methyl]propane-1,3-diyl}bis[azanediyl(2-oxoethane-2, 1-diyl)carbamoyl-6-oxa-3,9, 15-triazabicyclo[9.3.1]pentadeca-1 (15), 11, 13-triene-12,3,9-triyl])tetraacetic acid,
2,2',2",2'"-{[2,2-bis({2-[3,9-bis(carboxymethyl)-6-oxa-3,9,15-triazabicyclo[9.3.1] pentadeca-1 (15),11,13-trien-13-yl]acetamido}methyl)propane-1,3-diyl]bis[azanediyl(2-oxoethane-2,1-diyl)-6-oxa-3,9,15-triazabicyclo [9.3.1]pentadeca-1 (15),11,13-triene-13,3,9-triyl]}tetraacetic acid,
(2*R*,2'*S*,2"*R*,2'"*S*)-2,2',2",2'"-({2,2-bis[(2-{3-[(1*R*)-1-carboxyethyl]-9-[(1*S*)-1-carboxyethyl]-6-oxa-3,9,15-triazabicyclo[9.3.1]pentadeca-1(15),11,13-trien-13-yl}acetamido)methyl]propane-1,3-diyl}bis[azanediyl(2-oxoethane-2,1-diyl){6-oxa-3,9,15-triazabicyclo[9.3.1]pentadeca-1(15),11,13-triene-13,3,9-triyl}])tetrapropanoic

acid,
(2*R**,2'*S**,2"*RS*,2'''*SR*)-2,2',2",2'''-{(2,2-bis{[({3-[(1*RS*)-1-carboxyethyl]-9-[(1*SR*)-1-carboxy ethyl]-6-oxa-3,9,15-triazabicyclo[9.3.1] pentadeca-1(15),11,13-trien-12-yl} carbonyl)amino] methyl}propane-1,3-diyl)bis[carbamoyl-6-oxa-3,9,15-triazabicyclo[9.3.1] pentadeca-1(15),11, 13-triene-12,3,9-triyl]}tetrapropanoic acid,
(2*R**,2'*R**,2"*RS*,2'''*RS*)-2,2',2",2'''-({2,2-bis[({3,9-bis[(1*RS*)-1-carboxyethyl]-6-oxa-3,9,15-triazabicyclo[9.3.1] pentadeca-1(15),11,13-triene-12-carbonyl}amino)methyl]propane-1,3-diyl}bis[carbamoyl-6-oxa-3,9,15-triazabicyclo[9.3.1]pentadeca-1(15),11,13-triene-12,3,9-triyl]) tetrapropanoic acid,
2,2',2",2'''-{[2,2-bis({3-[3,9-bis(1-carboxyethyl)-6-oxa-3,9,15-triazabicyclo[9.3.1]pentadeca-1(15), 11,13-trien-13-yl]propanamido}methyl)propane-1,3-diyl]bis[azanediyl(3-oxopropane-3,1-diyl)-6-oxa-3,9,15-triazabicyclo[9.3.1]pentadeca-1(15),11,13-triene-13,3,9-triyl]}tetra propanoic acid,
2,2',2",2'''-([2,2-bis({[3,9-bis(carboxymethyl)-4-methyl-6-oxa-3,9,15-triazabicyclo[9.3.1]penta deca-1(15), 11,13-triene-13-carbonyl]amino}methyl)propane-1,3-diyl]bis{carbamoyl[4-methyl-6-oxa-3,9,15-triazabicyclo [9.3.1]pentadeca-1(15),11,13-triene-13,3,9-triyl]})tetraacetic acid,
2,2',2",2'''-([2,2-bis({[3,9-bis(carboxymethyl)-4-ethyl-6-oxa-3,9,15-triazabicyclo[9.3.1]penta deca-1(15),11,13-triene-13-carbonyl]amino}methyl) propane-1,3-diyl] bis{carbamoyl[4-ethyl-6-oxa-3,9,15-triazabicyclo[9.3.1] pentadeca-1(15),11,13-triene-13,3,9-triyl]})tetraacetic acid,
2,2',2",2'''-{[2,2-bis({[3,9-bis(carboxymethyl)-5-methyl-6-oxa-3,9,15-triazabicyclo[9.3.1]penta deca-1(15), 11,13-triene-13-carbonyl]amino}methyl)propane-1,3-diyl]bis[carbamoyl(5-methyl-6-oxa-3,9,15-triazabicyclo [9.3.1]pentadeca-1(15),11,13-triene-13,3, 9-triyl)]}tetraacetic acid,
(2*R*,2'*S*,2"*R*,2'''*S*)-2,2',2",2'''-{(2,2-bis[({3-[(1*R*)1-carboxypropyl]-9-[(1*S*)-1-carboxypropyl]-6-oxa-3,9,15-triazabicyclo[9.3.1]pentadeca-1(15),11,13-triene-13-carbonyl}amino)methyl] propane-1,3-diyl)bis[carbamoyl-6-oxa-3,9,15-triazabicyclo[9.3.1]pentadeca-1(15),11,13-triene-13,3,9-triyl]}tetrabutanoic acid,
(2*R**,2'*S**,2"*RS*,2'''*SR*)-2,2',2",2'''-{(2,2-bis{[({3-[(1*RS*)-1-carboxypropyl]-9-[(1*SR*)-1-carboxy propyl]-6-oxa-3,9,15-triazabicyclo[9.3.1] pentadeca-1(15),11, 13-trien-13-yl}carbonyl)amino] methyl}propane-1,3-diyl) bis [carbamoyl-6-oxa-3,9,15-triazabicyclo[9.3.1]pentadeca-1(15),11, 13-triene-13,3,9-triyl]}tetrabutanoic acid,
2,2'-({2,2-bis({[3-(1-carboxyethyl)-9-(carboxymethyl)-6-oxa-3,9, 15-triazabicyclo [9.3.1] pentadeca-1(15),11,13-triene-13-carbonyl]amino}methyl)propane-1,3-diyl}bis {carbamoyl[9-(carboxymethyl)-6-oxa-3,9,15-triazabicyclo[9.3.1]pentadeca-1(15),11,13-triene-13,3-diyl]}) dipropanoic acid,
2,2',2",2'''-{[2,2-bis({[3,9-bis(carboxymethyl)-8-methyl-6-oxa-3,9,15-triazabicyclo[9.3.1] pentadeca-1(15), 11,13-triene-12-carbonyl]amino}methyl)propane-1,3-diyl]bis[carbamoyl(8-methyl-6-oxa-3,9,15-triazabicyclo [9.3.1]pentadeca-1(15),11,13-triene-12,3,9-triyl)]} tetraacetic acid,
(2*S*,2'*S*,2"*S*,2'''*S*)-2,2',2",2'''-{(2,2-bis[({3,9-bis[(1*S*)-1-carboxyethyl]-6-oxa-3,9,15-triazabicyclo [9.3.1]pentadeca-1(15),11,13-triene-13-carbonyl}amino)methyl]propane-1,3-diyl)bis [carbamoyl-6-oxa-3,9,15-triazabicyclo [9.3.1]pentadeca-1(15),11,13-triene-13,3,9-triyl]} tetrapropano and
(2*R*,2'*R*,2"*R*,2'''*S*)-2,2',2",2'''-{(2,2-bis[({3,9-bis[(1*R*)-1-carboxyethyl]-6-oxa-3,9,15-triaza bicyclo[9.3.1]pentadeca-1(15), 11,13-triene-13-carbonyl}amino)methyl]propane-1,3-diyl)bis [carbamoyl-6-oxa-3,9,15-triazabicyclo [9.3.1] pentadeca-1(15),11,13-triene-13,3,9-triyl]} tetrapropanoic acid
and stereoisomers, tautomers, N-oxides, hydrates, solvates, or salts thereof, or mixtures of same.

**8.** The compound of formula (I) according to any of claims 1 to 7 in the form of a complex with $Mn^{2+}$ and stereoisomers, tautomers, N-oxides, hydrates, solvates, or salts thereof, or mixtures of same.

**9.** The compound of formula (I) according to claim 8 selected from the group consisting of:

tetramanganese(2+) 2,2',2",2'''-{[2,2-bis({[3,9-bis(carboxylatomethyl)-6-oxa-3,9,15-triazabicyclo[9.3.1]pentadeca-1(15),11,13-triene-13-carbonyl]amino}methyl)propane-1,3-diyl]bis[carbamoyl-6-oxa-3,9,15-triazabicyclo [9.3.1]pentadeca-1(15),11,13-triene-13,3,9-triyl]}tetraacetate,
tetramanganese(2+) 2,2',2",2'''-({2,2-bis[(2-{[3,9-bis(carboxylatomethyl)-6-oxa-3,9,15-triazabicyclo[9.3.1]pentadeca-1(15),11,13-triene-13-carbonyl]amino}acetamido)methyl] propane-1,3-diyl}bis[azanediyl(2-oxoethane-2,1-diyl)carbamoyl-6-oxa-3,9,15-triazabicyclo[9.3.1]pentadeca-1(15),11,13-triene-13,3,9-triyl])tetraacetate,
tetramanganese(2+) (2*R*,2'*S*,2"*R*,2'''*S*)-2,2',2",2'''-{(2,2-bis[({3-[(1*R*)-1-carboxylatoethyl]-9-[(1*S*)1-carboxylatoethyl]-6-oxa-3,9,15-triazabicyclo[9.3.1]pentadeca-1(15),11,13-triene-13-carbonyl}amino)methyl] propane-1,3-diyl)bis[carbamoyl-6-oxa-3,9,15-triazabicyclo [9.3.1]pentadeca-1(15),11,13-triene-13,3,9-triyl]}tetrapropanoate,
tetramanganese(2+) 2,2',2",2'''-{[2,2-bis({[3,9-bis(1-carboxylatoethyl)-6-oxa-3,9,15-triazabicyclo[9.3.1]pentadeca-1(15),11,13-triene-13-carbonyl]amino}methyl) propane-1,3-diyl]bis[carbamoyl-6-oxa-3,9,15-triazabicyclo[9.3.1]pentadeca-1(15),11,13-triene-13,3,9-triyl]}tetrapropanoate,

tetramanganese(2+) (2*R*,2'*S*,2"*R*,2'''*S*)-2,2',2",2'''-{(2,2-bis{[2-({3-[(1*R*)-1-carboxylato ethyl]-9-[(1*S*)-1-carboxylatoethyl]-6-oxa-3,9,15-triazabicyclo[9.3.1]pentadeca-1(15), 11,13-triene-13-carbonyl}amino)acetamido] methyl}propane-1,3-diyl)bis[azanediyl(2-oxoethane-2,1-diyl)carbamoyl{6-oxa-3,9,15-triazabicyclo[9.3.1] pentadeca-1(15),11,13-triene-13,3,9-triyl}]}tetrapropanoate,
tetramanganese(2+) 2,2',2",2'''-{[2,2-bis({[3,9-bis(carboxylatomethyl)-6-oxa-3,9,15-triazabicyclo[9.3.1]pentadeca-1(15),11,13-triene-12-carbonyl]amino}methyl)propane-1,3-diyl]bis[carbamoyl-6-oxa-3,9,15-triazabicyclo [9.3.1]pentadeca-1(15),11,13-triene-12,3,9-triyl]}tetraacetate,
tetramanganese(2+) 2,2',2",2'''-({2,2-bis[(2-{[3,9-bis(carboxylatomethyl)-6-oxa-3,9,15-triazabicyclo[9.3.1]pentadeca-1(15),11,13-triene-12-carbonyl]amino}acetamido) methyl]propane-1,3-diyl}bis[azanediyl(2-oxoethane-2,1-diyl)carbamoyl-6-oxa-3,9,15-triazabicyclo[9.3.1]pentadeca-1(15),11,13-triene-12,3,9-triyl])tetraacetate,
tetramanganese(2+) 2,2',2",2'''-{[2,2-bis({2-[3,9-bis(carboxylatomethyl)-6-oxa-3,9,15-triazabicyclo[9.3.1]pentadeca-1 (15),11,13-trien-13-yl]acetamido}methyl)propane-1,3-diyl]bis[azanediyl(2-oxoethane-2,1-diyl)-6-oxa-3,9,15-triazabicyclo[9.3.1]pentadeca-1(15),11,13-triene-13,3,9-triyl]}tetraacetate,
tetramanganese(2+) (2*R*,2'*S*,2"*R*,2'''*S*)-2,2',2",2'''-({2,2-bis[(2-{3-[(1*R*)-1-carboxylatoethyl]-9-[(1*S*)-1-carboxylatoethyl]-6-oxa-3,9,15-triazabicyclo[9.3.1]pentadeca-1(15),11,13-trien-13-yl}acetamido)methyl]propane-1,3-diyl}bis[azanediyl(2-oxoethane-2,1-diyl){6-oxa-3,9,15-triazabicyclo[9.3.1]pentadeca-1(15),11,13-triene-13,3,9-triyl}])tetrapropanoate,
tetramanganese(2+) (2*R**,2'*S**,2"*RS*,2'''*SR*)-2,2',2",2'''-{(2,2-bis{[({3-[(1*RS*)-1-carboxylato ethyl]-9-[(1SR)-1-carboxylatoethyl]-6-oxa-3,9,15-triazabicyclo[9.3.1] pentadeca-1(15),11,13-trien-12-yl}carbonyl)amino]methyl} propane-1,3-diyl)bis[carbamoyl-6-oxa-3,9,15-triazabicyclo [9.3.1]pentadeca-1(15),11,13-triene-12,3,9-triyl]} tetrapropanoate,
tetramanganese(2+) (2*R**,2'*R**,2"*RS*,2'''*RS*)-2,2',2",2'''-({2,2-bis[({3,9-bis[(1*RS*)-1-carboxylato ethyl]-6-oxa-3,9,15-triazabicyclo[9.3.1]pentadeca-1(15),11,13-triene-12-carbonyl}amino) methyl]propane-1,3-diyl}bis [carbamoyl-6-oxa-3,9,15-triazabicyclo[9.3.1]pentadeca-1(15),11, 13-triene-12,3,9-triyl])tetrapropanoate,
tetramanganese(2+) 2,2',2",2'''-{[2,2-bis({3-[3,9-bis(1-carboxylatoethyl)-6-oxa-3,9,15-triazabicyclo[9.3.1]pentadeca-1(15),11,13-trien-13-yl]propanamido}methyl)propane-1,3-diyl] bis[azanediyl(3-oxopropane-3,1-diyl)-6-oxa-3,9,15-triazabicyclo[9.3.1] pentadeca-1(15),11, 13-triene-13,3,9-triyl]}tetrapropanoate,
tetramanganese(2+) 2,2',2",2'''-([2,2-bis({[3,9-bis(carboxylatomethyl)-4-methyl-6-oxa-3,9,15-triazabicyclo [9.3.1]pentadeca-1(15),11,13-triene-13-carbonyl]amino}methyl)propane-1,3-diyl] bis{carbamoyl[4-methyl-6-oxa-3,9,15-triazabicyclo[9.3.1]pentadeca-1(15),11,13-triene-13,3,9-triyl]})tetraacetate,
tetramanganese(2+) 2,2',2",2'''-([2,2-bis({[3,9-bis(carboxylatomethyl)-4-ethyl-6-oxa-3,9,15-triazabicyclo[9.3.1] pentadeca-1(15),11,13-triene-13-carbonyl]amino}methyl)propane-1,3-diyl] bis{carbamoyl[4-ethyl-6-oxa-3,9,15-triazabicyclo[9.3.1]pentadeca-1(15),11,13-triene-13,3,9-triyl]})tetraacetate,
tetramanganese(2+) 2,2',2",2'''-{[2,2-bis({[3,9-bis(carboxylatomethyl)-5-methyl-6-oxa-3,9,15-triazabicyclo [9.3.1]pentadeca-1(15),11,13-triene-13-carbonyl]amino}methyl)propane-1,3-diyl] bis[carbamoyl(5-methyl-6-oxa-3,9,15-triazabicyclo[9.3.1]pentadeca-1(15),11,13-triene-13,3, 9-triyl)]}tetraacetate,
tetramanganese(2+) (2*R*,2'*S*,2"*R*,2'''*S*)-2,2',2",2'''-{(2,2-bis[({3-[(1*R*)1-carboxylatopropyl]-9-[(1S)-1-carboxylatopropyl]-6-oxa-3,9,15-triazabicyclo[9.3.1] pentadeca-1(15),11,13-triene-13-carbonyl}amino)methyl] propane-1,3-diyl) bis [carbamoyl-6-oxa-3,9,15-triazabicyclo[9.3.1] pentadeca-1(15),11,13-triene-13,3,9-triyl]}tetrabutanoate,
tetramanganese(2+) (2*R**,2'*S**,2"*RS*,2'''*SR*)-2,2',2",2'''-{(2,2-bis{[({3-[(1*RS*)-1-carboxylato propyl]-9-[(1SR)-1-carboxylatopropyl]-6-oxa-3,9,15-triazabicyclo[9.3.1] pentadeca-1(15),11, 13-trien-13-yl}carbonyl)amino] methyl}propane-1,3-diyl)bis [carbamoyl-6-oxa-3,9,15-triazabicyclo[9.3.1]pentadeca-1(15),11,13-triene-13,3,9-triyl]}tetrabutanoate,
tetramanganese(2+) 2,2'-({2,2-bis({[3-(1-carboxylatoethyl)-9-(carboxylatomethyl)-6-oxa-3,9, 15-triazabicyclo [9.3.1]pentadeca-1(15),11,13-triene-13-carbonyl]amino}methyl)propane-1,3-diyl}bis{carbamoyl[9-(carboxylatomethyl)-6-oxa-3,9,15-triazabicyclo[9.3.1]pentadeca-1(15), 11,13-triene-13,3-diyl]})dipropanoate,
tetramanganese(2+) 2,2',2",2'''-{[2,2-bis({[3,9-bis(carboxylatomethyl)-8-methyl-6-oxa-3,9,15-triazabicyclo [9.3.1]pentadeca-1(15),11,13-triene-12-carbonyl]amino}methyl)propane-1,3-diyl]bis[carbamoyl(8-methyl-6-oxa-3,9,15-triazabicyclo[9.3.1] pentadeca-1(15),11,13-triene-12,3,9-triyl)]}tetraacetate,
tetramanganese(2+) (2*S*,2'*S*,2"*S*,2'''*S*)-2,2',2",2'''-{(2,2-bis[({3,9-bis[(1*S*)-1-carboxylatoethyl]-6-oxa-3,9,15-triazabicyclo[9.3.1]pentadeca-1(15),11,13-triene-13-carbonyl}amino)methyl] propane-1,3-diyl)bis[carbamoyl-6-oxa-3,9,15-triazabicyclo[9.3.1]pentadeca-1(15),11,13-triene-13,3,9-triyl]}tetrapropanoate and
tetramanganese(2+) (2*R*,2'*R*,2"*R*,2'''*S*)-2,2',2",2'''-{(2,2-bis[({3,9-bis[(1*R*)-1-carboxylatoethyl]-6-oxa-3,9,15-triazabicyclo[9.3.1]pentadeca-1(15),11,13-triene-13-carbonyl} amino)methyl] propane-1,3-diyl)bis[carbamoyl-6-oxa-3,9,15-triazabicyclo[9.3.1] pentadeca-1(15),11,13-triene-13,3,9-triyl]}tetrapropanoate.
and stereoisomers, tautomers, N-oxides, hydrates, solvates, or salts thereof, or mixtures of same.

**10.** Use of a compound according to any one of claims 1 to 9 for diagnostic imaging, preferably for magnetic resonance imaging.

**11.** The compounds according to any one of claims 1 to 9 for use in diagnostic imaging, preferably for magnetic resonance imaging.

**12.** Use of the compounds according to any one of claims 1 to 9 or mixtures thereof for the manufacture of diagnostic agents, preferably for the manufacture of contrast agents for magnetic resonance imaging.

**13.** A method of imaging body tissue in a patient, comprising the steps of administering to the patient an effective amount of one or more compounds according to claim 8 or 9 in a pharmaceutically acceptable carrier, and subjecting the patient to magnetic resonance imaging.

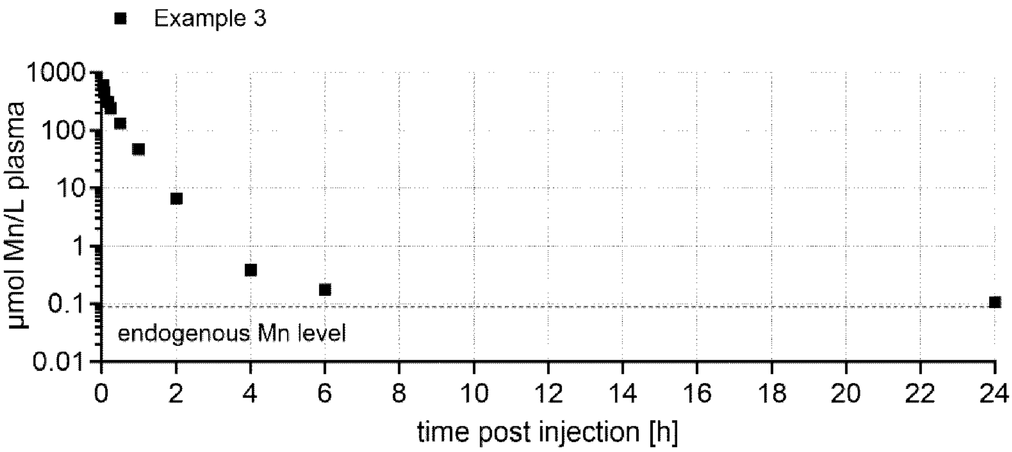
Example 3
1000
100
10
1
0.1
0.01
µmol Mn/L plasma
endogenous Mn level
0
2
4
6
8
10
12
14
16
18
20
22
24
time post injection [h]

Figure 1.

Europäisches Patentamt
European Patent Office
Office européen des brevets

# EUROPEAN SEARCH REPORT

Application Number
EP 24 21 2482

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/089849 A1 (DEBRECENI EGYETEM [HU]) 1 June 2017 (2017-06-01)<br>* page 1; claim 1; examples 1-13 *<br>----- | 1-13 | INV.<br>C07D498/08<br>A61P43/00<br>A61K31/395 |
| A,D | WO 2021/043926 A1 (GE HEALTHCARE LTD [GB]) 11 March 2021 (2021-03-11)<br>* paragraph [0001] - paragraph [0022]; claims 1,11; examples 11-23 *<br>----- | 1-13 | |

TECHNICAL FIELDS SEARCHED (IPC)

A61P
C07D

The present search report has been drawn up for all claims

| Place of search | Date of completion of the search | Examiner |
|---|---|---|
| Munich | 15 April 2025 | Seelmann, Ingo |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 21 2482

This annex lists the patent family members relating to the patent documents cited in the above-mentioned European search report. The members are as contained in the European Patent Office EDP file on The European Patent Office is in no way liable for these particulars which are merely given for the purpose of information.

15-04-2025

| Patent document cited in search report | Publication date | Patent family member(s) | Publication date |
|---|---|---|---|
| WO 2017089849 A1 | 01-06-2017 | EP 3380484 A1 | 03-10-2018 |
| | | US 2018354969 A1 | 13-12-2018 |
| | | WO 2017089849 A1 | 01-06-2017 |
| WO 2021043926 A1 | 11-03-2021 | AU 2020340541 A1 | 21-04-2022 |
| | | BR 112022003242 A2 | 17-05-2022 |
| | | CA 3152191 A1 | 11-03-2021 |
| | | CN 114423761 A | 29-04-2022 |
| | | CN 119080772 A | 06-12-2024 |
| | | DK 4025259 T3 | 20-01-2025 |
| | | EP 4025259 A1 | 13-07-2022 |
| | | EP 4512815 A2 | 26-02-2025 |
| | | ES 3010301 T3 | 02-04-2025 |
| | | FI 4025259 T3 | 13-01-2025 |
| | | HR P20250045 T1 | 14-03-2025 |
| | | JP 7644095 B2 | 11-03-2025 |
| | | JP 2022546554 A | 04-11-2022 |
| | | KR 20220058574 A | 09-05-2022 |
| | | LT 4025259 T | 10-02-2025 |
| | | PL 4025259 T3 | 03-03-2025 |
| | | PT 4025259 T | 24-01-2025 |
| | | RS 66436 B1 | 28-02-2025 |
| | | SI 4025259 T1 | 31-03-2025 |
| | | SM T202500026 T1 | 12-03-2025 |
| | | US 2022315616 A1 | 06-10-2022 |
| | | WO 2021043926 A1 | 11-03-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017027834 A
- WO 2021043926 A
- US 5560903 A


### Non-patent literature cited in the description


- **LAUFFER RB et al.** Paramagnetic metal complexes as water proton relaxation agents for NMR imaging: theory and design. *Chem Rev.*, 1987, vol. 87 (5), 901-27
- **CARAVAN P et al.** Gadolinium(III) chelates as MRI contrast agents: structure, dynamics, and applications.. *Chem Rev.*, 1999, vol. 99 (9), 2293-2352
- **FRENZEL et al.** *Invest Radiol.*, December 2008, vol. 43 (12), 817-28
- **KANDA et al.** *Radiology*, March 2014, vol. 270 (3), 834-41
- **TOFT KG et al.** Metabolism and pharmacokinetics of MnDPDP in man.. *Acta Radiol.*, July 1997, vol. 38 (4), 677-89
- *Curr Opin Pharmacol.*, October 2014, vol. 18, 18-27
- **BOEHM-STURM et al.** Low-Molecular-Weight Iron Chelates May Be an Alternative to Gadolinium-based Contrast Agents for T1-weighted Contrast-enhanced MR Imaging. *Radiology*, February 2018, vol. 286 (2), 537
- **SNYDER et al.** A Class of FeIII Macrocyclic Complexes with Alcohol Donor Groups as Effective T1 MRI Contrast Agents.. *Angew Chem Int Ed Engl.*, 03 February 2020, vol. 59 (6), 2414-2419
- *Pure Appl Chem*, 1976, vol. 45, 11-30
- **S. M. BERGE et al.** Pharmaceutical Salts. *J. Pharm. Sci.*, 1977, vol. 66, 1-19
- **T.W. GREENE** ; **P.G.M. WUTS**. Protective Groups in Organic Synthesis,. Wiley, 2014
- **GREENE** ; **WUTS**. Protecting groups in Organic Synthesis, 1091-1115
- **GREENE** ; **WUTS**. Protecting groups in Organic Synthesis, 699-704
- **T.W. GREENE** ; **P.G.M. WUTS**. Protective Groups in Organic Synthesis. Wiley, 2014
- **W. HAYES et al.** *Tetrahedron*, 2003, vol. 59, 7983-7996
- *CHEMICAL ABSTRACTS*, 3392-07-2
- *CHEMICAL ABSTRACTS*, 68331-44-2
- *CHEMICAL ABSTRACTS*, 7773-01-5
- *CHEMICAL ABSTRACTS*, 84028-88-6
- The Chemistry of Contrast Agents in Medical Magnetic Resonance Imaging. Wiley, 2013
- **DORATO et al.** *Regul Toxicol Pharmacol.*, August 2005, vol. 42 (3), 265-74
- **BANKER MJ et al.** *J Pharm Sci*, May 2003, vol. 92 (5), 967-974
- **BURDEN N et al.** *Lab Anim.*, October 2017, vol. 51 (5), 457-464